# EUROPEAN PATENT SPECIFICATION

(11) **EP 1 173 465 B1**
(45) Date of publication and mention of the grant of the patent: **22.11.2006**
(21) Application number: 00923227.3
(22) Date of filing: 10.04.2000
(51) Int. Cl.: C12N 15/12, C12N 15/11, C12N 5/10, C07K 14/705, C07K 16/28, C12Q 1/68, G01N 33/50

(54) **NOVEL HUMAN VOLTAGE-GATED POTASSIUM CHANNEL**
NEUARTIGE MENSCHLICHE KALIUM-POTENTIALABHÄNGIGE KANÄLE
NOUVEAU CANAL POTASSIQUE HUMAIN POTENTIEL-DEPENDANT

(30) Priority: 14.04.1999 US 129274 P
(43) Date of publication of application: 23.01.2002
(73) Proprietor: Merck & Co., Inc., Rahway, NJ 07065-0907 (US)
(72) Inventor: PETRUKHIN, Konstantin, Rahway, NJ 07065-0907 (US); CASKEY, C., Thomas, Rahway, NJ 07065-0907 (US); LI, Wen, Rahway, NJ 07065-0907 (US); METZKER, Michael, L., Rahway, NJ 07065-0907 (US)
(74) Representative: Horgan, James Michael Frederic
(86) International application number: PCT/US2000/009587
(87) International publication number: WO 2000/061606

(56) References cited:
- WO-A-99/07832
- DE-A- 10 013 732
- DATABASE EMBL [Online] EBI, Hinxton, Cambridgeshire, UK.; BONALDO ET AL: "UI-M-BH1-anr-g-09-0-UI.s1.NIH_BMAP_M_S2 Mus musculus cDNA clone" Database accession no. AW049888 XP002207983
- DATABASE EMBL [Online] EBI, Hinxton, Cambridgeshire, UK.; KUBISCH ET AL: "Homo sapiens voltage-gated potassium channel KCNQ4 mRNA, complete cds." Database accession no. AF105202 XP002207984
- DATABASE EMBL [Online] EBI, Hinxton, Cambridgeshire, UK.; ADAMS ET AL: "CIT-HSP-2326F6.TF CIT-HSP homos sapiens genomic clone 2326F6, genomic survey sequence" Database accession no. AQ038612 XP002207985
- DATABASE EMBL [Online] EBI, Hinxton, Cambridgeshire, UK.; ADAMS ET AL: "CIT-HSP-23366C19.TF CIT-HSP Homo sapiens genomic clone 2366C19, genomic survey sequence" Database accession no. AQ075537 XP002207986
- TINEL NORBERT ET AL: "The KCNQ2 potassium channel: Splice variants, functional and developmental expression. Brain localization and comparison with KCNQ3." FEBS LETTERS, vol. 438, no. 3, 6 November 1998 (1998-11-06), pages 171-176, XP000425858 ISSN: 0014-5793
- SCHROEDER B C ET AL: "KCNQ5, a novel potassium channel broadly expressed in brain, mediates M-type currents" JOURNAL OF BIOLOGICAL CHEMISTRY, AMERICAN SOCIETY OF BIOLOGICAL CHEMISTS, BALTIMORE, MD, US, vol. 275, no. 31, 4 August 2000 (2000-08-04), pages 24089-24095, XP002169158 ISSN: 0021-9258
- LERCHE C ET AL: "MOLECULAR CLONING AND FUNCTIONAL EXPRESSION OF KCNQ5, A POTASSIUM CHANNEL SUBUNIT THAT MAY CONTRIBUTE TO NEURONAL M-CURRENT DIVERSITY" JOURNAL OF BIOLOGICAL CHEMISTRY, AMERICAN SOCIETY OF BIOLOGICAL CHEMISTS, BALTIMORE, MD, US, vol. 275, no. 29, 21 July 2000 (2000-07-21), pages 22395-22400, XP002169157 ISSN: 0021-9258
- JEGLA T J ET AL: "CLONING AND EXPRESSION OF HUMAN KCNQ5, A NOVEL MEMBER OF THE KCNQ POTASSIUM CHANNEL FAMILY" SOCIETY FOR NEUROSCIENCE ABSTRACTS, SOCIETY FOR NEUROSCIENCE, US, vol. 26, no. 1/2, 4 November 2000 (2000-11-04), page 71401 XP001030837 ISSN: 0190-5295
- KANANURA ET AL.: 'The new voltage gated potassium channel KCNQ5 and neonatal convulsions' NEUROREPORT, vol. 11, no. 9, June 2000, pages 2063 - 2067, XP002929889
- WANG ET AL.: 'KCNQ2 and KCNQ3 Potassium channel subunits: Molecular correlates of the M-channel' SCIENCE, vol. 282, 04 December 1998, pages 1890 - 1893, XP002929890

### SEQUENCE LISTING

<110> Merck & Co., Inc.
<120> Novel Human Voltage-Gated Potassium Channel
<130> 20430 PCT
<140> US00/09587
   <141> 2000-04-10
<150> 69/129,274
   <151> 1999-04-14
<160> 43
<170> FastSEQ for Windows Version 4.0
<210> 1
   <211> 125910
   <212> DNA
   <213> Homo Sapiens
<220>
   <221> misc_feature
   <222> (1)...(125910)
   <223> n = A,T,C or G
<400> 1
   ctggagtgag gcgcgggaag atgcctggtc cttgcctcgc ggacttggca gccgcgtcct      60
   gcgggtctgt ccactgaact gctgaggact gcggcggtgg cctgagggag agccgccggg     120
   gcaagcaggg ggcccggatg agcctgctgg ggaagccgct ctcttacacg agtagccaga     180
   gctgccggcg caacgtcaag taccggcggg tgcagaacta cctgtacaac gtgctggaga     240
   gaccccgcgg ctgggcgttc atctaccacg ctttcgtnnn nnnnnnnttc cttttctatt     300
   cttattatta atatatgatc ttattattaa taatataaag gaatagcaaa tgagaatcca     360
   tgagcaatat cagaccatga aaatgagcca gtggctgagt aacaaccaat taggacactt     420
   gatagtttag caaagttgcc aaacaggaga cagactcggc tcctttgaac gaagagtgac     480
   tgcagtgtgg attccccaga taggagagca agaacatact ttctgggcct ctctcaggat     540
   cgttgtttgg gaaggaagtt gtatgggaaa ttcacaaact cttagatgct aacatttaaa     600
   tgcagcatgc cacacacaca aacccacaaa cacaaccttt ttttcatcaa taaaattgca     660
   gaggagcccc atttgcacag tatatcacat tgtattttaa tatccaaaat ggctagtccc     720
   ttccagagtt tttatgagtt aatgtgtgct aatttaatgg gcctggtgct ttattcattt     780
   gaagcaagaa attaagtctg tgataataag gtaaggttct tatcagattt ctctttttgt     840
   tgttttacag ttttctcctt gtctttggtt gcttgatttt gtcagtgttt tctaccatcc     900
   ctgagcacac aaaattggcc tcaagttgcc tcttgatcct ggtaagtgaa acatgaacaa     960
   gaacgtacat gaatgttgta taagaactgc ctataacatt tatactatgc atcttatcct    1020
   acaaaaaaat cctatctaaa aaagagttac tgagaaatat aaaaatgtca aagattactg    1080
   aaacatttgc ccaccaattt aacatgtagt caatccttag aaatatatag aaatgttcag    1140
   gattgctatt acacagcaat atcttgtgtt gtagatatat cataaataga aggcaatatt    1200
   agaaagcagt tttaagtatg tttatctatg ctaataaaca aattatataa gaagaatcag    1260
   tatctatgag gcctctcatt atattgtgaa agactataga gtagagagca ttttccaata    1320
   actgtaattt ggcagtagct aaatataatt ggccaagaac tatgaacata tggcacctca    1380
   taagaaaata gaaggctcct tcatgctctt ttcaaccaac agactgcatt atgagttttg    1440
   ctgctaatgc agttacctgg tgataaattc tgcagtttgc tctgtttcca ttatgctgtc    1500
   aatcctcaac cacacagaat tgctcaattc actttnnnnn nnnnnacgag gtcaggaggt    1560
   cgagaccatc ctgcctaaca cggtgaaacc ccgcctctac taaaaataca aaaaattagc    1620
   tgggcgtagc tactcaggag gctgaggcag gagaatggcg tgaacctggg aggcggagct    1680
   tgcagtgagc cgagattgcg tcactgcact ccagcgacag agccagactc cgtctcaaaa    1740
   aaaaaaaaaa aaaaaaaaaa gagtatactt gatttatggc atgagtggtc ttgaatgatt    1800
   ttgatggatg actggaaaca attagagata taaataaata gcacagaatc atgacagatt    1860
   tcatgaagaa tacactgtga agattcacat ggttaataac attgaaatta ttaaaataaa 1920
   agagactgca tatattagat ttttctttgt gatctagttg ttcaaagcag cagaaaactt 1980
   taaattttcc ttaattttga aagtgtgatt aatggaatat tgttacaatg ccattgattt 2040
   atatactttg aggatagtta acttctttat gtttattaga aattgcactg agagctaata 2100
   tgcagtttct attggtggtg atatgttctg ttaacagggc ctcctgtcag tttttattct 2160
   gagatttctg cctttcctgt tcttttgcat gagcctaact gactgagttt caatatcaag 2220
   ttctgaaagc aagtgagcat actagtgtgc atgcaatgtg cacagaaaga tgtgttttcc 2280
   tacctctcaa agctcaccaa aggatattac tcatattgcc acaaagtaca tttacaccaa 2340
   taacacatac tttggtaatg ggaaaaataa ataatcttag gtaataaaat gcactttggt 2400
   gcttataaag gaaaataatc atcacaggta gaagaggagg aaggcaagac taggttttga 2460
   ttcaaaatgt tttctgtttt gctaggaagt ctagaaggaa tttggtcaaa tgacctgagg 2520
   aggcaaggag atttctccca gtggcacatg gcagtccaca agaagactag gggcagggaa 2580
   acaaagcaag attgtatctg atttctacag gactccaaca ctaccctgcc aatacccttt 2640
   tctcctctga aagccatgat cccaaaggtt tccatttctt ccgggttttt cacattctac 2700
   caacaagcag aatgtcttcc ccactgacag ctcaacagtt gagtttttcg gggttctacc 2760
   ccttcatcaa cacagccttt gctttcctgg ttactgagta aataaaacac actggccact 2820
   tctgcttagc cttcaggagc cactctggct ccaccctgaa gttccatttc ctccaagaac 2880
   taagacattt ggagattctg cctaaaatat gcttaaataa aagggataaa ctgtctctca 2940
   ctttctttcc cccacagctt gtcttaagaa caacggtgta accattagtg aaaaatcctt 3000
   tcaggctagt aaaatgtaaa aagagaagag gtatcgggta acaacaaatg aaaataaaat 3060
   cattcagtgg tagaaaccat aggagtgaaa ttaggtagtt actgtcctcc agcttacatt 3120
   aatcaaaata agcccacact gtcactaaga catcttgcag taagaagcca ttcaccctct 3180
   cattgcaatt agtgtcataa agctgaatag acaggtcaga aatgaaaatc ataggccaat 3240
   taagttattc tcatttttcc acctccagct agctcttttt gagaattttg attttaatag 3300
   ataataaaac agtatgctaa ttgggtggtg ctgagcctga agccgtgaac tgaaggaaac 3360
   ttcaacaaac atttggctag ggcctgctct aagcagaggg gctacgacaa tgggtaaggc 3420
   atttctctag aaaccagcac tcagcagaat ttctggcacg agattttgtg agttttctag 3480
   tggtgtgtct tatcttcccc caatagatta taaactcctc gagggtgaca gaacacattg 3540
   ctgatcttta tatcctgcat gtttatggta ttcggtagac atttataaaa tgaattcatg 3600
   aaaagcagaa catctagaaa tgaagtcaac attgcagaga agttagctat atgaaactct 3660
   gtaaataccc tcaaggaaga aggaagaagc agattttctg gaaatcaagg gacctcccag 3720
   tataagagca attcctttgg tccaaagaag ggcagctata agaacagaga cataactcct 3780
   ccgtaagaaa agataaaaat tctgaaagtt ctctaagaac atgcccagcc ctgacctgcc 3840
   tctcactctc agtagcatgg aagccgtact atctcttatc ttgacacaag catccacttc 3900
   aatccacagg acaaatatca ccaatatgag aagataagat ctgttttata gtcaacttgg 3960
   ctgacatgta ctaatttctc aaggtcagga tagctctcta aatagaaata cttgtttgtt 4020
   ttaaggaatt ttatttaaat ttgtgtatct taaattttaa ttttgatctc ggctcactgc 4080
   aacctccacc tcccaggttc aagcaattct cctgcctcag cctcctgagt agctgggatt 4140
   acaggcacct gtcaccatgc ctggccaatt tttgtatttt tagtagagac cggatttcac 4200
   catgttaacc aggctggtct caaactcctg acctcaagtg atctgcttac caaggtctcc 4260
   cagagtgctg agattacggg catgaaccac tgctcccagc ctcatgtagt tcttaagagg 4320
   aagaaaagcc tatagattag tgagaagtag acaaattagc aatttgaatc aaatgaaaac 4380
   ttggtttgat ttcattcatt ttggagacac tttcggtgtt tccattttga tctgattcag 4440
   gacactgatc ttcgattcta agttgcactg gttaatttgt gattatttta caaaatcata 4500
   acagaataca tatctgggtc cagttcaagg tacagcaagc cattttcaat gtttccagct 4560
   tggtgtttat gattcataca tgaatcattt gggcatgcag acacacccaa atagaaacct 4620
   caaaccaatt aaggtataaa taggtttagg atgggggagg atggacaaca aaaaaaacct 4680
   cagtaaggct cctccacaaa gggcaccact tcagcttggt cccatgggca gacttcacct 4740
   gtggcaggtg aaggggaaag cctaagacat cctgtgccct gatgcagatg tgacttacag 4800
   aacataaaac gtaaaggcag aagggatcat ctgggtggct gctcatacta ggcaggcatt 4860
   atagctgctg acaagagcca acccatccct aaggatctaa aatcactaca agagaggacg 4920
   gaagtcctct catcatggca tcccaactag taactgggat gcatagggcc actccaggta 4980
   ggcagcgagg ggtaaaccag gaatgtcctg ctgttcccac ctcttttccc caagcagggg 5040
   gcacccactt cctgcctcca gcccatatgg tctcaattta tggcctcaat acctgataaa 5100
   aaaccagccg tgatccttcc ttagtaacaa agtgtcaatc agtcatttgg aatggggcat 5160
   tttcaaacta ttgcctccct atgatcttct gaagaacctc tgggataaat gaacattgtt 5220
   atttaggtat ggttaaatga attacttttc aggagcagat ttatatatta acaggtgtga 5280
   aaaacttgaa tggataaatc atttataaag aaatttcagg aatcaactaa tcccaagggg 5340
   aaagacccac taagacacta gattttgctg gagctactca aacaaattca tctatcatat 5400
   ctacctaagc cttccaatca atgagactag ccgcagtgac actgtctgtc aaaactacct 5460
   cagtcatttt tttccaaggc aggaagcatc catacccctt cacaccttaa tctaatttcc 5520
   cctatctcca ccccactccc acttctatga cccccttccc cccataccca cccccgaagc 5580
   atctactagc caacttagtg aagttctgtc acgctctcac aaccacctct gtgcagcaat 5640
   gattctgtaa atatccatgt gtcctcaaca ccaggtcaaa ttagtccctt tggtaaaaac 5700
   attcattccc tcaaattctc ttccaataca ctaatatacc ttcccaaaaa gtaaggagaa 5760
   gcttgaaagc tagctggatt gatgatggta tgtgatgttc tataagttat agttagtaag 5820
   catgttttta ggatattttt ctgctctcca aagagacaca attccggaag atatttactt 5880
   ttgtgtattt ccacattttg gtttaagttt ggagccatct ctagatctta atttcattcc 5940
   cctaatatgt gttatactag tagaattttc caaattacat agaattataa ctgcaactct 6000
   tctgactgat gccttttttt tgcattttat gatgcagttt acatcacaaa ttctttccct 6060
   gcagggatta tgtaaagagg catgttgacc tgctagccct atgttacttt aagtatatgc 6120
   acacacacaa aaaggaacaa aaacagctgg gaattgatta tgttgatatt ctgaataaaa 6180
   gcaatagttc taattatgta tgtctaatta gccacagctc ttcaagaatt gctgcaaatg 6240
   tcacagggtt tataatgtcg gcatttcatc ttcacgaaaa tgctatttga tggcataaaa 6300
   ccagaaaaaa ctaatggtca cagaagacag cttgtagatt agacaaaggt cactgtgttt 6360
   taatgaacag tgctgttaat taatgagaaa acaactggta catgagcttt taagcattgt 6420
   gaatttgtac ccaaaaaatc aatctgccta aaacaatttt aagtagctaa aaaacaaaat 6480
   aacggcaaga acataattta aacctcaaat ggtacagcag agttatatgt atcaattaaa 6540
   ttgaatcaca gttctcaggt gtgacatatg aagaggcttc tttaatgcct ggaaaagagg 6600
   gttaatatgg attggtattc tcaatacata ttgtagataa aattcaagac tagctctacc 6660
   actgcctctt ttcttttttt tttttttttt ttatttgaga cagtatctcg ctctgttgcc 6720
   caagctggag tgcagtggca tgatctcggc tcactgcaag ctccgcctcc cgggttcacg 6780
   ccattctcct gcctcagcct cctgagtagc tgggactaca ggcgcccgcc accacaccca 6840
   gctaattttt tgtattttta gtagagacgg ggtttcacct tattagccag gatggtctcg 6900
   atctcctgac ctcgtgaccc acccgcctcg gcttccgaaa gcgctgggat tacaggtgtg 6960
   aaccaccgcg cccggccacc actgcctctt aggcttctta atttccttat catttaagaa 7020
   gaataagaaa atgcttctat gttttaccaa aattctgtga ggacaaatga ggaaccattg 7080
   taactcctac aaggtgagtg ataaaaataa tacacattta ttgtctttgc ttttgtaaag 7140
   agttatccaa gccaagcttc taggggctta aataaggaag gacaggacca ttgttaataa 7200
   catcaagttt ccactacagc tttcctccaa acaagtcaaa tattctgaat attattcact 7260
   aatctcttta gctgccattt cagtaaatag cgagcatttt atttcaacta aaaccaagca 7320
   agagaaaatg aactgcttta tcctgaggta cagcagcaaa ggcaccagaa cttgtctcat 7380
   ggcttaccca gcaagggtca gaagaaccat ccctaattta aatcatctcg actgaatgta 7440
   acagattttt gtattttcaa ctcatatgaa aataaaacaa tgagacctca tccaaagggt 7500
   gatttagaga gtacctctaa acaaaacaca gtgaaaatag acccagcatc tggatttgaa 7560
   gaacacattt cctcttacga gtctatccca ttgtctagat tgctggcaat ggctttttta 7620
   aatttaaatt gttattgaga taattgtaga ttcacatgca gttgtaagat atagtagaca 7680
   taccctgtgt atactttacc caatttccgc aaaaggtaac attttgtaaa actatagtat 7740
   aatatcacaa ccaggataat aatattgata cagctcacaa atctcattca gatttctcca 7800
   gttttacttg aatacatttg tgtgtgtgtg tgtgtgtgtg tgtgtgtgtg tgtgtattgg 7860
   ctctgtacag ttttatcact tgcagagatt agtatatcta aaacaacaat caagatattg 7920
   gacagttccg gccgggcacg gtggctcact cctgtaatcc cagcactttg ggaggccgag 7980
   gcaggcagat cacgaggtca ggagatcaag accatcctgg ctaacacggt gaaaccacgt 8040
   ctctactaaa aatgcaaaaa attagctggg cgtggtggtg ggcgcctgta gtcccagcta 8100
   ctcgggaggc tgaggcaggc gaatggcatt aaccagggag gtggagcttg cagtgagccg 8160
   agatcgcacc actgcactcc attctgggca acagcgcgag actctgtctc aaaaaaaaaa 8220
   aaaaaaaaaa gatatcggac agttccattg tcacaaggat ctctcaagtt accccttgct 8280
   aaccacatcc accttctcct tcacacttgc acaccccttc ccttctccag cctgacccca 8340
   gcagccacta atctgttctc catttctgaa atgtttttat ttcaaaaatg ttatataaat 8400
   ggaataatac agtgtataac tttttaagac tgacttttct tgcactcaat ataattccct 8460
   ggcaattcat ttatgttact ctgtgtatca atagttcatt catttttatt attgagtagc 8520
   attccatggt atggaggcac cagagcttgt ttaaccatcc tcatggtgaa ggacatctgg 8580
   gctgtttttg gggtctggtc attatgaata cttcttctgt gaacattcat gtacaggttt 8640
   ttgtacaaac ataagttttc atgtctctga cacgaatgcc caagagtaca attgctgagt 8700
   catatggtaa ctatatgtcc agttttataa gaaacgacca tgctcagaag gaccatactg 8760
   ttttacattc ccatcagcag tgtttgaatg atccagcttc tccgcatcct ccccagcatt 8820
   ttgtgttgtc actatttttt gccactattt tttattttag ccattctgct agctgtgtag 8880
   tgataccatt gcgatctaat ttgcattttt ctgatggcta atgatgtcaa ataacttttc 8940
   atgtgcttct ttgccatatg tgtaacttct tttatgtatg tcctctttta tgaatatcct 9000
   atttatacct ttgccaattt tctaattgag tttttggttt tttactgttg agttttaagg 9060
   tttctttaca tattttagat attagtcctt tgtcagatat gtggtttaca aacattttct 9120
   cccagtctat ggcttgtctt ttcatcctta gtacctgggc tctcacagag taagttttta 9180
   cttttgatgg agtccaattt ctcatttttt ccttttataa cttctgcttt tgatgtcaag     9240
   attaagaact ctttgcttag tccaaatccc aaaaatatct ccattttttt cctaaaagtt     9300
   ttattatttt atgtttaatt tttaaacccg tggtccattt ttaaatgatt atcataagat     9360
   aggaagtata gattaaggtc cacttttttg cctatagatg tccaattttt ccagcatcat     9420
   ttgttgaggg cccttcttcc tccattgaaa tgcttttgca cctttaaaac aaatcaattg     9480
   agcatatttg tgtgggtcta gttctgaatt ctctattctg ttcactgttc tatatgtctg     9540
   tatgttccat atgtctatcc ctccaccaat ccacagtctt gattactgga gctataatag     9600
   taggctttaa tatcaggtaa agtgattgat ctcgttttat tcttctttgt cagcattgtt     9660
   ttagctattc caggtcctgt gcatttccat ataaatttta aaataagttt gtatctaccc     9720
   cccaaaaaaa actttgctga gattttgaca ggaattttat tagacctgta gatcaatttg     9780
   ggaagaattg acatctttgc tatgatcagt cttccaattc ataaacacgg tatttctcct     9840
   cacttattta ggtcttttct gatttctttt atctgcattt cataatctca atgcacataa     9900
   accgtatgtg tttttctaag tggatattta agttattttt atttggaatt attataaata     9960
   ataattttgt gtttctaatt ttcatttcca catgatcatt gttagtataa agaaatatga    10020
   ttaattttta tattgatcat acatcgtgca gccttagtga actcacttgt ttcaagagtg    10080
   tcttcgtaga tttcttggga tttttctgta gaccatcatg ccatttgtaa atagagacca    10140
   ttttatgttt acctttccaa actgcatggt tttttttctt ttatttggct tattgcagca    10200
   actattaata taacttacaa tactatgttg aataagagtg atgaaagtat acatcttggt    10260
   tgttcttaat gttggggaaa agcattcagt ctttcactat taagtatgat gttaagtttg    10320
   ttgtaaatgc tatttatcga attaaactgt taaacctctt ttcctaactt gccaagagtt    10380
   ttttttaatc ataaatgggt gttggatttt gtcaaatgct atttcgtcat caatttatgt    10440
   gatttttttt tagctttatt atataatgca ttatattgat tgatttcaac cattgagcta    10500
   gccttgcata cctggaataa atcctacttg cctatatatt gactgattga ctgtttgcta    10560
   atattttgct gaggattttt tgtgtctaag tttatgataa atatgggtat gtagtttttg    10620
   ttttgctttg ttttattgtc tttgtctggt tttggtgtca ggataatact gacttcataa    10680
   aatcattgag aaagtttttt ctcctctatt ttctgggaga aattgtgtga aaatgatata    10740
   ttcttattta aatgtctggt tgattttgcc agtgaaacca tttgacctgg acatttctgt    10800
   ttggggagat tttaaactat gagttcaatt gctttagcgg ttatagggct acctagattg    10860
   tctatttctt ctttgttgag ttttggtagc ttgtagtttt aaaggatttg gtccatttct    10920
   tctaagttat caagtgtatg tgcgtaaagt taccatagta ctccttcatt ttttcagtgg    10980
   cgtagaatct atactgatat cccctattac attcctgaca ttggggattt tcatcagctt    11040
   tcaagaaaga gatttatcaa ctttattgat tttctcaagg aaccactttt ttgtttcatg    11100
   gattttccat attgttttcc tcttttcagt ttcattaatt tttgctcttt tctatgttat    11160
   ttccttctat ctgcttcctt tgggtttatt ttacccttct ttttccaatt tcttgaggta    11220
   ggtactcaga ttattaatat gagaactttc ttcatctaca atgtaagcat ttagtgctat    11280
   aaattttcct ctcagcactg cttttactgc atcctacata ttttgttatg tcattttttt    11340
   attttcattc agttcaattt ttttaaaatt tttgagactt tctctttgac caatggacta    11400
   ctgggaatat tgtttaattt ccacatgttt agagatttgc ttgttgtctt tctgttattg    11460
   atttctagtt ttattccatt gtggcttgag aacacattct gtctgatttt attcttttaa    11520
   acttgttgaa atttgtttat gacccggaat ataatctatc ttaataaatg tgcatgggtg    11580
   ctttttaaaa atgtgtattc tgctgatgtt gggtgaaatg tctcatatca gtcagatcct    11640
   gcaggttgaa tgtgttgttc aaatagttca catacttgtt aattttcatt ctagtagtgc    11700
   tatgcccata gttctgagca aggataaggt gggttgatga agttcccaaa tacgacttaa    11760
   catttgtcta tttttccttt cagccctatt ggttttgctt tatgaatttt gagactgtct    11820
   tgtcattttc ccctcacctt cttaaaaaaa aaagcagcaa gcaatatttc tgaacaatag    11880
   ggcttgaata aagaacttgt aaatgtactt tatcctgcct tactgcagca gaggaatagt    11940
   gcagtgacct tggcatacct aagaacaatg ggcatatatc tgtcatttgt taagaacaat    12000
   ggacatattg gtgtcatctg tttccatgaa aaagaaaaat tactacacag tagtgttcgg    12060
   gggctcaggc cacatttggg attttcttgg gatcccttat aatatgagta ctcacaactg    12120
   gagtgccaaa tgtcctggca taagcaggaa ttaggggcag ggattaaaaa gtggatctgc    12180
   cattcaacct aggttgggca caggcacaac tacatgggca ttttgggcct gagtatgttt    12240
   tccatatttg gaaagatgtg ctggattaac aagaaatgat ctggcacttt agatacctta    12300
   ataaatgaac agactcaact acccagatat acattcaaaa ccaaaacaaa gccaaaatca    12360
   aacaaaacct tcggattatt aatgaatgta tgttgaccct aattttggtt atctgtgcat    12420
   aaagtcatct ttgaaggttt atcctataag taattttgaa taactgtgag taagcagtga    12480
   tgatattggg tatttatttc ccatcatgag aaatatttgt ggattactta gtgcaaaata    12540
   agagaaggat agaatgatat agctctacta ggcatttcaa aagtgcagca tatcatcaca    12600
   tgctctaatt aaagaaacag acatttgcct gaaatttgca acaaaaatgc cacttttata    12660
   aatttggtaa cagtttttat cctgcgagta agaatgggag aatggggagt aggttagaat    12720
   gtttactttc tctgctttgt atttctgtga tgtttggaag ttttaataaa gacaatatat    12780
   tacttttaca attaagctat tataacaaca atctataaat attgttctac attataaatc    12840
   ttgaatcaac cctcgtttta aaacaagaaa gtgaattatt ttttaaagga aaaacaattt 12900
   tgataaataa agtgcctgtc cctatagaga cagcaaggtg actgtgctaa gcttgctcca 12960
   gaaatgcttt cgttttacaa attccatttg gtttttcatt ctatcatcga ctgtttaatg 13020
   agccactcta gctggccttt ccccagggtt aactcacaac tcttcctaaa taatctcagt 13080
   cattttcctg gagcaggtgt tgttttctag gcagctcttg ccaaagggca gcataataat 13140
   taacaagctt cagatctgga ggattcaggg tctcatttct ggcaaaggat atgtgtgtca 13200
   cactgtaatg cttttgaaac ttactgtttt ataaggagat tttgacactg taacgaaaga 13260
   attcagtata cttcgaaata tttgcttctt tttctctaag aacaactctc gaagatttag 13320
   agattatgtc actccttgca gcaaaatgaa gctctatttt ctgtaaaact tgccattata 13380
   tttcaggatc gctattttat ttcagatatt tcacatgtta ctgggtagct aaagtcagct 13440
   acactataaa ttccagacga gacagatcca ttattcagcc tgcacattta gtattgaaac 13500
   tgactcccaa atttacgaag cattaatgac ttgaaaagga atagcattcc atgataggaa 13560
   atgcagacag tatgaggttc aaatgctgtc cagatttagt ttgatctgga gaaaggaaat 13620
   gttcttaact ttagatatta acaactagat cctttagaca aagtgcctaa atgtccaaaa 13680
   atatgcatag agcttcttac tgtggtttgc tccataatgc ttctctctga tatgtcttat 13740
   ctgatatttt aggagttcgt gatgattgtc gtctttggtt tggagttcat cattcgaatc 13800
   tggtctgcgg gttgctgttg tcgatataga ggatggcaag gaagactgag gtttgctcga 13860
   aagcccttct gtgttatagg tgaatatcag agtctcagat acctggacta tgacaccaac 13920
   attcttgtct tctatctcct gttttgcaat atttgattaa tattaaaagc taacatccat 13980
   ggagtactta tcatggaccg ggcactcttg tgtcttgaaa atgttgactc atttgatgtt 14040
   gtctccacag cattatgagg tagatatttt tattatcccc attttacagt tgaagaattt 14100
   gagacaaaga gagacggagt tagttgctga atgtcataca gctactaagt ggtagagctt 14160
   gaattttagc caggcaatct ggctctagaa tacccactta tatcaattaa cttatatgga 14220
   aatacatgtt tctccagtgt tgccctaatt taggggaaca ttgatttcag tgagtataaa 14280
   aaaccctttt tgctctacaa tatgacacca gtttatgctc tggatcagct ggaatagata 14340
   gccatacatt ggtactggta atgcatatca attaacattc ttcaagaatg attataagta 14400
   tggtttgctt aatgcattgg taactgtaga ctggacaaag tgctctacac tctgaaatat 14460
   gaatccccca ccctccaatc caaaataccc aaatacatag cacatctctg cacctcccta 14520
   gctgccattg tttctgagag acatcattca cctgggttct agcttgaaat tgtacttcct 14580
   aatgaagatg ccatagagca ttctggaatt aagatctagc atttcccagt aatatttaaa 14640
   agtattataa aatggaaaag tctatatata aaagctatta ttattattat ttagtagcta 14700
   aatttggctc tcttgctttt ttgtctttct cacttcctag gcagccattc aaatccaaat 14760
   actgaaaatt cttgatgagg cttatttatt tcaactattt tcaacctgaa aaagtaggtt 14820
   tttaatttac aaaatagttt aggctatctt ctacagctac cacaggtttt ctgttttgtt 14880
   ttaggagtca ggaattgttg cgtaatcttc tcagtttcct cccttactcc tgttccctat 14940
   ggtgggggca gagtggattt aaggaagaaa gtgtatcacc atccatgggc actgttaaga 15000
   agaatccttt attccatact tctttcatag tatgcacatc tcaaaagtct ccacttatta 15060
   aaccagaatt tatggaaatg tggctgtgtg tgaggcactg gtgttacaat agtgaatcta 15120
   gtggaattct cagttaaatc tatcttaaaa taccttgttt ggccctcacg aatgcagtct 15180
   gtttgtctgt cttctccaat aataggcttt actgcttttt ggaagccatc atgctaaggt 15240
   atggagcaaa gtcggtggta actccaagcc ttgcaggttc ataaagcccc aaggcatatt 15300
   agtgaaggca ttagattttt ctccctggag atgctttttt ctctctgaat tctctactca 15360
   ggcagcatta caaaggactg taacctgatg tgaaactact gcacaacaag tactttctgt 15420
   cccataaggg gcccaaacaa aacaattcac aaggcagatg gttagccaag attcactcaa 15480
   cttctacagg ataaggtcag ttcatggatg actggaaaaa tgtttcattc cagattttaa 15540
   aaccaattaa aaattggttt tgttccaatt tcagaagcaa tgccaatgta tctctggcct 15600
   ccatgcgaaa aagaaagctt tttcccagat gtctgctgcc ttttgatagc tgttttcagc 15660
   tacggataca tcaacacatc ttagtccttt agtctttctc ttcctgcctc agatccttgc 15720
   aggtcttgat ataatatgta gttctaaaaa tgatgttatg ttatctataa tcttacttgc 15780
   ctttggatgt atcaatagtg accttaaaaa tttagtatga tgcctggcgc agtcactcct 15840
   gcctgtaatc ccagcccttt ggaaggctga ggaaggcaga tcacttgagc ccaggagttt 15900
   aagaccagcc tgggatggaa agacctgtct ctacaaaaat ttaaaaatta gctgggcatg 15960
   gtggcataca cctgtagtcc cagctactca gaaggctgag actggaggat catttgagcc 16020
   caggagtttg aggctgcagt gagccatgtt cctgccacta cactccagcc tgagtaaaaa 16080
   gagtaagacc ctgtctcaaa aaaatactag tatgacatgg tttacgcata attgcagttt 16140
   tgttaaccct tcagaccttt tctttattta gatattttac ctctaggaaa ttaattgctt 16200
   tggaagaaaa tttcatggat aaatatataa tgaatcccca ttagaacaaa gtctagattt 16260
   gtacttacag ttaggataat tatgaatttt ttagtctttt attaaatgta catgtttgac 16320
   ccatggtcca gctgaatgga tgttatagtg aagcatgttc aaatgtggct tatcagtatg 16380
   aggtcagtaa aatttaacct acacaaagga atcaaacatc accttcctga aggcagcaag 16440
   ctgtaccaga tgactgttga gactatttct aggcccgttc aacaggttca taaaaacgct 16500
   tgatgtaact ggtaaaatac taaagtgaac actgtagtcg ccttgttcaa cctaatttac   16560
   tgtgacaaaa tgaaaaaaga aatagcatct cttatctgtc cctgataact gttctgaatg   16620
   ggtgtttgga aatatagcta aaacctccca aaacttcatg tatttgtaag atttgattac   16680
   tctgaacatt ccaagtacac acccaaatat ttttagaagt gttgagcatt cgagaaaaga   16740
   acatagaaaa gattgtggta gactggctgt gccttcaacc tgggaaatgc tttctgtaag   16800
   cacaccaatg tttttctttc cagttggaaa tacactgaag ctgctagagt ggtcagctaa   16860
   caagccccat gtactattgc accctattga caaaggccca attgatagag gttttatggg   16920
   ctaattactg aaggttttag catgtcccct tctgaactgc ccaccttcct tacccctgca  16980
   ctctagccaa aaccaagagc atttcaaaag cccactgatc tcacaaatcc taaaatcatt   17040
   acatatttgt gtttggaaaa catattttaa acaattataa cataagggaa gaaataaatc   17100
   tatttaactt tttttaatta ggtactttgt ttcattacta ctgtacattg atgtatgtct   17160
   gcttggtttt aagcatttgc tttgaaaatt agtaaaatct tatctagaac ataaaattct   17220
   tcctcatatc tatgtgttca atttttatct ctcccctaag actcttctgt cataataaca   17280
   ttctatagct atgagcacta attgctgtat tgctgatgtg gatagtcaag tgtgttttgg   17340
   gatgaccacc caagctgccc ttcatttacc cagactggct gctttatgat ttctctatgt   17400
   gaaaacacaa aacactcacc caataacatc ggcataaaga atttgtgaat aacctctaag   17460
   actgatttca tagtcattct tttttctagc tttcattact gacctaccca aaaattgcaa   17520
   atagacctaa ggctgaaact tggatcacaa taatgagcct gatgtgtcag gaagccaaca   17580
   aattacttct cacagtggct ttttttagat tccttggtga cgctttccaa aggcagatat   17640
   aacagggcca tatttttcag tcttgcaggg agtctcagtg agaaaatacc agcctgttgt   17700
   tagtcttgga tctgcatttg atgctaagtg taagttgatt tcttattgtt attttaacag   17760
   tttattttca gtgtaattat ttccacaaga ggacaaaaga ggaataaaat aaaattttat   17820
   tttatcctac gtggataaaa taaaattgaa gcctttatta aaatcgacca catggctgag   17880
   actactctaa tatatctatg atttcatctt attaaaaggc aacttaagtt gctaatgtgt   17940
   tcaaaagtta gatcagactc ccaaaactgc cactgtgttc taaattgatt ttgagacatc   18000
   atttttctgt ttctctgtct tctctatctt ttgttctaag agacttactc ttcaatggca   18060
   ttattttcta taccttctac tcccttttcc ctttagtaga aattatatta tttcacttat  18120
   tttctgttag tcatacactc ttctgcaact tattacttct aagtgttcaa actccctcag   18180
   tgcatgtaga ttccttccat gtgattaact ggaccatctg aggacaatta aaacctgttg   18240
   gattggctga tcacaaaaca ttaaaacaat tttaatccct ctagttagta atagctgata   18300
   tttattgaga actactatat attaggtact actttaagca ctttataaat atggtcttat   18360
   ttaattctac ataagtacta taatgtcctt atttatttta ttttatttta ctttacttta   18420
   ttttattcta ttttatttta ttttatttta ttttatttta ttttatttta ttttatttta   18480
   ttttttgagg cagagtttcc ctctatcacc caggctggag tccaaatggc atagtctcag  18540
   ctcactacaa cctccgcctc ctgggttcaa gccattctca tgcctcagcc tcccgagtag   18600
   ctgggactac aggcacacgc caccacaccc agctaatttt tgcattttta gtagagatag   18660
   gatttcaccg tgttggccag gctggtcttg aactcctggc ctcaagtaat ccacccgcct   18720
   cagcgtccca aagtgctggt attacaagtg tgagccacca cgcctggcct ttataatgtc   18780
   ccattttata gatgaggaag taagacaaga ctgaagactt tatagatgag gaagcaagac   18840
   aaacttaaaa agatgatagg tgaaagaacc tggatttatt tctcatgatt tcactctaga   18900
   aaaatataca tacccaactg gaaaattgtc tttactgttt attagcattt taaaattaaa   18960
   tttaattgaa ccttatctat tgtcatcaac ttgtaaacag aagaacatga tactattatg   19020
   cttatcatta gtaattcaag atctttaaag aaaagccagg ctttatagaa cattaattgc   19080
   agtatcagcc ttaattatat caactaaatt atgttaccta ttgctgctct ctaacctgaa   19140
   tgggaaatta ggacctaaaa tagacttact tacctgtatc aaacttgcat atactcttca   19200
   gttgttgtta tcatgaggct ccctgtgtat ttccaaattg gagacctctg gagagcaagt   19260
   aacattactc gcaggactac cttcaaatgt tttttaacat taataaacta tcggtaattt   19320
   agataattgt tgacctggta cacaaacttg aaagttgaac ttacaagaca gcttgattag   19380
   ctgatatggt tggaccacat gaagaaaatt tatttccgaa gaggaaaatg aataaggatt   19440
   atttcccact ctttctataa actttagtag agcactatga tatactgtat taacattctg   19500
   tactatcaca tgttacataa gaaaattcat tcacagacag ggtatttatt atgtgtcaaa   19560
   cataatctta agtataaagg atacaaaagc gactaaaaca cggtccctgc ctgtaagaat   19620
   agaatctcct gtgcccttag agaatggcaa aacttaaagg aggtatacta taattatggt   19680
   gttacagagc aaaggtgatg gaaattgaag tagtcctaga aagaaggcta tggaatatca   19740
   tgagtgattg tataaaacaa agaagcaaag gactaccccc aggttttcaa attatgtttg   19800
   agtcatccac tgaacattta tacaatttct accatgtcca agcatgctta tggatgctgg   19860
   agaggctgta tggcaccaaa tgggcaaggt cttccttctt atagagataa tattcgtgtg   19920
   gacaaagaaa aacaatacac aaatatgttt ttgaaaaaca agataatgtc atctacagat   19980
   aagcacaatg aagaaaataa aacagaaaga gtagaggcta ccttagcttg ggtggtctga   20040
   gaaagtcttt ctaagtagtt gacacttgag ttgacctgaa tattaagaag aagccagcca   20100
   tgaggaggtt tagacacaga tctttccagg ccagggaata gtaattgcaa ggattattaa   20160
   cacttgatga gcttgggttg tacaaggtgg agacataagg tctcgtggct ggagctcaat 20220
   gagcagtggg gagaatagca gaaggtgata ggggagacag gcagaggtca caccctgaag 20280
   gccatgtagg ctctgctacc ctgaatcaag gttccttcta aagacaggcc accaggcaaa 20340
   ggtcctacgg gttgctggca ggcctaatat gaatactatt caggttatga acagcagcat 20400
   taatcaggca acaaaaagta tggctgttta atgccctggg ttttttagtc atgtatgcct 20460
   gttggaactt catatcagca atatacaaat tattacccat ttatgtttat aaaaaacata 20520
   aacataagtt tataaggaaa taatcctctg aattttgttt ctatatttct gcccttagaa 20580
   acatgaaatt ttacattgtt ttttaaaatg ttatttttct atgaggaact ggaattagaa 20640
   tgtgatggaa acttttttac agccccagtg gtatcaccag taagcagttc ctaagatcct 20700
   ccttgccaag ggactctatt ccatactctt agttatgatg attgcagtgc agaattattt 20760
   cattaaagat tgtttatttc ttttgagaaa ttgtcccttt ttgccttgag atgttatctg 20820
   aaactgtatg atcaataaga agcattaaac tgtcactatg gggtaaattt tcaaagtggt 20880
   gcctgcaatt tgccagtatg agtcccatta acttctgtgg aagtcctgca gttaaacccc 20940
   tgcacatggc tttgaaaatg ttctacaagt ttgtctttgt ggtgcctttt ttatttttaa 21000
   taaatatcac agagcaactc agtgcatagt tgattgcagt atttaagaac gtggataatg 21060
   tactgtgcag gaggagactt ttaaaattag attttaaagc ttcatttcta ttaagagtca 21120
   caacaacaat ataaatacat ctttagttct tcaaagagca cctttctata aaggaatttg 21180
   gagctcaatt agaatataga gcttataaag cagcaccatg tccctctgag acattgttta 21240
   cttcactcta tagatggaaa agtcgaggca ctgtgaagtg gggtgctttt gtccttagcc 21300
   cataaatcag cagtaaagct aggaatagca tgctggcctc ccaatagctc attgctctaa 21360
   agcactgcct cttgttagag tcaaatttaa ttatagtata atccgtttaa tctacatgtg 21420
   gagaaataat gaaggagcag gaaaaatgaa tttcttaaca gatcagtgga aaaccatatt 21480
   acatagatta gcaactgtct tttcaagatc cgtattttcg gcatgtacta gatggtatct 21540
   gatgccatga aagcaaatat tctaattcca ctggccagaa ctggcaaaga acttcttatt 21600
   ttcctcactt tgaaacataa gtaaaggggt tccacttgaa aattaataag ccatgtgata 21660
   tgattcgatt cctgtcccca aatctccctc cttctagggg ccattgtcta aagtctaaaa 21720
   gaaaacatac caggcacagt ggctcatgcc tgtaatccca gcactttaaa gggagagcaa 21780
   ggtaggagga tcacttgagc ctaggagttc aaaatcatca acctgggcaa cataatgaga 21840
   ccctatctct acaaaaaaaa caaacaaaaa caaaaacaaa caaaaaaaac tgtaatagct 21900
   gggtgtggtg gaacacactg gtagtctcag ctactcagga ggctgatagg agaaggtaac 21960
   ttctgcccag gaattcaagg ctgcagtgaa ttatgattgc accactgtac tgcagcctgg 22020
   gcaacagagt aagacagaga gagagagaga caaagagaaa ggaaggaagg aaagaaggaa 22080
   ggagggaagg aaggaaggaa ggaaggaagg aaggaaggaa ggaaggaagg aaggaaggaa 22140
   gggaagaagg aaggaaggga gggagggaag gagggaggga aagaaaggaa aggaaagaaa 22200
   ataaatgtaa ctattataaa agatgttcaa tgttttattt tggaataact ttagacttac 22260
   ttagaagttg caaaaatagt acagaatttc catgttcaaa tgtactcttt acctaacttt 22320
   ccccaaaata ttttacctac gtatactgca attatcaaaa ccaggatatg aatacatact 22380
   attagctata gtctttattt gaatttcacc agtttttaca tgcgctcaat ttttatatac 22440
   actcctataa aattttctca catgtatata tgcatttaac caccaccaca atcaaaatac 22500
   aaaacttttc catcacccta aataaacttg gaatgatata ctttgtatta ttcctttaca 22560
   ggtactccct ctcttcaatc ctaacctctg ggaaccacta tatctgttct gtttcactgt 22620
   aattttgtca tttttataat gttatataaa tgaaattgta aagtagtaat cttttgaaat 22680
   tggctttttc ttacacagca tgatgctctt gagatccatc caagtgcatc aatagtgcat 22740
   tcctgtttat tgcttaatag aattccatga tctagatgta acacaatttg tatattcatt 22800
   tacctaatca aggtcatttg ggctgcttct agtttttggc tattacaaat aaggcagcta 22860
   tgtacattca tgtgtaggtt tgtgtatgaa cgtaagtttt tatttctcta gggtcatata 22920
   gtgctagttc acctttatca gaaactgcca aactatttcc ttttaaaata tagaaatgcc 22980
   aatgcaaaca tgtgcatgga ggtcagcagc cccaccccct gccccacgct gccactgctg 23040
   ccagtgtgaa catatgcatg gaggctggca gccccacgct caccactagc ctgcccctgc 23100
   actgacactg ctaccagcat gagcgtgcac atggaggccg gcagccccat ggccaccagc 23160
   actctgcccc agttgatgag cattcacccc accacactgc cacttgctct tggcactgct 23220
   cttggcactt acaaatgagc acagatcctg ctgccaccac cccaacaaag tggtttggct 23280
   ggcaccaccc atcagagtat tgtggccaga ggtgcaggaa caccttagcg cctccacttt 23340
   agcagtttcc taacctcaag gggccagaga ataaagtcag agatccagta acagcctccc 23400
   aaagttgcag catacagccc aaaagttctg agctgagcct taccccctaa attcttccag 23460
   aaatgaagcc agtcaactga acccacctta tatgagattc aaaccttcca gggcatcaaa 23520
   gaagatagag gcaaaaaaaa aaaaaaaaaa aaaaaaacta tccaaaggac agcaatttca 23580
   aagactgaag gaaaatcagc ccacacagat gataaagaac cagtgcaaga gctctggcaa 23640
   ctcaaaagtc agagtgcttt cttacctcca aacaactgca gtagttcccc agcaatggtt 23700
   cttaaccagg ctgaaatgac aaaaagagaa atcagaatgt ggataggaac agagatcact 23760
   gagattcagg agacagtgaa aacctaatcc agggattcta aggattacaa caaagtgata 23820
   caggagatga aagacgaaat ggccacttta agaaagaaca gaactgatct gatagagctg 23880
   aaaaactcac ttcaagaatt ccagaataca atcacaaata taacggcaga atcgatcaag 23940
   ctgaggaaag aatctcagag cttgaatatc gcttctctga aataactcag tcagacaaaa 24000
   ataaagaaaa aaagattaaa gaagaatgaa caaaatctcc aaagtatggg attatgtaaa 24060
   gacaccaaat ctatgactca ctgatgtccc tgaaagagag ggagagaaag caagcaactt 24120
   ggagaacata tttcaggata tcatccatga aaattccccc aacttcacta gagaggccaa 24180
   cattcaaatt gagaaaatgc agagaaccct tgtgagatac tatacagata accatcccca 24240
   agacacatac ttgtcagatt ctctaaggtc aaaacaaaac aaaaaatgtt aacagcagct 24300
   aaagagaagg ggcaggccac ctcccaagga aaccccatca ggctaacagc acacctttca 24360
   gcagaaactc tataagccag cagagattga ggacctatat tcagcattat taaagaaaag 24420
   aaattccaaa caagaatttc atatccagcc aaactaagct tcataaatga aggagaaata 24480
   agattctttt caggcgagca aatgctaagg gaattcatta ccatcagacc tgccttataa 24540
   gaggtcctaa agagagtgtt aaatatggaa aggaaagatt attactggca actacaaaaa 24600
   cgtgcttaag tacacacacc attgatgcca aaaagcaacc acacaaacag atctgcataa 24660
   taaccaatta acaacacaaa acaggatttg atccttcaaa tccacacata tcaatattaa 24720
   ccttaaatgt aaatgggcta aatgcccaat taaaaggcac agagtggcaa gttggacaaa 24780
   gaagcaagac ccaacagtat gctgtcttga atagacccat ttcacatgca gtgacgcaca 24840
   taggatcaaa ctaaagggat ggagaaaaat ctaccaagca aatggaaaac agaaaaaagc 24900
   aggagttgct attctacatt caaacaaaac agactttaaa ccaacaaaaa tcaaaaaaga 24960
   taaagaaagg cattacataa tggtaaaggg ttcacctcaa aaggccagac ttaactatcc 25020
   taaatagata tgcatccaaa acaggagcac ccaaattcat agagcaagtt tttagacacc 25080
   cacaaagaga tttagataac cacacaataa tagtgggaga ctttaacatc ccactggcag 25140
   tattagacag gtcattgagg cagaaaacta acaaagaaat tcaggacctg aatttgacac 25200
   ttgactaaat agacctaata gacatctaca gaaatctcca cccaaaaaca ggagtgtata 25260
   tattcttctc atctgcacat ggcccataat ctaaaattga ctaatcagcc ataaaacaat 25320
   ccttagcaaa taaaaaaaaa tcataccaac cacactctca gactacagtg caataaaaat 25380
   agaaattaat ggaaattaac ctgcttctga atgacttttg ggtaaacaat gaaattaagg 25440
   tagaaattca gaaattattt gaaactaatt agaacaaaga tactacatac cagaatctct 25500
   gggacacagc taaagcaata ttaagaggga agtttataga gctgaatgcc tacatcaaaa 25560
   agttagaaag atcttaagtt aacaaccgaa catcacacct agaggaaata gagaaacaag 25620
   agcagatcga ccccaaagct agtagaacac aagaaatcaa aatctgcact aaactgaagg 25680
   aaattgagac gcaaaaaaac atacagaaga tcaatgaatc caggacttgg ttcttcgaaa 25740
   gaataaataa gatagataaa atgctagcta gactaataag gaaaaaaaga gagatcaaaa 25800
   taaacacaat cagaaatgac aaagggatgt taccacctac cccacaaaaa tttaaaaaac 25860
   cctcagagac tactaaaaac acctctatgc acacaaacta gaaaacctag aagaaatgga 25920
   taaattcctt gaaacataca accttccaag attgaaccag gaagaaattg aatcctggac 25980
   agagaccaat aatgagttcc aaaattgaat cagtaataaa aagcctacca accagaaaaa 26040
   gcccaggacc agagggattc acagctgaat attacaagaa gtataaagaa gagctggtac 26100
   cattcctact gaaactattc caaaaaacag aggaggaggg actcctccct aactatgaaa 26160
   ccagcatcat cctgatacca aaacctggca gggacacaac aacaacaaaa aacaaaactt 26220
   caggccaaat ccttgatgaa catagatgca aaaattctca ataaaatcct agtgaaatga 26280
   atccagcagc acatgaaaaa gctaatccac cactatcaag gaggcttcgt ccctgggaca 26340
   caagtttggt tcaacatata caaatgaata aatgtgattc atcacataaa cagaattaaa 26400
   aacaaaaacc acatgatcat ctcaataaat gcagaaaagg cttttgataa agttcaacat 26460
   cccttcacgt taaaaaccct caacaaacta ggcactgaag gaacatactt caaaataata 26520
   agagccatct ataagaaact cacagccaac atcacagtga atgagcaaaa gctggaagca 26580
   ttcttattaa acaccagaac aaaacaagga tgtcctctct caccagtcct tttcaacata 26640
   gtactggaag tcctggccag agcaatcagg caagagaaag atataaaagc catctgaata 26700
   gaggggaagt caaactgtcc ctgattccag tcagtatgat tctatgccca gaaaccccca 26760
   taatctctgc tccaaagctc cttggtttga taaacaactt cagcaaagct tcaggataca 26820
   aagtcaatgt acaaaaatca gtagaatccc tgtgcaccaa caacatccaa gctgagagcc 26880
   aaatcaagaa tgctatcccc caagcctgcc accatggcca cctacaaact ggtgcagatc 26940
   cagcacagcg agagtgagtg gaacctggag aaccacttca gcggctggta cgacgccatc 27000
   ctgagcccag cgggccatga ggcggcaaag caggcactgc gagatgctgg ctatgagttt 27060
   gacatctgct tcacctcatt gcagaagaga gcgatcccga ccttctggac agtgctagat 27120
   gccattgatc agatgtagct gccaatagtg aggacttggc gcctcaatga gcagcactat 27180
   ggggctctag ccggtctcaa taaagcagaa actgctgcaa aacatagtga agcccaggtg 27240
   aagatctgca ggtgctccta tgatgtccca ccacctccga tggagcccta ccatcctttc 27300
   tacagcaaca tcagtaagga tcacaggtat gcagacctca cagaaaatca cctaccctcc 27360
   tgtgagagtc tgaaggacac tattgccaga gctctgccct tctggaatga agaagtattt 27420
   ccccagatca aggaggggaa atgggtactg actgcagccc atggcaacag cctctggggc 27480
   actgacaagc atctgaaggg tctctctgaa gaggctatca tggagctgaa cctgccaact 27540
   ggcattctca ttgtctatga attggacaag aacttgaagc ccatcaagcc cagcccacgc 27600
   agttcctagg ggatgaagag accatgtgca aagccatgga agttgtggct gcccagggca 27660
   aagccaagaa gtgaaggcca gcaaacaggc accctccctg cccattgcat ccatctgtcc 27720
   ctccctcctg aacatgtcac actgaccaca tctatagaca tcttgagttg cagctgcaga 27780
   tggggaccgg tggctcccat tttcatttta gccattttgt cttctgcacc cactcccttc 27840
   atacattcta gtcagaatag cacttctagg gcacaggttc tcagtctaag ctgtggaaaa 27900
   gcccccgtta tccaagagag ttcaaagata gtgacttggg tttttgcaag tgctttgttt 27960
   actaaggact tgtgaggagg agccatgctg agctacgacc aatgaggaga agcaagagag 28020
   cctgtctgcc cccaggagct agtcctgtgc ttgtctgtag tcaggccact gcctgggggc 28080
   tctagtcatc ccagtggaag atgaatgtaa cctgcatggt gatgtgacag ctgtttcctc 28140
   cctgacccca ttacatttac ttttatttaa aaaaaaaaaa gtatagtgta tataaataat 28200
   acaaaacaat aacccttcta agggtttctc gtggtggttg aaatagtccc acatgtggtc 28260
   atcagaacat aagccattcc tcataccaat atggggtaag ctccttgacc tttgaggggc 28320
   aggagtgctt catgctgtgt gttttagaat ccctccctgc cttgtttcat ggcagtgaaa 28380
   tgcctcttgg tcctctccaa gtgtgttttt cactgatttc tgaatcatgt tgcagttgct 28440
   tggccctgcc acataggtct agtgttcatt tgagcataac tgtactaaat cctttttcca 28500
   gatcagtata ataaaggagt gatgtgcaat aaaaaaaaga atgccatccc atgcacacca 28560
   gttagaatgg tgatcattaa aaagtcagga aacaacaggt gctggagagg atgtggagaa 28620
   ataggaacat ttttacactc ttggtgggac tgtaaactag ttcaaccatt gtggaagaca 28680
   gtgtggtgat tccttaagga tctagaacta gaaataccat ttgacccagc catcccatta 28740
   ctgggtatat actcaaagga ttataaatca tgctgctata aagacacatg cacatgtatg 28800
   tttattgcag cactattcac aatagcaaag acttagaacc aacccaaaag tccatcaatg 28860
   atagactgga ttaagaaaat gtggcacgta tacaccatgg aatactatgc agccataaaa 28920
   aaggatgagt tcatgtcctt tgcagggaca tggatgaagc tggaaaccat cattctcagc 28980
   aaactatcac aaggacagaa aaccaaacac cacatgttct cactcatagg tgggaattga 29040
   acaatgagaa cacttggatg cagggtgggg aacatcacac actggggcct gtcatggggt 29100
   ggggggaagg ggggagggat agcattagga gatataccga atgtaaatga caagttaatg 29160
   ggtgcagcac accaacatgg cacatgtata catgtgtaac aaatctgcac attgtgcaca 29220
   tgtaccttag aacttaaagt atagtaaaaa aataaataaa taaataaaaa tttaaaaaat 29280
   gaataaaata catagaaata cagctaacca ggtaggtgaa agatctctac aacaagcatt 29340
   ataaaacact gctcaaagta atctgagatg atataaacaa aaggaaaagc attccatgct 29400
   catggatagg aagaagcaat attgttaaaa tggccatatt gcccaaagca atttacagat 29460
   tcagtgctat tcctatcaaa ctaccagttc ttcacagaat tagaaaaaaa aattatttaa 29520
   aaatttgtat gaaaccaaaa aaaagcctga atagccaagg caattctaag caaaaacaac 29580
   aaagccagaa gtatcacatt gtgtgacttc aatctatatt acaaggctac agtaaccaca 29640
   acagcatggt actggtacaa aacacataga ccaatggaac agaagagata gcccagaaat 29700
   aatgccacac acaaaccatt tgatcttaaa caaacctaac aaaaacaagc aatgggaaaa 29760
   gaaattccta ttccataaat ggtgctggga taactggcta gccatatgca gaagattgaa 29820
   attggacccc ttgtttatac catatacaaa aatcaactca agatgggtta aaggcttaaa 29880
   cataaaacct aaaactggcc gggcacggtg gctcacgcct gtaattccag cactttggga 29940
   ggccgaggca ggcggatcac gaggtcagga gatcgagacc atcctggcta acacggtgaa 30000
   acgccatctg tactaaaatt acaaaaaatt agctgggcga ggtggcgggc acctgtagtc 30060
   ccagctactc gggaggctga ggcaggagaa tggcgtgaac ccgggggggc ggaggctgca 30120
   gtgagccgag atgctgccac tgcactccag cctgggcgac agcgagactc cgtctcaaaa 30180
   aaaaacaaac ctaaaactat aaaaaccctg gaagatgacc tgggaaatac cactctggac 30240
   atataggacc tggcaaagat tttatgacaa agacaaaagc gattgcagca aaaacaaaaa 30300
   ttgactaatt ttgtcaatta aactaaaggg atctaattaa actaaacagc ttctgcacag 30360
   caaaagaaac tatcaacaga gtgaacagac aacctacaga ataggagaaa atatttgcaa 30420
   actatgcatc tgacaaaggt ctattatccg gaatctgtaa ggaatttaaa caaatttata 30480
   agcaaaaaaa caaacaacac cattaaaaaa tgggcaaagt ccttgaacag acacttttaa 30540
   aagaagacat acacgtggcc aagaagcata taaaaaaatg ctcaacatta ctaatcttta 30600
   gagaaatgca aatcaaaacc acaataagat accatctcac aaaagtcaga atggctatta 30660
   ctaaaaagtc aaaaaataac agatgctggc aaggttatgg agaaaaagga atgcttatat 30720
   actgctggtg ggaatgtaaa ttagttcagc cggtgtggaa agccgtttgg caatttctca 30780
   aagaactcaa aatggaatta ccatttgacc cagtaatctc actattgggt ttattctcaa 30840
   aggaatgtaa ctcattctac cttaaagaca tatgcactca tatattcatc acagtactat 30900
   tcacaatagc aaagacatga aatcaaccta catgcccatt gatggtggat tggataaaga 30960
   aaatgtggta catatacacc ataaatacta cacagccata aaaaagaaca agatcatgtc 31020
   ttttgcaaca acatggatga ggctggaggc cattcaccta agcgaactga cacaggaaca 31080
   gaaaaccaaa tacacatgtt ctcacttaca agtgagagct aagcattgag ttatatggtc 31140
   acaaacaaga gaacaacaga cgcgtaagcc tacttgagga tggaaagtgg gaggagggag   31200
   agcataaaaa aactacctat caggtactat gctttttacc tgcgtgatga aataatctga   31260
   acatcaaacc cccataatat acaattcacc tatataacaa acctgcacat gtaccacaga   31320
   acctgaaata aaagtttaaa aataaatcat aaaataaaat gttgaaatgc ctagaatgtt   31380
   tcatagcatt aaattagacc atgctgtctt aaatgacatc ttattttcat agaaatcatg   31440
   tttctgcttt ttctgttttt ctatgaaaat ttttttaaca agcagaagtt aacgaaatgt   31500
   atattttacc acttgggtca agaaacaaat gaaagtattg cccattaagg agcagttctt   31560
   catactgtga taaatgaatg taagtattat gtttacatag atgaagtata aagagaccaa   31620
   ttaatgtaaa aagccaatac tgtacaaatt cctgaaggac cattatttaa atctaacttt   31680
   aatttttaga aagtccttat tcaagcaaac tttactgaga ccaatactaa ttatactttc   31740
   ctgctaaaag atacagataa tgacattagt tagagcctta aagtactact ccaatggaat   31800
   aaaatattta aaattgccac caagacccag tggtgcttta tagagagttg ggtccaatat   31860
   tcatggttct aatgtgatct aaaaagaagt cgtttgttct ccttaattca acacctctca   31920
   gaaattgccc tattcttaaa actattctct aagactactt ttcctccaag actctgccaa   31980
   atgtctcccc ttggatcttc caccataacc aacaaaaaca ttattttctc aaagttagaa   32040
   acaaccctgt gtggccaaaa tatatgaatc ccaacttcat tctcactctg gttgtaagaa   32100
   atgctggaat aattaattac tcatttggaa aaaattaaag ctgtatccat atctatctta   32160
   ccagattcca agataaactc caaataaatc aaagatttaa attaaataag accataaaat   32220
   actgggcaga agaaattggt aaactctctt agaaccttgg aatctggaaa gattttctaa   32280
   ttataactaa aaatacaaaa gtcataggag aaaatattga tagaatacat aaaatcaaag   32340
   aatactgtat gataaactaa acagcaacgt caaaagacaa atgtggaaaa atatttacaa   32400
   ctcctactaa aaaatagaaa ctaatcacct taaattataa ggagctctag aaatcaagaa   32460
   aaacccaaca acctaacaga aaataaaatg gatatgaaca gactggtcac agaataagaa   32520
   atacatatat acctcttaaa catacccagt ctcactcata aaaaagaaat gcaaatgaaa   32580
   gcattctctc acctatcaga ttggcaaaaa tctgcagttt gaccacacac tttattgatg   32640
   agactgcaag gaaataggca ctcacatgcc acaggagtgc aaaggggtat cacccttatg   32700
   gaagagtgtc aatatctacc aaaattactt aagcatttac tctttgaccc aggaagtcta   32760
   cttcctaata atttcatcaa tttgtagtaa atgttcacta tagctgaata aactattgta   32820
   aatccaaaca ataaaataca gtggaagacc tttctgcaca gctacagaag aatcccaaga   32880
   tatatgaaat aaaagaaaaa tgaaatgtaa tactgcatat atagtgtgct acttttgtgt   32940
   aagaaaggag aaaaataaaa gttgactttc atattgcttg tgtttgtata aagaaacatt   33000
   gaaataattt ttacaaaaag aagagaataa aaatgtttaa ctgaggatag cgaatgagga   33060
   cagcatgaat ggaaacgggt aggagtaaga tttttcagca tttacctttt tatgtcattt   33120
   tgatgtttaa aatataaata tgttaactat tcaaaaacta aattaaaaga attattgcct   33180
   ggcacagatg aagtacagtt tagatgttga ctattattat ttttgtatag caatatttgg   33240
   atgggttaat gggctcatat tatgtacagt tgttccgaat ctactcacac tccaggcact   33300
   tttgtcattg caaagaggga caatgccaaa gataatttac cataggattt ccatgtgcac   33360
   ctcctcagtt ctaggctgaa gtaacataga atggcatttt taactcacga aaaactctgt   33420
   tctaaaaggt gctttattat ttgccttaat ttgctcttca tatatttcct taattttaaa   33480
   cttagaattt tagttgtaac ttttcaaagg attgcccaag aagatgcaat gagatgatac   33540
   atgtaaagtg cttagcttaa tgcctggtgt ttaatcaata ctccataact cttagtggta   33600
   gcaaacagaa tcataaaagt gatgtgttca gtgaagcctc taatcaatcc tagaatcgat   33660
   acctacaatc agaaaaaaat tatcaactca gaccatcttt gtatgttttt gtttattatt   33720
   tttcctcaaa atatcttcaa taaggcattc ctagaatttg agagtctgtg tatcttaaat   33780
   aagaatgcat tttattttaa gaggtaaatg gaaggagcaa gaaccaagac aggtactttt   33840
   tgagtctgga gtccttagat ataataagta atttcaggaa tggtatgtat ggtacctaaa   33900
   ggatgtggtt taattagaag ggcttttgaa gctttcttgt agactggttg ttcattatgg   33960
   cttcatctat gtttctccca gaaggcttcc ctggcctctt aactaagtta attgtccctg   34020
   caatgtgctc cttgtatttc ttctgtcata atattcatca ctcatgattg tggttactcg   34080
   attcagcgtc ttctatgcaa accatgcttt cagccttgtg cagacagaag gcatccatct   34140
   ggtccacacc agccatgccc tttaatatta tatgcccagg tcctagaagt ttctggccac   34200
   aatgaatgtg tattgaatga ataaaaaatt gagaaaactg caagctagta aacagtcaca    34260
   tgagaagcgt catcccccgg gcccccatca caacccctac acacacaaag taaaaacaac    34320
   acagtcaaca tgagcaaata gagaaaaaaa atgtggcaat tattcaagca atgcaaggtg   34380
   aaaaatagcc cctttgcctc agttatcaga ggttttcaaa aggatatttt ttgcctgcat   34440
   tttttaatct aattgcttca attattttat ttggtcatgg gaacatgatt atatttttta   34500
   ataaagtgct taatttgcat gcatgctttg aattgtaggt tcttttacag gaggcttgtc   34560
   tactcccata ttgccttttt ctcaattatt ttacttctgc cttttgcaaa atgttttaaa   34620
   tatttgattt tagcccctac cacagactcc aaatctgtgc ttggaatttg gagatcaaag   34680
   tactgtagct aatccttagg acatttgcca ttgtgtgact taagaatcca tctcttatca   34740
   agtttctgtg gttttctgga gtatttacag gtcacaatgt caaaatgtgc acatctgaaa   34800
   ctatttttga attatgcttt ctttatactc ttccatattt agttaatggg aaaatatcca 34860
   gctcctgaat ggtattgaac tcgattaggc catctccagt ctccttataa aagattccta 34920
   agttgtagta tcacatgttt tcaataaacc ttgtttttgt tctcctttaa acagagcgtg 34980
   tcttattcaa aaagctcatg acagtaggaa aggaattgat aatgaaaaga aagtgagtcc 35040
   gaaggaggat ttttcctcat agctttagga tttgttataa gacaatgtgt ttcaccgcct 35100
   gacctgcagc ttcaggagtt ggttgccatg aattctgatc ctgtttctgg aagtaattta 35160
   tcgcattatc ttgtacaagc ttctctggga cactctttct ttccttttaa gcacagataa 35220
   gagcagcaat acactttgca gcgttcataa acagcaatac ataaaagtaa atatgaaatt 35280
   agaaagttcc aaaaactgct ctaatctagt gccagtgagg gaaaattttg gtaaggattt 35340
   tataagtaaa gatgggagtt agatgcataa ttatatgtga cctgtttggt taataatccc 35400
   actgaaatac agatagatga tagatagata gatgatagat agatagatag atagatagat 35460
   gatagataga tagatagata gatagataga tagatagata gatagataga tgatagatag 35520
   atagatagat agatagatag atagatagat agatataata gatctacaca tggcaacaag 35580
   caagattttc aaaattataa tgaatgtcag gtatttattc ttggcttata tttactcaaa 35640
   ttcactcttt aaagtgctat ctttttattc ttcacaggag gaaaagtcta ttttcacacc 35700
   ccaatgttta cccctaagga ttagccaagt gcctaaactg tatccatttc atatttccaa 35760
   aaataggaaa ttgtgcattg aaaaatattg agagccaggt gaaccaggta attctaaatt 35820
   ttcccagata atacttagaa tgaattccca tccataaatg tcctttgcta agtgacgttt 35880
   catctcaagt ggcatctcca ttgtaacatc cgagtaacac agtctattca aagaacttcc 35940
   tgaagatatc ctttcaaaaa cttttagcag taggtgatga ttaatcataa tctgtatgtc 36000
   atctgaaaat aagtatcaag tttcttaaag aaaaattaca tatcccaaca ctactaaaca 36060
   taactggctg ctaagtttca gagggtccat atcatttaac tgcttagagt aagtgcaaaa 36120
   agggagtatt ataaattagg tattgtagtt ttgctctgta aatttaaacc cataatgcca 36180
   ttgtttggct gttgatgttt aaatattcaa gctatgacct tgaataagat atccaagcat 36240
   ggaatttcat cataggatgt tgggatttcc agcagatgtt tcaaatttga ttattcattg 36300
   gtggaatcca aatctttttt tttttaatct gcagtcaaat actggtatgg ggttctgttt 36360
   agaaatcaaa actgactgca gctttgtcat ttggtatgtg tgaaaatagg tactgaagaa 36420
   aattttaaac atctgatggt atcaacgtgc aaaattgctg ttcctaaaag ctgtctcaaa 36480
   ctttctatat aaaagaatat aatataaagc cactgtatca ttgccctaaa ccatgttcta 36540
   aagcatcaga aaatacacat gacttttcat ctcttcgatt tattgaaaac tgatggaatt 36600
   acacctaggt aatttactca agatactaga ctatgcctta aataggatgt gcctcctact 36660
   cattcatgat gactctcagg ggacaaatgc ttctttcctg ttttataaat acagaaacac 36720
   agcttcctta cctccttcag tctgtaaccc acatctgaaa ttaaatgttt gtgatcacac 36780
   tttgaagcaa tttgtttcag taggttactg atattgcaag ggattcgtcc tgtagcatga 36840
   aaaaaaaaaa aaaaaatcta ctgcctgact ctgcatccct gacctttccc ccagactcca 36900
   gccccattca ccatctacca aatgcctact agccatctca aaattaacat ggccataacg 36960
   gacaagtagt tctgcccttc aaatagtgct cctcacctat ccccagtggt atttccccag 37020
   cttagtaaat agcactttat tcacctgatt gcttagacca aaaaccctga agtcagcctt 37080
   agactccctc gtttgtctca tcccacagtc agtccttcag caagtcttgg cctctccacc 37140
   ttcacaatgt gcaccaaatc cagctccttt acagctcctc ctgactttaa cctcactctt 37200
   gtaatgctgc tctcaccctt tgtccagacc acagcagtgg cttcttacct ggccttccag 37260
   cttcctctct gacatctggc ttcctttctg gcatccagcc tcttcctggt tggtttccca 37320
   aaggtaacca aaatgatctt gtaaaataca catctgatcc tgcactctgc caaaaaccct 37380
   ttccctgccg ctatcatata atacttagaa ggacatctaa agctattccc aaagctgtcg 37440
   tgatctggct ggagccttct ctcactccgc tcaagctaac ttgatctcct tgccttccct 37500
   tgaacacact gaacacactt ccacctcaag gtctttttat atttttttgc ctggaaccat 37560
   ttttccttag atatgatcac ggtcacccct tcacttcgtt gggtcatggc tcctctgaga 37620
   tgctttccca ttgagacaac cgaaaatact aaaaacggcc gggctcagtg gctcacgcct 37680
   ataatcccag cactttggga ggccaaggtg ggtggatcac ttgaggtcag gagttcgaga 37740
   ccagcctggc caacatggtg aaaccccatc tctcccaaaa atacaaaatt agcctggagt 37800
   ggtggtgcac acctgtaatc ccagctactt gggaagctga ggcaggaaaa tcgcttgaac 37860
   ccgagaggcg gaggttgcag tgagctgaga tcatgccatt gcactccagc ctgggtgaca 37920
   gagactgtct caaaaataaa ataaaaacta aaatattaaa aacatgttaa ttataagatg 37980
   tttgctaaat tttacctgaa tatcaaccat ttgcaagtag ttatattccc tattcttaat 38040
   aatactttac ttcttggaac ttcacattaa gatagagcac ccgcccctct gtgactccac 38100
   cctcatcctg ctctgcattt ccccagggtt tttgtcacta cctaacatta tattctgtac 38160
   ttgtttattg ctttaacatc agcctacccc aggagaacat aagctccatg agggcagaaa 38220
   actcttctct tttattcact gctctatacc tcatgcttgg cttatagtag acatgcaaca 38280
   aaaaatattt gtcgaatgaa ctaatgaatg aataaataaa ttgccttaac acttaatttg 38340
   agaaaagtca tatccaaaac actccttctg atgcttcctc gttcaaccag tatccaaaac 38400
   aaaaaatgta ccacttagaa tttgagaaat tcaaagttag aaaggatcct tgagacttgc 38460
   cagttcaatc caactatttt aaaggtatgg aaaccagccc tgagagataa gtggtgacaa 38520
   agctagttaa tggcgaacag ataattaaac taggcatagt tcaatgttct ttatgcttcc 38580
   atgctttaca tctctgtttc ttttgaaagt tccttctgta tacataagca aatataatgg 38640
   aaccagaaca cttctattcc ttcttatctt gctgctctgt tggcaataaa acatctggca 38700
   catgatatac atgggacact aaaaaaatta taaatttctg cagaaaaggg ctcacattga 38760
   taaggcagat tgacctctct gtctcaatct gctgtcccat tgggacaacc taaaatataa 38820
   aaaacatgtt aattataata ttttcactaa attttacctg aatatcaact atttgcacgt 38880
   agttatattc cctattcttg ataatacttt acttcttgga atatcacatg taagcctttg 38940
   cttgactttc tacatccctg tttactaggc tttctatgac ataaaagcta ccaaaagaga 39000
   aacacgggaa aaaaatatat aagtgctctg gaaacagaaa ttagtttgat ttttggctcc 39060
   atttaccagc taaatgactt tggataagat tcttggactt tcttagtatc aaattactaa 39120
   tctagaaaag tgaagggaaa ggtatatcta taaaacattt ttttttagat tgagagttaa 39180
   catgatacca ggcacactaa ggcagactaa aggctttata tgtattattt catttaatca 39240
   tcccaatagc cctgtgagaa aaagggatct attccctatt tgcaggtgag gactccaaga 39300
   ctcagcaaga ttaataggcc caaggtcaca caactaagaa aggacagaac tagaaatacc 39360
   tgggctgttg cacatgaagt ttatactcat aacctctgtg cctccttgaa gggctattat 39420
   ggagaaacaa tgaaataaag tctgtgtact gagcatcccg cacagcctca tatatcagag 39480
   gtactcaata aatgttgccc atttctcttc tgttttccca ccactgacta aataaattac 39540
   cttggccaat gtactaacct ttctaggcca tagcttcttt tttaaataag ttatggttct 39600
   cttttttctc attacaaaag caatacatgg ctattaaaga tcatttggac aaaagatata 39660
   tatatatata taaaactaaa atttgtacgg aatcttacca cctaaagata atcaccatta 39720
   atatgcaaat gtatgtattt atactattta tttttctata tactataaga atacataatc 39780
   tggccaggtg caatgactca cacctgtaat cccagcactt taggaggcca aggctggtgg 39840
   atcacttgag gtcaggagtt cgataccagc tcgaccaaca tggtgaaaca tcgtctctac 39900
   tagaaataca aaaattagtt gggcatggtg gcaggtgcct gtaatcccag ctactgggga 39960
   ggctgaggca ggagagacac ttgaatccta gaggtggagg ttgcagtgag ctgagattgc 40020
   gccattgccc tctagcctgg gcaaaaagag ggaaactccg tctcaaaaaa taataataat 40080
   aatacataat attttgggag agcaaaactg aaatcctgct atcctgctat gtaatcagtt 40140
   atttaaataa tgatgtttag aaacatttta tctcacaata ttcttctaag acataatgtt 40200
   ttgtggctgt atcgcatttg tggtaataat aatagtaata ataaattagc tcaaatgtat 40260
   gttagtattg tactgagatg tttcacagtt aacttattca attcttgcaa ccctatgaca 40320
   taggtacctt tattatctac attttacaga taaagaaact gaggcacaga aagattcagt 40380
   cacttaacaa ggtaacccag ctattaagca gcagagccag gattggaact gaggcacatg 40440
   agcaccacag cccatgctct ggaagggata tagcatgttg ttttttatta atccatcctc 40500
   attagaaatc tggattggtt ctcatttttt atactgttac taatagtgtt aggtatgagc 40560
   tgggaggaga aaaaaacaat agtttaataa gtatgctgct gcaaacttgt taacatctac 40620
   aattttatca tacatcccta cagtttaaat tcctgaaata agttattcac attgttaatg 40680
   gttttgtcaa ataatgtcaa attgctcccc caaaaaatac cataccaatt tattcttaca 40740
   gaaaatcttc atcaaataac tttttttctc tgccttgcca acattgacaa ttcatttttt 40800
   taaactttgt cagtttgata gaataaaatg gtacctttct agtattttaa atttcaaaac 40860
   tagaaattgt aaagaacttt tctatatatt aattagtcat tgtttcttct tcctaggata 40920
   aattcgtcta tgtgccttct gtccaatttt ctactagcat gttttcttag tgttttatgt 40980
   ttatatatta aagatattaa tcttttgttc atcatcatat atgttgccat ttttttccag 41040
   gtttgttttt tgcatttcaa tctgtgtaga gttctttttt aatgtatgga attttcgtgt 41100
   gttcgtgcct tcacttctca actgcaaaat atgggagatt aaactatatt gtttttaaga 41160
   cctttaaaat tataacattg tattctattt ctttcctaat tactacaaac aaataactta 41220
   tcaggtaagt atctatcaaa taagttttga acatctacag tatgatatgt acggagctag 41280
   tcactgaggt tacaaaaaaa aaagcacgtc tgcatgcagt tggtttctta tagcttacta 41340
   tagaagagac cagcaagtaa acagactact agtataatca tgggtaataa gtgttatgac 41400
   aggagcatac ctcagacctc tgggcacaag tggccttgac tggttgatat ctgtgctgag 41460
   ttccagaagc aagcagaagt gagccaggta aagaagggag aagggccttc taggcagagc 41520
   agcagcattt tatagagcac tttgaatgcc aggtagcaga atttgatctt tcttctatgt 41580
   attggaaggc cttttttaaa tcacagaggt agtataatca tcttaacatt ttcatgtggt 41640
   agtagaatac aaagtggttt agagaaagga aaaacaggag agtgaaaacc aggccataca 41700
   ttgtagggat gaccaagaga gaagtagcgt agcctgaact aaggaggaga caggataaat 41760
   ggaaagagaa aattagatat gaaacatact atggaagcag aatcaacaaa agtaaaccac 41820
   tcagtagata tgagacgagg aagacaataa agaaggagat gttaatccgt gaatttaata 41880
   tgaggtctgt atcagaattt tgttgtttaa cttgattcat ttattctgaa attcacttgg 41940
   taaaaataaa tacgtttaca ataattgata ctactttgaa aaagaataac atttgtgtca 42000
   cacgaatata ggaataggca ataaaacata atagaatccc aaaataggct tctcaataag 42060
   tatatgcaaa catttggaga atgacagagt ttttatttag atggtattgg gaaaattgac 42120
   tttcatatgg gataaaaagt aagattagtt ctctacttca ccacagagac aaacaaaaat 42180
   tccgttaaaa ctaatgattg tataagttag agtaatgctt agttattata taaatctcca 42240
   aattctagta actaaataaa atagaagttt atttcttact tgtgtaatag tccaatgaag 42300
   gtgttctttg tttggggttg gggtatatgg taaaggctct gttccatgca gttattcagg 42360
   aacccaggct gacaaaggca gtgccatctt cagcacatgg tttctaaggg cattcttagt 42420
   accaaactgc aactgacata gaaaaagaga aagtgataag ccatatgtgc ttgccctcaa 42480
   ctgggaagtg gcatacatca tttccattcg tcttcattaa ctagaattag tcacctcatc 42540
   tcacccagaa gcaaggggtc ccgggaaatg agacctctag atgggcaacc acttctcaga 42600
   gataatgctg taccttagaa gggaagcaca tatctgtggt agacagctag ctacctttac 42660
   cacaaagact attctgaata gtaaacataa attcattaac taaataagga tgtaagtgtg 42720
   ccaaaataaa atataggaaa atatttttat aatcttggtt ggacaaagtc attctagata 42780
   acttaacttc tgtgacttaa aaatgacatg aaatccaggc atctaaaaca ggagggggat 42840
   ggttttgttt ttcccactgt atttgcaaag attgaagact tataatatcc caggtaataa 42900
   atgagtgtgc ttagaaccat gtctatcaca tagtgaacag taaacattag atttactagt 42960
   actattttta ttattattag cattcctttt tattagcaag gagagagaaa taagtgtttc 43020
   catacattgt tagtgagaat tggtaccatc attttgaagg catcttgata tcaatcacaa 43080
   atcaaaatat gccaagaatt cagtctcaag aaatttagcc ttaaattcct tatcctaaaa 43140
   ataaatgtac aggactattc gtttcagcat cattttaata gcaaaaccaa aagtaaccta 43200
   aaaattcatt aataagtgtc ttagtctatt tgttttataa taaaggaata cacaaagctg 43260
   ggtattcata aagaaagtag gtttatgtgg ctcacagttc tgcaggctgt acaaaaagca 43320
   tggtgcctgc atctgctcct ggtgagaacc tcaggctgcc tccactcatg gcagaaggtg 43380
   aagtggggcc aacatgtgct gagatcacat gacgagagat gaagcaagac ataagtgcca 43440
   ttttcttttt aacaaccaaa tcttaaggga acttacagag ccagaactca gtcattactt 43500
   caaggacggt accaagccat tcatgagcga tcctccccca tgaccaaaac acctcccatt 43560
   aggcttcacc tctaacactg gggatcacat ttcaacatga tatttggagg tcaaatatcc 43620
   aaactatagc ataagtaatt ggttaattaa ataaattagt gtcctacctt atatacattt 43680
   gtattgtttt gatattttac tttataactt taaagtgtat attttattaa aattattcaa 43740
   aacttagctt atagtgaggc cactagaata attcaaaaaa ctggaacaaa aaaatgtggt 43800
   gggagagggg gatataaaca tcaagaacat tgtatttgtc gtacttctga ttgtctcaga 43860
   gagaacaaga tgaagtgagt ttaaagggaa agaggtaaat ttatgttagt tattagggaa 43920
   aactttctga gactatagaa taacttttaa tttacaaaaa ttttaaaagc ctgaagtctc 43980
   cagacagttt ctagaagtca agatataccc tctttgtggc ttacttacct cgagagggtt 44040
   tatctgtcct tataactact agtttgaaag tgttcaacca tgctttgttt gatccagatg 44100
   atttagttct tttcattgtg attttacatt gagccatgtt atactttcta atacatgcat 44160
   tatgcagtgt aattgtagca acaaaacaca gtccatcagg ttcagatgta caattggcct 44220
   tgctctaacc caatccttga gttcacaata gggaagcttt ggatgtaaat aatgaaggct 44280
   ttttggtatt tcagtgacta acacctttta gacagggtcc atggtgatca attcatattg 44340
   attttgctga ctaaataata cttgagcaaa gttccagtgg catctaaaaa taagctcttt 44400
   cacagtcctt atgtgtctta aatatcatta tggtatttat tttgttcaaa tctttgcagt 44460
   cacctcaaat ttcaagaatt catgtgtaaa tcattctaag tgatgcagta aattagtgaa 44520
   cctacttcct aataccttgt aaacctatat ttcacatttc tactgggctg cttataatca 44580
   atagctcttt cttcactact tcccaacttg ttctaatttg tttcctttga gagaggtaga 44640
   ttttaagagt tctggattac atgctttttt ttttattcat gtgacttaga gctcctcctt 44700
   gtttattagc agttctattt ctttctctag ccccattttc tcttgaagca gccataaaag 44760
   taccatctgt ttttcagcat cattttattt ctgctttctc ctccctgcac ttttctcttc 44820
   tctgaatgct ttatgcgata acaatttatt cctgatttat tatccacctg ctttcatagc 44880
   ctcccccttt ctgttcctcc tccatcccta tcccaccctg gatggagtgg tataccataa 44940
   ttcttaagga cacagctttg gagtcaagta agggccagct caaccactta tgcatgttag 45000
   tgacttgacc actatgagcc tcaatttctt catttataag aaagagaagg ttataatatc 45060
   atctactaca taggttggct tgaagaatta aataaaataa tgtacatcag ggtttctcag 45120
   ccttggcact attgacattt ggggctgagt cattctttgt tctggaggct gtcctgggca 45180
   ttgtaggatg gttagcagca accctagtct ctaccactag atgctcgtag caaccccctt 45240
   cccagctatg acaatgcaaa atgtctccag acattgctaa atgtcccagg gaggcaaaat 45300
   tgtccccatt cggaatcatt gatgtatata aaacatttag cacagtgcct ggtacatagt 45360
   agggacttga tcaataattg tgatggatgg atggtcagat gaatgattga atattcattt 45420
   ttaatacaag tgtctaatat aattcttgac agtcaattaa ctacttaata cataaatgga 45480
   taaacaaatg aacctgaagc cagtgagctt caagggctgt ccatagtttc taccttacac 45540
   caaaatgtgt tatcttcact aaaaacaagc ttcctatgtt tcaaaaaata ttttagacaa 45600
   ttaactaact gcacagctta agctactccc ccaatttaat atatatgcat atactttaaa 45660
   taatagggag attttaaaat aaactctaat ggatgatagc tataacaatt atgttgctat 45720
   agaattcagt gctagaaatt gaagtgatcc tttgattaac ctgagtattt taggttgata 45780
   tcccagtcaa caagccattg atgtggtaag agaacaaagc tcagccaact tttaagtgtt 45840
   ttttaatcta atttatctta tttaaatatt tattgagtgc ctactatatt tcaggcactg 45900
   ttctaggtac tcaggataca attgtgaaca aaacaatgat aaaacactgt ggaaccacca 45960
   ttctatgaaa tagtagatca caaaaatagt aaatacataa atagaagtaa aaataaagac 46020
   tactcataac ttgtaaaata ctagcctagg aaatagtcta ggtggtactg acagcaaaat 46080
   tccagtggat gactgtaacc caaccagaaa catttttgtg actgcactca ctgtttgcca 46140
   tttggttgct catctatgat ttcagtttac tgaactctaa ctttctcgtc tgatctgaat 46200
   tacaatgagg gtattaaatc aaatctgtgg ttacctttgc atattttgga acttcaaaca 46260
   tttagattgt gattcactgt gtgcataatt ttttaaattg tctcaaatca acaatctttc 46320
   tcttactttt ctaggttttc tctaatgaac taactttata gcatactttt tgtgctgtag 46380
   tctaacaaac cgatagctga gtttttttaa aaatgaatac aatatatgct ttttctgcct 46440
   cgcttttttc atctgtaaaa tgggatctgt gggataccta acacataaag tcattgtaag 46500
   gattaaataa ggtaatgttt attaaaaaaa aaaaaaacct tagaactgaa gaaaatgcca 46560
   agcacatagt aagttttaag tgtagtattt tctgcacccc tactagccat gtacttattt 46620
   ctagtatttc caatatttta gcatagtaaa tgaggtagta tatctttttc ttttctttct 46680
   acacttggga gaaagcagga ttacaatgaa ataaaagcaa aatgtatgtc caacagggtc 46740
   atataagtaa tgtctgtttg taaaccacta gtctcaggag taaatccctc aacatgatga 46800
   aaaccaagag atgtgtttgc acataactct ttttataaag tttttttaat tgcaaagaca 46860
   tagtcgttgt tataaacttc caacatacag aaattaataa tttacaaagt gaaaagcccc 46920
   tttatgcata tattcacttc agctcactcc ctttagaaat aattaatagt aataatcagc 46980
   gtatagtctc actttttttt tttttttgag acgcagtctt gctctgtcgc taggctggag 47040
   tgcagtggca cgatcttggc tcactgcaac ctccgcctcc tgggttcaag ggattctcat 47100
   gcctcagcct tccaagtagc tgggattaca ggcacgtgcc accacaccca gctaattttt 47160
   gtatttttag tagagacagg gtttcaccat gttggccagg atggtctcga tctcctgacc 47220
   tcatgatccg cccaccttgg cctcccaaag tgctgggatt acaggtgtga gccactgcgc 47280
   ccagcctagt ctcaaatttt tttcctagtc atatactaat atatatttct attaaaataa 47340
   aatcacattg tacatactgt tttgcaactg gcctttattc acttaaaata tgtctttggt 47400
   gtcagtgtca acacatttag ttccttcttg tttccaacag ctgccaagag tggtaactct 47460
   ctttggctct ctgggtactt acccacaaaa gcacatgcat ttagagctgg tggaaaaagt 47520
   ccttcagggc ggacaccggt ggctcacacc tgtaatctca actctttggg aggccaaggc 47580
   tggcagatga tgtgagctca ggagttcgag accagcctgg gcaacatagc aataccccat 47640
   ctctacaaaa aaatacaaaa attagccagg cgtgttggtg catgcctgta gtcctagctc 47700
   cttgggaggc tgaggtggga cacagaggtt gcagtgagca gagctcatgc cagtgcactc 47760
   cagcctgggt gatagagtga gaccctgtca aaggatggaa agagggaaga aaggagagga 47820
   gaggggaaga agccctttgg ctgaaacttg cactgccaac aactgacccc ccctctctct 47880
   caaagcactg acccaaaaag gggcaccagc agagaggctc agaagatctt ctcagtccac 47940
   accaggagac cagacctttc tcagtgcagg gagaaggatg agctgaatga ggaatactca 48000
   cttatacagc agttaatcca gaggatgaca gaaacattac aaatgtgaca tccttgaaat 48060
   ggaactcctc tttggagtgt ggaagcccag tcatgcacaa cccacttgga gcaggaagga 48120
   aattgtattt gaggatgtta aagtcaagct aacttttatt aaaattgcca caggaggcca 48180
   ttcatcccca cttcacttat cccacttcaa agcctacttc ccatcactca gatgtttact 48240
   tatggagtga ttaagttttc ttttatcttt tctatgcttt catcagatgc tctttttctc 48300
   ttttttgctt agaaaaggca gattttttca gcctaatgtc aatatgtcaa acattaagta 48360
   ttcattccct tgcaatgcac agtgtttata cttacatacc ctattataac aaatgtaata 48420
   ttatttaatt atgcagcaaa cttacacata attggtgaac aggtctaaag aatactctat 48480
   acaggaagca tagagtattc agcttagctg aggcctttgt taaacaatat gcaaagttgc 48540
   aggtgccaca ttagggaatt ttgggggaaa ggaaatgcaa acttatttgg caggaaaaca 48600
   agagaaatga ttatatctca cagcactttc aagctggaca catttacatg cctctgtcac 48660
   atcacccaga agctgtggtg ttgagggctg aggcaggaga tccttttgcc acaggaaaaa 48720
   ggatgaaacg agagacattg tgaaaggttg ccccaaagtc ttaaggctcc ctgaaacctt 48780
   gggttttttg aacaccattg tccaatctgt tgaaggttaa ataatacgtt gttcaaaatt 48840
   gcataggtaa agcgcagaat ggccaaaggg tcagaactac agcaataggt aggtcccatt 48900
   ctctgccttt tcattcttca gtcttcggaa atgccacaga ggccaagtgg cactaggtgc 48960
   agctgggaat caagatctgt ttatctgtac cttaaatagg tcaaagtgaa ccttttggaa 49020
   atattgtttt ggtaaataaa aattcctggt cgtgctaact gtggctattt cgacctgttt 49080
   ctttcagata ccattgttct tatcgcttca atagcagttg tttctgcaaa aactcagggt 49140
   aatatttttg ccacgtctgc actcagaagt ctccgtttcc tacagatcct ccgcatggtg 49200
   cgcatggacc gaaggggagg cacttggaaa ttactgggtt cagtggttta tgctcacagc 49260
   aaggtaagat ttgctctctg aatttaaaaa cacaattttt gaaacttttt cattgatcgc 49320
   tgtaatagtt aataaacctg tcacaagaaa acttagtcat tgcagaaaat ggttaaaaca 49380
   attttgggac atgatgtaca atggattatt taagagccat cataaacatc caagaagtag 49440
   taaagtgaat agaatcctca tagaattctt ttttcctttt tcaaagattt cccacctcta 49500
   aaaatctttc attcctttta tatctaggaa ttaatcacag cttggtacat aggatttttg 49560
   gttcttattt tttcgtcttt ccttgtctat ctggtggaaa aggatgccaa taaagagttt 49620
   tctacatatg cagatgctct ctggtggggc acagtaagta taaaaataca ttttttattt 49680
   attggatgtt gtgaattgtt tttttttaat acaacgtaat ggttcctatg gatggtttca 49740
   ataaaaatat taaattgcga aagagagtta tatggaaaat aaatactact tattattgtc 49800
   taaaggcttt ccacatcttt ttaggccaac taagttgtct tgtaaaccat cattaatatt 49860
   tcttgagtgc tagattgttg ccaaaattaa aaatatataa aatatataaa tatatatcat 49920
   ttttaaaatg gtgattaaaa cattgatagt ttgtttattt gttttaactt tgtggagcac 49980
   aaatttaaat gatcaattgt tgcattgatt ttccgctctt caaaagttta tctggggtta 50040
   aaaaatgaat ggaagctgtg aataaaattg cagcacaaag aaatgaaaaa gaagactggt 50100
   aaccatctgt gagatcagag attatagtca cctaatttca aaactttcct ctcaaaacct 50160
   actttttctt tatattatta aagaataatt ttccaaataa ataaagtaaa atcatgattc 50220
   ttacatagaa agaaaattaa cacatgctaa acattcattt aactcatttt aaaagtttaa 50280
   gtcattatta atgaagaaac cagtaagatg ttaaaaccag ttcaaagcac tatccaaagg 50340
   acagaaacat ttaaagatca ggaatattga aatcaatgtg caaatagagg caaaactggc 50400
   tccttccaca gtgtgggagg gaagtcagtt caacctccac taggcagaat ttatttacat 50460
   gtccataggc aagaattatt ttgcattgct accagttatc aacaacttac agagttgtaa 50520
   aatagctcaa aggctatgaa aagagcagag ccccataaaa tcagaatcca aaaacctgga 50580
   agggtgtact ctgaacaacg catagttttc cactgaagca aaaattttcc ctgcattgta 50640
   aagaatttat gaaacagtca ttggagctat gccgtaataa gaatgacatg ggatatagga 50700
   gtgctttatg caatgtataa attacatttc tttttatttt attcctttaa ggtattccaa 50760
   aggggagtta tatgaaaata atcatagcca aacatggtgg ctcacgcctg taatccaagc 50820
   actttgggag gctaaggcgg gccagttgct tgagctcagg catttgagac cagcctgggc 50880
   aacatggcaa gaccccatcc ctacaaaaaa tacaaaaatt agccaggtgt ggtggcacac 50940
   tcctgtagtt ccagctacct gggaggctga ggtggcagaa tcacttaagc ctggcaggta 51000
   gattataaat gcttgtctaa agaatgtcca tatcgttttg gttcaaaaaa ccccgtgagt 51060
   tctcttttaa accaccctta gtatttcttg agttctagat tgttgccaaa ataaaaatat 51120
   atcattttct taagctgtga tttaaacaat gataggcttt ttatttgttt gtttagtgaa 51180
   gcacagagtt accaattgtt gcattgttcc actagtcatc aaaagctcat cagcaagacc 51240
   cagaacattt gtcagagagg gggaaaattt gttagggctg tatttagcag aacttgttgt 51300
   atttgttgat tgcatagcag caataaaatg aataaaaatg agaaatacat tcacctaaag 51360
   attcgaattt ttttcttaag ttatctaagt aaatgtctac aagattatgc cattggtttc 51420
   aaaggattcc gttggtttaa taatgtggtt tccggctgct cttcactatt cacaagcaat 51480
   aatgggcatt tcgaaaaaga gtggaagctt gaaagagagc agttatctga cattcttaaa 51540
   atacttttta tatgtagaga gcaaatgaaa atctgtgact catgatggga taaactgtaa 51600
   actgagaaaa caggaggctt ttcttgccag caaggggaag acagtttatc atcccagcct 51660
   tcaaagcaat ggtttgaatt caagtttaat ttttgcttct cttggataat tctaatgtat 51720
   ttgacttact gaggcaccca ggtgcccctt ttgccactga gatgcagttg ttggtgagtt 51780
   tagcctggga gctcattatc tttcttttta ttagtcttcc atttctcctt attacaaaaa 51840
   agaattgctg tattgtgtgt taaacagtgt gtgggaaact cttaatatac ctctcagtta 51900
   gtcatcagtt tgtttctttt gccagaataa cccacacggt aggttttcaa aggtcatatc 51960
   tgtgactttc aggggaagaa acattttcat tgatttgcct ttcagttctg ctaatttgac 52020
   tgacatttaa cccgtactta acaggttcct gaatggccta attatgtttc tatttttaaa 52080
   aaaaatctag tcttgaatat gagagtaaaa attttataag agatcaaact tcgaagaatt 52140
   ttttaaggcc ttattggtcc gtagcaggtt tgttgatatt aactgactca ttaatgaaat 52200
   tgtctgaatc ctaaaaagac agaagaagca aacaaacaag agagctcatt gtttctattc 52260
   attttaagaa gaacatatac tagattccta gtgggaaata aaataaattt agactattat 52320
   accttttatt tatacattaa tcaagacctt agtgaaaatt atggttgggt atttcttagt 52380
   taaaattgcg catgttgcac ctaatggaaa ccttaataac tatggaagta tccagaataa 52440
   atagggaaat gatagcatcc taaatgagag tatttctaag tgttacactt tgatgaaaaa 52500
   aggaaatccc ctatattaaa agatcccaca gtatcaacat taagtatcaa aatataggtt 52560
   ttgttccaca gatacgctgt gccagtgaat tttcaatact aactctggaa ctccttccat 52620
   taagatagtg agatctgtcc cctagatcaa catttggagg gatatcatgg tgggatcagt 52680
   gctgcccaaa aaactttgat ccctgaaagc agtttcatat tagtgaatag caatacctag 52740
   ttcattgagt aagtgaaata gatataaaag acctggtacc aagaacatag gaagtgccca 52800
   atcaaatatg agagctattg ttggtgtaga catcattgct gtggaagtta aatgaaagca 52860
   cctttaatac aaaaggaaca aaaaaagagt tatgtgtttt gatgcagacc cacagtttga 52920
   acacagaaga ggcttgggaa ttggaggttg tgatgaaaag atgtcctggg ttctgatgtg 52980
   gcaactaaag ttgcattgat ggcagaaaat ggaagaggga agtagctcta gtagctgagg 53040
   cactgagagc tgctggaaaa ggggatcttc tgattgttgg atagagctcc atttattatc 53100
   attttctatt gttatttttc tcatttttgg ttgttccaga tttattctga aataatttaa 53160
   gaatgggttt ccctggagtt attttctgtt attaaatcct tcctgttttg tgggagtgat 53220
   ttttctcttt ttgatctgct ttagatttat tttgaaataa cttaagatca ggttttcctg 53280
   cactccagcc tgggtgcttg agtgagaccc tgcctcaaag actcccttgg ggagaattat 53340
   accgtgccac ggaaatctac caacagtata ttgttcacct gaacacatta gctataaact 53400
   aatgtacata gatttactaa aaacataatt gtatcttcaa aatcaggtca ataaatattt 53460
   gttgatttgt tgcctgaaag aaagaaggat ttatatctcc tgatttggaa ttaattctgc 53520
   tattgaaaag aaaaaaaact ttagcctaac cagtgtctta aattataatc tgaccatagc 53580
   ctcagccatc tcatttaaaa aaaaaatcaa gaaaagtctg gaccagagtt ttcatttgtt 53640
   tttcagtatt atttctgagg aagaattcct gaatgctaaa cacacgaagg tgcccccact 53700
   ccacctcaga ttttcctaac ataattacaa ctttatgcca acatttccaa cccacagatt 53760
   ttcagaatgt attttctttc acctgtgtga ctgtatttga gcataggaaa cataagggcc 53820
   aaaatatatc taagctgttt ctatctgcaa gacgccttca aataaatggt ccagctgaag 53880
   agatggtaca aaataccagg gattcctgag gccagctgtg gggaggtagc agggcaaagc 53940
   agaccatccc tgcccagatc ctcatccgcc ttgttaccct gcagcctgaa aaggcagctg 54000
   ccagcttctc agtttaagaa ctgtggcaag aaatggcagc ttctcactta aggagaatac 54060
   tactctgcct agaactttac tgaaagagat ggtggacatt tttgtcactt gtaatttgtt 54120
   tttaatttta aaaagtaaat tcaaaacgtc atgaccttac ctatgatatt ataaagatca 54180
   tactcaaaag tcacgagtat gaatcacaga agatttttat aataaaaatg taatctatcc 54240
   agtgcatgtt caccagcata tctcttacaa gctctggaat gaggaaaaaa aatggaatta 54300
   tgtccaatat cgtgatgtaa agatccatct ctctattctc ctgcttctca tctcttacac 54360
   acacgtatat ataagcaaaa tgtcacgctc acatgggtgt gggaaatcaa agctcacgaa 54420
   acactattta gtttacttgc ccacttatgt aaatattgta tttgttttag aatagagtag 54480
   aagctctcag aacttatttc ctcttcactg actggccaga ttaaccaaca ctcctcactc 54540
   ccacaaaaca tactgaggct tgcagagcac aaaatgctct ttagtatcca cagaattctg 54600
   ttctgaacat tgtttgaatg cttgcaaaga gtcagcatgt tcattaccaa accttttttt 54660
   tttttttttt tttgagacag ggtctcactc tgtcacatag gttgcagtgc attggtgcaa 54720
   ttatagctcg ctgcagcctc aacctcccag gttcgagcag ccctcccgcc tcagcctcct 54780
   gagtagctgg gaccccaggc acatgccacc atgcccagct aattttttac taattttttg 54840
   aagaaaaggg gtctcaccat gttgcccagg ctggtctcaa actcctaggt tcaagcaatc 54900
   tgtctgcctt ggcctcccaa agtgctggga ttacaggcgt gagccactgt gcccatccca 54960
   ttcctaaacc tttttatgtc agttgtattt gtaattataa tggcatggtt taattttata 55020
   ttattgcata aactgatgac atatggtaca gaaagaaaga tggatggttt acgaaaatag 55080
   aatgctttag aaaagcttga taaatgcaaa atactaaata tactaaaata ctaaaaatac 55140
   ttcaaaatca ggggtgaaca cacaactatg tgggggaaaa atcttaaata tttaggactc 55200
   tgttctcaga tagctcccac aaaagttgag tttatgcacc actaaaaaaa aagttaattt 55260
   taaaaaacat taaaattaag gaaaataaat gatgcattat gagtgagatt tatgcaaaaa 55320
   agataaggca gaaccccaat tgcagaacac attcttcatc aagaatatta catttatgtg 55380
   ctttaggcta aaataaaata tgcagcatat aagtctcttt gtgattatct gcattagtca 55440
   aatttctgac taagcaacca agtagccatc ataaaagttt caattaagaa gacttctctg 55500
   tgtattctca tgcattaata tataatatca gaaaattatc tcaccaacag agaataacaa 55560
   ggctgtctca taagaacatt tattttacat tgattcaact gctttatggt tctactaatt 55620
   cccttgcagc acatttgcta tgcagagtaa aagtctgatg tattgtgatt cacttttaac 55680
   actgtatata ttagaagaga attaaatgct ttgactcagg aagtatcaaa aatatattag 55740
   tacaaaaaga acccattaac cgaccacact ttagttcttt ctgtcatccc aaacccaccc 55800
   aatccctgtc atccccacct acctagccca tatgtagtca tgaagcatcc tcatggttca 55860
   cccgagatga acagagtcac tgggtttaat tccagcacca tcacccatgg gactcaaccc 55920
   ttgcacccta atgccatatg cacatataga actggcagag aattacacaa caaagggggc 55980
   gggatgtgat ggaagtatct tcaaaggagc aactacatta tttatttaat gttggattcc 56040
   cagtctaaag atagtgtaaa gggattattt atttaatgtt ggattcccag cctaaagaca 56100
   gtgtaaaggg agtgtgttat ttatttgttt ttctttataa cagtaatcca ttttttccaa 56160
   aatcagctct ggctctttaa cttcatttgg cccatattac tgtgttaact gtatactgtt 56220
   aagtgataat agccacctgc ccttctgcct gggagtcact gacgtacgca gtttgtaagc 56280
   atctgcaata cagagatgat gagatacaat cgaataagtc atcttcccat gccatgccat 56340
   caggatgctg ctgggttctt gaagatctgt gagaaatgtt tctagaaaac gaactgactg 56400
   cctcatcacc caattcaatt gtgaagtcta gatgtgattt cctccaagcc cctaaagatc 56460
   gcatatgcag cctctccagg ttctctgaaa catgctgcta ttccagaatc tccccactta 56520
   ttctgaagaa cgggtgccct tgggttcttg tgtgggcctg ccctttctcc gaccctgaac 56580
   ctaaatgaaa ctaatcatta ctgggttcta ccagtgaaaa gattcataac atttttggaa 56640
   ggcagaaaga ggtttattat ttagcacaag catgggagaa taagaaccaa gagggctcct 56700
   gctactcagt tcatgtacaa aggatcaact gataaacatt agcagggcct cccaagtgct 56760
   aggaaaagaa aagaaatata atgtagttgt acagttccaa ggagactcac tggtacttca 56820
   agggcagctt gacctgaaag tccagaaaaa tcaatgaata tctatgcagc gctcatctaa 56880
   ggatggttta tcaaatgcag ctggtccttg acttaaaatg tcaaagccag atcaagcaga 56940
   tgggtcaggg tgaatacctg gaaaaaatta acatcttttt attgtaagca catcttaaag 57000
   acttcataat tttatgtctg gtgctatctt tctgttcaca tcatgaagcc caggtgtcct 57060
   ctttgccacc acagaaggga aatgatgcat gcacgctcca ggcaaaaaac gagggaaacc 57120
   cacaccaact ccctctctgg catagagggg gacaaaactg ccaccctgtt gccagatcct 57180
   tcccaagcct aagctgccaa caaaaggccc ctttgcttta accaggaaac atccctagct 57240
   catttgaaat cagaggggag aaagttattg aggctggggg ttcagttgct ggctgttcag 57300
   gtcccttgca ttcagtgaaa tgtgatcatc ccggaagcct gaactggagt gtagggagaa 57360
   agtgggcagg gtctttgctc acccccagag gaactccaga gtgctgttct cagatttcac 57420
   aaccttctat tctcccccgc aaaccacccc tgtaagagaa aggggcaacc ctaaattcag 57480
   aatgaaagaa agcaggcact cttttctact caggatgaaa cactgagcac attgtcactt 57540
   ggctgtgtcc cattcttcag agttagagtc agtggtttaa aacccactct gtgagcactg 57600
   ggccgagcaa atgtattatt ggaatgaaaa gcaaaggaaa ccacgaaatg taacattttt 57660
   gctgtgacca aaccatgaaa gttcttcttc agtctattat tattaataaa ccgatgtaaa 57720
   aggattaagc tatttttcag aaaaagtata gtagattaat atctgtccca catatataaa 57780
   gattaagaat aaaattgacc tggaaatttt ttttctgcat tctcaaggaa ttcttttgag 57840
   aattccatat taatactgct gatgacatta gattaatttg gctcctgtaa agttctacta 57900
   catattccct ctctctgaca ttgaatgagt tcttccaacc agcagacctc ttattaacct 57960
   gtccttgacc ccttctcaga ccttcacagc ccaagcctct ttgcctattc caagcctctg 58020
   actccccaca catgatccca ccttctcctt gtctataatt gaaaggacag acataagcat 58080
   tcctaatcct tccctccact cacactgagg ccacatcata cccccactgt ggaagcgctg 58140
   tcctttcaca ctgcctgggc taacccctcc aactgtgccc ttaaccgaat tccttcttgg 58200
   gtactttacc ccatcaggaa gccgccaccc cttccctctt cagtctcacc ctctctacaa 58260
   acatgcagaa gtacatcctg gaaagcctcc cttcaagtca gagattcctg ggagttcttg 58320
   ccctctacat ctcacttcag ttgttgccaa tgttcttcaa agtattattc atttatttac 58380
   taaatattta gtgagtttct ctcactttgt ggcaagcatt atgctagatg ttggggaaat 58440
   aaagagcaag ctgaggttcc actttctagt taaggaaata agaatcccta cccaaagaaa 58500
   aatacaacag actatcactc acgttaatgt tattacgaaa caaggagctg taatatagag 58560
   taatcagagt ctctactgaa gataggtagg aaaggcctct ccaatgaggg agtattttgg 58620
   ataagacctg aaggatgaaa aggagccctg ggaagagtga ggaaaatggc aattccagct 58680
   gagggaacag cttgtgcaaa ggccctgagg cagaaagatc ctggcttgtt tgaagagcca 58740
   aaaggccact gtcattaaag tgcagtgagc aagggtaaaa tcaaaatgat cttaggtagg 58800
   tggagatggc ttaattagta gcttagaatt tattttgaat tcaacagcta gccattgaag 58860
   gattttttag atggaaataa gaaaatgata ccccttgcag gtatatgaag aaggaactag 58920
   aggaaagcaa aagcaaaagc agagagagca attaagaggc tactatagca gcctggtcta 58980
   ccatggtggc agcaaagaca gagaaaaggg ggtgaatttg caaaaaccta aggagatcga 59040
   atcaacagga cttgctgata gattccgtgg ggggagtgag ggaaatgaat agttaggatg 59100
   attctgaggc ttctgacatg agcaagtgga tagattggtg gagctcttgg ttaagacagg 59160
   aaggaatata tttggtagaa ataagagttc tgttttagac acattaagtt aaatgactgt 59220
   gacatctaaa tggaactgtc aactgcagat atgtgaattg ggaactcaga aaagtggtct 59280
   ggcctagaga tataaatttg gagtgtttag cataaaagtg gcatttaaat ctatgagaac 59340
   atttaagatc acctggaaag agagaaaaaa gaggggatag aaaagaaagg aaagtctaag 59400
   aagaatacaa tttttaaaag aagaaaaaga cttagaacaa aaccttaaaa atccagtgtc 59460
   caaaggttga aaagggtaag aggagccaca gaaaaagagc caccaaggaa tacaaagaga 59520
   aaaccatgga agtgaagaga agagtgcttc tagaaagagg tggtcagctg agctgaatgc 59580
   ttctgaaagg tctggcatca tagaatttgt ctccctcttt atcaaacctg cttctgaatt 59640
   gttccccctc tttcaaagcc caggtaaggt ggcatgacca gagcaaccac tttctcagtg 59700
   attgccagtg accacctatc tccaccatat tcagtctctt tttctcaatg ctcattgtcc 59760
   ttgttttctt tttctctctc tctctctttt tttttttttt tttccagatg gagtttcact 59820
   ctgttgccca ggctggagtg caatggtgtg atcttggctc actgcaacct ccacatccta 59880
   gattcaatca attcccctgc ctcaccttcc tgagtagctg ggattacagg catgcaccac 59940
   cacacctggc tagttttgta tttttagtgg agatggagtt tcaccatgtt ggccaggctg 60000
   gtctcaaact cctggcctca ggtgatccac ctgcctcggc ctcccaaagt gctgagatta 60060
   caggcataag ccaccgtgcc cagactcact gtccttgttt tctattaggc tgtgacatca 60120
   gagattacct ggtctttgtg aaactctcca ttctctattg agtttccatt tatataaccc 60180
   tttcccgatt gttctacttt tgaaaaactt gaaaaacatg acatccaaag acattcttct 60240
   ctgactttcc tttcccttcc attccttaaa tgtctgtgtt ttttgaggtg tccctggacc 60300
   ctttctgtgt accttttgta atctcagtta tgcacatagc tttaacatca ccctgttact 60360
   gattctaaaa cccaaatctc acgccaaacc aaatttctct ctcaggactc aagctctcat 60420
   tctcagtttc ctgctagaca tttctgcatt agtcatactg acatttgaaa aaccattatt 60480
   tgcaaaacta aactcttcac ctttactcca aaaccttctc tttttcatta taatgctttt 60540
   atttatggtg caaccatctt cccacttaaa attttcaagt cactgttaac ttagtctttc 60600
   cttttacctc aatcaataaa tttatttcta ttgaacttcc taaacatttc cctttactct 60660
   ccactctcat agtgtcttaa ctggtctctg cacctctcat ttcttcctat ttcaatctct 60720
   cctgtacatt tgctgccata atttcagctt ctaaaagcac tactctgatc acattatgta 60780
   ctttgcttac aaaaaccttc cgtcagtatt tctgggctgc aaaacaagag agcagaatcc 60840
   tatcccaccc atagggcttt cattccttta ttcagtaaat atatattgag tgactactgt 60900
   atgccaatca ctgtgcaagg tgcttggatt cagcagtaag ataaagtccc aacccacata 60960
   caatttactt tctagttggg aaggcaaaca ataaacaatt taatcaatat acaatatcat 61020
   ttcaggtatt gatacattct atgaataaag atgaacagaa taaggattta ggaaacagtt 61080
   ggtagggaag tagctttttt tgttgttgtt gattttcgtg gggttttgtt ttgtctttat 61140
   gagaaaatga ctgttattat tatacagtgt cccccagcga ttatttttct gtaaattatt 61200
   ggggaacagg tggtttttgg ttacataagt aagttctttc atggtgagtt gtgagatttt 61260
   ggtgcaccca tcacccaagc agtatacact gcaccctatt tgtagtcttt tatccctctc 61320
   ctccttccca tcctttcacc ttgagtctcc aaagttcatt gtgacattct tatgcctttg 61380
   catcctcaga gtttagctcc cacctatgag tgagcacata ggatgtttgg ttttccattc 61440
   ctgagttact tcacttagaa caatagtctc caatctcatc taggtggctg caaatgccat 61500
   taattcattc ctctttatgg ctgagtagta ttccatcatg tatatatata tataccacag 61560
   tttctttatc cactcattga ttgatgggca tttgggttgg ttccacattt ttgcgattgt 61620
   gaattgcgct gctataaaca tgcatgtgca agtgtcttct tcatataatg acttcttttc 61680
   ctctgggtag ataaccagta gtgagattgc tggatcaaat ggttctactt ttagttcttt 61740
   aaggaatctc cacactattt tctatagtgg ttgtgctagt ttacattcca accagcagtg 61800
   tagatgtgtt ccctgttcac cgcatctcca ccaacatcta ctatttttcc acttttttat 61860
   tatggccatt tttgcaggac taaggtgtat cacattgtgg ttttcatttg catttccctg 61920
   atcattagcg atgttgagca ttttttcatg tgtcttggcc attcatatat catcttttga 61980
   gaattgtcta ttcatgtcct tagcccactt tttgatggga ttgtttgttt ttttcatgtt 62040
   gatttgtttg agttcattgt agattctaga tattcatcct ttgtcaaatg tatagattat 62100
   aacgattttc tcccactctg tgagttgtct gtttactctg ctgactgttt cttttgccgt 62160
   gcaaaagctc tttagtttaa ttaagtacca gcaatttatc tttgttttta ttgcattcac 62220
   ttttgggttc ttggtcttga aatccttgcc taactcaatg tatagaaggg tttctccaat 62280
   gttatcttct agagtgttta tagtttcagg ctctatattt aagtccttaa tccaccttca 62340
   gttgattctt gtatgtggtg agaaatgagg atccagttta attctcctac atatggctag 62400
   ccaattatcc cagcaccatt tgttgaaaag ggtgtccttc gcttctcaaa agaagacatt 62460
   tatacagcca acaaaattat gaaaaaatgc tcatcatcac tggtcatcaa agaaatgcaa 62520
   gtcaaaacca caatgagata ccatctcaag ccagttagaa tggcgatcat taaagagtca 62580
   ggaaacaaca gatgctggag aggatgtgga gaaataggaa cacttttaca ctgttggtgg 62640
   gagtataaat tagttcaacc attgtgcaag acagtgtggc aactcctcaa ggatctagaa 62700
   ctagacatac catttgaccc agcaatccca ttactgggta tatacccaaa gtattataaa 62760
   tcattctact ataaagacaa atgcacttgt atgtttattg cagcactttt cacagtagta 62820
   aagtcttgga accaacccaa atgcccatca atggtagact ggataaagaa aatgtggcac 62880
   atatacacca aggaatacta tacagccata aaaaaggatg agttcatgtc ctttgcaggg 62940
   acatggatga aactggaaac catcattctc agcaaagtaa ctcaagaaga gaaaaccaaa 63000
   caccacatgt tttcactcat aagtgggagc tgaacaatga gaacacatgg acacagggag 63060
   gggaacatca cacaccaggg cctgtcaggg gttagggggc tgggggaggg atagcattag 63120
   gagaaatacc taatgtaaat gatgagctga tgggtgcaac aaaccaatat tgcacatgta 63180
   tacctatgta acaaacctgc actttgtgca catgtacccc agaacttaaa gtataataaa 63240
   aaagataaaa ttcagcctta aaaaaaagaa aaagaaaaag aaaagggtgt cctttcccca 63300
   ttttatgttt ttgtttgctt tgtccaagat cagttgactg taagtacctt ggtttgtttc 63360
   tggattctct attctgttcc attggtctat gtgcatattt ttataccagt accatgctgt 63420
   cttggtgact atggccttgt agtttgaaat caggtaatgt gatgcctcca ggtttggttg 63480
   ggtttttttg tttttgttgt tgttgttgtt tggttggttg ggttttttgt tgttttgttt 63540
   atttgtttgt ttgttttggc ttagtccttc tttggctatg tgggctcttt cctggttcca 63600
   tgtgaatttt atcattgttt tttctaattc tgtgaagaat gatggtggta ttttgatgga 63660
   aattgcattg aatttgtaga ttgcttttgg aagtatggtc atttttacaa tattgattct 63720
   acctatccgt gagcattgga tatgtttcca tttgtttgtg tcatctatga tttctttcag 63780
   cagtgttttg tagttttcca tgtagaggtc tttcacttcc ttggttaggt atattcctaa 63840
   gttttgtgga tttttttttt gtttggtttt tttttgtttc gttttgtttt gtttttttgt 63900
   ttttgtagct attgtaaagg ggattgagtt cttgatttga ttctcagctt ggtcgctgtt 63960
   ggtgtataga agagatactg atttgtgtat gtgaattttg tatccagaaa gtttgctgaa 64020
   ttcttttgca agttctagga gctttctgga ggagtcttta gggttttcta ggtaaacatt 64080
   catatcatca gcaaacagca acagtttgac ttcctcttta atgagttgga tgccctttat 64140
   ttcttcctct tgtctgattg ctctggcaag gacttccagt attatgatga agaggagtga 64200
   taagagtggg catccttatc ttgttccagt tctcagaggg aatgctttca acttttcccc 64260
   attcagtatt atgttggctg tgggtttgtc ataggtgact tttattactt ttattacatt 64320
   gttatattat gacattttat tgtcatagat gtcctttgtg taccagtttt gttgagagtt 64380
   ttaacgataa aggaatgcta gattttgttg aatgcttttt ctgcgtctat tgagatgatc 64440
   atgtgatttt ggtttttaat tctgtttatg tggtgtatca catttattga cttgcatatg 64500
   ttaaactatc cctgcatccc tggtatgaaa cccatttgat catggtgtat tatctttttg 64560
   atatgctgtt agattcggtt agctagtatt ttgttaacaa ttttagcatc tatgttcatc 64620
   agggatattg gtctgtagtt ttcttttttg attatgtcct ttcctggttt tggtattagg 64680
   gtgatactgg cttcatagaa tgaattgggg agggttcctt ctttatcttg tggaatagtg 64740
   tcaataggac tggtatcaat tcttctttga atgtctagta gaattctgct gtgaatccat 64800
   ctggtcctgg actttttttg ttggtaattt tttaactacc atttcaatct cattgcttgt 64860
   tattggtcat tcaggctatc taattcttcc tgatttaaag taggagggtt gtatttttcc 64920
   agaaatttat ccatctcttc tagattttcc agtttatgtg cataatggtg ttcatagtag 64980
   ccttgaatga tcttttgtat ttctgtggtg tcagttgtaa tatctcccat ttcatttctt 65040
   cttgagctaa tttgcatttt ctctcttctt tttatggtta atcttgctaa cggtctatca 65100
   attttattca tttttaaaga accagctttt tgtttcattt atcttttgta ttttgtttgt 65160
   ttgtttcaaa ttcatttggt tctgctctga tcgtggttat ttcctttctt ctgctgggtc 65220
   tgggtttggt ttgttcttgc ttctctagtt ccttgaggtg tgaccttaga ttgtctgttt 65280
   gtgctctttc agactttttg atgtaggcat ttatggctat aaactttctt cttagcatcg 65340
   cctttgctgt gtcccagaga tcttgatatg ttgtgtcatt gttgccattc aattcaaaga 65400
   attttttaat ttccatcttg atttcatttt tggcccaatg atcattcaag agcaggttat 65460
   ttaatttcca tatatttgca tggctttgaa agttcatttt ggagttgatt ttcagtttta 65520
   ttccactgtg gtctgagaga gtgcttgata taatttcaat tttcttaaat ttattgaggc 65580
   tcattttgtg gcctgtcgta tggtctacct tggagaaagt tccatgcact gaatagaatg 65640
   tatattctat ggttgttgga tggaatgttc tgtatatatc tgttaagtct atttgttcca 65700
   aggtatagtt taaatccatt gtttcattgt tgaccttcag tcttgattat ctgtccagtg 65760
   ctgtcagtgg agtattgaag tctcccacta ttattatgtt gccacctgtc tcatttctaa 65820
   ggtctgttag taattgtttt ataaatttga tagttccagt gttcagtgca tatacgttta 65880
   ggattgtgat gttttcctgt tggactaggc cttttatcat tatataatgc cctctttgtc 65940
   tttttaaaat gctgttgctt taaagtttgt ttgtctaaga ataggtactc ccgtttgctt 66000
   ttggtgtcca tattccaaaa atgtcttttt ccacaccttt accttaagtt tgtgagactc 66060
   cttatgtgtt aggcgagtct cttgaagtca gcaggtagtt ggttggtgaa ttcttatcaa 66120
   ttctgcaatt ctatatcttt taagtggagc atttaagcca tttacattca acgttagtat 66180
   tgagatgtga ggtaccattc cattcattat gctatttgtt gcctgtatac cttggttttt 66240
   tgttttttgt ttttgttttt taaattgtat ttttgtttta taggtcctgt gagatttatg 66300
   ctttaaagag gttctgtttt gatgtgtttc caggatttgt ttcaagattt agagctcctt 66360
   ttagcagttc ttgtagtggt ggcttggtag tggtgaattc tctcagaatt tgtctgtctg 66420
   aaaaagactg tatctttcct tcatatatga agcatagttt tgttggatac aaaattcttg 66480
   gctggtaatt cttttgcttg aggaggctga agacaaggcc ccaatacctt ctagcttata 66540
   gggtttctgc tgagaaatct gctgttaatc taataggttt ttatttatag gttaccaggt 66600
   acttttgtct cactgctcct aaaattcttt cctttgtctt aactttagat tacctgatga 66660
   caatgtgcct aggtgatgat ctttttgcaa tgaatttccc aggtgttctt tgtgcttctt 66720
   gtatttggat gtctaggtct ctagcaatac tggggaagtt ttcctctgtt attccctgaa 66780
   atatgttttc caaactttta gatttctctt cttcctcaag aacactgatt attattagct 66840
   ttggtcatct aacataatcc cagacttctt ggaggctgtg ttcgtatttt cttattcttt 66900
   tttctttgtc tttatggagt gggttaactg aaagaccttg tcttagagct ctgaacttct 66960
   ttcttctact tgttcaattc tattgctgag actttccaga gcattttgca tttctataag 67020
   ggtgtccaaa gtttactgaa gttttgactg ttttttattt atgctatcta tttccttgaa 67080
   tatttctccc ttcacttctt gtatcatttt ttggatttcc ttaaatcggg cttcacatat 67140
   ctctggtgct ccctgattag cttaataatt aacctcctca attctttttc aggcaaatca 67200
   gggatttctt cttggtttgg gtccattgct ggggagctag tgtgattttt tgggagttgt 67260
   aaagaacttt gttttgtcat attaacagaa tcggtttctg gttccttctt atttgggtag 67320
   gctctgtcag agggaaggtc tagggctgaa ggctgttgtt cagattcttt tgtaccacag 67380
   ggtgttccct tggtgtagta ctctccccct gttcctatgg atgtgacttc ctgagagctg 67440
   agctgtagtg attgttatct atcttgtgga tctagccacc caacaagtct tccagactcc 67500
   gggctggcac tggggattgt ctacagagtc ctgtgatgtg aaccatctgt gggtttctca 67560
   gccgtggatg ctagcacagt atttagggtg tctcctaggt ccttcaggag caatctgctt 67620
   cctgcagggg gtctgtgggt cctctctatt ttcgttttaa aggcagactt tgtaatagga 67680
   cacactagtg ggcaatttat agttgtatac tcataagcaa actgtttaaa ttatctgaat 67740
   ccaagtttcc tcatatgatc acaagatgag gtcccacaac aggtcgtctg caagctgagg 67800
   agcaaggaag ccactctgag tcccaaaacc ccaaaagtag ggaagccgac agtgcagctt 67860
   tcagtctgtg gtcaaagatc caagagtccc aaaattgaag aacttagagt ccagtgttca 67920
   aggacaggag gcatcccgga tgggagaaag atgtaggctg ggagactaag ccagtcttgg 67980
   gaagtggctt aaattaggtg atctgggatg gttttctgtg gagctgacat ttcagcaggt 68040
   acctgaatgc aaacctggaa gtaacagaaa ttcagaaaga gaaaagcaag ggcaaaggtc 68100
   cttaggcaaa aaaaaaaaaa aaaaaagagc ttggtgtgct gatgaatagg aaggcaggag 68160
   gggcagaagc agaatgagag agagcatggt agaagaccat atctgagaag ttagctgagg 68220
   tcaatcatga agaaccctgc aggccatgga ggagctggga ctttgttcta agagatgtgg 68280
   ggaacgcctg gagcagtctg atcataggag aggcatgata ttcaaagaca ttccctggcc 68340
   tagctttagc atatctagtt aaccttctct ttactaatcc tttacttttc tttcattgta 68400
   aattgagaaa ttatagttgt atatatttct ggggtagaaa gtgatgctat gatttacaga 68460
   cacaatgtgg aatcattaca tcgtgctaat taacatatcc accacttcaa atacatatca 68520
   atttttgtgg tgtggctggg tgcagtgact cactcctata atcccagcat tttgggaggc 68580
   caaggcaggg ggatcatttg aagtcaaaag ttcaaaacca gcctggccaa catggtgaaa 68640
   ccctgtctct actagaaata caaaaattag ctgggtgtgg tggcggatgc ctgtaatccc 68700
   agctactcaa gaggctgaaa caggagaatc acttgaacct gcgaggcagt ttgcggtaag 68760
   ctgagaccgt gccactgcac tccagtctgg gtgacagagt gactcagtct cagaaaaaaa 68820
   aaatgtggta agagaatttg aaatttagtc ttagtaattt tgaaatgcac aatacacatt 68880
   actaactgta ttcaccacac tgtgcaatag atctcatata tatatatata tatatatata 68940
   tatatatata tacacacaca catacacata ttgttcctgt ttaactgagg ctttgtgtcc 69000
   tttgaccatc atctccccat tcccccaacc cccagcctct ggtaaccagc atactactct 69060
   cttctaccat gggtttcatt gtttcagatt ccccatgtag tgaaaacatg cagtatttac 69120
   ctttctgtgc ctggcttatt gcagatcatc tcattagatg cagaaacatc atttgatcaa 69180
   attttatttc ataataaaaa tcctcaacaa attaggtaca gaaggaatgt acctcaacac 69240
   aataaaaagc atatattata agccaataac taacattata ctccacagtg aaaaattgaa 69300
   atcctttcct ctaagatcca gaacaaaata aggatgccca cttgagcaag atctagtgct 69360
   gtgctggctt caggtctaac ccagtgcagt ctcagtgatg gtggccacaa ggctgcttgt 69420
   gtcgcccctc accaagctcc aggcacctca gcacagagaa agagagactc gtttgtttgg 69480
   gagaatgtaa ggaagagaac aagagttttt gcccagtaat ccagagaatt ctttcagatc 69540
   ttattcaaga ccaccaaggt gatacctcta caagtctaca agaaccatag cattactggg 69600
   cttggggtat cccttaatgc agaaatgcct gcagtgacca aaaacttaga tcataatact 69660
   gaagtccctt caaataccag gaaagccttc ccaaggagga tggctacaaa caagcccaga 69720
   ttacaaagac tacaataaat acctaactct tcaatgccca gacactgaca aacaccacaa 69780
   cctcaagatc atccaggaaa acatgagaca ctcaacataa cacagagaat gaattcagaa 69840
   ttttatcaga taaatttaac aaataaattg aaataattaa aaagaatcaa gcagaaaatc 69900
   tggaggtcaa aaatgcaact gacatactga agaatcatca gagtccctta atagcagaat 69960
   tgatcaagca gaagaaagaa ctagtgagca tgaagacagg ctatttaaaa atacatggtc 70020
   agcagagaca aaagataaaa gaataaaaaa gaaggaaatg cccaaaatat ctataaaata 70080
   gcttcaaagg ggcaaatcta agttattggc cttaaaaagt agggagagag agagaaagtt 70140
   tattcaaagg gataataaca gaacttccca aacctagaga aagatatcaa tattcaagta 70200
   aaagaaggct atagaacacc aaacagattt aacctcaaag cattcactaa taaacttgca 70260
   aaattcaagg ataaagaaaa gattttagaa gcagcaaaag aagagaaata aataacgtga 70320
   aatggagctc caatacatct ggcagcagac ttttcagtgg aaaccttaca ggccaggaaa 70380
   gagtggcatg gcatatttaa agtgctgaag ggaaaaaata ttttatcctt gaatagtata 70440
   tctggcaaaa atagccttca tgaaggagaa ataaagactt tcccagacaa acaaaaactg 70500
   aggcatttca tcaacaccag acctgtccta caagaaatgc taaagtgatt tctttagttt 70560
   gaaagaaaaa aggcattaag gaaaaactaa aaaccatcta agggtagaaa actcactggt 70620
   aatagtaagt acacagaaaa acagaatatt atagcactat aattgtgcta attatttata 70680
   ttttaagtag aaagacaaaa agatgaactt atcaaaaata actacaacaa cttttcaaga 70740
   tatacacagc ataataagat aaagtagaaa caacaaaaag tttaaaagtg ggatgacaaa 70800
   gttaaaatgt agcattttca ttaattttct ttttgtttat ttgtttatgc aatcggagtt 70860
   aaattgtgat cagtttaaaa taatgggtta taaggtatta tttgcaagcc tcatagtaac 70920
   ctaaaaatca aaaaagcata caatggatac acacaaaaaa ataaaaagca agaaactaaa 70980
   tcataccacc agagaaaatc actttcaatg aaaggaagac aagaaggaaa gaaaaaagca 71040
   agagaagacc acaaaacaac tagaaaacaa ataacaaaat ggcaggagta agtccttact 71100
   tatccataat aacactgaat ataaatggat taaactctcc aatcaaaaga cacaggattg 71160
   aatggattca atggattaaa attaaatttt aaaaagcaag acccaatgat ctgttgacta 71220
   caagaaacat acttcatcta taaagactca catagattga aaataaaggg ataaaaaaag 71280
   atatcccatg caaatggaaa ccgaaaaaga gcaggagtag ctacacttag atcagacaga 71340
   ataggtttca agacaaaagc tatcaaaaga gacaaggaag gtcattatat aatgataaag 71400
   gaatcaattc agcaagagga tataacaact ataaatatat atgcacccaa cactggagca 71460
   cccagatata taaagcaaat attattagag caaaagagag agacataccc caagtcaata 71520
   atagctggaa acgtcaacac ccaactttca gcattggaca gatcatccag acagaaaatc 71580
   aacaaagaaa catcaaactt aatctgcaca atagaccaaa tggacctaat aaatatttat 71640
   agaacatttc atccaacagc tgcagaatac tccacacact tctcctaaac acatggatta 71700
   ttctcaagga tagatcagga taggccacat gttaggccac aaaacaagtc ttaagacatt 71760
   cagaaaattg aaataatatc aagtatcttc tctgaccaca attgaatgaa actaaaaatc 71820
   agtaacaaga gaaaatttgg aaactataca aacacatgta aattaaacaa tatgctcctc 71880
   aatgaccaat gggtcaatga agagattaag aagcaaattg aaaaatgtct tgaaacaaat 71940
   gataatggaa acacaaccta taacaaaacc aatgggatgc agcaaaagga gtactaaaag 72000
   ggaagtttat aactaaaagt gcttacatca gtaaagaaga aaaacttcaa ataaacaacc 72060
   tattatgcaa cctacaagag caagagcaaa ccaaacccaa agttggtagg agaaaggaaa 72120
   taaagatcag agcagaaata aatgaaattg aaacaaagaa aatacaaaag attaacaagg 72180
   ccaggcactg tggctcacgc ctgtaatccc agcactttgg gaggctgagg caggcagatc 72240
   atgaggtcag gagaccgata ccatcctggc taacatgatg aaaccctgtc tctactaaaa 72300
   aatacaaaaa aaaaatagct gggcgtggtg gcaggcgcct gtagtcccag ctactcggga 72360
   agctgaggca ggagaatggc gtgaacccgg gaggcggagc ttgcagtgag cccagatcac 72420
   accactgcac tccagcctgg gcaacagagt gagactctgt ctcaaaaaag aaaaaaaaaa 72480
   aagataaaca aaagaaaaag ttgttttttg aaaagataaa caaaaatgac aaacttagcc 72540
   agactaagaa aaaaggagag aagacccaaa taaataaaat cagagatgaa aaaggagaca 72600
   ttaaaactga tactacagaa attcaaagga tcattagagg ctactgtgag aaactatata 72660
   ccaataaatt ggaaaaccta gaagaaatgg ataaattcct atatacatac aacctaccaa 72720
   cattgaacca tgaagaaatc caaaacctga acagaccaat aacaagtaat gagattgaag 72780
   ccataaaaaa aattatccca gcaaagaaaa acctgaaacc tgatggcttt actgttgaat 72840
   tctaccaaac aatgaaagaa gaactaatac caatcctgct caaactattc caaaaagtag 72900
   aggagaaaca aatacttcta aactcattct atgaggccag tgttatccta ataccaaaac 72960
   cagacaaaaa atgcatttaa aaaagaaaac tacaggccac catccctaat gaacattgat 73020
   ggaaaaatcc ttgacaaaat actagcaaac cgaattcaac aacacattaa aaagatcatc 73080
   catcatgacc aagtgagatt tatctcaggg atgcaaggat ggttcctcct atatttagaa 73140
   cacaaaaaag atgctcattt tcaccactgt tattcaacat agtactgaaa gtcctagcta 73200
   cagcaatcag agaagagaga aataaagagc atccaaattg ggaaggaaga agtcaagtta 73260
   tgcttgtttg cagatgatat gatcttatat ttggaaaaac ctagagtccc catcaaaaaa 73320
   ctattagaac tgataaacaa attcagtcaa gttgcagaat acaaaataaa atacaaaaat 73380
   cagcagcatt tctatatgcc aactgcaaac aatctgaaaa agaaattgag aaaataatcc 73440
   catttacaat agctacaaat aaaattaaat acctaggaat taaccagaga agtgaaagat 73500
   ctctacaaag aaaactatga aacactgatg aaagatatta aagaggatac aaaaaaagga 73560
   aagatatttc atgttcatgg attagaataa tcattattgt taaaatttcc atatttctca 73620
   aagcaatcta caaattcaga gcaattccta acaaaaatac caatgacatt cttcacataa 73680
   atggaaaaaa cagtgctaaa atttatatgg aaccagaaaa tacccagaag agccaaagct 73740
   atcctaagca aaaagagaaa aactggagaa atcacattac ctgacttcaa attatactac 73800
   agagatatag taaccaaaac aaaatggtac tggcataaaa acatatactg accaatggga 73860
   cagaagagag aaaccagaaa caaatccgta catccacagt gaactcattt tcgacaaaag 73920
   taccaagaac atgcaacgag gaaaggacag tctcttcaat aaaaggtgct ggagaaactg 73980
   gatatccata tgaagaagaa tgaaattaga cccctatccc ttgccatatg gaaaaaaatc 74040
   aaatcaaaat gggattaaaa agttaaatct aagacctcaa tctatgaaac tactacaaga 74100
   aaacatgagg gaaattctcc agggcaagga tttcttgagt aatatcccac aagcacagtc 74160
   aaccaaggca aaaatgaaca aatgggatca catcacgtga aaaagcttct gcacagcaaa 74220
   ggaaacaatc aacaaaatga agagacaacc cacagaatgg gagaaaatat ttgcaaatta 74280
   tccatctgac aagagattaa taaccagaat atataaggag ctctcatgac tgtataagaa 74340
   aaaataaaat aatccaattt taaaacgggc aaaaaatcta aatagatatt tctcaaaaga 74400
   tgacattaca aaccacaaac aggcatatga aaaggtgctc aatataatta atcatcagag 74460
   atatgcaaat caaactgcaa tgagatatca tctcactcca gtaaatgaca ggcactaaca 74520
   aatgctggca aggatttgga gacaggcaat aacaaatgct gacaaggatt tggagaaaag 74580
   gacaggcaat aacaaattct ggcaaggatt tggagaaaaa ggaaccattg tacactgttg 74640
   gtgggaatgt aaattagtat aatttagcca ctatggagaa gagcttggag gtttctcata 74700
   aaactaaaaa tagagctacc atgtgatcca gcaatcccac tgctaggtat attcccaaaa 74760
   gaaaggaaat cagtatatca aagagatatc tgcactctca tgtttattgc agcactatac 74820
   acaatggcca agatttggaa gtaacccaag catccatcaa cagacagata aagaaaatgt 74880
   ggcatatata cacaatggag tactattcag ccattaaaaa gaatgagaac ctgtcatttg 74940
   cagcaacatg gatgcaagcg gagctcattt tgttgagtaa aataagccag gcacagaaag 75000
   acaaacttca catggtctca tttatttgtg gaaactaaga attaacagtt aaggattaag 75060
   aaaatgtggc acatatacac catggaatac tatgcagcca caaaacagga tgagttcatg 75120
   tcctttgtag ggacatggat gaagctggaa accatcattc tcagcaaact atcgcaagga 75180
   cagaaaatca aacaccacat gttctcactc ataggtggga attgaacaat gagaacactt 75240
   ggacacagga tggggaacat cacatactgg ggcctgtcat ggggtggggg agaggggagg 75300
   gatagcatta ggagaaatac ctaatgtaga tgatgagtta atgggtgcag cacaccaaca 75360
   tggcacatgt atacatatat aacaaacctg cacattgtgc acatgtaccc tagaacttaa 75420
   agtataataa ttaaaaaaaa ttaacagtta aactcacaaa gatagagagt agaatgacgg 75480
   gtaccagagt atgagaaggg tagtggagag agtgagcaga gagggaagcg gagatggtta 75540
   atggacacca aaaaaaacca gaaagagtga ataacgtcta gtatttgaaa gtacaaaagt 75600
   gtgactatag tcagtaataa tttaattgta cattttaaaa taattaagag tataattgga 75660
   ttatttgtaa cacaaaggat aaatgcttga ggtgatggat acctctttta ccctgatatc 75720
   atcattacat atcgcatgca agtatcaaaa tatcccacat aactcataaa tatatataac 75780
   tactgtgtac ccacaaaaat taaaaattat taataaatta ttagtttgat ttaaaaaaaa 75840
   caaggatgct cacttttgcc acttctattc aatatagtag tggaagtcca tgccagagca 75900
   gttagacaag agaaagaaat aaaaggcatc caaataagaa aggaagaaag gaaatttttg 75960
   ctgtttgctg acaacataat cttatatata gaaaacccta aagattccag aaaaaaactg 76020
   ctagaactaa taaattcagt acagttacag gagacacaat caacaccaca aaaatcacta 76080
   gcatttctat acactaacaa caaactttcc aaaaaagaaa tcaagagagc aatcccattt 76140
   acagtagcta caaaaattaa aatacttagg aataaattta accaaggagc tgaaagacct 76200
   gtacacagta gtctataaaa tattaaaaaa ataaattgaa gaagtcacta atccttttta 76260
   aatccaccat gccccagtcc agctgaacaa ctcatacttt tcagaatcta ctctatgtgt 76320
   tctcactgtt ctgcagctag ccacctcttt ctcctcccta gatcaccttc tttatcattt 76380
   ctgcttctca aaatgttacc cctctttcaa agcccaggta tgatgacatc tccctagtaa 76440
   agctccctca acacctcaag cttataataa ctaatctctt ccttgaattc ccatagcagg 76500
   ttatttgtac ctccattata atacctctct catcctgttt tgtattttaa ttattcatgt 76560
   gtggcatgtt tctcttatta ggccataaat atcttaggac aagaactatg ccctcatttt 76620
   tgttccacta caaatcttaa cacatacctt gcacacacca gaccttcagg aaatgcctgc 76680
   taaggtaacg aagggacagc ctaaattaac ataaattatt atttgtgtac tgatccagtc 76740
   ctaaatccat ggtgctatca tttctgtatc actgatggtt atccacttac ttaaataatc 76800
   tttccttaaa tacacttaga aaagacggaa aactgtgtta taagggagga acaagtatgt 76860
   aggttattcc tttccttaaa caaagagaag ttctgtcatg cgtcacggaa aattaaagca 76920
   cagtaataaa aagcacgaac aagatttgtt gaataatgat ttagtggaaa cgttgcttgc 76980
   ctcctgttca taggtcctaa ctttcctctc aagtacttaa gctgcacatt ctatttattt 77040
   tgtttctaga ttacattgac aactattggc tatggagaca aaactcccct aacttggctg 77100
   ggaagattgc tttctgcagg ctttgcactc cttggcattt ctttctttgc acttcctgcc 77160
   gtgagtatct ttgcaccaat aaagcagttt aaattagatc ttagagactt attagagtga 77220
   gctctcacaa attcgtatgc ttgggaacat atctatatca tttacaaata taatttttcc 77280
   gatataatgt tgacttttct aaatatatct acttggataa tttataaaat atggtcttcc 77340
   cttggagaca cttaatctga gactttattt aatgtaggct catactttgt gaccaaataa 77400
   tcattcagat ggctcacttt ttaggcaaca tgctactaag acatttaaaa tatttgcaaa 77460
   gatttaaagc tatattcttt taaaactgat ccttccaatt tggtttataa tgtgtgctac 77520
   ttaagagaca gatgtgcaca atggagtatt tttctcttca gatccatttg agtgaaaagc 77580
   atatgacctt aaagggtgtt aaacatgtca gatgatttat tcattgtgtc atcatcattg 77640
   atttttttaa tgagcagttt ctgtatctta tcagaataaa gggcacattt atctgccccc 77700
   aagcttctga ttattttctc cttagtgaat gaagcctgcc tttctcatgc ttttattctc 77760
   ttgtgtcttt aaaggcagct gttcaaaata ttacagtttg cccccatttc caagttcttg 77820
   cttttgttcg ctccacctgg ggtagggaag gaggagccaa taaaaaggag ggttaacgcg 77880
   gtgggagcag aagtgcttaa ggaaaataga tccagtcttg ctccagacac agctcaagct 77940
   gtgccaacca gagaccccag agcacctctc caaagctctt ttaggtggta gggcttctcc 78000
   tctgcctatt aataagtgca gagaacaccc ttgtcccacc agtgcccaac ttgagattca 78060
   ccagatcgtt ctgccccaga acactcaaaa tcacagaaaa gagactgaaa aggccatgca 78120
   tatgcttggg tactagacct cagtttgaat acatctttgt cacatactaa ctatatgact 78180
   gctttagttt actagggctg ccttaacaag taccacagac tgggtgactt aaacaataga 78240
   aaggtattgt ctcacagttc ccaagactgg acgtccaaaa tcaaagcatt gagaaaccac 78300
   gagagccatg agagaaagaa ctgttccagg cctctcttct tggcttatag atggctcttc 78360
   ttcctgtgtc ttcacatcat ctgtgtccaa atttctactt cctagaagaa caccagtcat 78420
   atttgattag ggtccatcct aatgacctcg ttttaacttg attacctctg taaggaccct 78480
   attttcaaac aaggtcacat tctgaggtca tggggattag gacttcaaca tatgaatttt 78540
   ggaaggacgc aattcagcct gaacagtgac cctgaataag tttctttttt aatagaatgg 78600
   aggaagaatt attgagccca gagaaatttt gtgcggacca atgtaataat gtaagtcaag 78660
   tgcttgtcat gaagagaact ctcaataaat gttacttccc tttgcctgta tgaatctaga 78720
   aatacatgtt tcctggaaca aaacgatgaa atccaggaga attaaaaaaa aaaatcttac 78780
   gtatccaagg caacttcatc tggcaatctc aacaattcag aacagagaag agattcactc 78840
   aataatcaga ttccctacag gaaaatacct ttagaaacaa tacaacgcag tcttgggttt 78900
   tttcccaaaa taatggcaca aaataattgc aacttttgtg tttatattta tcctgaggcc 78960
   acatgtagag caacctctat gattcagtga aaaactatga acaaggaggt aggcaacctc 79020
   tgggctgttg aaatagacaa caggctatgt tctgtcctca taggagagcc acttgagacc 79080
   taattcataa cctagtcttc attcagagaa aattctgata aactcaccta tatggtttca 79140
   caattctttg aagattataa acaataaata tagaggaaga tgatagaaca aacactgcaa 79200
   cagaaagaaa tggtaaaaga ctaacagcaa agaagggaaa aggaaatctg tgagtagcaa 79260
   gcagacatca atgttcaaaa tgtgtatcct gtcagtaaaa ataaaattaa attaaaaggg 79320
   aaaacaaaat gtatgaagat ctagggccaa ttagtgttag ggtcaacaaa cctctggaag 79380
   ttggctgcac ccagcacagc aaaaaggcag tccccataat gaaactctaa actatcacag 79440
   ggaggttaaa tatgtcacta ggttaagtta aaggtcagag actaagtagt cattcttgga 79500
   aatgtttcca ataaagtaag tatgaaattt ttatatgcaa attaaattaa cctatgttgg 79560
   aaatctgaaa attttgcttt cacagaaaat tacatttctt aagaacccca agaaaagtaa 79620
   cctctactaa ggatctttta ctgaaacttc acaatatttt ctgagtcctt atttattaaa 79680
   tgaaagactg aatggtccat atataccttt agaaaaaaac aagattgcca gagattttat 79740
   tatatcctgt gtatctgcca accaaaagag ttgtctgatt gaaatttagg aagacacatt 79800
   gacattttgc tgagaaagct agaggtggat tgtgggctgc ccctatgtag ggttcctccc 79860
   acccaggaca cctgagaacc aagaaagaaa agagcctttc tccatcttac cctcaaagca 79920
   gaagggccat cctgcacatc acagagtcat acaagccacc catccaaagc gccactgtgg 79980
   ggtcacttcc cctggtgccc gctccatctc tgggctaatg agctcacaga agaagcccca 80040
   gctcccctgt gttaccctga tatgcaggtc tttctccttt ttagagtgct cccttgtctt 80100
   ctgctagata ttcctgctaa gagtattttc attgatgaag aaattgagtt ttatagagac 80160
   tgaattcctt gagacctcca gctagtaaat tcagtgcaag aactagtgct gtttgactca 80220
   agatattaac cattccatta aaaatattgg ccactgtaat ccttaacaat attaactaaa 80280
   tgtaattatt gcatataata acatgttaaa aaccgtttaa aaaaacaggg catcttgctg 80340
   ggcgtggtga ttcacgcctg taatcccagc actttgggaa gccgaggcgg tcggatcaca 80400
   aggtgaggag atcgagacca tcctgcctaa catggtgaaa ccatgtatct actaaaaaat 80460
   acaaaaaatc agccaggcat ggtggcgggc acctgtaatc ccagctactc gggaggctga 80520
   ggcaggagaa tgatgtaaac ccaggaggcg gaacttgcag tgagccgaga tcgctccact 80580
   gcactccagc ctgggcaaca gagcaagact ccgtctcaaa aaaaataaaa aataaaaaaa 80640
   ataggatgtc tttcagcatt gcctatttgc tatgtgtggg ttacaaatta ggttcattta 80700
   atccagaaga taatgaaggg taaatttctt tacatttatt ggtgtcagta tttgaaggga 80760
   tgacatatat gttcaaagct taagccatgt tttatgtgtg tatatacaaa tataatagac 80820
   cttattttca cagacttctg tttaggtgca ccaagctgtg tactaagaga gaggtggaaa 80880
   aaaattggaa aggagccatg atgagacaat aagcagacct ggtttaataa ctgctgtaat 80940
   gtacagtctg gcgcaagaaa tacgtagtga acatttacca ttcactaata tatgttaccg 81000
   agcaatgcca atgtgatttt cagcacattt tagattgttt ggcagatgat aaatttgtgc 81060
   tatgtagggc ctgtctttat tttccaaagg ggattaaact ccgaacctca catccacatc 81120
   ataagtgctc aaaatggtag ctagtgctgt taattcaatg tctgcaaaat ctaaattatg 81180
   ctccatacaa gtggattata cacatatatg cacataaatg catgtatata tactacatat 81240
   tataaatata tgccagaaat tttggtattt catattctct tacatgtcta cactttatga 81300
   aaagtagtat taaagggaaa ataaagaaat ctttcctaaa tgatcttaca cagcagaaaa 81360
   aaattagaag ttacaaatga cttaagttgt tctggattta gaaatggagg ggcattttta 81420
   taacacctaa atggagggaa atgttgttta aatctataaa ccagatcaca gagaccaggt 81480
   ttttataaag cttagtttgt acaatcacaa cctgtatgag atagagatct tagacagcgt 81540
   aagacagtca ctgaaaagga gagatggaaa acagaacctc tgatgtaatt tctaatttca 81600
   tagtacagtc atttatacta attaataatg gagtagatca ttgctcacga gtagcaaata 81660
   gtattatcag accactgcat attttaaaca tgattgcatt acttattgtc tggttattgt 81720
   cactgatgta ctcagaagtg tggaatctca agataagaag gaaggttaaa ggttatctag 81780
   agcccaaaaa tatctcagcc aagttgttat tcagttgaga cttaacacct tctagaaatg 81840
   aaaaacccac cacctctcca gacagcctat agcatctttg aaccatcttt gagctactct 81900
   aaaaataaaa agaaaagaaa agcaaccatt ccttacctgt agcaaatact gacttagtgc 81960
   ttataaaatg tccatctcta acgaaagggt tagactcctt gaagtttaat aaaaaactaa 82020
   taatttatag ctagcaagta aaatccacag acttgaaagc agcagagctc ccagccccat 82080
   gctaataaac tcaagagtta aagacaaagc ccagagactg aatttagcaa gctccaaaag 82140
   ccaacccaag gctgagacaa agcatcttgt ttttacaaat gttagtagac cagagcaaat 82200
   tgaaaagagt gggggacatc aaagaaccag aattttggca gatcaagatg ataagcagaa 82260
   gaaaagcaaa ctggcccaaa actaatgtaa acctagaagg gaatactaat cttactaatc 82320
   ccaaaaactg aataggtgag ccaaaataaa aaaggcaagg aatggtaaaa gtgtgaatta 82380
   aaatgggaat gtgctttgca atgatccacg ttcttgtctc tagtgtacaa atgggcaaaa 82440
   tagaaaacct tctactctgt atcttcaaaa ctgagtatcc ctgctctcca ttttactttt 82500
   cttttagtag gtgtacagtt ataacattat aaagaactac agctgggaca gattttatac 82560
   agtcctctct cctccatttc atgaatagag aatctaactt tcagagtctc atcaaagtgc 82620
   tagaagtatt cccatgttca cagcagtgag actttaactc cctcagattt aaaatccagc 82680
   tctgtcccaa gttgaaattc atgttctcta cattccatca ctggatgaga ccattcaatc 82740
   taacttaggc aaaaagaaat gtcacagaga aggccacaca cacaaaaaaa aattttattg 82800
   tgattttatt tataatcatg gtgcctatgc tataccacaa aagataataa atgacaatgc 82860
   ctaattgact tactaaagaa cagaagttgt atctcctggc agtattgccc aacaatttgc 82920
   cattggctat tggtattata atggagataa atactgctta ttttaataag taacatgttt 82980
   atggacaggt tacataccgt ttgtcttctc tcagactcat tttagtgact ttttaatatt 83040
   tgtattattt aacagtgctt ttgaaatttt tgagtgccat ttttgtattt tgttccaggg 83100
   cattcttggc tcaggttttg cattaaaagt acaagaacaa caccgccaga aacactttga 83160
   gaaaagaagg aacccagctg ccaacctcat tcaggtaaat gtcaatgtaa taggtagatg 83220
   gcaacatttg tgtccatagt gcttgatggg tcattttcca atctctgtgc ttcattgctt 83280
   ggtagaacta ctgctttgtt ccttttcata aaatgaagtc agaacttaat gtacagtctt 83340
   ggatttggac agtcttggat ttgttcctcc tctcccaaac tgaaacacat gtatttttag 83400
   caaatggtcc atgcattgtt cagactaagt aaccatgtca gcaaggccat gcaaaggtta 83460
   gcagttgtgt ggctcgtgga attatctaat atgtcttatg aattacttga tgaaacgtaa 83520
   ttgatcatta agtgatggag cagaacaggg cattgttccc tgcattccaa ttactttctc 83580
   cagccagatg tatgttgctg ttttaaagaa caacatattt gtttagaatg tcaaggttta 83640
   atacctgtga aattgctttt agtgccatct agggcagtta gcggaaatag ctatcctatt 83700
   ttgagagctg atgggactat ggaatacatc agtgtttgag aatattaaac aataagtata 83760
   taatttgaag ctagctccta atacataggt tgttatttag tatttcattt tcttttcact 83820
   aactcaggat tagcttcagc actaaaagga atgcttctcc ttttgccatc catatatttt 83880
   ccatcctaga tccaggttaa gaatcacaaa atatattttt tgttagattc ataatgtaga 83940
   gttgacattt agttatggac aaaataattc tacaatctaa agtttctccc ctcctgctag 84000
   ggaagtggaa gctaccgaat ttctaaactg gagggtcttg aagtggtgct ccaaggccct 84060
   aggaaacttg ccttaaagct gcagtaacgg gcacactgtt ttgttgctgt tgttgttttg 84120
   ttttattttt tgtttgtttt gttttttttt ttttgagaca gagtcgcgct ttgtcgccca 84180
   ggctggagtg cagtggcgcg atctcggctc actgcaagct ccgcctcccg ggttcacgcc 84240
   attctcctgc ctcagcctcc cgagtagctg ggactacagg cacccgccac cgcgcccggc 84300
   taattttttg tatttttagt agagacaggg tttcaccgtg ttagccagga tggtctccat 84360
   ctctcaacct cgtgatccgc ccacctcggt ctcccaaagt gctgggatta caggcatgag 84420
   ccacggctcc cggccacaag cacactgttt taatttagat tgtaggtttg ggtgggtggg 84480
   ttggtgggag ataaccacca caacccctac ctttagtgtt gatctgtgac tgtgatatat 84540
   gcagtgcctc ccacagacat ggagctgggg aaagaagggg gcagtatggt tcagttatat 84600
   catgaaggag atctgtttat atctaatcat taaatgagga tattaatttt taaatcaagc 84660
   tattctgcaa ctgttgacaa gaaagtagac catcaggcat gacttccagt gcaaatcact 84720
   gagaatctct atttttttac attatttttt acaatttttt ttttttacat attctttaca 84780
   ggttttaaaa ggtcactaaa gagaaagtaa actctatttt cttatatgaa gttggagttt 84840
   aagatctgat gccaaacaat aatcagcaga atgaaaagag gagtcacaca ctttaggaca 84900
   cacgcaaaat accccaaggt ccagaattca taggctttca tcctttagca ctccttaact 84960
   ggttacacat gttccaatac ttctaataag tttttctctt tactgttctt atcctttaat 85020
   tttccttcac tctagaacaa cgttttccaa cctgtgagtg cagaagttat tgcatgggtt 85080
   cctttcatcc gtctgagcac caagctctgt ctacatatgg aaccaataat acattttaca 85140
   caataaaacc tcttggaata tcagctgttt tgttagaaag tgagctcttt gaggaatttc 85200
   tcatgcacat atcaattgtg atgatacaaa gctgtgccgc tcaatacagt aaccactagc 85260
   tacatgtagc tttttacatt taaattaatt aaaattaaaa accaagtttg ccaatggcac 85320
   tagccacttc tcaagtgttc aatagctaca ggtggctatc ggctgccata ccatacaaga 85380
   gaaatataga gaataactgc aggaaatctc ctgatctaac ttgtcattat tgactactaa 85440
   actgtgcaac aataatttcc aattgaactt gaaaataaat tgttatttct acagcaaatc 85500
   attggagcag aggcatttat atgtctacag tcaaaatgag gcaaggcctc cctcacaggt 85560
   aaccctgtgg aggacaggct aattgaacag ttttacttgg aggcctagaa ttttatttta 85620
   tggaactggg ttatgtaaca tatattgtga tcagtaattg aacttgtaca aatgcaggta 85680
   acaatgaagt aaaatcgtga gtttttattt tacttaaaac tccaacttaa atgttgttct 85740
   taacaaaaat aatgcaaaat ggaatgcttg aagcattcaa gagtactttt aaaatctaat 85800
   catagtttca ttaatcaaag tagaatctac taattttaaa agcaagtgct ccttcattta 85860
   aatgcaatta atttattaca catagaaata ataatataat tattaatttg ttcattgttc 85920
   ttccttgcta tagtctttag acagaattca aggctttaag ccttgattat atattataga 85980
   aatttatatt tcaggatgta atagattgaa aagcaaactt catttaatca tcctttcacc 86040
   ttcttaccca ataaacctga cttggtctgc tgaacacaca aaccatcaag cccaaaagcg 86100
   tctcatttat tttaggccag agtcactgtc ttcttaaaag ggcaactgag atgcccaaat 86160
   tgcttcatca tttggttggt gagagaacta aattaaccaa catttcaggg ttttaggaaa 86220
   aaaatttaaa accccagaag cttgcagcaa atttagctgt gtcaatatct ttgcatgatt 86280
   cttttaaatt tgcttttcat ttcaagctgg agcagtctgg aaaaagaaat tagctctcag 86340
   taatgttttg aatacctgcc caaatgtcac aacttttatt aataatgctt aagactcatg 86400
   aagtgctttt tagtttgcag ggtgcttcta atacattatg ttacttgatc ctcgagacaa 86460
   gcctaggagg agctaggact caaatccctg ctgtctgaat ctgaatccat tacccctttc 86520
   aaaaagcagc atttacatcc tcataaatat atgctttatt agaaacagaa acagggaatt 86580
   aagtaaaagt ttgtgaaata aatgttggta gggatagatt agaagatgtt gctttgatgc 86640
   caactgaccc aaaagaaaaa tatatatatt tgtcttgaaa ctttggcttc ccctagtaaa 86700
   atgacttagt ctccacccaa ttaccctaaa gcgaagccac tagtcattaa gctcaagcca 86760
   cttatataga gattttacat ctttttagtg ctccagtcat aaataaaaac agtgccaggt 86820
   gtggtgactc atgcccgtaa tcccagcatt ttgagagggc aaggtgggag gattgcttga 86880
   gttcaagatc agtctgggca atatagtgag accacatttc tttaataaat aaataaataa 86940
   gaacagactc agaaaaaaaa atcttagatg tgaaaaacat gaaggcagga aaaaaaacag 87000
   aaggtaagat aaagttgtga aaatttccct gaaactagaa taaacagaga ttgaaaataa 87060
   agcaaaaaag ataagaaaca tgaagtgtcc atccaggcat tccaatatcc agctaataaa 87120
   gatgtctcag ctcaagtgca gggaaactaa gaggaagatt ttatcaaaga aatacaacat 87180
   tatgattctc cagaacagga ggacaggagt ggccacagta gaagagctca ccaagtgtcc 87240
   agcactgtga accaaaaact tcacaccaag attatcacag taaaatatca gaaaacttac 87300
   caaaagggga tcctaaatgc tttctgagca ggacagagcc aaaggaacaa gaataaggat 87360
   gacataggat ttctcattag taatccaaaa attagaagac aatgaaacaa tactttcgaa 87420
   attatagaaa ttattttcaa cctagaacgc tatatccagc caaaccacca tcaagtgtga 87480
   gagcagcacc aaggcacttt taggcatgca agatctcaaa aattaatcta ccatttacct 87540
   ttactcaaga aactttgtaa atagaacttc tcttccacaa caaggggata agccaagaga 87600
   gaaagagaca tggagtcaag aaaacaagag attccgcaca ggaaagagca aaggggattt 87660
   ctatgataat agggaaggga agtcccaaga caacagctat gcaggccttg agaacagaat 87720
   gagaaggagg ctgagagctc caggagaaag atcttcaagg aagcgaaaga atggagttct 87780
   cagatttcat gatgcatttg accagaggaa ttttatagtt ctgttggaaa gatctggaat 87840
   gtaatcataa caggtacata taggcaaatc aagaaattaa aatagggcaa ttataactcc 87900
   aaaaaaacca aaaacttgta taagaaagaa agagcagcca tagtacatca cattgctgaa 87960
   ctgggaacag cagttacatt gtcataataa tattaaatag taacttagct aagaattgtg 88020
   atattaaaat gttgatagga tgaaaggagg ggaagagggc atgtaagtgg gcaatatcat 88080
   tatcgttcag gataggaagt taattgataa tgtctaaaat tgaaaaatca agaaatataa 88140
   ctataagcat aatccttaaa aataagcatt cttggctgga cgcaagtggc tcatgcctgt 88200
   aacccccgca ctttgagagg ctaaggtggg aggatcactt gaggccagga gttcaagacc 88260
   aacctgtgca acatagcgag accccacctc tacaaaaaaa ttttaaaaag tcaagtgcaa 88320
   tggcacacac ctgtagtcct cacacacctg tagctactca ggaggctgaa gccagaggat 88380
   tggttgagcc tgggagtttg aagctgctgt gagctataat tgtaccactg cactccagcc 88440
   tgggtgatag agtatgaccc tgtctcaaaa ataataataa taataaataa ggattcttaa 88500
   aaatagaagc aacaactata agaattgaag gggttgcctc tgaggaacag aaagtagtgt 88560
   gaagaaagat gtggaaaggg attgctggtt catagcctaa aactttcagg actttcacat 88620
   tttaaactct gtaccttgaa aaaataagct ctgtgttcat tattcctttg gatggtcagc 88680
   tgaaacgttc taaaccattt ccagtaagga tcaaacatgc cataatggta atacttgcaa 88740
   gacaaacaaa atactcccaa aaaaactgaa ttatactggc cttgtactta ttgagcaaag 88800
   tccaaatctc tttgactgcc tagagcacca gcatcacctg ataaatggca aagttggctc 88860
   tcattttgct tcttgttgat ttgtcctttg aattgtatac accatgtaaa ttaaccatag 88920
   gaaatccctc taagtgttat ttttctcttg ccaactactg tgtgattttg gctttaaata 88980
   taggaacatc agctactgga tcacagggat gtgggttcaa agttcagcta ctagaaacaa 89040
   tttgctaaga gagacctaag aacaaaataa agttattaga gtgtaacggg catagagttc 89100
   cactttccac cttcattttt actgttaact aatatcagcc atgtgacaga tgccacctgc 89160
   accttccata cctctcattt aaaaggcatt tttgggagat gagtaatgaa gggaacaaga 89220
   gcttagctgt tgggatcaat ctgagagaaa aatagcaaga catcaggtct cattgtaaag 89280
   aaaatcttta attttctcag agtggcctct tttgccccca gttctttata acattcacag 89340
   gcaatgcctt ggtcccattc ttttgttgtt ctgttgtttt ggattttttc actggtatct 89400
   aataatgtgc agacctgcca tatgcttcta gttctacagg agtcttgctt tcaatgtatc 89460
   aaaaatcata tatgtgtgac atcttctatg gaaattgttt aattaaggca gccgtctgtg 89520
   tcatcacaac attagaggat ctctgggcat cactactaca gaagaagctg caatcctaaa 89580
   tccatgtgtt ttccttaatt attctaaatc actctataaa ttgtgcaggt ataatatttg 89640
   caaagattaa catttcaaga tgatgcaaca atagttgcat tatataagat gtcaagatac 89700
   ttagcacaaa tttccacaga tttgtagatt aaatcagcat gagccatgta atgaaggcaa 89760
   ggaactttgg ttcaatcatt taaaccttcc tcaaaaatat aaaaagtccc tcagtttcta 89820
   ccactatatt tttctagttt ttttatgtca ctttctgaaa tttcattgaa gtacaatgtg 89880
   catgcagaaa agagcacata tcttaggaga actgagcaca cctgcataac cagcactcag 89940
   ataaacagag caggacctcc tgtgtacccc ttcgcaatca ctatcccata aggataacca 90000
   ctgtgctacc ttctaacagc gtagaatagt tttccctggt ttttgtacat tatggaaatg 90060
   tactccttgg tgtcttcttt ttctcactca acgttattca catgaagttc atccatgttg 90120
   ttgtgtgtag ctatatgtac tattgccata tagtattgta ctatgtaaat ggtgtcacaa 90180
   attttctgtt tattctactg tttctgggta ttttggaatt tccagcttta gtttattaga 90240
   aaccaacata tagtacatat cttttaatga acatatataa gcatatattt tgaaaatata 90300
   cctaggagtg tgtagttgta tcataggata cttaaatgtt cagctttcgt agaaacttcc 90360
   agttttccca agtagttgtg tgaattttta acttttattt agcatacatt tattcaatga 90420
   tattctcagg aaaaggaata ttgttagatc acattttctt cattatggat ttcacaatca 90480
   tgactgatgt gttatgtact agggatgtgg cccatttaaa tgaattggtc tgccaggcat 90540
   ggtggctcat gcctgtaatc ccagcacttt gggaggccga ggtggcagat cacctgaggt 90600
   gaggagttca agatcagcct ggccaacata gtgaaaccct gtctctacta aaaatacaaa 90660
   aattagctgg atgtggtggt gcgtgtctgt agtcccagct actcaggagg ctgaggcacg 90720
   agcatcgctt gaacccagaa ggcagaggtg gcagacagcc aagattgtgc cactgcactc 90780
   cagcctgggg gacagaggga gaccctgtct caaataaata aataataaat aaataggttt 90840
   gtcacataaa ataaaatgtt ttgattaatc tactataata aaaaagtttt ctctgagggg 90900
   actggagtaa atacctggcc attagagatg tttgcagact cattaaaaag aaagaaattt 90960
   accaaaatct atatgggaaa gtaaggtcgg gcaatctctc aaatgagcag ttcagatgga 91020
   agcttttcca ataacagtct tcaagcagtc agcaagtctc cgtggaggga ttctatgact 91080
   gacagccagg caatctgaga tattgtctca actccaccaa ctttgtataa acctcttcat 91140
   ttatctgagc cctttttttc tcacctacaa aacaggaatc ttaattcaca ctttgatggt 91200
   gaggatcaca taaaaatact ttgtcttatg tatgccaggt gtaattatat gaaatggaga 91260
   gagttgatgt tttacatcaa agagctataa acatattctt agatcatggc ataaatccgt 91320
   aggtgttttg cagctataca cctagagagt acattttccc catcttgttt tagttctgct 91380
   tcttgatatc actatcttca tttttagcat tagaagtatt acatagttca aggatttcat 91440
   taattcaatc actagcatac cctggtgttc attagttaac tagttagctt actagttgca 91500
   taatggattt tcattagtcc tttgcttctc ccttatcaaa tattgattaa agatcaatct 91560
   ctagaatgta tgctcatgct caagtcgctc acctaactac tatcttggat attagcattt 91620
   aaccagtcaa tcattacttt ttgttttgga taatatagta tctttccctt accaattcaa 91680
   aaattacatt atattgcact ttttgggcct tttgcttctt ggtatgttgt tttgaaaaga 91740
   agctctggcc aggcacagtg gttcatacct gtaatcccag cactttggga ggccaaggca 91800
   ggcggatcac ttgaggtcag gaatttgaga ccagcctggc cagcatggtg aaactccatc 91860
   tctactaaaa gtacaaaaaa aaaaaaaaat agccaggcat ggtggtgggc acctgtagtc 91920
   ccagctactc tagaggctga ggcacaagaa tcacttgaac ccagaaggca gagattgcaa 91980
   tgagctgaga tcgcaccact gcactccagc ctgggcaaca gagtgagagt ccatctcaaa 92040
   ataaataaat aataaaataa aataaaataa aagaagttct gatgtggtgc aatatgttta 92100
   acagccagct gaaaatgcca aagtagcctt cttcctcctt tgggtattta tcagggctaa 92160
   gtataagagg caaaagattc ttcatgtgta gtaacttaat catttctctt tttattttat 92220
   tctaaatcct gctctctttt ttctttttca tgtccccatc tatgccacat gcagtgtgtt 92280
   tggcgtagtt acgcagctga tgagaaatct gtttccattg caacctggaa gccacacttg 92340
   aaggccttgc acacctgcag ccctaccaag taggtatcag tgtgacagct gccactgtag 92400
   ttgagtcttt tcaagtctgt taaacaaacc atacccacac acacaaaaaa aggtgttaat 92460
   gtttggcttc tcttattctc tgttgctcct agagagattc aaacctattt agtagcagtt 92520
   ctggcacaaa actaaatgga atcagtatca ttcctcacaa tccacagact ggggttaaag 92580
   attaaaataa tgtaaatttt atagagattt atttttacta tttcatgctt actttctaat 92640
   gtctcatttc gtaaagccca ccagcatcct aaaggtaagc accacagttc ttgaaaaaca 92700
   aggattattt attttgcacc catcgcctga ccccaagaga tgagtgctta gagagtaatc 92760
   catctttgta cgtttagatc tctcaaagcc atttctcatc tggatcctct ctggatggtc 92820
   ctcaagcttt tacttgcaga tcaaactcac atcagtggca aaaaaagaga aaaataggag 92880
   tcctaatagg attatagttt acaatgaggc aatgggaccc agcggattaa aatttggaat 92940
   ttgcattcac aattttaaag taagactact tttcttcaca tttcattggc caaagtaaat 93000
   cactcagcca tgcctaaatt caaagaaaat ggggaagtac aattttacct tttaccatgt 93060
   gcctagaagc aggcaagaac caaggacatt atggacagtc ctaatgatta ccacagagat 93120
   ctgtaattaa aaaaaaaaaa aagggcttag tcttggagtc ttggaaacct ggactccagt 93180
   tcaactatca cagaaaatgt taaatcatat tcaccaccta attaaagaag tcctcattga 93240
   aataccgaag cagtaagtca tgagaaaggc tttctgtgag tcacccagct tgattagcgt 93300
   agtcagttcc tcaggttgtt caaaagttat taaattgctc atccgacatt tgagagcacc 93360
   agctgctttt cttcatagcc caacaaaacc ttaatcctag tggcttcatg ggattcggga 93420
   cacctaacta ctgtgactca caaaaaacca cctgttcgta ctgatcttgg gagatatcag 93480
   ctgaggaaac ctccatgaca ttccctggca gtaccgtctt aatggaggtt tgtgatgcag 93540
   cagaatgcat accattggga agcaccagat ccatcattaa ctacattact tccctgttct 93600
   gcccttggcc tcagttttcc cacccacaaa gagaaggaca agagttgggc aagatgattc 93660
   ctaagctcta atgggaaatg cctcctggtg ggtggtatct aaaattcttg tcagcaccta 93720
   ggacagagat attcatagca ctccagggat ttcagagagg atggaacctt agcatggata 93780
   gaagcatgga tgctgttgaa tgcatgtttg gattaaaatc ctcttttcat ttagatcttg 93840
   aagtttagtt gaacttttta tggagtttaa gaccctttca acattttgat atcaaaaaac 93900
   cgaaaataga acctaactcc tgtgccattt attccttggg agtatgaacc aaaaataaaa 93960
   attatataga aattgtattt cttgtcttca tgaagtttgc taggtaaatg taaaaaggga 94020
   atctgagtag gaaaactgat agagaattct caagtgacag aggagagtgg tctattatca 94080
   tatgtgatta tttggcttcc cacttactgc agtttttgga ttaaaagtca ttaaaagcct 94140
   ggaaaaactg tttaataatg agtcaaattc ccccagtaaa tcaactatta ttttattcac 94200
   ataagttctt tctactcctc tgatattttc cctgtcttaa gtgggggtgg aggtggagga 94260
   ggatttatgc aaacacctgt gttcctcatg tcctactgtg cctttccctc actttccttt 94320
   tcctgcaaac tcatgaacca tggggctcag ctccattgaa tgaagaacag ttatttgtcc 94380
   agaatgacac agcagtcact ggtaaagttc atgggaaact agatgtccta acatctctcc 94440
   ccttccagtg atgcttcatt gcttcaaaat gcagaggttc ttaatccttg ttgatgtttt 94500
   ttgttaaacc attgagaatg tgatggaaat tatggggtct ttcccaagaa agataatcaa 94560
   tgcacgagaa aatcaatact ttgcctacaa ttttaatgta ttcacagagt ccctgaagcc 94620
   aagttcaaac ttttgtttta caaagcaaac ttgctttaat caagtgacaa taattttcag 94680
   tgacttagag ggatcttttt acatttaagt ggcttaccga atggagagtg ttgtagtaag 94740
   aaacagacta caattactat tttatttaca ttgtgtattt tcctttagca ttcagcctaa 94800
   gaacttctga tgggaaagat catttattca gtcatgtatg cattcaacaa ttactcattg 94860
   agcacctatg atgtgcaagg catagttcta ggcactgaga atgaagcagt tggagtaaaa 94920
   aaaaaaaaaa agttagaatc tctggccttg ggaagtatgt attcaaggga tgttgggata 94980
   gacaatataa ataaataaat ataggatatg ttagatggca ttaagtgcta aggagaaaag 95040
   taaaataaag aaggaattta gggagcatca gaggctataa tttcaaatag gtggccaggg 95100
   ggaagatcaa tttatcaaca atatcatcat catcatcatc aatgttactg ttatcattaa 95160
   aaaacaccag ccccatgctc ttgttcccca gttttaaaga catagtggga tgctttgatt 95220
   ccactaaaca ggatgaaaga gaggtctaag aaacagctgt gcagggcaga gagaaatggg 95280
   aggagggaca ctcaaacctg ccagggatac aaccagcagt tccttactct ctcttttcag 95340
   gtctggtttg tctcttacat acactctaga ggtcttagca attctatttc aaagttttat 95400
   tttaataaca gaaggatgta tagaagcaag tacttttctt gattttctcc tcaaagagag 95460
   tgaagctgca aagggcaaaa cacaggtaaa tatgctattt agggaatgag tcagaaattt 95520
   tatgaggcta tggggaatta tcaagctcca aaaacacctt gtggtacctt aagaagtggg 95580
   gatttggaga ttatctaacc taaagccttt gatttactca taagaaaacc aaaacctgca 95640
   agcaaataag gtgcctgccc aaaggcacac agctcgtttg caaagagtac agaccctaca 95700
   ccatggtgct tagacctaca tttgctaaaa tgagttatta caaatgccac ccaacagtat 95760
   aggctaaaag agttttggta aatattctat gtaccttatt tactccaagg aaatttagat 95820
   tttatacgat acaaaaaaaa atctgcctca gcaagtgact gagatacaat tcattaatga 95880
   atccaggttt agaaaccaaa gtctgccaga tgcacagaga acagttatgt tgtaactagc 95940
   aacattctaa gagcccttgc taattctggt tagatcagaa ccactttttc ttagccatgg 96000
   atagctttgg ctaatgaaaa gtctattgtt ttttattcta tgtgggcagt cttggctttg 96060
   ccaagtaaat gttgtgctat ctctggtctg tatcacaaag aatatatgtt agcaagttga 96120
   gtacatttat tataaagaac actgcttttg tggatcttgc atgaaaagag ttacttaagg 96180
   tatttgattc cccaatctcc aatttggcca ttatcaagtg ctataaatat attcactggt 96240
   ttatcatcat ttctcttctg cctgcacgca cctgaaggaa agaacaaggg gaagcatcaa 96300
   gcaggtttgt gatttctctc ttgctacatg tttgtttata aatctgatac ctaccaggtg 96360
   ttttaaatgt gtctcatgtg agtaaaatcg atcaacaaat acaagatgat taaaagtgac 96420
   ctttcacaag attgcctgca aagattgcta ttttattgat atttcgcccc ttagtatcaa 96480
   agtgtctttc cctaactcac tccctgcttt ttcaaaatct gcagggcatg gaacatttgg 96540
   aggtggggga ggaggggaac tgatcttgga ttagtccttg ctgttgtttt cccagacaga 96600
   agtcttatca aagtattcaa atatggtaat ctagagaatg aaagaaagaa attttttttc 96660
   tgaatgtaaa aagtccaggc agcagtctag gatacactcc ccttcactag ggtccttctt 96720
   ttggagtgat atatatatat atatatatat atatatatat atatatatat atatatatat 96780
   atacacacat atatatatca tatacatata cacatacata cattatatgt atatgtatat 96840
   atacctatac atataatctt ttttttttgg caagatgaaa ccacctctct gtgacatctc 96900
   cagatctcca gcccactgag tgctgaggtc tactcaagtg actcaaagag ccccatcccc 96960
   atcttttagg aagaagacaa ccctcccaaa aattccccaa ataatttatt atcagcttcc 97020
   catgacttag atggaatgcg tcgtcagatt acagcagaga attaaaccta gagggaatac 97080
   gaggtgaaag aaaaatttct tgatctgtgt taatgagtac caggaagtgc ctagatttga 97140
   gagcaaagcc ctgaaaaaca gagccaggtg gaacccagta ttaatgatgt gaagagtggc 97200
   agaggcagaa ccagcaagga gaatcccagg tgcctggaaa ggaagaagag atacattttt 97260
   tttcctatca ttgtcactgt gtttccataa agaaatgaag gcatccacct acccagagtt 97320
   ggtccctaag tggaggccag tgtgggccca gggattgttc ttttagctta gtactattat 97380
   aataattata ttaataataa tatatttatg caatactgaa gccagttttg aaagttcact 97440
   tggtgtatta aggctctgga ttatccattt ttgcattatg tttatatgta aagataaaca 97500
   aatgaaaaca taaatgcatt cgtctatgat gggagatagg cacgatagga tatacaacat 97560
   caggcacatc ttctgcgtag gtaacctacc caagggatgg cattaaagtt ctttctctta 97620
   tttggtagca tagagttaga gccaagggca ttggatattc cttggagata tttccagaaa 97680
   aaagtcattc ttccccacct aagaaacgtt tttgaccaaa gagaggttct agtcattaga 97740
   ttcggaatag tctttgccta gacactggaa gagacaagct gaccaattct agacttttag 97800
   tccattttct agattccata cagagctcac catccttctg ttgtgacaat tttacttgaa 97860
   aaaagttaca caaaataaat catagccatt ttaaggtagc ttatagttgc tttggtgata 97920
   ttttaaaagc tttcattgtc tcaggagctt tccatttact aattatctca aagggtgatc 97980
   tgcctccaaa tccaaaggac agagaccatc tttggcacaa attccagagc tacctgagag 98040
   tgcgatcacc catcttagtt tcatcctcca aaacggagct gttaacacct aaaagagaag 98100
   tttgtaaatg acatggaggt gcttgaataa gtgtaaactc tatggcaatc ctgaaggagg 98160
   ggacaccttg ggctggcaca atgatattgt actgttttta aatgttagca cagcagaaaa 98220
   caaagacaaa attaaagtgt cactgttgag cttctgctca ctagtccaaa aacaagcttc 98280
   ctattcagtt atcactattc aaaaaaacta ccaaccaaat tttccacatt tatgatgttc 98340
   gcatatatct acgacattta cctctctgca tgcagcagac tgaggttgtc aaagataact 98400
   cacccatagg tttaaatatg tacaaaacca ctgaagtggg ttgatgactt agagaatcac 98460
   acatggaaat ttcttgcttc tctgtggtca ttgaacattt ggcaaaaacc accttgaaat 98520
   aagtcctgca ctattactta gcctagattc attatttaat atcttatatt tcatacactt 98580
   aggtgtccta tcttccaact agctgtcaca tcaccctggg taaagctatt acagaatgtg 98640
   ctcaataaat aattcttgga tagagtaata aaatggccct gacctcaagt agtttgtggg 98700
   aagagataaa acaccaacaa aaaatgggaa gagaggagaa atataaaaag agaaataaaa 98760
   aattaaaaac attgcaataa tctaaggaaa gtaagctgaa gctcccattt aatattatgt 98820
   aagctttcac cttgttgtgc tccatacaca gaatttataa tcctgcataa tgaatgcaga 98880
   aaggctattg taataacaat agcaacagta ccatcttaga aagtaacaaa gttgtcctta 98940
   cccaagcaaa atatcacaaa gtaaaatgca tttataatga gagttcttcc ttacagaaga 99000
   gtagatctgg gtccagagta ttgttaatta atgcttgacc ccatttcaaa ggaactaatt 99060
   aacaccaccc aactgcagag taatttactg cctttgggac cactgagtta ttgatggtga 99120
   gtcagaaagg cagaggagac aagcttcagc ccaagcagga gccaacattt tcacctttta 99180
   gcccagagct tggaatctcc taagtccttc ttcatggttt ttctttgtgt tacaaactga 99240
   gagaaaccca agtgtagatg aactcaacac agcagccaac atccgcctct gggtgcctgg 99300
   cactctcacc aaaggacgga gccatgactg tagagtgggt catacacaga gaggcttttc 99360
   tttctgcaac attgattaag atgtcgaaca catttataga acccttagtt aactacattc 99420
   ccggctttgg aatgttcttg ttaaaaaggg attttgtctt tctacactga ggctagggta 99480
   tgtttaaaca aaatttgcct acgatgtctt gggctaagga aaaatgtcac attatttatg 99540
   acagatccaa aacttttctt taaaaataca atggccaagt gcagtgcctc atgcctataa 99600
   tcccagcact ttgggaggcc aaagagagtg gatcacctga ggtcaggagt tcgagaccag 99660
   cctggccaac atattgtgaa accccctcta ttttaaaaat acaaaaatta gttgggcgtg 99720
   gtgctgcatg cctgtagtcc cagctacctg ggaagctgag gcaggagaat cacttgaacc 99780
   tgggaagcag aggttgcagt gagctgagat cacaccactg cactccagcc tggacgacag 99840
   agcaagactc catctcaaaa aataaaaaat aaaaaaaaat acaagccatc aatctgaaga 99900
   agagcttttt aaatatagct caactgagca atagtgaaaa tactgtttca tcagttcttt 99960
   ttggtacatt caggtacaaa ttactgttag tctcaactga gcctgctgtt ttttcctcta 100020
   ctccaatcat aaaatacttc tttagcatca tttaatcact catttctttt cctctgaatt 100080
   cccactaaaa cagtactaat ctcatatatg tggtggtaaa tgtgtttatt gatttcattc 100140
   tattaaaaaa ttagttggaa aagtcatttt taaaatggga caagtttttt tctgttttga 100200
   tataaattgt cattttcaga gaactgaatt tacctctgaa tccatctgtc cagaataagc 100260
   caatattctc accttgaggt tttatgtggt tctcccttcc tccttgacca gattatcaaa 100320
   tagtagtcag tcatcacatt gatacaagaa ggcttttcct tctatctacc taagttttat 100380
   tgctacaaaa acagattata ttatctgagg ttttgagatt atctatacat cttgctgcct 100440
   ctagttactc tgtcttctta atcaacaatg gttggctata tgcctgcttg atacttaagc 100500
   ttccttaatg gcaattttaa aagctttgct agaacccttt cagatttctg agtagactaa 100560
   agttgcttta ccaggtacct gtgagactgt tgtaaagatg catgtcttaa acgttaaggc 100620
   aaaaatgata tttttatatt acattacact cttgtgctgt gagccaccga gtgctttttc 100680
   tgaaccactc ctgtgtggtt ggcttttggg ggaaggggga gttctgaagc tgtgtgtggc 100740
   tcttggagag aactctagaa ccctggtgga agactgtgtg ctcttatatt ttttctgcat 100800
   ttattttctg ttatttcaat tttctttctt ctgtgtgcaa tgaatgatct gcatgtgttg 100860
   ttaaactaaa ctcaataaga ctagattttg gtttttttcc aacatagtca acttgtgtcc 100920
   ttattggcaa aatgttccat tgtttcaaaa acaatctttc caaaatggtc tctccttctg 100980
   ttcacattta ttgatagagg aaacggattt agtcttctaa caacacatca taggacttaa 101040
   aataagaaac gtacttttct ttcgtttctt acaaaccagc aatctagaat cttttcttct 101100
   gttttgttga acatatattc tatctgacaa ttggttacct atatcaacta taaatgcaga 101160
   tgtttcacac aaaccaagaa cttacgtttt caaatttcaa caattaatgt catgaattta 101220
   ttgatttctg tagttattct ttgaagttct aaagtacgtt attaatcatt tatactatgt 101280
   gttattgata tttattacag gggtagctat attctaaagt atttattctt acatgctaac 101340
   acagagacta tcatcatttg tatgtaatgc ctagttcaaa tccaattttt caacctagcc 101400
   gttaagatca ctggatatct ttcaagcagc acaattaaac aacatagtac tctctgctta 101460
   aatagtctgt gtacttctat attaaaaaca ataaagccaa atggctttgt ttttataaaa 101520
   gaatccctga gcactttcct attcgtgaaa tgcttagtaa tgtgctgctc tatttccctc 101580
   actcctagta agttctgtag taataagcag aagctcttca gaatgtacac ctcacggaag 101640
   cagaggtacc cagcatccgg gctattgaca tgagatttga gaatgccaac taacctcagg 101700
   tgcatgacca ggttacaccg ccaccccacc tgagccctgt tcagagactc actctttcta 101760
   gggacttctt gaatgaggtt ttcaacccgc ttgaaatttt ccttgttgcc tgttgcctgc 101820
   tttgatactc tccaccgctg ggacaaatag tcggcgtccc gcgtagaact ggcagtcagc 101880
   cctttggcgc ctccttgtgg ttggacaact gctgagctgc tgcacgcact ggattgttat 101940
   ggctcagttg ccatattgtg cctgaggcga gaatttgtag aatgcttatc ccatgaaggg 102000
   ctaaaaccct aattaagtat aacaataact acagcattct aagtcccaga ggattactgt 102060
   ggggttatga tgatgccaca cagttgcctg gcagagaaca gccggtagta gactgcatgc 102120
   taggccattt gaataacctg tacatctggt tcctagagat gagagaattc tctcttgcag 102180
   ggtgacagtg caggctctgc tgagcgtggt cctcagctgt aactgtggga ctgtacccta 102240
   agagagatgc tccgatcaat gaccacagca attttttttt tactttctgc aaagcaagac 102300
   ctccttgttg gtctcttggg gataatcctt ggcattcttc ctaaaaatac tgttacataa 102360
   atcttcaaaa atcatattcg tagatgtcac caaaaaaaag gcaggaaaat aatgaagact 102420
   tcagtgtttc agtgaagagt ctatattttt aatcaattca atgtgtgttt tcagtaacag 102480
   tcacctgatc agcttaatag tcatctaatc aagacgctgt gttgattttc aaagggaaac 102540
   aaagtagaaa aataataaag tgagcttttt gtaagaggac acaagaacag atctttgcct 102600
   tggaaaagaa aattttgcct ggttaaaaag ttcttgatta gaatcttatt cttaactttc 102660
   atataattat gtcaaaagag aaagtgaaaa cattacattt tttctaattt aacatgtaat 102720
   atgccgagtt tattttaaaa taattcttgg gaatctttaa aataatagac attagcaaat 102780
   atccatgtag ttgagaagga caaaagggaa atgttactta gaatttattt ttgtcaactt 102840
   taagtgcttt aaatgatact aagatttaga gaatattact cacattttcc aatttattca 102900
   taaggtaatg tcaaaatggt tcatagaggc aatttttaaa aatattttta taggtatgat 102960
   ttttaaaata cttttattat aaacaaacta tggtggattg aatgctaata ctttcatttt 103020
   ggatgttttt acttttttag taattgaatt tttttaattt ttcatattaa gtgtttttat 103080
   gatggtttat ccatttctta tgtatttagt tgacatagaa aagagatata tcttgaaagt 103140
   tggagaaaat aatttgatat taactaaaac caacctttta tagtaattag catctgttag 103200
   ttccagaaaa ggtaatagat aaaatcaaat atagtgagga ccaattgtaa tgatatctat 103260
   gtcaacttga gctattcatt aatctgaata aaggtgcaga agtgagcata tgcttcctga 103320
   actagatcat tgtaaattta aattatggaa cagatgtgtt gaaactaaaa agagatttga 103380
   aacttttaca atttaaaaaa aaaaaagctt gagaaaagta gatatttttg ggcaccatcc 103440
   ctttgacaga tttgtttaaa taattgaagg ctttgggatg acatggaaaa cctagcctga 103500
   gaattaaaag atgcaggttt atgatgttat tgttgctctt gtttaatgta atctatcatt 103560
   taatcacaca tgtgtcaagc acagataaac tcttccactg ttgggattgt tgtaaagaac 103620
   tcttgaaccc acctgacctc atcaccaaac tgccccgcct gttctttgca tgtgccttca 103680
   gcgagtcatg ctggacttag ctgtgtctgc atttcagagg ggagctgtca gttgcatggt 103740
   caacttgttt attgtttcca gaactccctg accaccagtt catgatttga tttgttttca 103800
   tgtaaacaca cataaaacgt ggtgcccttt tagaatatcg tttgttaagc aactaggcat 103860
   ggaaaaataa atgttactgt ttcttgttag ctacaaagca catatccact aaacacattt 103920
   gctgttggca tgctgcagtt ggggacagtg gacattgcgg taagtggaca aaacaaggcc 103980
   tccacttcgt ctcaaataag ccttatccat gagaacgctg ctttcatacc ccacaaaggc 104040
   tgcggcttta ctctctcaga gtagctgctc tgagagactt ccctcagact cattgcccag 104100
   gatttcttca ggccctgcag agtcctgggc ctctcacatt gatgacagta tcaccttcta 104160
   atgaatggca taatttcact ataaaggaaa caagaaaaaa aaacagaatg ggtacacaaa 104220
   gcttcttaca gttcatttga ttcccacaag aatcccttag ctgtgttgct atgtttatct 104280
   ccaagttaga gatgagaacc ctagcccccg tgtgtctaat cccaacctgc ccttcctagc 104340
   acatcatagc atatctcttg attaggaaag tgtgaagatg cactttggat tttatgattg 104400
   ttgttaatgc ttcttttatc agcccacaac aatttgatct tagccggcat cagagaactg 104460
   aatgcctgaa caaatataat ttaccagtag ctgccacagc aaatcaataa cctgtttact 104520
   agcaggaaat tgtgttagtg aacataacat ctcattctct tcatcagtca tcacattcat 104580
   tatctgttgg ggctgttcta tcttgtgtgg ttttcatctc acaagaatga atgtaagcat 104640
   aaaagaaatc taatgttaag tgttgtgtgt ttaatattat gtatgtggtg ctaccaactg 104700
   ctatgagatt gcatgaattt atggataaca taatcttgcc tggaatataa aatgcatgcc 104760
   aaatgtaggt atatatgtaa ctgtcatccc tgtttacctg tagaaatgtc tagaaattac 104820
   aaagagatat gctcccaaac aaaaaataat tttttaaatc tcaaaagttg aggttaaagc 104880
   taaaattacc caactgtttt tcaagtgaat tccaaaacag ttcttagtta tctacagtgt 104940
   aattgtgtac aaaatgctgt atgacctcag tcttttctac aatgaatcca agaaggatgt 105000
   atgtacctgt agaatgagat tgatctcgaa tgcaaatata gagattagat aaagctcaaa 105060
   ttttaaataa gggaaaaata attgctatgc tcctacgtgc tgaaaacatc ttgtctattg 105120
   aaatatatga ccacatgagc ttcatcaaat tacttaccca agcaaagtgg aagaaattgc 105180
   ttgaaatgga ataatcatgc ctctgttctc cacagtcaga agctaagttt taaggagcga 105240
   gtgcgcatgg ctagccccag gggccagagt attaagagcc gacaagcctc agtaggtgac 105300
   aggaggtccc caagcaccga catcacagcc gagggcagtc ccaccaaagt gcagaagagc 105360
   tggagcttca acgaccgaac ccgcttccgg ccctcgctgc gcctcaaaag ttctcagcca 105420
   aaaccagtga tagatggtaa gccctgtttt tccataacca tttttaattg gataggctat 105480
   agtgagtgag attatgaagt aattaattct ccatagtgcc acttgaagaa agaagaaaac 105540
   aaccccagag aaagaattgt ttgtttgttt tattggaagt ttattcattg ccttgggcaa 105600
   atgtacagaa cataagttta cttttggaaa ccactttggc agctaaattt acattccaaa 105660
   gaagctgaca aaatgttgtt aattcttcac ttgtatctac aaatttattt ctcagacaag 105720
   ggccacatta agatcaatgg gactgtcatt ctgatgtatt tgtgacgtct gaccagcaaa 105780
   ggtccaagac ccagggacaa ccaggtgctt gctctgtgaa tatcttgaaa cacagtaagg 105840
   aggatgatca tttctggtca acaatgaagg tgctctttcc ctctctcaca cactccatct 105900
   accagcaaat cagagaccac tgcttatctt ccttagagag gttcctgttg aaagttattt 105960
   cgaagtcttc caatgataag taaaattcaa gttcttctcc caagaggaat taacctattt 106020
   tatttatcat attatgtagc agacattcac ttgccaacac attttattga gtgccacata 106080
   attttcatgt ttataaaata aaatatttta tttttcattt aagataagct tggctttcct 106140
   tttaactagg cccccctgga tctcttatac tgggcaagga accaaaatca aatagcattt 106200
   cccattaatg cataaaaact acaaaggcta tcagtccttc tgttcccttt tcctaaataa 106260
   cgtgtatttt cccaggccct caaagctcga ggaataaaat taacaaccag gggtcataat 106320
   cacaatgatc tgaggatccc tgcagtgaag tccctggaac cgcagtccac gggtccctct 106380
   cagcttactc aaaggtcctc agggatatct cttccacgct caagtgagca acacaagtca 106440
   cctcttcacg cctcactcca cacccctgtg ggtgccatgc tcttcagggc gacccctagt 106500
   ggctgcactg acgtggctcc tcctggcgga gcttgcaggt cccatatggg cgcaagacgg 106560
   ctgatcctgc catgcttccc cgcagcctgt cctgtgctaa ggaaggaaaa acctccgcca 106620
   ccaacacggg acactcctcc atcgagccac ttgcaggatc tgagtcactt gtggcacagc 106680
   ttttcagtgt cctctagttt cctcactctc ctcctaggat tttggctgaa agcccttgag 106740
   cagctgagtc aagagttacc agtgacaggg ctttatgttg cactcaaaat aggcttgctg 106800
   aatataaatg ggtacatcct ttctggaggg caatttggca aaatatatta aaagtcttta 106860
   catatgtcta agcctagaca catcccacct accaattcta tttctaggat ataatagata 106920
   caatgaaaca atgtcaggca ttagaaatga tgctctaaat taaaaattat tgacacagaa 106980
   agatgataac aaaaaaatca ttccatttat atatattata taaattcatg tatctatagg 107040
   aacaagtctg gaaagattta caagaatgtg tgaacagtta cctttgagtg gtgagatcac 107100
   agttatattt tgtttcttca ttttttaatc tgtatttcct aatatttcta caatgaacag 107160
   gtaaagctct tgtaataaga aggcaataaa ggattttctt ttttaaaaaa aaaaaagccc 107220
   tcagtttaga aaaacaaaat gttaggggga aaaaaagaaa aaaaaaacta gcccttcaag 107280
   aaaaaaaatt agcccttcct ttcacacact gccctctccc ctgcccctac tctaggggcc 107340
   cttgccctga ctgttactca cctaacacct ttccttaagc tgatttcctc ccaagtggta 107400
   gaaggagcag cttgtcttaa tggtgggaac cagtctttgt tgagtgcaga catagactat 107460
   gtaatgatgt ttaatagctt ctactacaaa tggaacttgt attagtttgc tcaagctgct 107520
   ataacaaagt actagatgcc ttaaacaaca aaaatttatt ttctcacagt ctagaggcta 107580
   gaaggccaag tttaaggtgt cagcagggtt ggttccttct gaaggctttc ttcttggctt 107640
   tagatggtct tctccctgag tctttacata gacttttctc tgtacccagc catgtgcaaa 107700
   tttcctctac ttataaggat accaatcata ttggattagg agccactcta ataacctcat 107760
   tttaatttaa taacatctct aaagacccta tctccaaaca gtcgcattcg gaggtactag 107820
   gggttaggac cataacatat gaattttgga gggatgcaat tcagcccata gacaaagctc 107880
   atgctcaccc acatcacagc atccctggaa agctgagatg tgtccatatc ttcatccccg 107940
   gccctggttt cacactgcat tagaaatcat acattacata ttgcaatgga tccatattct 108000
   tctaacatgt gcttcaaaaa gcattcaact ttgtagtgta aggtccttac tgttggctga 108060
   gcatatttaa ataaaaaatt catagaatct ggatttttaa tttggaaaac aaattctact 108120
   tgtactgttt ggccagagga aaagaatttt tcacagtatc tagagtcacc actatctatc 108180
   acaatatttt cagtatctgc aaagatgtct cagaggtaca gaaacagaag tcctattctc 108240
   taccaaatca caaattggag aggtctcctg tcatctattt tctaggtcat ttcatacgag 108300
   tacagttttg tttgtttaca aaagaattgc ttctcccttg ttgatgccca ttcaattaat 108360
   tctgagtttc ccaagtgacc agaggaccaa ttgctgttct gttgaaatta ctttgttggc 108420
   tacccacctg caagagatag tagctcctgc ctcctcattt aatccaatag tccagaatct 108480
   ttacctctga ttgaattcaa tgttgcgtct tgggaaactg gaagttcttt ttttacaaga 108540
   aaaaaaatta agtagctgtc aaataaatgt agacttcagt tatacatctt tacttatagt 108600
   acctctttag gttttaagta tttctaaaat catctactta ctaccataga aaataatata 108660
   taacacttat agccagtttt caattattcc aggtctattt gcccattcac ttctggggct 108720
   gttttcttct cctctgctct cctcacatat tttcagcatt ccattgccca ttaacatgct 108780
   caggagaata gacaaataaa agaaagaata atggaaactc agatcaacct ttaagttaat 108840
   aacaaaaagt tatatttgga aaataataga cttgcaattg aaagatagta taatagagaa 108900
   aaaaaaatat tcagggttaa tctgtaaatt ttaataattc ctgacaaatc tgatactctt 108960
   tactacagac agtagtgttt tcataattta atgactcttt aagaaggtta ttaaatatcc 109020
   atggcatttt tctcttgcta taatttttaa taagattaat atgttacact taaaaaggac 109080
   gcagttgtct ctcaatattc catacttgtg tgtaccagaa aagtagaaag tagcaggcag 109140
   atgctgaagc cacaaatgac aaacgaaaga aagtaaactc taaaggactg atgtggtgtt 109200
   atcataccaa atttattttg actgctatga tatgaatcat atttttaact gataaattct 109260
   ataataaaag atagaaatac tctattcatt gacagtatgt aaccctcttc acataatttt 109320
   agaaattagg taatatagaa atatttttgt caagtatatg agtaataata atgataaaac 109380
   gaacatgtat tgaattcttc ccattggcat gtgttgttct aaattcttta cctctattat 109440
   tttatttaat tctgtcaata cccctatgga gtagctgagg tattatcccc attttatgga 109500
   tgaggaaact aagagttgaa tctaagtaac ttgcccaagg tctagcaggt ataagtaaag 109560
   gagccaccat gcacatgcag gcaattgaat ttcacggtcc tcctacaaac gtattctaag 109620
   atgttctcct agatattata tcaatttccc tttaagaatc tttcatttct tcccctcccc 109680
   caacccccac caccctcttc ttgccgctgc tcttccaact tcaatcggtc agattcccct 109740
   tcccatataa gccacttgac catgaccatg ccactatccc atctttctcc cagcctcagc 109800
   tgccaaacct ctcaactaca tcatctcaag gcagccagag ctagaagatt aggcaaattg 109860
   gggaaatggc ctttcaatga ccgttctctg ttttgatcaa gtatctggga agagactcag 109920
   gataacagcc atactggtct gtgtctattt tccctggagg gaggccaatg gtagggtcat 109980
   ctgaggcctg tttcaagatg caaaagatag tgtcagaaga tgcacaggtg ttgccaacag 110040
   acaagcaagt ggaaggtggc agattgcaag cgagaaatgg agcaagcagc caaaatccac 110100
   aagaatgata ctaaggtaga gctaggagaa aatcttcgga tttctggtga ctagtttgct 110160
   acgaaggagt cctacactta ctgaacccca ctgcatttgg gagtgcctta cagaattgca 110220
   atcaaagagt acaataacct ggacagtgaa ggagaaggac gtagctttag ggagctccac 110280
   cttacagtgc ttgatattag ctaaacccaa cgttctgtag taattagcat gttcgttcca 110340
   gaagtggcaa tagatctgaa aatctgtctc acatacatgt ataaccatgc atgttttaac 110400
   aagtctaaca ttgtttctgc ttaaggagaa attaccattc ctgctgagca atcctcagtg 110460
   ctcatgttag ctgccatgga aggtacatcc tcatcaggtg tttatgggaa aaactggcac 110520
   agcctgggtt caggagatgt ttagggttca ccagggtggg atctgggtga gatgaagatg 110580
   cagaatatct tccgattgtg ggggtccctc aaggctagtg cctttgttcc aggcactgat 110640
   ggtaattcct ctctcttcac tacccttcat gttcctttct cctctcagcc agtgtaccaa 110700
   ttaggatgca tttagcagca ggtcacaaaa gaagcagatt caactacctt aaactataat 110760
   gcaatttata ataccacata gaaagaggct cagagataag acagctctcc acagagcctc 110820
   agggctctgg cttcatttct ctgattctct gagtcttctt ttgacttttt ccatctgtct 110880
   gcctgcctcc tcctcaggct ggaagcaaaa tggctacagc agttgcaata tcttctatag 110940
   atgcaacact ttcagaggca gaaagaggcc atcttatgtt tgtatctctt cttaaaagtg 111000
   aggagaactg tccattcaca tcttattttg cagaatcctc atgcctaaaa caatcaccag 111060
   catggaggat gggaccgcca tcattggctg tcaccagtca ggacccagca gctggatctc 111120
   ggccagactc ccctaaagca catgatcaag tgtgggggga agtagctgga taaatttaag 111180
   gatttaggtt tggggattgc atttggggaa agcaactttc catagtataa tatttgatat 111240
   agccaagaaa cctcttccac tttggggtga gggtaaggag tcagcatgtc cttaaacatg 111300
   gtgctttctc tcaaatattc tgtctatatg tcatttctgt tcctctaaat gtccgggctt 111360
   ttaaaaacat aaaagccaga tttataattt ttttcacttt tctctcatca catcatttcc 111420
   atgaaacaac aagcatagac tagactgtct ccttgaattt caaatgaaag gcaaagtggg 111480
   taaaggaaca actagcaaaa ataaataaat tagtatttca atcatataat ccttcctcac 111540
   atacagtcag cccataaatg ttctatcacc accaccacta agggaaaact ggtacagttc 111600
   ccttcctctc cacctcctca gcagcttcct gacacctcgc tcctcaagct gtttctcttg 111660
   gtgactcctt cttccttctg aagttaataa ctaacccagt ctcacctaaa aatagcaaaa 111720
   aagtagaaat acaccatgtg gggtgccaga accctcaagt cttcttaaag atagttcatc 111780
   cccttcctct cttctcttaa cttctaccta ggttctaggc ccaccagtct accacaaccc 111840
   ttgtttgaaa atatcaagat cactgaaaac agtgacaaaa aggcagcatt ttaatgcaat 111900
   ggttttgaca actatcctga actaacagtc tcttgtatcc ataatggcct attttcttcc 111960
   aagcagtata aatcacagac tatcagtatt ttctatttga ctaatttatg cacacgagct 112020
   ttcatcttac actaatcctc ttttatgctt attttccaat ggcatcttcc tccctcatgt 112080
   ttttctcagt ctccccagtt gacatttttg gtgtttccac aaaatgccaa gcaccacgct 112140
   gcacatagca tttttcctga ctttccataa tgatggccca acttcagcca ggccttgatt 112200
   ataattttcc acatgatgct tcctggcact ttctgaaatt gcctgataat gactctgcat 112260
   tgtgttgaca gaaataattc ttgttaaagc agtatcctat tagggcttcc acttaacctg 112320
   tttgtgcaga tttctggcct gggattctga ctgagtaggg cacccattcc tgtcatcctc 112380
   tatttgtaac aaaatctgag ggttttcttt aatgttaaga acaaaaggat ttattgagaa 112440
   tgactggatc taaactctag aaatttattc ttcatattta actctatatt cagcgtaatt 112500
   gttggagtaa ttctatgcaa aaccctacaa agggtattca gtaggattct aaaacgcact 112560
   ttcagtactt ccttaactta ttggtaaaaa ccattacctg aagaaattgt ctcaattctg 112620
   atccataggt gtcttttttc tttagttgtc caggttcttt attcagtccc tcaggcatct 112680
   tcattcaaag acatatccta ctcctctgct gtgtttcata tgtactcttg tccccctaag 112740
   atctgcttct cctattgggt ggagagtgac ctctcaatct ctggtttcag accccaagtg 112800
   actacgttat ttcttggatt tctgagaaga gttgtgtctt agtttgtttg gactgctata 112860
   aaaaacatac cataaactag gcagcctata aacaacaaat atttacttct cacagttctg 112920
   gaagctggaa gtccaagatc atggtgctgg aagattctgt gtctggagag ggcccacttc 112980
   cacgtgatgg aaagggcgag gttcctcttg ggtctcttta tgagggcact aatcccattt 113040
   attagggctc tgccctcata gcctaaccat ctcccagagc ccccacctct taatactatc 113100
   actttgaggg ttagggattt aacatataaa ttctgggggg acacaaacat tcagaccata 113160
   acaggttgct tcttccatta ctttttgtga aatgcttgtt cttagttttt agtgacgggg 113220
   aaatgctttt ctattttgat aagactgaag ttccacctgg ctttctactc taatccaagt 113280
   gctctttgta ttatagaaca ggatttctgt cagatttctc taggttctgt cccatggttt 113340
   gttgttactc tacagtgata aggaaaatta tattctgttt ctgttggtgg ataaaatagc 113400
   ttcctgcttg agctctgaat ggatttcatc tcctcccttc cagcagaaac tgagctgcca 113460
   aatgaccttc gagtgctgcc ttaaccatac cttttggctt ctgtagatcc aggtttcatt 113520
   gagaaaccca gggttataaa ctggagccct atattttgtt tcatcagcaa agtgtgtgtg 113580
   catgtccaca ttgatacttt aaaatgaatc tgaatgctgt attagttatc tattgttaca 113640
   tagcaaatta ccctcaattt agtggtttga aataacacat acttataatc tcacagtttc 113700
   tatggtcagg tatccagaag cagtttagct gaatggttat ggctcaaaat ctcatatgag 113760
   gttgcagtca agctcttggc tggagatgct gggagaggac ccattaccaa gctcaactga 113820
   gcatttgcta gcaggccttt tatttccttg ccacattatt cacaccctag gttgcctgaa 113880
   tgtccatatg acatgcagct ggcttccccc agagagagct gagagagtac gcagagagaa 113940
   gctgcagtct tttatgacat aagctcagaa gtgttatgcc atcgcttctt tcatgtgtaa 114000
   gtggtcacaa agaccaaccc tgtacagtgt aggcagggac tatacagggg ggtaatacca 114060
   ggagatgagg gccactgggg accatgttag agtcaggcta ccacaaatgc cttcagatag 114120
   attatgtctt ttaaattgca gatttcaacc agtccctatt gtctttccag gaacccaaac 114180
   caccaattcc ccattccttt cgacctctcc gttacctacc tgcctttaac gacatgggtt 114240
   tataacttct ggtctaacca ttttatgtcc agtatccttt gtattatatc tcaaaaaaaa 114300
   aaaaaaatcc tggcctcaac ctatagctac gcaattatat tccactctta gtgcagactc 114360
   tcaaaaggaa tattaacaaa atattgagta tgtgattact gcagcaacaa gttaaataaa 114420
   atgaggaggc caggtgcgat ggttcatgcc tgtaatccca gcactttggg aggctgaggg 114480
   ggatggatca cctgaggtca ggagttcgag accagcctgg tcaacatggt gaaaccctgt 114540
   ctctactaaa aatacaaaaa ttagccaggc gtggtggcgg gcacctgtaa tcccagctac 114600
   tcaggaggct gaggcaggag aatcacttga atccaggaag aggaggttgc agtgagctga 114660
   gattgcaccc ttgcactcca gcctgggcga caagagcaag actccatctc gaaaataaat 114720
   aaataaataa attgagggag caggacatga actagtcagt tgcaaaacca aagtgtcata 114780
   acttctaacg cagctctctt tcttgtacaa tgtatggccc ctcttcagct tttcaagagg 114840
   ttgaagatat gttcttggtt gcttcaaagg gcagaagcag aactaaaagg tgtgtgatac 114900
   caatatttag ttcaatataa tggcagctta gcacttagag actccacagc aattgagtgg 114960
   attccataat tggaagcatt caaatcaaag ttggatgatc aacagtcagg aatttctgga 115020
   agcttaagga gatcaaacca gaaaacctct aagaaatttt cttccaaatc tgagttttta 115080
   taatctgctc ttctggacca atgcaaagta tcttccttag ccattaactc gtatcctcac 115140
   tagcaattct atactaagaa atagaagagg aaagcaagga ttggctgagg aatgccagga 115200
   gtgagttttc tgtccttacc ctagtttcac gtctctttga taccgagaaa agtgctaccc 115260
   gctgcttctt gcctctcatt cctcccactg tttcttttcc actttcactg tcagccatag 115320
   tcctttctgt cctaagaagt gtatcaggtg tgtcccaccc accattgtga ggtttatgtg 115380
   tgtgctccat agcggctcat tcttagaact tgaataagcc ctccactgga tttcaattca 115440
   tatcagaagg tatttgaggg ctgctactca gagctagcat gtttctgggc atttagaagg 115500
   cagggaggag gcgtgacaac tgtcacccca aagaacttac tgtttgtctg taagttttac 115560
   caccccagcc cacctcagtt acagtattga ttggcactga ggggaacaaa aggcaccttc 115620
   ctcattcttc agacttcata gttttcagaa ataacctgtg ggtgacgtcc aaaattcgca 115680
   caaaataagc acaaaattaa ccattacaca aatcacagaa tttagcccat atttaagaga 115740
   agcacttttc agctgcatgg tgatttttcc ttcatatatc tcaaattatt tccttcaaag 115800
   tcacacttag ttgtctgctg cataaacaga aaccaatatg acacaatttt aaaattatat 115860
   ttaatcgctt tcatggtgtg ctctacttgg taaagaaggg gggaaacaag gttaagtaaa 115920
   ggccaggatc ctttttctgc aatggatgac tgacatggga aagagatgag ttacccaggc 115980
   tttgcttttt gctacctgca gtataaaact ccctgacatt gtcccaaatt gcttttacag 116040
   gtattttatt ttcaacatct aatttgttcg gcttttcttc ccttgccatc tttttcttct 116100
   tttgccatgt ataatgcata acaacaattt ctgaactcca aaagcataga aacctcagaa 116160
   taacattacc tgggaaaaat taatctctct acctccagct accaagagaa tcatttgcac 116220
   atgaaaccct gagatttgcg tctttcatta atctaatgca gtagcaattg ttataccaca 116280
   tcgttctatt ggaattgatg actaatcatt cccaaaactc ttttgaaact attgttccca 116340
   taattttgtg ttttgattga aaaatgtcat gaccctatat ctcttcatgt tatctgttgc 116400
   atagctaacc ctctccctca gatttagtgg cttaaaacag caaccatttg tttagcccac 116460
   aattctgtta ggcagttctt ctggtcttaa gtgaactgac tcatgctctg tgttcagctg 116520
   ctgtgttggc tggtgctgac tggactaagg tggtctcagt tagaattact gctctttgct 116580
   catgtggtct ctcatcctcc agcaggctgg gctggttcac atggtggtgg tgggttccca 116640
   agagcagtac attgatatct gaaggcctct tgaagttcag gctcaaccta tcacagtata 116700
   atgtccacta tgtcctgttg gtcaaagcaa atggcaagga cagcccacat gcaagagaca 116760
   gagaaagaga ttccacctct tcatgtgaga agctgcaagt gtcgtggcca gtattgcaat 116820
   ctaccatccc agccatttaa agcttatttg cttttagtat tttcaacatc ctgttggttc 116880
   tatgaagaca gtgtcagtgt ctaattgact tttttatttc tccctgtagt atccttcact 116940
   caaagagcct aagaaagaaa aggaaaatag cattgttagc acctcatgta tcgaggcatt 117000
   ggctagacat tttctatgaa ttggctcatt taatacacac atagccattt tgcagataag 117060
   gaaattgaga ctcagaaaat gtaaataact tgcccacata attcagtcta aatgccaaaa 117120
   ctggggtttg aagttgcact taggtctaag ttcaaagctt tttatgttct accatgcatg 117180
   ctgcttagca tgttttcttt catataattc atgctgctaa tgaaacagta taaaccagct 117240
   gtatgttaac tgctagtgaa atgatttatt gtcttctttt tcagtaaata tggcaagtct 117300
   gctcaattta ttccctttat cattgcatac actacaaata ttctctccct ataccttcac 117360
   atgtcaatta aggtatgcaa cagtaatttc catttttttt aaacaatgct tatgttacca 117420
   tctctagaca agttagtcca gatgattttg gggttaacat gttggcattt cctggcacta 117480
   ttactgctag ctgaataaga gttgaaagtt ggggaaaaat ttaccagatc tgtattagtt 117540
   ttttcgcaca ctgctataaa gaactacctg agactgggtg atttataaag aaaagtttaa 117600
   ttgactcaca gttccacaac ctgtacagga agcacgcctg ggatgcctca agaaacttac 117660
   agtcatgtca gaagagcgaa agggaaggaa gcgtgtcttt acgtggtagc agaagagaaa 117720
   gagacagaga gaatgagaaa gtgagagtga agggggaagt gccacacttt taaaccataa 117780
   tccaccccca tgatccattc acctcccacc cagtccctcc cccaacattg ggaataacaa 117840
   ctcaacatga gatttgggtg gggatacaga gcgaaaccat atcaagatct atttagaatt 117900
   ctccagcaga tttgattaac aatatagatt tgtgattctg caatttgtag ggtcattata 117960
   aaacatggct gccttgattg caatcagata tctctgaata tggttcaaag gaaacagatc 118020
   tcgttcttgt ggagatagga ttacataact ccctctcagt cattttgcca ttgattcaga 118080
   atgtttctcc ctaagatgaa gtactgtttt agcttagaat ggtaacagca attcatcacc 118140
   aggcacagtg gctcacacct gtgatcccca cactttggga ggcccaggcc tgtggatcac 118200
   ttgagcccag gagttagaga ctagcctagg caacatggca aaaccccatc tctacaaaaa 118260
   atgtaaaaat tagctgggca tggtggcaca ggcctatagc cccagccact tgggaggctg 118320
   agaaatggga ggatcttttg agcccaggag gttgaggctg cagtgagcca tgatcgcatc 118380
   actgcactcc atcctgggca acagaacaag tccctgtctc aaaaaaaaaa aaaaaaaaaa 118440
   aaaaaaagca attcattgat ctcatctagt ttgactagtt caattataca tgtgagaagg 118500
   ctatctgaga tgatgtcagg tgtggagcag tgactatccc agtgatctga atgtgattga 118560
   ccacttggct tcaccaggat tatctcactg tttggttcta ataaaggcag tcattctagt 118620
   ctatgagttt acctcccttt tgctacaaac acttcagtga tatactattg aaactataaa 118680
   aatccataaa aattggtttt tgtatgtgag ttttaaaaaa ttgttttcac caggctgtga 118740
   gttttatgtc tttgttttct attgggatac taatagtgct atgactatag tatgttctca 118800
   aggaaattct tatgaaaatt gaattaatcc gacacagtgc atggttttgg agtaaaacag 118860
   aacttgtttt aaatcctagt tcccccacta ggttgggtga gatgagacaa acgacttaac 118920
   ctcactgcca ctttaatttg ttctctctct ctctttaggg tggtagaaat gatacctacc 118980
   taatagggtt acgtgagtaa taataagata taatgtgtgt aaaattcttg tacaatgccc 119040
   agcacagaat aggtgctcaa tatattaaat atattgttat tatttattaa ttaattaatg 119100
   ctgatttctg cattaatttt gactaattga aatcctggac ttgcttaatt tgcatgtatt 119160
   tgtcttccct tcttccagta cttgatggca atttctacct gtggtaaatg gaacgttaaa 119220
   agttatatgc ttatacaaac tttagtcctt tattccttgg agtgagtttt aagagtcctc 119280
   atgttgaata attcatgtaa tcacagaaat aatactatgt atcagggacc gcttccttta 119340
   ttaaagataa aggcagtgga aatactgcct ttgtcagaaa tacttcagtt actctgagag 119400
   agagagacat ttgtttacta tatgacagca cagtgcatgg caaataatag aagtttggaa 119460
   aatatttatt ttgaatgaat gaataatgaa cacattgtga acaaataaat ggtttactat 119520
   ttaattattc aggaatcaat taagctttat taataaaaat tgtttggggg ttttaaaaag 119580
   cccacacaca aatgagaggc atcacagagg caaacggtat gacattatta aaatcgttat 119640
   tgctgaatca cagttcagca ataagcaagt gtttgaggca ttcaaataag tgagtatttg 119700
   agtgactcta agaagataat gaaactcctc cactgtttta cagttgcaat aatttttaag 119760
   gaacatataa aaacatattc tagtgtttcc atttgaataa agatagtata tataacatat 119820
   gcaattaaaa agaagaaaga aaacaaattt taatgtgttt ttcttttcac cttatgggca 119880
   gttactagat gtatnnnnnn nnnnagcttc atcttgcccg tagaaaacag ttcagaaaag 119940
   tattcatatt taaaactggg gaggctgtag aaaaagaaga aagaataata ttggcaagac 120000
   aattggctga ccttatacat atcttggttt atcagcttac tctgtgagta tcccgccatt 120060
   tccacgtgag aaaaattcag caacatgttt caaattgcgg atggtggtgt tatttaactg 120120
   gcaatattct cttgcagctg acacagccct tggcactgat gatgtatatg atgaaaaagg 120180
   atgccagtgt gatgtatcag tggaagacct caccccacca cttaaaactg tcattcgagc 120240
   tatcaggttg gtgaaaatct tgaacaacgt gattcagaga catacttatg attaattgaa 120300
   ttactatgtt gctaagttct ttttctgcag gtaaatatcc tggaataacc actttccctt 120360
   tcctattcat nnnnnnnnnn aatagagtgt tttgcaaaca catttgtatc tcaaaaatgc 120420
   atgtagttcc ataaaagacc attttaacag cctttatatt ttttaaggga tgctattact 120480
   agatgttgta tcttggactt ctggagattg catggaaaag ttagaattgc attttgactt 120540
   taacttacat tcagggtttc aacggacatt aaaaaggcaa aacacttagt gttcagcctc 120600
   ttttttagtg tgtttttcta ttcttaaggt gttgtctcac atgagatgtt cttaaattgt 120660
   ccactgtaat ctcaggcttt tatattacac atctgcagaa gccaaaaata atttgatgca 120720
   tgacttttcc ccccagagtt tcttattata tttactggta aactattttg gtaacttata 120780
   ttaacagagt tataaagatt aatatgtaat atgttctcat acagaattat gaaatttcat 120840
   gttgcaaaac ggaagtttaa ggaaacatta cgtccatatg atgtaaaaga tgtcattgaa 120900
   caatattctg ctggtcatct ggacatgttg tgtagaatta aaagccttca aacacggtaa 120960
   gcaatggaaa tgtcattcct tgtaaaggaa tgggcataga acctatctag gaagaacttc 121020
   tctttagtag agtaaacctc tgaactccat atttttcatt catttttagt gggcaaatgg 121080
   aatttgcttt aaacattgat tttattttgc ttttaacata gatataactc aggcattcaa 121140
   agcatccaac tgtctcattt tccttatctg taaaaatggg aagaaagtta gttcttgcct 121200
   tatagggttg tcatgaggat tgaacaagtt aatgcatgta attcataaca tgtaacaggc 121260
   ctggtactgt gtctaataaa catcaatggt tgttgctgat gtttgaatta ttactgctgc 121320
   tgatcctaaa ttatctaaat tccatttatt atctaaattc taatcatcct ttgggaataa 121380
   cgtactattt ctccttcatt aagtttcatc caaccatcca acctgtgaag acgggccttt 121440
   ctctgacctt tatacagtag cagagtctac tgcagagcac aagagcactc agaatctcgg 121500
   tgtgctgggc caaagttgag acttggccct tgtgttgttt cctggttgca agtgtgttca 121560
   ccttgctctg gactaagact gaggtccttc attcatgtcc tgtacggttc tacaaatata 121620
   ctatcctcct agtagcatct tattgagtga ttaaaagaaa tgaattccac atgttaattc 121680
   caaccacata tggttgattg tgattatctc aaagccaaaa tattcagtaa attcagtttt 121740
   ttaagagcct tgctttatta ccagcacaaa ttaccagcat ttgtgattgt atagtgccca 121800
   aaatgcaagg actgagactt gacttttttt tatgcccccc agcacactta gcccagtgtg 121860
   aggcatataa tgggccctta gtaaaatacc tgttcatcaa ttgttataga aataacaatt 121920
   tgttaattca tttagcaggt aattattgag cccatcatgt gtgccaggac ctgtggacat 121980
   ccagagattg ataaaataat gacccagacc ttgtagagtt tgcagtttag taaattagat 122040
   gnnnnnnnnn ngaacatctg aacatgacca gagattgtgg tcatattgca atctctgcct 122100
   agatggccat ggccttctaa gttctttaat aaaactgtct acagctccca aggttttata 122160
   aagttgaact gctacagaat ttatagaacc aatcttgggt ttctagctca tgtatcttct 122220
   ccaacttgaa ctccgaaaga aaaagaccat ggctttctta tctgcttatc tttcacacat 122280
   gtacaataga tgctcataga ataatctgtt ctattaatgt aagatctcta tagttatgat 122340
   ttttgtcata ttttgaaatg gttaaattca ggaactgcct gatcagaggt agaaacatat 122400
   tactttgtag tttaggacac ttttacttcc agaagatcac ccataagtga tttcatcttg 122460
   gaccagaata atgagaaaat taatttcatg tgtaatctca tgttcaaata agcatatcaa 122520
   ctggaacaag ttctttctta tttcatctta cataaattga agataactgt atttaatttt 122580
   tttttccaaa actcttcatt ctgacctagg gcaatctcca ccttcagccc tcataatcag 122640
   atctcctctt tctctgatag tgttgatcaa attcttggaa aagggcaaat cacatcagat  122700
   aagaagagcc gagagaaaat aacagcagaa catgagacca cagacgatct cagtatgctc  122760
   ggtcgggtgg tcaaggttga aaaacaggta caactcaact acgctgggta tctttttagc  122820
   cagaattttt ttaatcaaaa tttattatag gcaattgtat gtattccagt gattatttta  122880
   aaataaataa aatcacaaaa agagtgaata aaattaaatt ttctttgtgt gtagacagtg  122940
   ctctctgaaa cactctggat ttccctttaa ggaatagaaa atctcatctc atcaattaat  123000
   agttaattgg aaacaactat agatttccaa tagttgaaaa tcgtttaaaa ataacaaaga  123060
   tctattcttc atttgtgttt cagttgtttt tattgtttct tttttaggtc attaattttt  123120
   tttttttttt tttnnnnnnn nnngggacta tgggcatgca ccaccacacc tggctgattt  123180
   ttgtattatt tttggtagag acgaggtctc accatgttgc ccaggctggt ctcaaactcc  123240
   tgggctcaat tctcctgcct cagtttccca aagtgctggc attacaggca tgagccactg  123300
   cacccagcct ggaatggaaa gtcttaaagt tgcattttta tagtgcttat atgactttta  123360
   agaggcacta tttaaagaag tggtggggga gagatgctaa taaagactgt atagaagcca  123420
   aagtgtccct ctcttcctaa gtcctcctct ggccccatca ttgctaaatg ttacattcac  123480
   taagggtcaa gactgcatcg tactttatat tcaatatttc tttaaatgtg ttggcacacc  123540
   ttgacttcat ttttcaaaat taaaaaatga agtccattct taataacaga ttatactcat  123600
   gtgtaaccat tttcctcact tctaggtaca gtccatagaa tccaagctgg actgcctact  123660
   agacatctat caacaggtcc ttcggaaagg ctctgcctca gccctcgctt tggcttcatt  123720
   ccagatccca ccttttgaat gtgaacagac atctgactat caaagccctg tggatagcaa  123780
   agatctttcg ggttccgcac aaaacagtgg ctgcttatcc agatcaacta gtgccaacat  123840
   ctcgagaggc ctgcagttca ttctgacgcc aaatgagttc agtgcccaga ctttctacgc  123900
   gcttagccct actatgcaca gtcaagcaac acaggtgcca attagtcaaa gcgatggctc  123960
   agcagtggca gccaccaaca ccattgcaaa ccaaataaat acggcaccca agccagcagc  124020
   cccaacaact ttacagatcc cacctcctct cccagccatc aagcatctgc ccaggccaga  124080
   aactctgcac cctaaccctg caggcttaca ggaaagcatt tctgacgtca ccacctgcct  124140
   tgttgcctcc aaggaaaatg ttcaggttgc acagtcaaat ctcaccaagg accgttctat  124200
   gaggaaaagc tttgacatgg gaggagaaac tctgttgtct gtctgtccca tggtgccgaa  124260
   ggacttgggc aaatctttgt ctgtgcaaaa cctgatcagg tcgaccgagg aactgaatat  124320
   acaactttca gggagtgagt caagtggctc cagaggcagc caagattttt accccaaatg  124380
   gagggaatcc aaattgttta taactgatga agaggtgggt cccgaagaga cagagacaga  124440
   cacttttgat gccgcaccgc agcctgccag ggaagctgcc tttgcatcag actctctaag  124500
   gactggaagg tcacgatcat ctcagagcat ttgtaaggca ggagaaagta cagatgccct  124560
   cagcttgcct catgtcaaac tgaaataagt tcttcatttt ctttccaggc atagcagttc  124620
   tttagccata catatcattg catgaactat ttcgaaagcc cttctaaaaa gttgaaattg  124680
   caagaatcgg gaagaacatg aaaggcagtt tataagcccg ttacctttta attgcatgaa  124740
   aatgcatgtt tagggatggc taaaattcca aggtgcatcg acattaaccc actcatttag  124800
   taatgtacct tgagttaaaa agcctgagaa accaaacaca gctaatgcta tggggtgtat  124860
   gaatatgtca agtttaggtc atttagaaga tttgacactg tattttgaaa ttatgggagt  124920
   aaacaccttc aaatttcagg catttctgct ttgtgactaa atacaaacta cattttcaag  124980
   attaggccat aatgtatatt taaacacaat ggctatcaac agctgctaat aaggtatcaa  125040
   ctaaagcaga attggggaat aatagaaatg gctgcttatt tcaagatata tttgccaacc  125100
   cattcctatt cagtcatttt attattaatg taatttgaat gtcaatttgt gtgcttttgg  125160
   tgatttagcg ctgtggcaag caattttgca catcattttc atgttgttct ttatgacaag  125220
   aatgttcttc aattagaaaa tgtgcaaata atgaaattca gggccagtga ggcaaataga  125280
   ctatctgaca tatttgactt tatgaaaaca tattgcctga tggcagaatc aactttataa  125340
   gtggtcaact tctacacaag cgtatgaaat actggtcagt agaacagcca ttgtgattgg  125400
   actggtttct ctgcaatggc gccaacccca ggcttgccaa tactgcctat gtaaagggca  125460
   agtgtgagaa gctattctca tttcgctgac atacaggtag gactatgggg gatgggacat  125520
   ttgagtggga ctgagatagg aaaggcttga aaagaaccca gaaacaccac caggaagttg  125580
   gcaaagtaaa agaaaatgac ttccccctca aagggcaatg agagggagag aaacaaacca  125640
   aaatagaaga actagacttt ttagaaaatg agtattgcta gggaattcaa ctacctaatc  125700
   ttcccttatt cttatatata agcagagaat ttttgcaagg tatttatttt ttaatatgcc  125760
   ctgaatgtct tttgctatta tgtgtacatt ttgcatatga aagtctaaaa cgaaacttcc  125820
   tttacttttt atactgtagt gaaaattttc tattcttccc aagaatgttg tcccaaatct  125880
   gaaattactg gttcaatttc ctgatataaa                                    125910
<210> 2
   <211> 3718
   <212> DNA
   <213> Homo Sapiens
<400> 2
   ctggagtgag gcgcgggaag atgcctggtc cttgcctcgc ggacttggca gccgcgtcct 60
   gcgggtctgt ccactgaact gctgaggact gcggcggtgg cctgagggag agccgccggg 120
   gcaagcaggg ggcccggatg agcctgctgg ggaagccgct ctcttacacg agtagccaga 180
   gctgccggcg caacgtcaag taccggcggg tgcagaacta cctgtacaac gtgctggaga 240
   gaccccgcgg ctgggcgttc atctaccacg ctttcgtttt tctccttgtc tttggttgct 300
   tgattttgtc agtgttttct accatccctg agcacacaaa attggcctca agttgcctct 360
   tgatcctgga gttcgtgatg attgtcgtct ttggtttgga gttcatcatt cgaatctggt 420
   ctgcgggttg ctgttgtcga tatagaggat ggcaaggaag actgaggttt gctcgaaagc 480
   ccttctgtgt tatagatacc attgttctta tcgcttcaat agcagttgtt tctgcaaaaa 540
   ctcagggtaa tatttttgcc acgtctgcac tcagaagtct ccgtttccta cagatcctcc 600
   gcatggtgcg catggaccga aggggaggca cttggaaatt actgggttca gtggtttatg 660
   ctcacagcaa ggaattaatc acagcttggt acataggatt tttggttctt attttttcgt 720
   ctttccttgt ctatctggtg gaaaaggatg ccaataaaga gttttctaca tatgcagatg 780
   ctctctggtg gggcacaatt acattgacaa ctattggcta tggagacaaa actcccctaa 840
   cttggctggg aagattgctt tctgcaggct ttgcactcct tggcatttct ttctttgcac 900
   ttcctgccgg cattcttggc tcaggttttg cattaaaagt acaagaacaa caccgccaga 960
   aacactttga gaaaagaagg aacccagctg ccaacctcat tcagtgtgtt tggcgtagtt 1020
   acgcagctga tgagaaatct gtttccattg caacctggaa gccacacttg aaggccttgc 1080
   acacctgcag ccctaccaag aaagaacaag gggaagcatc aagcagtcag aagctaagtt 1140
   ttaaggagcg agtgcgcatg gctagcccca ggggccagag tattaagagc cgacaagcct 1200
   cagtaggtga caggaggtcc ccaagcaccg acatcacagc cgagggcagt cccaccaaag 1260
   tgcagaagag ctggagcttc aacgaccgaa cccgcttccg gccctcgctg cgcctcaaaa 1320
   gttctcagcc aaaaccagtg atagatgctg acacagccct tggcactgat gatgtatatg 1380
   atgaaaaagg atgccagtgt gatgtatcag tggaagacct caccccacca cttaaaactg 1440
   tcattcgagc tatcagaatt atgaaatttc atgttgcaaa acggaagttt aaggaaacat 1500
   tacgtccata tgatgtaaaa gatgtcattg aacaatattc tgctggtcat ctggacatgt 1560
   tgtgtagaat taaaagcctt caaacacgtg ttgatcaaat tcttggaaaa gggcaaatca 1620
   catcagataa gaagagccga gagaaaataa cagcagaaca tgagaccaca gacgatctca 1680
   gtatgctcgg tcgggtggtc aaggttgaaa aacaggtaca gtccatagaa tccaagctgg 1740
   actgcctact agacatctat caacaggtcc ttcggaaagg ctctgcctca gccctcgctt 1800
   tggcttcatt ccagatccca ccttttgaat gtgaacagac atctgactat caaagccctg 1860
   tggatagcaa agatctttcg ggttccgcac aaaacagtgg ctgcttatcc agatcaacta 1920
   gtgccaacat ctcgagaggc ctgcagttca ttctgacgcc aaatgagttc agtgcccaga 1980
   ctttctacgc gcttagccct actatgcaca gtcaagcaac acaggtgcca attagtcaaa 2040
   gcgatggctc agcagtggca gccaccaaca ccattgcaaa ccaaataaat acggcaccca 2100
   agccagcagc cccaacaact ttacagatcc cacctcctct cccagccatc aagcatctgc 2160
   ccaggccaga aactctgcac cctaaccctg caggcttaca ggaaagcatt tctgacgtca 2220
   ccacctgcct tgttgcctcc aaggaaaatg ttcaggttgc acagtcaaat ctcaccaagg 2280
   accgttctat gaggaaaagc tttgacatgg gaggagaaac tctgttgtct gtctgtccca 2340
   tggtgccgaa ggacttgggc aaatctttgt ctgtgcaaaa cctgatcagg tcgaccgagg 2400
   aactgaatat acaactttca gggagtgagt caagtgcctc cagaggcagc caagattttt 2460
   accccaaatg gagggaatcc aaattgttta taactgatga agaggtgggt cccgaagaga 2520
   cagagacaga cacttttgat gccgcaccgc agcctgccag ggaagctgcc tttgcatcag 2580
   actctctaag gactggaagg tcacgatcat ctcagagcat ttgtaaggca ggagaaagta 2640
   cagatgccct cagcttgcct catgtcaaac tgaaataagt tcttcatttt ctttccaggc 2700
   atagcagttc tttagccata catatcattg catgaactat ttcgaaagcc cttctaaaaa 2760
   gttgaaattg caagaatcgg gaagaacatg aaaggcagtt tataagcccg ttacctttta 2820
   attgcatgaa aatgcatgtt tagggatggc taaaattcca aggtgcatcg acattaaccc 2880
   actcatttag taatgtacct tgagttaaaa agcctgagaa accaaacaca gctaatgcta 2940
   tggggtgtat gaatatgtca agtttaggtc atttagaaga tttgacactg tattttgaaa 3000
   ttatgggagt aaacaccttc aaatttcagg catttctgct ttgtgactaa atacaaacta 3060
   cattttcaag attaggccat aatgtatatt taaacacaat ggctatcaac agctgctaat 3120
   aaggtatcaa ctaaagcaga attggggaat aatagaaatg gctgcttatt tcaagatata 3180
   tttgccaacc cattcctatt cagtcatttt attattaatg taatttgaat gtcaatttgt 3240
   gtgcttttgg tgatttagcg ctgtggcaag caattttgca catcattttc atgttgttct 3300
   ttatgacaag aatgttcttc aattagaaaa tgtgcaaata atgaaattca gggccagtga 3360
   ggcaaataga ctatctgaca tatttgactt tatgaaaaca tattgcctga tggcagaatc 3420
   aactttataa gtggtcaact tctacacaag cgtatgaaat actggtcagt agaacagcca 3480
   ttgtgattgg actggtttct ctgcaatggc gccaacccca ggcttgccaa tactgcctat 3540
   gtaaagggca agtgtgagaa gctattctca tttcgctgac atacaggtag gactatgggg 3600
   gatgggacat ttgagtggga ctgagatagg aaaggcttga aaagaaccca gaaacaccac 3660
   caggaagttg gcaaagtaaa agaaaatgac ttccccctca aagggcaatg agagggag 3718
<210> 3
   <211> 846
   <212> PRT
   <213> Homo Sapiens
<400> 3
   Met Ser Leu Leu Gly Lys Pro Leu Ser Tyr Thr Ser Ser Gln Ser Cys
   1 5 10 15
   Arg Arg Asn Val Lys Tyr Arg Arg Val Gln Asn Tyr Leu Tyr Asn Val
   20 25 30
   Leu Glu Arg Pro Arg Gly Trp Ala Phe Ile Tyr His Ala Phe Val Phe
   35 40 45
   Leu Leu Val Phe Gly Cys Leu Ile Leu Ser Val Phe Ser Thr Ile Pro
   50 55 60
   Glu His Thr Lys Leu Ala Ser Ser Cys Leu Leu Ile Leu Glu Phe Val
   65 70 75 80
   Met Ile Val Val Phe Gly Leu Glu Phe Ile Ile Arg Ile Trp Ser Ala
   85 90 95
   Gly Cys Cys Cys Arg Tyr Arg Gly Trp Gln Gly Arg Leu Arg Phe Ala
   100 105 110
   Arg Lys Pro Phe Cys Val Ile Asp Thr Ile Val Leu Ile Ala Ser Ile
   115 120 125
   Ala Val Val Ser Ala Lys Thr Gln Gly Asn Ile Phe Ala Thr Ser Ala
   130 135 140
   Leu Arg Ser Leu Arg Phe Leu Gln Ile Leu Arg Met Val Arg Met Asp
   145 150 155 160
   Arg Arg Gly Gly Thr Trp Lys Leu Leu Gly Ser Val Val Tyr Ala His
   165 170 175
   Ser Lys Glu Leu Ile Thr Ala Trp Tyr Ile Gly Phe Leu Val Leu Ile
   180 185 190
   Phe Ser Ser Phe Leu Val Tyr Leu Val Glu Lys Asp Ala Asn Lys Glu
   195 200 205
   Phe Ser Thr Tyr Ala Asp Ala Leu Trp Trp Gly Thr Ile Thr Leu Thr
   210 215 220
   Thr Ile Gly Tyr Gly Asp Lys Thr Pro Leu Thr Trp Leu Gly Arg Leu
   225 230 235 240
   Leu Ser Ala Gly Phe Ala Leu Leu Gly Ile Ser Phe Phe Ala Leu Pro
   245 250 255
   Ala Gly Ile Leu Gly Ser Gly Phe Ala Leu Lys Val Gln Glu Gln His
   260 265 270
   Arg Gln Lys His Phe Glu Lys Arg Arg Asn Pro Ala Ala Asn Leu Ile
   275 280 285
   Gln Cys Val Trp Arg Ser Tyr Ala Ala Asp Glu Lys Ser Val Ser Ile
   290 295 300
   Ala Thr Trp Lys Pro His Leu Lys Ala Leu His Thr Cys Ser Pro Thr
   305 310 315 320
   Lys Lys Glu Gln Gly Glu Ala Ser Ser Ser Gln Lys Leu Ser Phe Lys
   325 330 335
   Glu Arg Val Arg Met Ala Ser Pro Arg Gly Gln Ser Ile Lys Ser Arg
   340 345 350
   Gln Ala Ser Val Gly Asp Arg Arg Ser Pro Ser Thr Asp Ile Thr Ala
   355 360 365
   Glu Gly Ser Pro Thr Lys Val Gln Lys Ser Trp Ser Phe Asn Asp Arg
   370 375 380
   Thr Arg Phe Arg Pro Ser Leu Arg Leu Lys Ser Ser Gln Pro Lys Pro
   385 390 395 400
   Val Ile Asp Ala Asp Thr Ala Leu Gly Thr Asp Asp Val Tyr Asp Glu
   405 410 415
   Lys Gly Cys Gln Cys Asp Val Ser Val Glu Asp Leu Thr Pro Pro Leu
   420 425 430
   Lys Thr Val Ile Arg Ala Ile Arg Ile Met Lys Phe His Val Ala Lys
   435 440 445
   Arg Lys Phe Lys Glu Thr Leu Arg Pro Tyr Asp Val Lys Asp Val Ile
   450 455 460
   Glu Gln Tyr Ser Ala Gly His Leu Asp Met Leu Cys Arg Ile Lys Ser
   465 470 475 480
   Leu Gln Thr Arg Val Asp Gln Ile Leu Gly Lys Gly Gln Ile Thr Ser
   485 490 495
   Asp Lys Lys Ser Arg Glu Lys Ile Thr Ala Glu His Glu Thr Thr Asp
   500 505 510
   Asp Leu Ser Met Leu Gly Arg Val Val Lys Val Glu Lys Gln Val Gln
   515 520 525
   Ser Ile Glu Ser Lys Leu Asp Cys Leu Leu Asp Ile Tyr Gln Gln Val
   530 535 540
   Leu Arg Lys Gly Ser Ala Ser Ala Leu Ala Leu Ala Ser Phe Gln Ile
   545 550 555 560
   Pro Pro Phe Glu Cys Glu Gln Thr Ser Asp Tyr Gln Ser Pro Val Asp
   565 570 575
   Ser Lys Asp Leu Ser Gly Ser Ala Gln Asn Ser Gly Cys Leu Ser Arg
   580 585 590
   Ser Thr Ser Ala Asn Ile Ser Arg Gly Leu Gln Phe Ile Leu Thr Pro
   595 600 605
   Asn Glu Phe Ser Ala Gln Thr Phe Tyr Ala Leu Ser Pro Thr Met His
   610 615 620
   Ser Gln Ala Thr Gln Val Pro Ile Ser Gln Ser Asp Gly Ser Ala Val
   625 630 635 640
   Ala Ala Thr Asn Thr Ile Ala Asn Gln Ile Asn Thr Ala Pro Lys Pro
   645 650 655
   Ala Ala Pro Thr Thr Leu Gln Ile Pro Pro Pro Leu Pro Ala Ile Lys
   660 665 670
   His Leu Pro Arg Pro Glu Thr Leu His Pro Asn Pro Ala Gly Leu Gln
   675 680 685
   Glu Ser Ile Ser Asp Val Thr Thr Cys Leu Val Ala Ser Lys Glu Asn
   690 695 700
   Val Gln Val Ala Gln Ser Asn Leu Thr Lys Asp Arg Ser Met Arg Lys
   705 710 715 720
   Ser Phe Asp Met Gly Gly Glu Thr Leu Leu Ser Val Cys Pro Met Val
   725 730 735
   Pro Lys Asp Leu Gly Lys Ser Leu Ser Val Gln Asn Leu Ile Arg Ser
   740 745 750
   Thr Glu Glu Leu Asn Ile Gln Leu Ser Gly Ser Glu Ser Ser Ala Ser
   755 760 765
   Arg Gly Ser Gln Asp Phe Tyr Pro Lys Trp Arg Glu Ser Lys Leu Phe
   770 775 780
   Ile Thr Asp Glu Glu Val Gly Pro Glu Glu Thr Glu Thr Asp Thr Phe
   785 790 795 800
   Asp Ala Ala Pro Gln Pro Ala Arg Glu Ala Ala Phe Ala Ser Asp Ser
   805 810 815
   Leu Arg Thr Gly Arg Ser Arg Ser Ser Gln Ser Ile Cys Lys Ala Gly
   820 825 830
   Glu Ser Thr Asp Ala Leu Ser Leu Pro His Val Lys Leu Lys
   835 840 845
<210> 4
   <211> 695
   <212> PRT
   <213> Homo Sapiens
<400> 4
<210> 5
   <211> 423
   <212> PRT
   <213> Homo Sapiens
<400> 5
   Ser Leu Leu Gly Pro Leu Ser Ser Ser Cys Arg Lys Tyr Arg Arg Gln
   1 5 10 15
   Asn Tyr Asn Val Leu Glu Arg Pro Arg Gly Trp Ala Phe Tyr His Phe
   20 25 30
   Phe Leu Leu Val Phe Cys Leu Leu Ser Val Ser Thr Ile Glu His Leu
   35 40 45
   Ala Cys Leu Leu Ile Leu Glu Phe Val Met Ile Val Val Phe Gly Leu
   50 55 60
   Glu Ile Arg Trp Ser Ala Gly Cys Cys Cys Arg Tyr Arg Gly Trp Gln
   65 70 75 80
   Gly Arg Arg Phe Ala Arg Lys Pro Phe Cys Val Ile Asp Ile Val Ala
   85 90 95
   Ser Ala Val Ala Thr Gln Gly Asn Ile Phe Ala Thr Ser Ala Leu Arg
   100 105 110
   Ser Arg Phe Leu Gln Ile Leu Arg Met Val Arg Met Asp Arg Arg Gly
   115 120 125
   Gly Thr Trp Lys Leu Leu Gly Ser Val Val Tyr Ala His Ser Lys Glu
   130 135 140
   Leu Ile Thr Ala Trp Tyr Ile Gly Phe Leu Val Leu Ile Phe Ser Phe
   145 150 155 160
   Leu Val Tyr Leu Glu Lys Asp Ala Asn Phe Ser Tyr Ala Asp Leu Trp
   165 170 175
   Trp Gly Thr Ile Thr Leu Thr Thr Ile Gly Tyr Gly Asp Lys Thr Pro
   180 185 190
   Thr Trp Leu Gly Arg Leu Ala Gly Phe Ala Leu Leu Gly Ile Ser Phe
   195 200 205
   Phe Ala Leu Pro Ala Gly Ile Leu Gly Ser Gly Phe Ala Leu Lys Val
   210 215 220
   Gln Glu Gln His Arg Gln Lys His Phe Glu Lys Arg Arg Pro Ala Ala
   225 230 235 240
   Asn Leu Ile Gln Trp Arg Tyr Asp Ser Ala Thr Trp Leu Ala Leu Thr
   245 250 255
   Cys Pro Thr Gly Glu Ser Ser Lys Arg Arg Met Ser Arg Gly Ser Lys
   260 265 270
   Ala Ser Pro Ser Glu Ser Pro Thr Lys Val Gln Lys Ser Trp Ser Phe
   275 280 285
   Asn Asp Arg Thr Arg Phe Arg Ser Leu Arg Leu Lys Pro Ala Ala Val
   290 295 300
   Glu Lys Gln Cys Val Asp Pro Lys Thr Val Ile Arg Ile Arg Ile Lys
   305 310 315 320
   Phe Val Ala Lys Arg Lys Phe Lys Glu Thr Leu Arg Pro Tyr Asp Val
   325 330 335
   Lys Asp Val Ile Glu Gln Tyr Ser Ala Gly His Leu Asp Met Leu Arg
   340 345 350
   Ile Lys Ser Leu Gln Thr Arg Val Asp Gln Ile Gly Gly Asp Lys Arg
   355 360 365
   Glu Lys Glu Asp Ser Met Gly Arg Val Val Lys Val Glu Lys Gln Val
   370 375 380
   Gln Ser Ile Glu Lys Leu Asp Leu Leu Tyr Leu Arg Gly Ser Ala Ser
   385 390 395 400
   Leu Gln Pro Phe Thr Ser Asp Tyr Ser Pro Val Asp Asp Ser Ser Ala
   405 410 415
   Gln Ser Ser Arg Ser Ser Asn
   420
<210> 6
   <211> 514
   <212> PRT
   <213> Homo Sapiens
<400> 6
   Met Val Gln Lys Ser Arg Asn Gly Gly Val Tyr Pro Gly Pro Ser Gly
    1               5                  10                  15
   Glu Lys Lys Leu Lys Val Gly Phe Val Gly Leu Asp Pro Gly Ala Pro
               20                  25                  30
   Asp Ser Thr Arg Asp Gly Ala Leu Leu Ile Ala Gly Ser Glu Ala Pro
           35                  40                  45
   Lys Arg Gly Ser Ile Leu Ser Lys Pro Arg Ala Gly Gly Ala Gly Ala
       50                  55                  60
   Gly Lys Pro Pro Lys Arg Asn Ala Phe Tyr Arg Lys Leu Gln Asn Phe
   65                  70                  75                  80
   Leu Tyr Asn Val Leu Glu Arg Pro Arg Gly Trp Ala Phe Ile Tyr His
                   85                  90                  95
   Ala Tyr Val Phe Leu Leu Val Phe Ser Cys Leu Val Leu Ser Val Phe
               100                 105                 110
   Ser Thr Ile Lys Glu Tyr Glu Lys Ser Ser Glu Gly Ala Leu Tyr Ile
           115                 120                 125
   Leu Glu Ile Val Thr Ile Val Val Phe Gly Val Glu Tyr Phe Val Arg
       130                 135                 140
   Ile Trp Ala Ala Gly Cys Cys Cys Arg Tyr Arg Gly Trp Arg Gly Arg
   145                 150                 155                 160
   Leu Lys Phe Ala Arg Lys Pro Phe Cys Val Ile Asp Ile Met Val Leu
                   165                 170                 175
   Ile Ala Ser Ile Ala Val Leu Ala Ala Gly Ser Gln Gly Asn Val Phe
               180                 185                 190
   Ala Thr Ser Ala Leu Arg Ser Leu Arg Phe Leu Gln Ile Leu Arg Met
           195                 200                 205
   Ile Arg Met Asp Arg Arg Gly Gly Thr Trp Lys Leu Leu Gly Ser Val
       210                 215                 220
   Val Tyr Ala His Ser Lys Glu Leu Val Thr Ala Trp Tyr Ile Gly Phe
   225                 230                 235                 240
   Leu Cys Leu Ile Leu Ala Ser Phe Leu Val Tyr Leu Ala Glu Lys Gly
                   245                 250                 255
   Glu Asn Asp His Phe Asp Thr Tyr Ala Asp Ala Leu Trp Trp Gly Leu
               260                 265                 270
   Ile Thr Leu Thr Thr Ile Gly Tyr Gly Asp Lys Tyr Pro Gln Thr Trp
           275                 280                 285
   Asn Gly Arg Leu Leu Ala Ala Thr Phe Thr Leu Ile Gly Val Ser Phe
       290                 295                 300
   Phe Ala Leu Pro Ala Gly Ile Leu Gly Ser Gly Phe Ala Leu Lys Val
   305                 310                 315                 320
   Gln Glu Gln His Arg Gln Lys His Phe Glu Lys Arg Arg Asn Pro Ala
                   325                 330                 335
   Ala Gly Leu Ile Gln Ser Ala Trp Arg Phe Tyr Ala Thr Asn Leu Ser
               340                 345                 350
   Arg Thr Asp Leu His Ser Thr Trp Gln Tyr Tyr Glu Arg Thr Val Thr
           355                 360                 365
   Val Pro Met Tyr Arg Leu Ile Pro Pro Leu Asn Gln Leu Glu Leu Leu
       370                 375                 380
   Arg Asn Leu Lys Ser Lys Ser Gly Leu Ala Phe Arg Lys Asp Pro Pro
   385                 390                 395                 400
   Pro Glu Pro Ser Pro Ser Gln Lys Val Ser Leu Lys Asp Arg Val Phe
                   405                 410                 415
   Ser Ser Pro Arg Gly Val Ala Ala Lys Gly Lys Gly Ser Pro Gln Ala
               420                 425                 430
   Gln Thr Val Arg Arg Ser Pro Ser Ala Asp Gln Ser Leu Glu Asp Ser
           435                 440                 445
   Pro Ser Lys Val Pro Lys Ser Trp Ser Phe Gly Asp Arg Ser Arg Ala
       450                 455                 460
   Arg Gln Ala Phe Arg Ile Lys Gly Ala Ala Ser Arg Gln Asn Ser Glu
   465                 470                 475                 480
   Glu Ala Ser Leu Pro Gly Glu Asp Ile Val Asp Asp Lys Ser Cys Pro
                   485                 490                 495
   Cys Glu Phe Val Thr Glu Asp Leu Thr Pro Gly Leu Lys Val Ser Ile
               500                 505                 510
   Arg Ala
<210> 7
   <211> 521
   <212> PRT
   <213> Homo Sapiens
<400> 7
   Met Gly Leu Lys Ala Arg Arg Ala Ala Gly Ala Ala Gly Gly Gly Gly
    1               5                  10                  15
   Asp Gly Gly Gly Gly Gly Gly Gly Ala Ala Asn Pro Ala Gly Gly Asp
               20                  25                  30
   Ala Ala Ala Ala Gly Asp Glu Glu Arg Lys Val Gly Leu Ala Pro Gly
           35                  40                  45
   Asp Val Glu Gln Val Thr Leu Ala Leu Gly Ala Gly Ala Asp Lys Asp
       50                  55                  60
   Gly Thr Leu Leu Leu Glu Gly Gly Gly Arg Asp Glu Gly Gln Arg Arg
   65                  70                  75                  80
   Thr Pro Gln Gly Ile Gly Leu Leu Ala Lys Thr Pro Leu Ser Arg Pro
                   85                  90                  95
   Val Lys Arg Asn Asn Ala Lys Tyr Arg Arg Ile Gln Thr Leu Ile Tyr
                   100                 105                 110
   Asp Ala Leu Glu Arg Pro Arg Gly Trp Ala Leu Leu Tyr His Ala Leu
           115                 120                 125
   Val Phe Leu Ile Val Leu Gly Cys Leu Ile Leu Ala Val Leu Thr Thr
       130                 135                 140
   Phe Lys Glu Tyr Glu Thr Val Ser Gly Asp Trp Leu Leu Leu Leu Glu
   145                 150                 155                 160
   Thr Phe Ala Ile Phe Ile Phe Gly Ala Glu Phe Ala Leu Arg Ile Trp
                   165                 170                 175
   Ala Ala Gly Cys Cys Cys Arg Tyr Lys Gly Trp Arg Gly Arg Leu Lys
               180                 185                 190
   Phe Ala Arg Lys Pro Leu Cys Met Leu Asp Ile Phe Val Leu Ile Ala
           195                 200                 205
   Ser Val Pro Val Val Ala Val Gly Asn Gln Gly Asn Val Leu Ala Thr
       210                 215                 220
   Ser Leu Arg Ser Leu Arg Phe Leu Gln Ile Leu Arg Met Leu Arg Met
   225                 230                 235                 240
   Asp Arg Arg Gly Gly Thr Trp Lys Leu Leu Gly Ser Ala Ile Cys Ala
                   245                 250                 255
   His Ser Lys Glu Leu Ile Thr Ala Trp Tyr Ile Gly Phe Leu Thr Leu
               260                 265                 270
   Ile Leu Ser Ser Phe Leu Val Tyr Leu Val Glu Lys Asp Val Pro Glu
           275                 280                 285
   Val Asp Ala Gln Gly Glu Glu Met Lys Glu Glu Phe Glu Thr Tyr Ala
       290                 295                 300
   Asp Ala Leu Trp Trp Gly Leu Ile Thr Leu Ala Thr Ile Gly Tyr Gly
   305                 310                 315                 320
   Asp Lys Thr Pro Lys Thr Trp Glu Gly Arg Leu Ile Ala Ala Thr Phe
                   325                 330                 335
   Ser Leu Ile Gly Val Ser Phe Phe Ala Leu Pro Ala Gly Ile Leu Gly
               340                 345                 350
   Ser Gly Leu Ala Leu Lys Val Gln Glu Gln His Arg Gln Lys His Phe
           355                 360                 365
   Glu Lys Arg Arg Lys Pro Ala Ala Glu Leu Ile Gln Ala Ala Trp Arg
       370                 375                 380
   Tyr Tyr Ala Thr Asn Pro Asn Arg Ile Asp Leu Val Ala Thr Trp Arg
   385                 390                 395                 400
   Phe Tyr Glu Ser Val Val Ser Phe Pro Phe Phe Arg Lys Glu Gln Leu
                   405                 410                 415
   Glu Ala Ala Ser Ser Gln Lys Leu Gly Leu Leu Asp Arg Val Arg Leu
               420                 425                 430
   Ser Asn Pro Arg Gly Ser Asn Thr Lys Gly Lys Leu Phe Thr Pro Leu
           435                 440                 445
   Asn Val Asp Ala Ile Glu Glu Ser Pro Ser Lys Glu Pro Lys Pro Val
       450                 455                 460
   Gly Leu Asn Asn Lys Glu Arg Phe Arg Thr Ala Phe Arg Met Lys Ala
   465                 470                 475                 480
   Tyr Ala Phe Trp Gln Ser Ser Glu Asp Ala Gly Thr Gly Asp Pro Met
                   485                 490                 495
   Ala Glu Asp Arg Gly Tyr Gly Asn Asp Phe Pro Ile Glu Asp Met Ile
               500                 505                 510
   Pro Thr Leu Lys Ala Ala Ile Arg Ala
           515                 520
<210> 8
   <211> 142
   <212> PRT
   <213> Homo Sapiens
<400> 8
   Arg Gln Tyr Leu Glu Arg Pro Gly Trp Tyr His Phe Leu Val Cys Leu
   1               5                   10                  15
   Val Thr Leu Glu Phe Gly Glu Arg Trp Ala Gly Cys Tyr Gly Gly Arg
               20                  25                  30
   Phe Ala Arg Lys Pro Asp Val Ala Ser Val Gly Ala Thr Ser Arg Arg
           35                  40                  45
   Phe Leu Gln Ile Leu Arg Met Asp Arg Gly Gly Thr Trp Leu Leu Gly
       50                  55                  60
   Ser His Glu Leu Thr Tyr Ile Gly Phe Leu Leu Ile Ser Val Tyr Leu
   65                  70                  75                  80
   Glu Lys Phe Tyr Ala Asp Leu Trp Trp Gly Thr Thr Ile Gly Tyr Gly
                   85                  90                  95
   Asp Lys Pro Thr Trp Gly Phe Ser Phe Phe Ala Leu Pro Ala Gly Ile
               100                 105                 110
   Leu Gly Ser Gly Ala Leu Lys Val Gln Arg Gln Lys His Phe Ala Ala
           115                 120                 125
   Leu Ile Gln Trp Arg Tyr Thr Trp Leu Ser Lys Glu Ala Ile
       130                 135                 140
<210> 9
   <211> 17
   <212> DNA
   <213> Homo Sapiens
<400> 9
   gggggcccgg atgagcc 17
<210> 10
   <211> 21
   <212> DNA
   <213> Homo Sapiens
<400> 10
   gaagaactta tttcagtttg a 21
<210> 11
   <211> 25
   <212> DNA
   <213> Homo Sapiens
<400> 11
   ttttctcctt gtctttggtt gcttg 25
<210> 12
   <211> 27
   <212> DNA
   <213> Homo Sapiens
<400> 12
   ccatcctaat acgactcact atagggc 27
<210> 13
   <211> 23
   <212> DNA
   <213> Homo Sapiens
<400> 13
   cctcaagttg cctcttgatc ctg 23
<210> 14
   <211> 23
   <212> DNA
   <213> Homo Sapiens
<400> 14
   actcactata gggctcgagc ggc 23
<210> 15
   <211> 23
   <212> DNA
   <213> Homo Sapiens
<400> 15
   caggatcaag aggcaacttg agg 23
<210> 16
   <211> 28
   <212> DNA
   <213> Homo Sapiens
<400> 16
   ccaattttgt gtgctcaggg atggtaga 28
<210> 17
   <211> 18
   <212> DNA
   <213> Homo Sapiens
<400> 17
   gacacagccc ttggcact 18
<210> 18
   <211> 26
   <212> DNA
   <213> Homo Sapiens
<400> 18
   gatgatgtat atgatgaaaa aggatg 26
<210> 19
   <211> 23
   <212> DNA
   <213> Homo Sapiens
<400> 19
   ctgatagctc gaatgacagt ttt 23
<210> 20
   <211> 26
   <212> DNA
   <213> Homo Sapiens
<400> 20
   aagtggtggg gtgaggtctt ccactg 26
<210> 21
   <211> 24
   <212> DNA
   <213> Homo Sapiens
<400> 21
   agaattatga aatttcatgt tgca 24
<210> 22
   <211> 23
   <212> DNA
   <213> Homo Sapiens
<400> 22
   aaacggaagt ttaaggaaac att 23
<210> 23
   <211> 23
   <212> DNA
   <213> Homo Sapiens
<400> 23
   acgtgtttgt tggcttttaa ttc 23
<210> 24
   <211> 21
   <212> DNA
   <213> Homo Sapiens
<400> 24
   tacacaacat gtccagatga c 21
<210> 25
   <211> 22
   <212> DNA
   <213> Homo Sapiens
<400> 25
   tgatcaaatt cttggaaaag gg 22
<210> 26
   <211> 23
   <212> DNA
   <213> Homo Sapiens
<400> 26
   tcacatcaga taagaagagc cga 23
<210> 27
   <211> 21
   <212> DNA
   <213> Homo Sapiens
<400> 27
   gtttttcaac cttgaccacc c 21
<210> 28
   <211> 23
   <212> DNA
   <213> Homo Sapiens
<400> 28
   agcatactga gatcgtctgt ggt 23
<210> 29
   <211> 25
   <212> DNA
   <213> Homo Sapiens
<400> 29
   aattccaaaa gtgtctgtct ctgtc 25
<210> 30
   <211> 22
   <212> DNA
   <213> Homo Sapiens
<400> 30
   ggacccacct cttcatcagt ta 22
<210> 31
   <211> 20
   <212> DNA
   <213> Homo Sapiens
<400> 31
   ggaagactga ggtttgctcg 20
<210> 32
   <211> 20
   <212> DNA
   <213> Homo Sapiens
<400> 32
   ggcaggaagt gcaaagaaag 20
<210> 33
   <211> 20
   <212> DNA
   <213> Homo Sapiens
<400> 33
   ggggatgtgc tgcaaggcga 20
<210> 34
   <211> 25
   <212> DNA
   <213> Homo Sapiens
<400> 34
   cccaggcttt acactttatg cttcc 25
<210> 35
   <211> 22
   <212> DNA
   <213> Homo Sapiens
<400> 35
   ccagggtttt cccagtcacg ac 22
<210> 36
   <211> 25
   <212> DNA
   <213> Homo Sapiens
<400> 36
   ttgtgtggaa ttgtgagcgg ataac 25
<210> 37
   <211> 20
   <212> DNA
   <213> Homo Sapiens
<400> 37 20
   ctttctttgc acttcctgcc 20
<210> 38
   <211> 20
   <212> DNA
   <213> Homo Sapiens
<400> 38 20
   aacacagaag ggctttcgag 20
<210> 39
   <211> 20
   <212> DNA
   <213> Homo Sapiens
<400> 39 20
   cagtcttcct tgccatcctc 20
<210> 40
   <211> 20
   <212> DNA
   <213> Homo Sapiens
<400> 40 20
   gcagccccaa caactttaca 20
<210> 41
   <211> 20
   <212> DNA
   <213> Homo Sapiens
<400> 41 20
   cattttcctt ggaggcaaca 20
<210> 42
   <211> 20
   <212> DNA
   <213> Homo Sapiens
<400> 42 20
   gccagaaact ctgcacccta 20
<210> 43
   <211> 421
   <212> PRT
   <213> Homo Sapiens
<400> 43
   Met Glu Thr Arg Gly Ser Arg Leu Thr Gly Gly Gln Gly Arg Val Tyr
    1               5                  10                  15
   Asn Phe Leu Glu Arg Pro Thr Gly Trp Lys Cys Phe Val Tyr His Phe
               20                  25                  30
   Ala Val Phe Leu Ile Val Leu Val Cys Leu Ile Phe Ser Val Leu Ser
           35                  40                  45
   Thr Ile Glu Gln Tyr Ala Ala Leu Ala Thr Gly Thr Leu Phe Trp Met
       50                  55                  60
   Glu Ile Val Leu Val Val Phe Phe Gly Thr Glu Tyr Val Val Arg Leu
   65 70 75 80
   Trp Ser Ala Gly Cys Arg Ser Lys Tyr Val Gly Leu Trp Gly Arg Leu
   85 90 95
   Arg Phe Ala Arg Lys Pro Ile Ser Ile Ile Asp Leu Ile Val Val Val
   100 105 110
   Ala Ser Met Val Val Leu Cys Val Gly Ser Lys Gly Gln Val Phe Ala
   115 120 125
   Thr Ser Ala Ile Arg Gly Ile Arg Phe Leu Gln Ile Leu Arg Met Leu
   130 135 140
   His Val Asp Arg Gln Gly Gly Thr Trp Arg Leu Leu Gly Ser Val Val
   145 150 155 160
   Phe Ile His Arg Gln Glu Leu Ile Thr Thr Leu Tyr Ile Gly Phe Leu
   165 170 175
   Gly Leu Ile Phe Ser Ser Tyr Phe Val Tyr Leu Ala Glu Lys Asp Ala
   180 185 190
   Val Asn Glu Ser Gly Arg Val Glu Phe Gly Ser Tyr Ala Asp Ala Leu
   195 200 205
   Trp Trp Gly Val Val Thr Val Thr Thr Ile Gly Tyr Gly Asp Lys Val
   210 215 220
   Pro Gln Thr Trp Val Gly Lys Thr Ile Ala Ser Cys Phe Ser Val Phe
   225 230 235 240
   Ala Ile Ser Phe Phe Ala Leu Pro Ala Gly Ile Leu Gly Ser Gly Phe
   245 250 255
   Ala Leu Lys Val Gln Gln Lys Gln Arg Gln Lys His Phe Asn Arg Gln
   260 265 270
   Ile Pro Ala Ala Ala Ser Leu Ile Gln Thr Ala Trp Arg Cys Tyr Ala
   275 280 285
   Ala Glu Asn Pro Asp Ser Ser Thr Trp Lys Ile Tyr Ile Arg Lys Ala
   290 295 300
   Pro Arg Ser His Thr Leu Leu Ser Pro Ser Pro Lys Pro Lys Lys Ser
   305 310 315 320
   Val Val Val Lys Lys Lys Lys Phe Lys Leu Asp Lys Asp Asn Gly Val
   325 330 335
   Thr Pro Gly Glu Lys Met Leu Thr Val Pro His Ile Thr Cys Asp Pro
   340 345 350
   Pro Glu Glu Arg Arg Leu Asp His Phe Ser Val Asp Gly Tyr Asp Ser
   355 360 365
   Ser Val Arg Lys Ser Pro Thr Leu Leu Glu Val Ser Met Pro His Phe
   370 375 380
   Met Arg Thr Asn Ser Phe Ala Glu Asp Leu Asp Leu Glu Gly Glu Thr
   385 390 395 400
   Leu Leu Thr Pro Ile Thr His Ile Ser Gln Leu Arg Glu His His Arg
   405 410 415
   Ala Thr Ile Lys Val
   420

### SEQUENCE LISTING

<110> Merck & Co., Inc.
<120> Novel Human Voltage-Gated Potassium Channel
<130> 20430 PCT
<140> US00/09587
   <141> 2000-04-10
<150> 69/129,274
   <151> 1999-04-14
<160> 43
<170> FastSEQ for Windows Version 4.0
<210> 1
   <211> 125910
   <212> DNA
   <213> Homo Sapiens
<220>
   <221> misc_feature
   <222> (1)...(125910)
   <223> n = A,T,C or G
<400> 1
<210> 2
   <211> 3718
   <212> DNA
   <213> Homo Sapiens
<400> 2
   ctggagtgag gcgcgggaag atgcctggtc cttgcctcgc ggacttggca gccgcgtcct     60
   gcgggtctgt ccactgaact gctgaggact gcggcggtgg cctgagggag agccgccggg    120
   gcaagcaggg ggcccggatg agcctgctgg ggaagccgct ctcttacacg agtagccaga    180
   gctgccggcg caacgtcaag taccggcggg tgcagaacta cctgtacaac gtgctggaga    240
   gaccccgcgg ctgggcgttc atctaccacg ctttcgtttt tctccttgtc tttggttgct    300
   tgattttgtc agtgttttct accatccctg agcacacaaa attggcctca agttgcctct    360
   tgatcctgga gttcgtgatg attgtcgtct ttggtttgga gttcatcatt cgaatctggt    420
   ctgcgggttg ctgttgtcga tatagaggat ggcaaggaag actgaggttt gctcgaaagc    480
   ccttctgtgt tatagatacc attgttctta tcgcttcaat agcagttgtt tctgcaaaaa    540
   ctcagggtaa tatttttgcc acgtctgcac tcagaagtct ccgtttccta cagatcctcc    600
   gcatggtgcg catggaccga aggggaggca cttggaaatt actgggttca gtggtttatg    660
   ctcacagcaa ggaattaatc acagcttggt acataggatt tttggttctt attttttcgt     720
   ctttccttgt ctatctggtg gaaaaggatg ccaataaaga gttttctaca tatgcagatg    780
   ctctctggtg gggcacaatt acattgacaa ctattggcta tggagacaaa actcccctaa    840
   cttggctggg aagattgctt tctgcaggct ttgcactcct tggcatttct ttctttgcac    900
   ttcctgccgg cattcttggc tcaggttttg cattaaaagt acaagaacaa caccgccaga    960
   aacactttga gaaaagaagg aacccagctg ccaacctcat tcagtgtgtt tggcgtagtt   1020
   acgcagctga tgagaaatct gtttccattg caacctggaa gccacacttg aaggccttgc   1080
   acacctgcag ccctaccaag aaagaacaag gggaagcatc aagcagtcag aagctaagtt    1140
   ttaaggagcg agtgcgcatg gctagcccca ggggccagag tattaagagc cgacaagcct   1200
   cagtaggtga caggaggtcc ccaagcaccg acatcacagc cgagggcagt cccaccaaag   1260
   tgcagaagag ctggagcttc aacgaccgaa cccgcttccg gccctcgctg cgcctcaaaa   1320
   gttctcagcc aaaaccagtg atagatgctg acacagccct tggcactgat gatgtatatg   1380
   atgaaaaagg atgccagtgt gatgtatcag tggaagacct caccccacca cttaaaactg  1440
   tcattcgagc tatcagaatt atgaaatttc atgttgcaaa acggaagttt aaggaaacat   1500
   tacgtccata tgatgtaaaa gatgtcattg aacaatattc tgctggtcat ctggacatgt   1560
   tgtgtagaat taaaagcctt caaacacgtg ttgatcaaat tcttggaaaa gggcaaatca   1620
   catcagataa gaagagccga gagaaaataa cagcagaaca tgagaccaca gacgatctca   1680
   gtatgctcgg tcgggtggtc aaggttgaaa aacaggtaca gtccatagaa tccaagctgg   1740
   actgcctact agacatctat caacaggtcc ttcggaaagg ctctgcctca gccctcgctt   1800
   tggcttcatt ccagatccca ccttttgaat gtgaacagac atctgactat caaagccctg   1860
   tggatagcaa agatctttcg ggttccgcac aaaacagtgg ctgcttatcc agatcaacta   1920
   gtgccaacat ctcgagaggc ctgcagttca ttctgacgcc aaatgagttc agtgcccaga   1980
   ctttctacgc gcttagccct actatgcaca gtcaagcaac acaggtgcca attagtcaaa   2040
   gcgatggctc agcagtggca gccaccaaca ccattgcaaa ccaaataaat acggcaccca   2100
   agccagcagc cccaacaact ttacagatcc cacctcctct cccagccatc aagcatctgc   2160
   ccaggccaga aactctgcac cctaaccctg caggcttaca ggaaagcatt tctgacgtca   2220
   ccacctgcct tgttgcctcc aaggaaaatg ttcaggttgc acagtcaaat ctcaccaagg   2280
   accgttctat gaggaaaagc tttgacatgg gaggagaaac tctgttgtct gtctgtccca   2340
   tggtgccgaa ggacttgggc aaatctttgt ctgtgcaaaa cctgatcagg tcgaccgagg   2400
   aactgaatat acaactttca gggagtgagt caagtgcctc cagaggcagc caagattttt   2460
   accccaaatg gagggaatcc aaattgttta taactgatga agaggtgggt cccgaagaga   2520
   cagagacaga cacttttgat gccgcaccgc agcctgccag ggaagctgcc tttgcatcag   2580
   actctctaag gactggaagg tcacgatcat ctcagagcat ttgtaaggca ggagaaagta   2640
   cagatgccct cagcttgcct catgtcaaac tgaaataagt tcttcatttt ctttccaggc   2700
   atagcagttc tttagccata catatcattg catgaactat ttcgaaagcc cttctaaaaa   2760
   gttgaaattg caagaatcgg gaagaacatg aaaggcagtt tataagcccg ttacctttta   2820
   attgcatgaa aatgcatgtt tagggatggc taaaattcca aggtgcatcg acattaaccc   2880
   actcatttag taatgtacct tgagttaaaa agcctgagaa accaaacaca gctaatgcta   2940
   tggggtgtat gaatatgtca agtttaggtc atttagaaga tttgacactg tattttgaaa   3000
   ttatgggagt aaacaccttc aaatttcagg catttctgct ttgtgactaa atacaaacta   3060
   cattttcaag attaggccat aatgtatatt taaacacaat ggctatcaac agctgctaat   3120
   aaggtatcaa ctaaagcaga attggggaat aatagaaatg gctgcttatt tcaagatata   3180
   tttgccaacc cattcctatt cagtcatttt attattaatg taatttgaat gtcaatttgt   3240
   gtgcttttgg tgatttagcg ctgtggcaag caattttgca catcattttc atgttgttct    3300
   ttatgacaag aatgttcttc aattagaaaa tgtgcaaata atgaaattca gggccagtga   3360
   ggcaaataga ctatctgaca tatttgactt tatgaaaaca tattgcctga tggcagaatc   3420
   aactttataa gtggtcaact tctacacaag cgtatgaaat actggtcagt agaacagcca   3480
   ttgtgattgg actggtttct ctgcaatggc gccaacccca ggcttgccaa tactgcctat   3540
   gtaaagggca agtgtgagaa gctattctca tttcgctgac atacaggtag gactatgggg   3600
   gatgggacat ttgagtggga ctgagatagg aaaggcttga aaagaaccca gaaacaccac    3660
   caggaagttg gcaaagtaaa agaaaatgac ttccccctca aagggcaatg agagggag       3718
<210> 3
   <211> 846
   <212> PRT
   <213> Homo Sapiens
<400> 3
   Met Ser Leu Leu Gly Lys Pro Leu Ser Tyr Thr Ser Ser Gln Ser Cys
    1               5                  10                  15
   Arg Arg Asn Val Lys Tyr Arg Arg Val Gln Asn Tyr Leu Tyr Asn Val
               20                  25                  30
   Leu Glu Arg Pro Arg Gly Trp Ala Phe Ile Tyr His Ala Phe Val Phe
           35                  40                  45
   Leu Leu Val Phe Gly Cys Leu Ile Leu Ser Val Phe Ser Thr Ile Pro
       50                  55                  60
   Glu His Thr Lys Leu Ala Ser Ser Cys Leu Leu Ile Leu Glu Phe Val
   65                  70                  75                  80
   Met Ile Val Val Phe Gly Leu Glu Phe Ile Ile Arg Ile Trp Ser Ala
                   85                  90                  95
   Gly Cys Cys Cys Arg Tyr Arg Gly Trp Gln Gly Arg Leu Arg Phe Ala
               100                 105                 110
   Arg Lys Pro Phe Cys Val Ile Asp Thr Ile Val Leu Ile Ala Ser Ile
           115                 120                 125
   Ala Val Val Ser Ala Lys Thr Gln Gly Asn Ile Phe Ala Thr Ser Ala
       130                 135                 140
   Leu Arg Ser Leu Arg Phe Leu Gln Ile Leu Arg Met Val Arg Met Asp
   145                 150                 155                 160
   Arg Arg Gly Gly Thr Trp Lys Leu Leu Gly Ser Val Val Tyr Ala His
                   165                 170                 175
   Ser Lys Glu Leu Ile Thr Ala Trp Tyr Ile Gly Phe Leu Val Leu Ile
               180                 185                 190
   Phe Ser Ser Phe Leu Val Tyr Leu Val Glu Lys Asp Ala Asn Lys Glu
           195                 200                 205
   Phe Ser Thr Tyr Ala Asp Ala Leu Trp Trp Gly Thr Ile Thr Leu Thr
       210                 215                 220
   Thr Ile Gly Tyr Gly Asp Lys Thr Pro Leu Thr Trp Leu Gly Arg Leu
   225                 230                 235                 240
   Leu Ser Ala Gly Phe Ala Leu Leu Gly Ile Ser Phe Phe Ala Leu Pro
                   245                 250                 255
   Ala Gly Ile Leu Gly Ser Gly Phe Ala Leu Lys Val Gln Glu Gln His
               260                 265                 270
   Arg Gln Lys His Phe Glu Lys Arg Arg Asn Pro Ala Ala Asn Leu Ile
           275                 280                 285
   Gln Cys Val Trp Arg Ser Tyr Ala Ala Asp Glu Lys Ser Val Ser Ile
       290                 295                 300
   Ala Thr Trp Lys Pro His Leu Lys Ala Leu His Thr Cys Ser Pro Thr
   305                 310                 315                 320
   Lys Lys Glu Gln Gly Glu Ala Ser Ser Ser Gln Lys Leu Ser Phe Lys
                   325                 330                 335
   Glu Arg Val Arg Met Ala Ser Pro Arg Gly Gln Ser Ile Lys Ser Arg
               340                 345                 350
   Gln Ala Ser Val Gly Asp Arg Arg Ser Pro Ser Thr Asp Ile Thr Ala
           355                 360                 365
   Glu Gly Ser Pro Thr Lys Val Gln Lys Ser Trp Ser Phe Asn Asp Arg
       370                 375                 380
   Thr Arg Phe Arg Pro Ser Leu Arg Leu Lys Ser Ser Gln Pro Lys Pro
   385                 390                 395                 400
   Val Ile Asp Ala Asp Thr Ala Leu Gly Thr Asp Asp Val Tyr Asp Glu
                   405                 410                 415
   Lys Gly Cys Gln Cys Asp Val Ser Val Glu Asp Leu Thr Pro Pro Leu
               420                 425                 430
   Lys Thr Val Ile Arg Ala Ile Arg Ile Met Lys Phe His Val Ala Lys
           435                 440                 445
   Arg Lys Phe Lys Glu Thr Leu Arg Pro Tyr Asp Val Lys Asp Val Ile
       450                 455                 460
   Glu Gln Tyr Ser Ala Gly His Leu Asp Met Leu Cys Arg Ile Lys Ser
   465                 470                 475                 480
   Leu Gln Thr Arg Val Asp Gln Ile Leu Gly Lys Gly Gln Ile Thr Ser
                   485                 490                 495
   Asp Lys Lys Ser Arg Glu Lys Ile Thr Ala Glu His Glu Thr Thr Asp
               500                 505                 510
   Asp Leu Ser Met Leu Gly Arg Val Val Lys Val Glu Lys Gln Val Gln
           515                 520                 525
   Ser Ile Glu Ser Lys Leu Asp Cys Leu Leu Asp Ile Tyr Gln Gln Val
       530                 535                 540
   Leu Arg Lys Gly Ser Ala Ser Ala Leu Ala Leu Ala Ser Phe Gln Ile
   545                 550                 555                 560
   Pro Pro Phe Glu Cys Glu Gln Thr Ser Asp Tyr Gln Ser Pro Val Asp
                   565                 570                 575
   Ser Lys Asp Leu Ser Gly Ser Ala Gln Asn Ser Gly Cys Leu Ser Arg
               580                 585                 590
   Ser Thr Ser Ala Asn Ile Ser Arg Gly Leu Gln Phe Ile Leu Thr Pro
           595                 600                 605
   Asn Glu Phe Ser Ala Gln Thr Phe Tyr Ala Leu Ser Pro Thr Met His
       610                 615                 620
   Ser Gln Ala Thr Gln Val Pro Ile Ser Gln Ser Asp Gly Ser Ala Val
   625                 630                 635                 640
   Ala Ala Thr Asn Thr Ile Ala Asn Gln Ile Asn Thr Ala Pro Lys Pro
                   645                 650                 655
   Ala Ala Pro Thr Thr Leu Gln Ile Pro Pro Pro Leu Pro Ala Ile Lys
               660                 665                 670
   His Leu Pro Arg Pro Glu Thr Leu His Pro Asn Pro Ala Gly Leu Gln
           675                 680                 685
   Glu Ser Ile Ser Asp Val Thr Thr Cys Leu Val Ala Ser Lys Glu Asn
       690                 695                 700
   Val Gln Val Ala Gln Ser Asn Leu Thr Lys Asp Arg Ser Met Arg Lys
   705                 710                 715                 720
   Ser Phe Asp Met Gly Gly Glu Thr Leu Leu Ser Val Cys Pro Met Val
                   725                 730                 735
   Pro Lys Asp Leu Gly Lys Ser Leu Ser Val Gln Asn Leu Ile Arg Ser
               740                 745                 750
   Thr Glu Glu Leu Asn Ile Gln Leu Ser Gly Ser Glu Ser Ser Ala Ser
           755                 760                 765
   Arg Gly Ser Gln Asp Phe Tyr Pro Lys Trp Arg Glu Ser Lys Leu Phe
       770                 775                 780
   Ile Thr Asp Glu Glu Val Gly Pro Glu Glu Thr Glu Thr Asp Thr Phe
   785                 790                 795                 800
   Asp Ala Ala Pro Gln Pro Ala Arg Glu Ala Ala Phe Ala Ser Asp Ser
                   805                 810                 815
   Leu Arg Thr Gly Arg Ser Arg Ser Ser Gln Ser Ile Cys Lys Ala Gly
               820                 825                 830
   Glu Ser Thr Asp Ala Leu Ser Leu Pro His Val Lys Leu Lys
           835                 840                 845
<210> 4
   <211> 695
   <212> PRT
   <213> Homo Sapiens
<400> 4
<210> 5
   <211> 423
   <212> PRT
   <213> Homo Sapiens
<400> 5
   Ser Leu Leu Gly Pro Leu Ser Ser Ser Cys Arg Lys Tyr Arg Arg Gln
    1              5                   10                  15
   Asn Tyr Asn Val Leu Glu Arg Pro Arg Gly Trp Ala Phe Tyr His Phe
               20                  25                  30
   Phe Leu Leu Val Phe Cys Leu Leu Ser Val Ser Thr Ile Glu His Leu
           35                  40                  45
   Ala Cys Leu Leu Ile Leu Glu Phe Val Met Ile Val Val Phe Gly Leu
       50                  55                  60
   Glu Ile Arg Trp Ser Ala Gly Cys Cys Cys Arg Tyr Arg Gly Trp Gln
   65                  70                  75                  80
   Gly Arg Arg Phe Ala Arg Lys Pro Phe Cys Val Ile Asp Ile Val Ala
                   85                  90                  95
   Ser Ala Val Ala Thr Gln Gly Asn Ile Phe Ala Thr Ser Ala Leu Arg
               100                 105                 110
   Ser Arg Phe Leu Gln Ile Leu Arg Met Val Arg Met Asp Arg Arg Gly
           115                 120                 125
   Gly Thr Trp Lys Leu Leu Gly Ser Val Val Tyr Ala His Ser Lys Glu
       130                 135                 140
   Leu Ile Thr Ala Trp Tyr Ile Gly Phe Leu Val Leu Ile Phe Ser Phe
   145                 150                 155                 160
   Leu Val Tyr Leu Glu Lys Asp Ala Asn Phe Ser Tyr Ala Asp Leu Trp
                   165                 170                 175
   Trp Gly Thr Ile Thr Leu Thr Thr Ile Gly Tyr Gly Asp Lys Thr Pro
               180                 185                 190
   Thr Trp Leu Gly Arg Leu Ala Gly Phe Ala Leu Leu Gly Ile Ser Phe
           195                 200                 205
   Phe Ala Leu Pro Ala Gly Ile Leu Gly Ser Gly Phe Ala Leu Lys Val
       210                 215                 220
   Gln Glu Gln His Arg Gln Lys His Phe Glu Lys Arg Arg Pro Ala Ala
   225                 230                 235                 240
   Asn Leu Ile Gln Trp Arg Tyr Asp Ser Ala Thr Trp Leu Ala Leu Thr
                   245                 250                 255
   Cys Pro Thr Gly Glu Ser Ser Lys Arg Arg Met Ser Arg Gly Ser Lys
               260                 265                 270
   Ala Ser Pro Ser Glu Ser Pro Thr Lys Val Gln Lys Ser Trp Ser Phe
           275                 280                 285
   Asn Asp Arg Thr Arg Phe Arg Ser Leu Arg Leu Lys Pro Ala Ala Val
       290                 295                 300
   Glu Lys Gln Cys Val Asp Pro Lys Thr Val Ile Arg Ile Arg Ile Lys
   305                 310                 315                 320
   Phe Val Ala Lys Arg Lys Phe Lys Glu Thr Leu Arg Pro Tyr Asp Val
                   325                 330                 335
   Lys Asp Val Ile Glu Gln Tyr Ser Ala Gly His Leu Asp Met Leu Arg
               340                 345                 350
   Ile Lys Ser Leu Gln Thr Arg Val Asp Gln Ile Gly Gly Asp Lys Arg
           355                 360                 365
   Glu Lys Glu Asp Ser Met Gly Arg Val Val Lys Val Glu Lys Gln Val
       370                 375                 380
   Gln Ser Ile Glu Lys Leu Asp Leu Leu Tyr Leu Arg Gly Ser Ala Ser
   385                 390                 395                 400
   Leu Gln Pro Phe Thr Ser Asp Tyr Ser Pro Val Asp Asp Ser Ser Ala
                   405                 410                 415
   Gln Ser Ser Arg Ser Ser Asn
               420
<210> 6
   <211> 514
   <212> PRT
   <213> Homo Sapiens
<400> 6
   Met Val Gln Lys Ser Arg Asn Gly Gly Val Tyr Pro Gly Pro Ser Gly
    1               5                  10                  15
   Glu Lys Lys Leu Lys Val Gly Phe Val Gly Leu Asp Pro Gly Ala Pro
               20                  25                  30
   Asp Ser Thr Arg Asp Gly Ala Leu Leu Ile Ala Gly Ser Glu Ala Pro
           35                  40                  45
   Lys Arg Gly Ser Ile Leu Ser Lys Pro Arg Ala Gly Gly Ala Gly Ala
       50                  55                  60
   Gly Lys Pro Pro Lys Arg Asn Ala Phe Tyr Arg Lys Leu Gln Asn Phe
   65                  70                  75                  80
   Leu Tyr Asn Val Leu Glu Arg Pro Arg Gly Trp Ala Phe Ile Tyr His
                   85                  90                  95
   Ala Tyr Val Phe Leu Leu Val Phe Ser Cys Leu Val Leu Ser Val Phe
               100                 105                 110
   Ser Thr Ile Lys Glu Tyr Glu Lys Ser Ser Glu Gly Ala Leu Tyr Ile
           115                 120                 125
   Leu Glu Ile Val Thr Ile Val Val Phe Gly Val Glu Tyr Phe Val Arg
       130                 135                 140
   Ile Trp Ala Ala Gly Cys Cys Cys Arg Tyr Arg Gly Trp Arg Gly Arg
   145                 150                 155                 160
   Leu Lys Phe Ala Arg Lys Pro Phe Cys Val Ile Asp Ile Met Val Leu
                   165                 170                 175
   Ile Ala Ser Ile Ala Val Leu Ala Ala Gly Ser Gln Gly Asn Val Phe
               180                 185                 190
   Ala Thr Ser Ala Leu Arg Ser Leu Arg Phe Leu Gln Ile Leu Arg Met
           195                 200                 205
   Ile Arg Met Asp Arg Arg Gly Gly Thr Trp Lys Leu Leu Gly Ser Val
       210                 215                 220
   Val Tyr Ala His Ser Lys Glu Leu Val Thr Ala Trp Tyr Ile Gly Phe
   225                 230                 235                 240
   Leu Cys Leu Ile Leu Ala Ser Phe Leu Val Tyr Leu Ala Glu Lys Gly
                   245                 250                 255
   Glu Asn Asp His Phe Asp Thr Tyr Ala Asp Ala Leu Trp Trp Gly Leu
               260                 265                 270
   Ile Thr Leu Thr Thr Ile Gly Tyr Gly Asp Lys Tyr Pro Gln Thr Trp
           275                 280                 285
   Asn Gly Arg Leu Leu Ala Ala Thr Phe Thr Leu Ile Gly Val Ser Phe
       290                 295                 300
   Phe Ala Leu Pro Ala Gly Ile Leu Gly Ser Gly Phe Ala Leu Lys Val
   305                 310                 315                 320
   Gln Glu Gln His Arg Gln Lys His Phe Glu Lys Arg Arg Asn Pro Ala
                   325                 330                 335
   Ala Gly Leu Ile Gln Ser Ala Trp Arg Phe Tyr Ala Thr Asn Leu Ser
               340                 345                 350
   Arg Thr Asp Leu His Ser Thr Trp Gln Tyr Tyr Glu Arg Thr Val Thr
           355                 360                 365
   Val Pro Met Tyr Arg Leu Ile Pro Pro Leu Asn Gln Leu Glu Leu Leu
       370                 375                 380
   Arg Asn Leu Lys Ser Lys Ser Gly Leu Ala Phe Arg Lys Asp Pro Pro
   385                 390                 395                 400
   Pro Glu Pro Ser Pro Ser Gln Lys Val Ser Leu Lys Asp Arg Val Phe
                   405                 410                 415
   Ser Ser Pro Arg Gly Val Ala Ala Lys Gly Lys Gly Ser Pro Gln Ala
               420                 425                 430
   Gln Thr Val Arg Arg Ser Pro Ser Ala Asp Gln Ser Leu Glu Asp Ser
           435                 440                 445
   Pro Ser Lys Val Pro Lys Ser Trp Ser Phe Gly Asp Arg Ser Arg Ala
       450                 455                 460
   Arg Gln Ala Phe Arg Ile Lys Gly Ala Ala Ser Arg Gln Asn Ser Glu
   465                 470                 475                 480
   Glu Ala Ser Leu Pro Gly Glu Asp Ile Val Asp Asp Lys Ser Cys Pro
                   485                 490                 495
   Cys Glu Phe Val Thr Glu Asp Leu Thr Pro Gly Leu Lys Val Ser Ile
               500                 505                 510
   Arg Ala
<210> 7
   <211> 521
   <212> PRT
   <213> Homo Sapiens
<400> 7
   Met Gly Leu Lys Ala Arg Arg Ala Ala Gly Ala Ala Gly Gly Gly Gly
    1              5                  10                  15
   Asp Gly Gly Gly Gly Gly Gly Gly Ala Ala Asn Pro Ala Gly Gly Asp
               20                  25                  30
   Ala Ala Ala Ala Gly Asp Glu Glu Arg Lys Val Gly Leu Ala Pro Gly
           35                  40                  45
   Asp Val Glu Gln Val Thr Leu Ala Leu Gly Ala Gly Ala Asp Lys Asp
       50                  55                  60
   Gly Thr Leu Leu Leu Glu Gly Gly Gly Arg Asp Glu Gly Gln Arg Arg
   65                  70                  75                  80
   Thr Pro Gln Gly Ile Gly Leu Leu Ala Lys Thr Pro Leu Ser Arg Pro
                   85                  90                  95
   Val Lys Arg Asn Asn Ala Lys Tyr Arg Arg Ile Gln Thr Leu Ile Tyr
                   100                 105                 110
   Asp Ala Leu Glu Arg Pro Arg Gly Trp Ala Leu Leu Tyr His Ala Leu
               115                 120                 125
   Val Phe Leu Ile Val Leu Gly Cys Leu Ile Leu Ala Val Leu Thr Thr
           130                 135                 140
   Phe Lys Glu Tyr Glu Thr Val Ser Gly Asp Trp Leu Leu Leu Leu Glu
   145                 150                 155                 160
   Thr Phe Ala Ile Phe Ile Phe Gly Ala Glu Phe Ala Leu Arg Ile Trp
                   165                 170                 175
   Ala Ala Gly Cys Cys Cys Arg Tyr Lys Gly Trp Arg Gly Arg Leu Lys
               180                 185                 190
   Phe Ala Arg Lys Pro Leu Cys Met Leu Asp Ile Phe Val Leu Ile Ala
           195                 200                 205
   Ser Val Pro Val Val Ala Val Gly Asn Gln Gly Asn Val Leu Ala Thr
       210                 215                 220
   Ser Leu Arg Ser Leu Arg Phe Leu Gln Ile Leu Arg Met Leu Arg Met
   225                 230                 235                 240
   Asp Arg Arg Gly Gly Thr Trp Lys Leu Leu Gly Ser Ala Ile Cys Ala
                   245                 250                 255
   His Ser Lys Glu Leu Ile Thr Ala Trp Tyr Ile Gly Phe Leu Thr Leu
               260                 265                 270
   Ile Leu Ser Ser Phe Leu Val Tyr Leu Val Glu Lys Asp Val Pro Glu
           275                 280                 285
   Val Asp Ala Gln Gly Glu Glu Met Lys Glu Glu Phe Glu Thr Tyr Ala
       290                 295                 300
   Asp Ala Leu Trp Trp Gly Leu Ile Thr Leu Ala Thr Ile Gly Tyr Gly
   305                 310                 315                 320
   Asp Lys Thr Pro Lys Thr Trp Glu Gly Arg Leu Ile Ala Ala Thr Phe
                   325                 330                 335
   Ser Leu Ile Gly Val Ser Phe Phe Ala Leu Pro Ala Gly Ile Leu Gly
               340                 345                 350
   Ser Gly Leu Ala Leu Lys Val Gln Glu Gln His Arg Gln Lys His Phe
           355                 360                 365
   Glu Lys Arg Arg Lys Pro Ala Ala Glu Leu Ile Gln Ala Ala Trp Arg
       370                 375                 380
   Tyr Tyr Ala Thr Asn Pro Asn Arg Ile Asp Leu Val Ala Thr Trp Arg
   385                 390                 395                 400
   Phe Tyr Glu Ser Val Val Ser Phe Pro Phe Phe Arg Lys Glu Gln Leu
                   405                 410                 415
   Glu Ala Ala Ser Ser Gln Lys Leu Gly Leu Leu Asp Arg Val Arg Leu
               420                 425                 430
   Ser Asn Pro Arg Gly Ser Asn Thr Lys Gly Lys Leu Phe Thr Pro Leu
           435                 440                 445
   Asn Val Asp Ala Ile Glu Glu Ser Pro Ser Lys Glu Pro Lys Pro Val
       450                 455                 460
   Gly Leu Asn Asn Lys Glu Arg Phe Arg Thr Ala Phe Arg Met Lys Ala
   465                 470                 475                 480
   Tyr Ala Phe Trp Gln Ser Ser Glu Asp Ala Gly Thr Gly Asp Pro Met
                   485                 490                 495
   Ala Glu Asp Arg Gly Tyr Gly Asn Asp Phe Pro Ile Glu Asp Met Ile
               500                 505                 510
   Pro Thr Leu Lys Ala Ala Ile Arg Ala
           515                 520
<210> 8
   <211> 142
   <212> PRT
   <213> Homo Sapiens
<400> 8
   Arg Gln Tyr Leu Glu Arg Pro Gly Trp Tyr His Phe Leu Val Cys Leu
   1               5                   10                  15
   Val Thr Leu Glu Phe Gly Glu Arg Trp Ala Gly Cys Tyr Gly Gly Arg
               20                  25                  30
   Phe Ala Arg Lys Pro Asp Val Ala Ser Val Gly Ala Thr Ser Arg Arg
           35                  40                  45
   Phe Leu Gln Ile Leu Arg Met Asp Arg Gly Gly Thr Trp Leu Leu Gly
       50                  55                  60
   Ser His Glu Leu Thr Tyr Ile Gly Phe Leu Leu Ile Ser Val Tyr Leu
   65                  70                  75                  80
   Glu Lys Phe Tyr Ala Asp Leu Trp Trp Gly Thr Thr Ile Gly Tyr Gly
                   85                  90                  95
   Asp Lys Pro Thr Trp Gly Phe Ser Phe Phe Ala Leu Pro Ala Gly Ile
               100                 105                 110
   Leu Gly Ser Gly Ala Leu Lys Val Gln Arg Gln Lys His Phe Ala Ala
           115                 120                 125
   Leu Ile Gln Trp Arg Tyr Thr Trp Leu Ser Lys Glu Ala Ile
       130                 135                 140
<210> 9
   <211> 17
   <212> DNA
   <213> Homo Sapiens
<400> 9
   gggggcccgg atgagcc 17
<210> 10
   <211> 21
   <212> DNA
   <213> Homo Sapiens
<400> 10
   gaagaactta tttcagtttg a 21
<210> 11
   <211> 25
   <212> DNA
   <213> Homo Sapiens
<400> 11
   ttttctcctt gtctttggtt gcttg 25
<210> 12
   <211> 27
   <212> DNA
   <213> Homo Sapiens
<400> 12
   ccatcctaat acgactcact atagggc 27
<210> 13
   <211> 23
   <212> DNA
   <213> Homo Sapiens
<400> 13
   cctcaagttg cctcttgatc ctg 23
<210> 14
   <211> 23
   <212> DNA
   <213> Homo Sapiens
<400> 14
   actcactata gggctcgagc ggc 23
<210> 15
   <211> 23
   <212> DNA
   <213> Homo Sapiens
<400> 15
   caggatcaag aggcaacttg agg 23
<210> 16
   <211> 28
   <212> DNA
   <213> Homo Sapiens
<400> 16
   ccaattttgt gtgctcaggg atggtaga 28
<210> 17
   <211> 18
   <212> DNA
   <213> Homo Sapiens
<400> 17
   gacacagccc ttggcact 18
<210> 18
   <211> 26
   <212> DNA
   <213> Homo Sapiens
<400> 18
   gatgatgtat atgatgaaaa aggatg 26
<210> 19
   <211> 23
   <212> DNA
   <213> Homo Sapiens
<400> 19
   ctgatagctc gaatgacagt ttt 23
<210> 20
   <211> 26
   <212> DNA
   <213> Homo Sapiens
<400> 20
   aagtggtggg gtgaggtctt ccactg 26
<210> 21
   <211> 24
   <212> DNA
   <213> Homo Sapiens
<400> 21
   agaattatga aatttcatgt tgca 24
<210> 22
   <211> 23
   <212> DNA
   <213> Homo Sapiens
<400> 22
   aaacggaagt ttaaggaaac att 23
<210> 23
   <211> 23
   <212> DNA
   <213> Homo Sapiens
<400> 23
   acgtgtttgt tggcttttaa ttc 23
<210> 24
   <211> 21
   <212> DNA
   <213> Homo Sapiens
<400> 24
   tacacaacat gtccagatga c 21
<210> 25
   <211> 22
   <212> DNA
   <213> Homo Sapiens
<400> 25
   tgatcaaatt cttggaaaag gg 22
<210> 26
   <211> 23
   <212> DNA
   <213> Homo Sapiens
<400> 26
   tcacatcaga taagaagagc cga 23
<210> 27
   <211> 21
   <212> DNA
   <213> Homo Sapiens
<400> 27
   gtttttcaac cttgaccacc c 21
<210> 28
   <211> 23
   <212> DNA
   <213> Homo Sapiens
<400> 28
   agcatactga gatcgtctgt ggt 23
<210> 29
   <211> 25
   <212> DNA
   <213> Homo Sapiens
<400> 29
   aattccaaaa gtgtctgtct ctgtc 25
<210> 30
   <211> 22
   <212> DNA
   <213> Homo Sapiens
<400> 30
   ggacccacct cttcatcagt ta 22
<210> 31
   <211> 20
   <212> DNA
   <213> Homo Sapiens
<400> 31
   ggaagactga ggtttgctcg 20
<210> 32
   <211> 20
   <212> DNA
   <213> Homo Sapiens
<400> 32
   ggcaggaagt gcaaagaaag 20
<210> 33
   <211> 20
   <212> DNA
   <213> Homo Sapiens
<400> 33
   ggggatgtgc tgcaaggcga 20
<210> 34
   <211> 25
   <212> DNA
   <213> Homo Sapiens
<400> 34
   cccaggcttt acactttatg cttcc 25
<210> 35
   <211> 22
   <212> DNA
   <213> Homo Sapiens
<400> 35
   ccagggtttt cccagtcacg ac 22
<210> 36
   <211> 25
   <212> DNA
   <213> Homo Sapiens
<400> 36
   ttgtgtggaa ttgtgagcgg ataac 25
<210> 37
   <211> 20
   <212> DNA
   <213> Homo Sapiens
<400> 37
   ctttctttgc acttcctgcc 20
<210> 38
   <211> 20
   <212> DNA
   <213> Homo Sapiens
<400> 38
   aacacagaag ggctttcgag 20
<210> 39
   <211> 20
   <212> DNA
   <213> Homo Sapiens
<400> 39
   cagtcttcct tgccatcctc 20
<210> 40
   <211> 20
   <212> DNA
   <213> Homo Sapiens
<400> 40
   gcagccccaa caactttaca 20
<210> 41
   <211> 20
   <212> DNA
   <213> Homo Sapiens
<400> 41
   cattttcctt ggaggcaaca 20
<210> 42
   <211> 20
   <212> DNA
   <213> Homo Sapiens
<400> 42
   gccagaaact ctgcacccta 20
<210> 43
   <211> 421
   <212> PRT
   <213> Homo Sapiens
<400> 43
   Met Glu Thr Arg Gly Ser Arg Leu Thr Gly Gly Gln Gly Arg Val Tyr
    1               5                  10                  15
   Asn Phe Leu Glu Arg Pro Thr Gly Trp Lys Cys Phe Val Tyr His Phe
               20                  25                  30
   Ala Val Phe Leu Ile Val Leu Val Cys Leu Ile Phe Ser Val Leu Ser
           35                  40                  45
   Thr Ile Glu Gln Tyr Ala Ala Leu Ala Thr Gly Thr Leu Phe Trp Met
       50                  55                  60
   Glu Ile Val Leu Val Val Phe Phe Gly Thr Glu Tyr Val Val Arg Leu
   65                  70                  75                  80
   Trp Ser Ala Gly Cys Arg Ser Lys Tyr Val Gly Leu Trp Gly Arg Leu
                   85                  90                  95
   Arg Phe Ala Arg Lys Pro Ile Ser Ile Ile Asp Leu Ile Val Val Val
               100                 105                 110
   Ala Ser Met Val Val Leu Cys Val Gly Ser Lys Gly Gln Val Phe Ala
           115                 120                 125
   Thr Ser Ala Ile Arg Gly Ile Arg Phe Leu Gln Ile Leu Arg Met Leu
       130                 135                 140
   His Val Asp Arg Gln Gly Gly Thr Trp Arg Leu Leu Gly Ser Val Val
   145                 150                 155                 160
   Phe Ile His Arg Gln Glu Leu Ile Thr Thr Leu Tyr Ile Gly Phe Leu
                   165                 170                 175
   Gly Leu Ile Phe Ser Ser Tyr Phe Val Tyr Leu Ala Glu Lys Asp Ala
               180                 185                 190
   Val Asn Glu Ser Gly Arg Val Glu Phe Gly Ser Tyr Ala Asp Ala Leu
           195                 200                 205
   Trp Trp Gly Val Val Thr Val Thr Thr Ile Gly Tyr Gly Asp Lys Val
       210                 215                 220
   Pro Gln Thr Trp Val Gly Lys Thr Ile Ala Ser Cys Phe Ser Val Phe
   225                 230                 235                 240
   Ala Ile Ser Phe Phe Ala Leu Pro Ala Gly Ile Leu Gly Ser Gly Phe
                   245                 250                 255
   Ala Leu Lys Val Gln Gln Lys Gln Arg Gln Lys His Phe Asn Arg Gln
               260                 265                 270
   Ile Pro Ala Ala Ala Ser Leu Ile Gln Thr Ala Trp Arg Cys Tyr Ala
           275                 280                 285
   Ala Glu Asn Pro Asp Ser Ser Thr Trp Lys Ile Tyr Ile Arg Lys Ala
       290                 295                 300
   Pro Arg Ser His Thr Leu Leu Ser Pro Ser Pro Lys Pro Lys Lys Ser
   305                 310                 315                 320
   Val Val Val Lys Lys Lys Lys Phe Lys Leu Asp Lys Asp Asn Gly Val
                   325                 330                 335
   Thr Pro Gly Glu Lys Met Leu Thr Val Pro His Ile Thr Cys Asp Pro
               340                 345                 350
   Pro Glu Glu Arg Arg Leu Asp His Phe Ser Val Asp Gly Tyr Asp Ser
           355                 360                 365
   Ser Val Arg Lys Ser Pro Thr Leu Leu Glu Val Ser Met Pro His Phe
       370                 375                 380
   Met Arg Thr Asn Ser Phe Ala Glu Asp Leu Asp Leu Glu Gly Glu Thr
   385                 390                 395                 400
   Leu Leu Thr Pro Ile Thr His Ile Ser Gln Leu Arg Glu His His Arg
                   405                 410                 415
   Ala Thr Ile Lys Val
               420

## Description

### FIELD OF THE INVENTION

The present invention is directed to novel human DNA sequences encoding a voltage-gated potassium channel.

### BACKGROUND OF THE INVENTION

Voltage-gated potassium channels form transmembrane pores that open in response to changes in cell membrane potential and selectively allow potassium ions to pass through the membrane. Many voltage-gated potassium channels have been identified. They are distinguishable by tissue-specific patterns of expression as well as by electrophysiological and pharmacological properties.

Voltage-gated potassium channels have been shown to be involved in maintaining cell membrane potentials and controlling the repolarization of action potentials in many cells, *e.g.,* neurons, muscle cells, and pancreatic β cells. They are important targets for drug discovery in connection with a variety of diseases.

Functional voltage-gated potassium channels are believed to be tetramers of four alpha subunits, each of which contains six transmembrane spanning segments. The alpha subunits making up a tetramer may be the same (in the case of homotetramers) or may be different (in the case of heterotetramers). The membrane-spanning alpha subunits making up the tetramers may sometimes be associated with additional, beta subunits, which may alter the behavior of the tetramers.

For reviews of voltage-gated potassium channels see Robertson, 1997, Trends Pharmacol. Sci. 18:474-483; Jan & Jan, 1997, J. Physiol. 505:267-282; Catterall, 1995, Ann. Rev. Biochem. 64:493-531.

Macular dystrophy is a term applied to a heterogeneous group of diseases that collectively are the cause of severe visual loss in a large number of people. A common characteristic of macular dystrophy is a progressive loss of central vision resulting from the degeneration of the pigmented epithelium underlying the retinal macula. In many forms of macular dystrophy, the end stage of the disease results in legal blindness. More than 20 types of macular dystrophy are known: e.g., age-related macular dystrophy, Stargardt's and Stargardt-like macular dystrophy, cone-rod dystrophies, atypical vitelliform macular dystrophy (VMD1), Usher Syndrome Type 1B, autosomal dominant neovascular inflammatory vitreoretinopathy, familial exudative vitreoretinopathy, and Best's macular dystrophy. For a review of the macular dystrophies, see Sullivan & Daiger, 1996, Mol. Med. Today 2:380-386.

Cone-rod dystrophies involve an initial loss of cone photoreceptors followed by the degeneration of rod photoreceptors. This loss of photoreceptors can lead to blindness. Cone-rod dystrophies appear to be a heterogeneous group of inherited disorders for which multiple chromosomal locations have been implicated (Evans et al., 1994, Nature Genet. 6:210-213; Kelsell et al., 1997, Hum. Mol. Genet. 6:597-600). In particular, Kelsell et al., 1998, Am. J. Hum. Genet. 63:274-279 found a candidate gene (CORD7) located at chromosome 6q in a four-generation British family affected with cone-rod dystrophy. A marker in 6q, D6S280, showed a high LOD score of 3.31 (at genetic distance = 0).

Stargardt-like macular dystrophy is an inherited, dominant retinal disease. Affected individuals have normal vision in early childhood but show impaired central vision either in late childhood or early adulthood. The first observable characteristics of the disease are flecks seen in the macula. This is followed by central atrophy, resulting in visual acuity decreasing to 20/200 or worse (Stone et al., Arch. Ophthalmol. 112:765-772 [Stone]). Stone mapped a gene responsible for Stargardt-like macular dystrophy to chromosome 6q. The marker D6S280 was observed by Stone to have the high LOD score of 5.5 (at genetic distance = 0).

Cone-rod dystrophy and Stargardt-like macular dystrophy appear different from a clinical perspective. For example, Stargardt-like macular dystrophy generally begins in childhood and involves white/yellow flecks in the retina while cone-rod dystrophy is an adult-onset disorder in which no flecks are present. Despite such clinical differences, both diseases may be caused by mutations in the same gene. It is not uncommon for different mutations in a single gene to give rise to clinically different disorders. For example, depending upon the particular mutation, mutations in the peripherin/RDS gene can give rise to either butterfly-shaped pigment dystrophy of the fovea, retinitis pigmentosa, pattern dystrophy, flavus maculatus, macular dystrophy, or central areolar choroidal dystrophy (Nichols et al., 1993, Nature Genet. 3:202-207; Weleber et al., 1993, Arch. Ophtalmol. 111:1531-1542; Wells et al., 1993, Nature Genet. 3:213-218; Reig et al., 1995, Ophthalmic. Genet. 16:39-44).

While studies of macular dystrophies such as cone-rod dystrophy or Stargardt-like macular dystrophy are valuable in themselves, such studies are also valuable in that they are expected to shed light on age-related macular degeneration (AMD). AMD is the leading cause of severe visual loss in older individuals. Genetic factors apparently play a role in AMD (Hyman et al., 1983, Am. J. Epidemiol. 118:213-227; Gass, 1973, Arch. Ophthamol. 90:206-217). It is believed likely that mild allelic variations of such earlier-onset diseases as cone-rod dystrophy and Stargardt-like macular dystrophy are responsible for some cases of AMD. Thus, understanding and developing treatments for these earlier-onset diseases should prove valuable with respect to AMD as well.

Salla disease is a recessive condition characterized by early-onset psychomotor retardation and ataxia that involves defects in the lysosomal transport of sialic acid. Leppänen et al., 1996, Genomics 37:62-67 (Leppänen) located the gene for Salla disease in the immediate vicinity of the marker D6S280. Leppänen screened a PAC library with the marker D6S280 and obtained three positive clones, among which were PAC 141B1 and PAC 224H23, strongly suggesting that the gene for Salla disease is present on these PACs.

### SUMMARY OF THE INVENTION

The present invention is directed to novel human DNA sequences encoding a voltage-gated potassium channel, KCNQ5, located in a chromosomal region that contains a gene associated with Stargardt-like macular dystrophy, cone-rod macular dystrophy, and Salla disease.

The present invention includes genomic KCNQ5 DNA as well as cDNA that encodes the KCNQ5 protein. The human genomic KCNQ5 DNA is substantially free from other nucleic acids and has the nucleotide sequence shown in SEQ.ID.NO.:1. The human cDNA encoding KCNQ5 protein is substantially free from other nucleic acids and has the nucleotide sequence shown in SEQ.ID.NO.:2. Also provided is KCNQ5 protein encoded by the novel DNA sequences. The human KCNQ5 protein is substantially free from other proteins and has the amino acid sequence shown in SEQ.ID.NO.:3. Methods of expressing KCNQ5 protein in recombinant systems are provided as well as methods of identifying activators and inhibitors of KCNQ5 protein function. Also provided are diagnostic methods that detect carriers of mutant KCNQ5 genes.

### BRIEF DESCRIPTION OF THE DRAWINGS

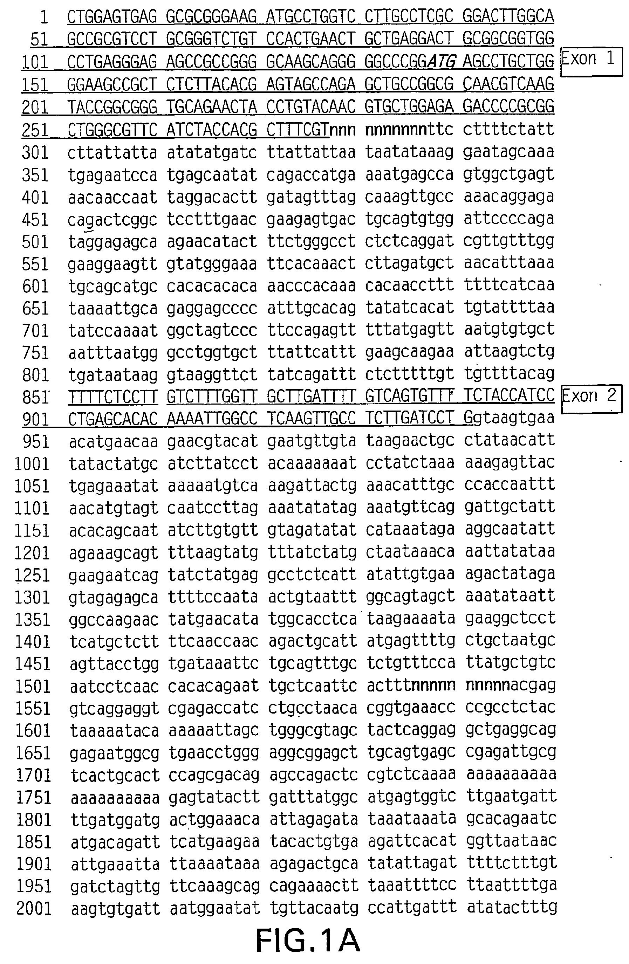

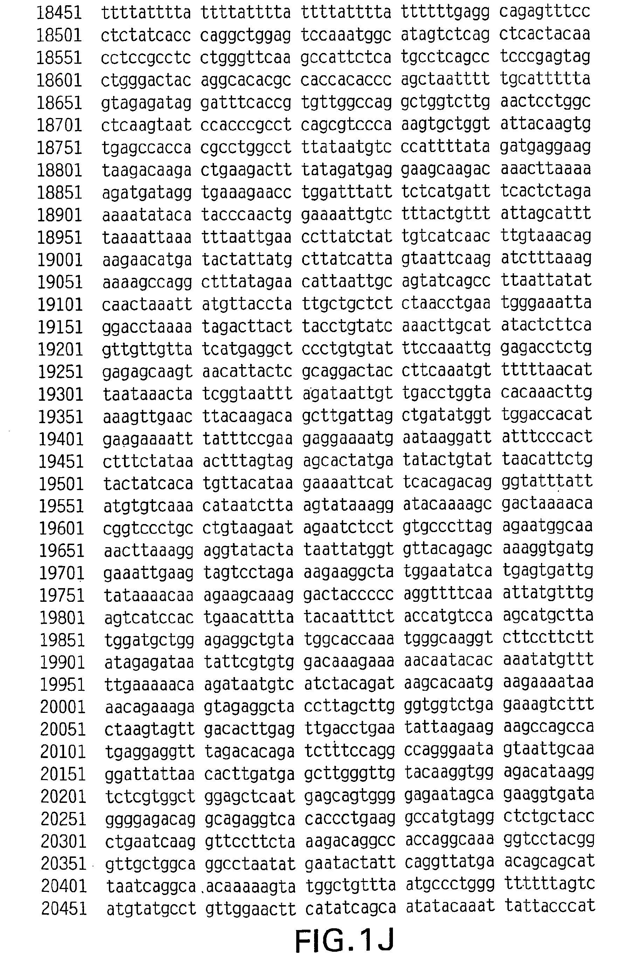

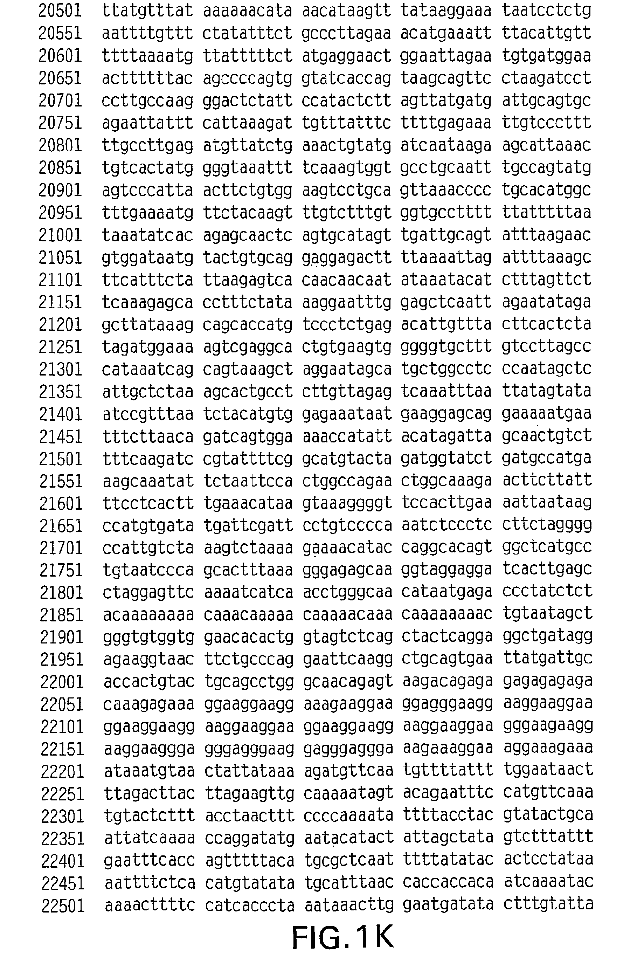

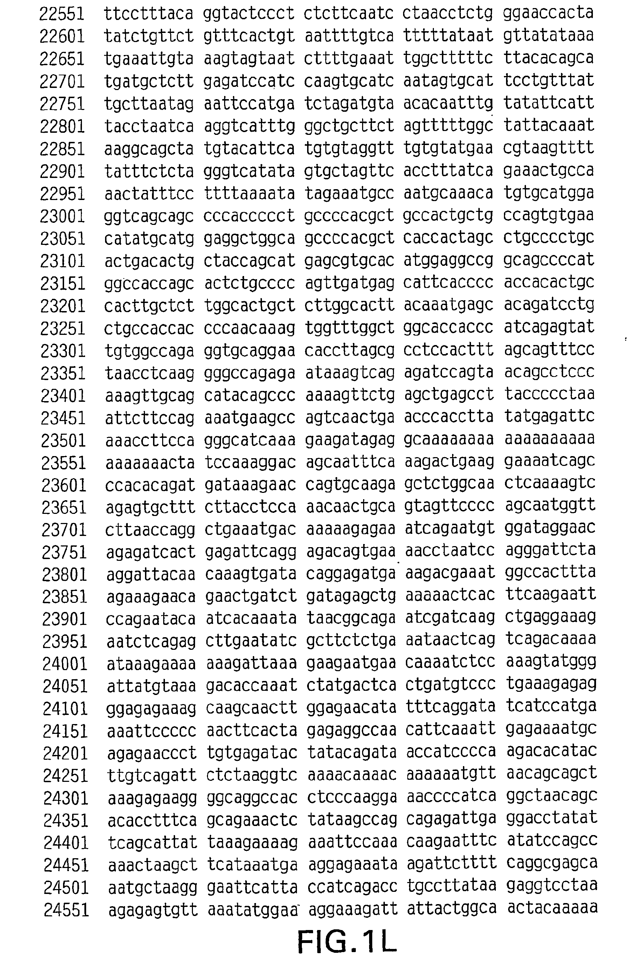

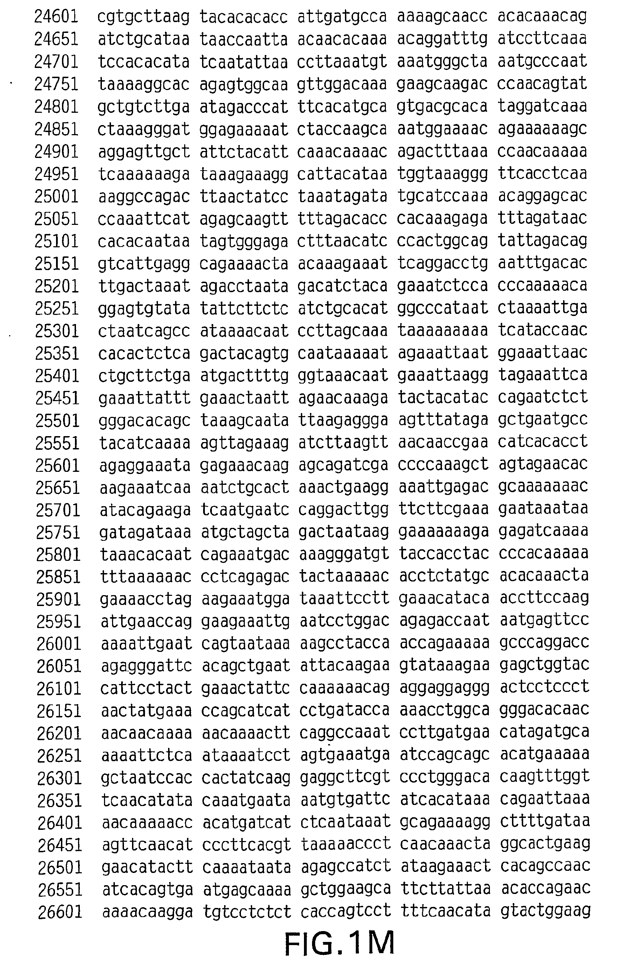

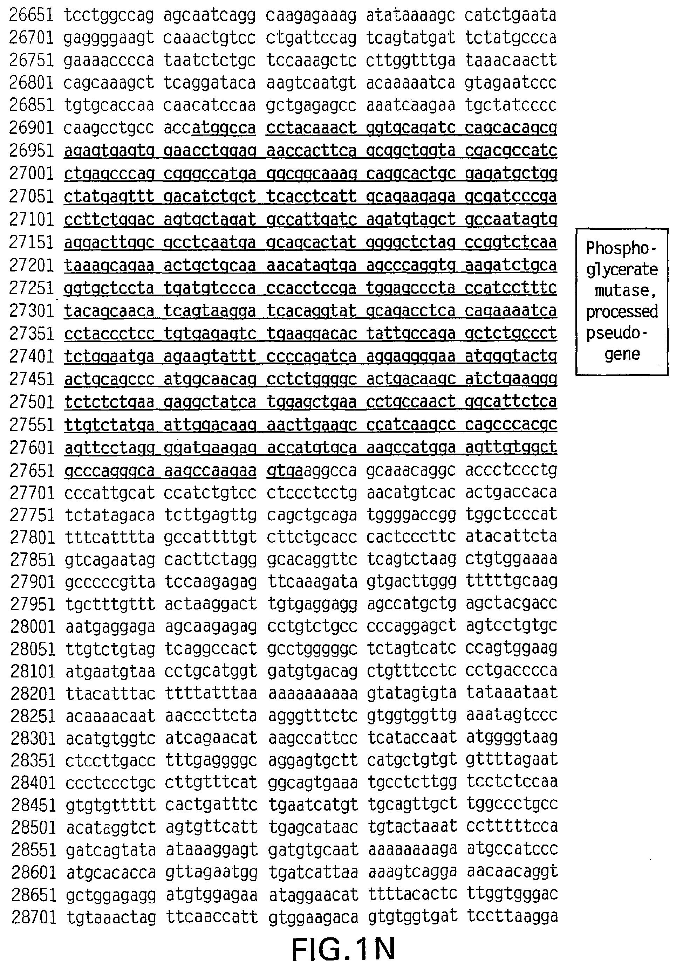

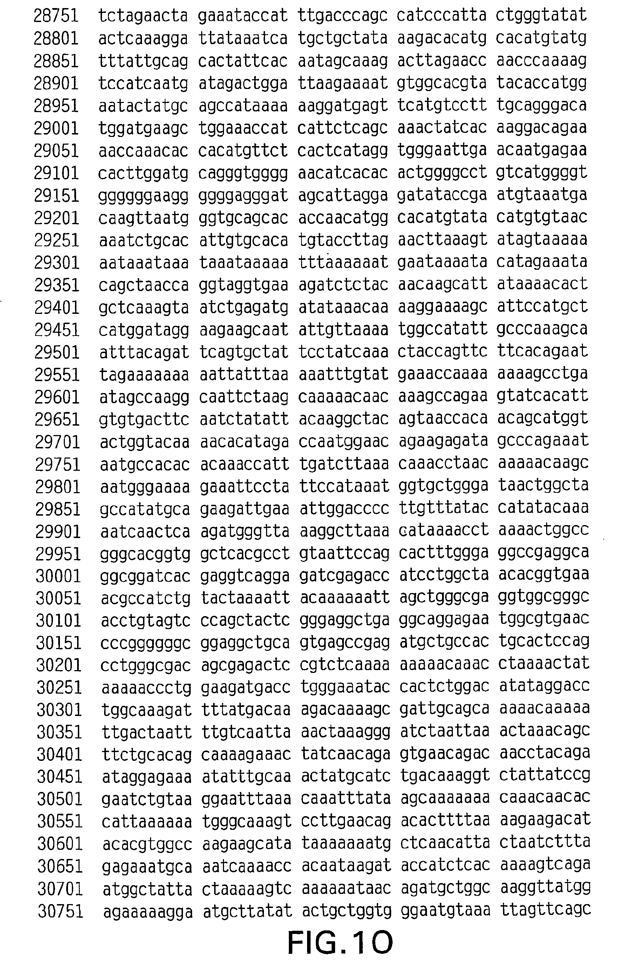

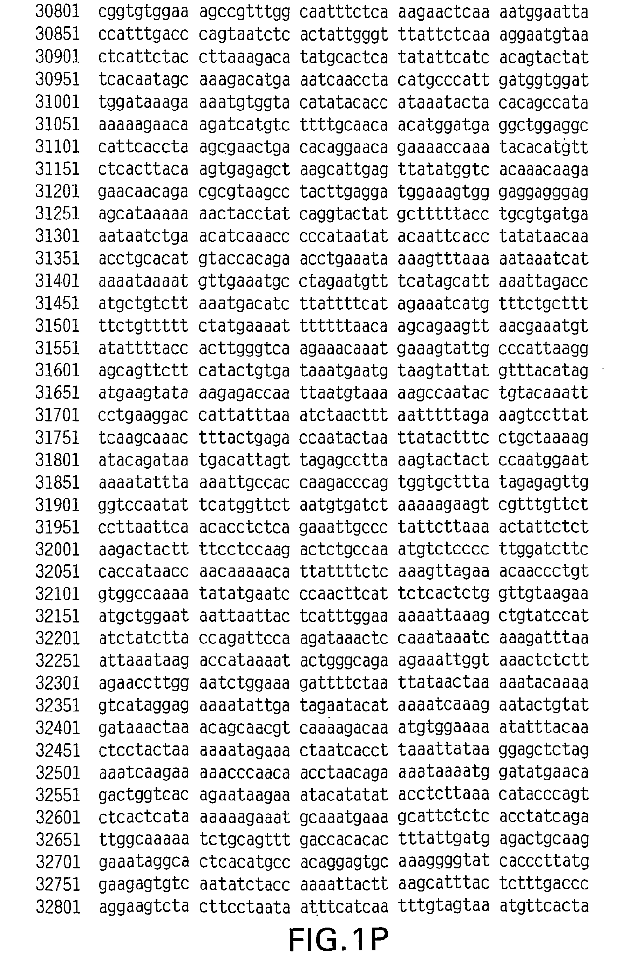

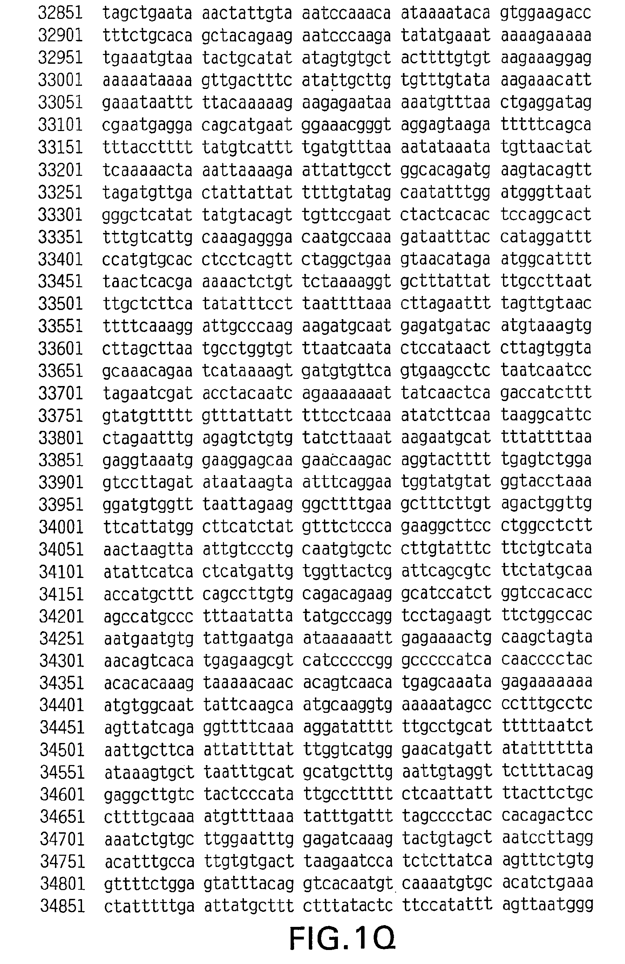

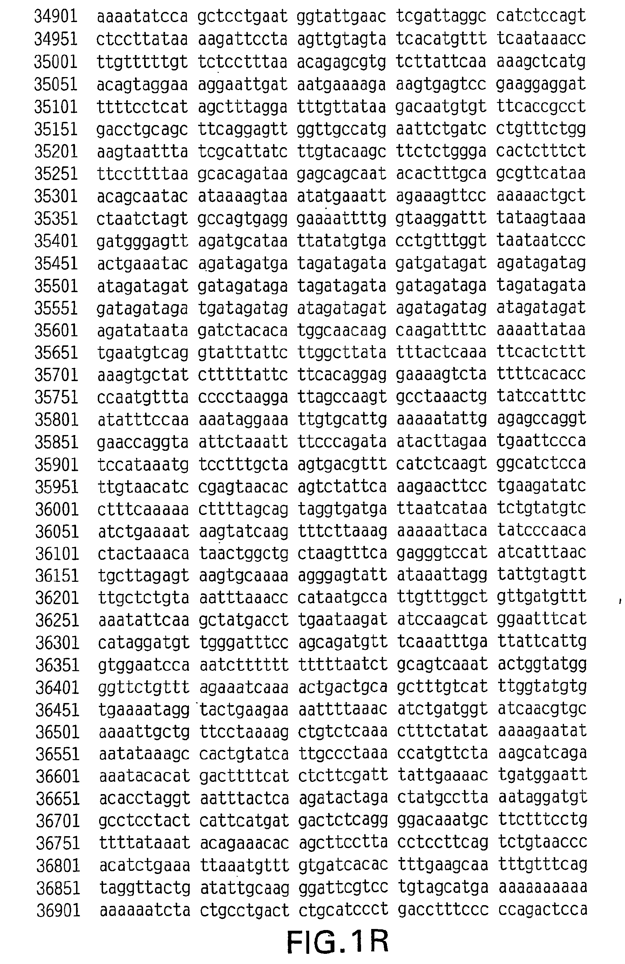

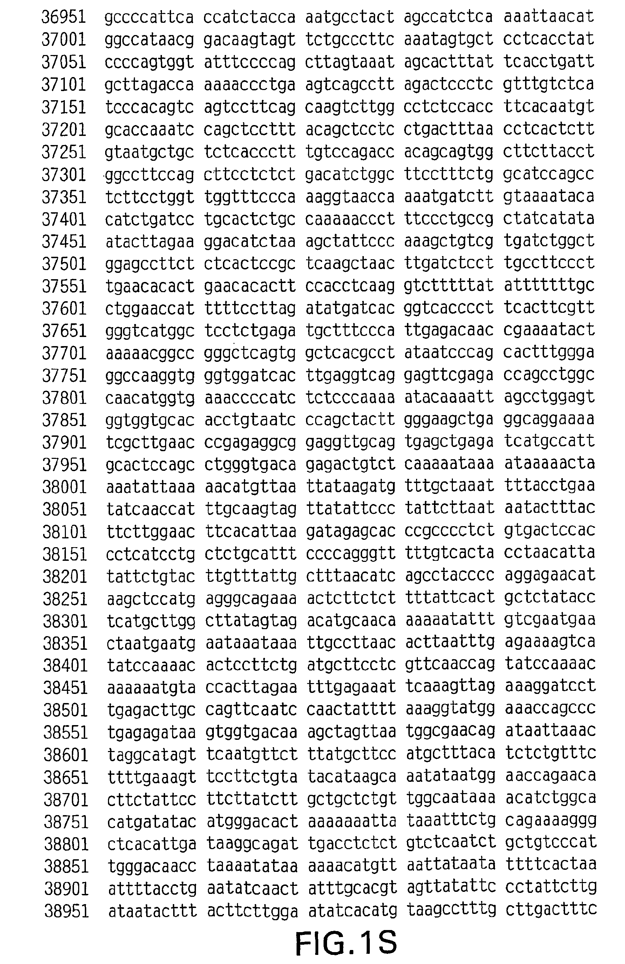

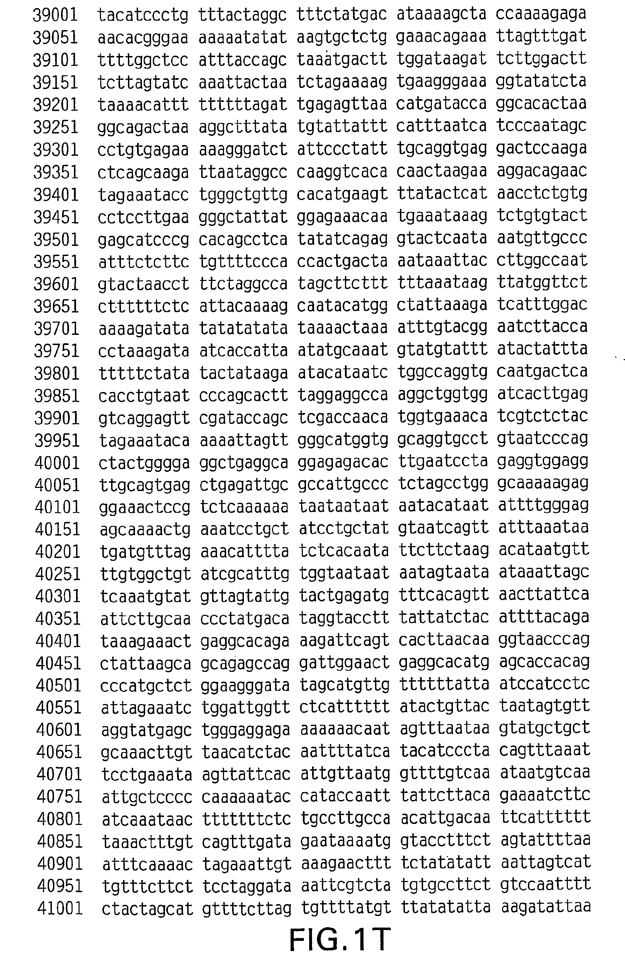

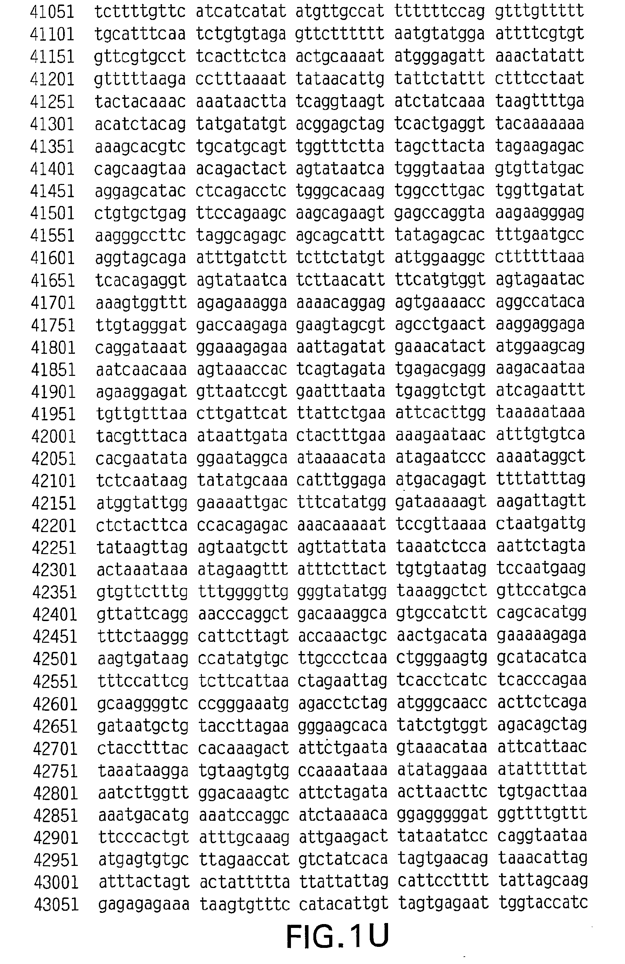

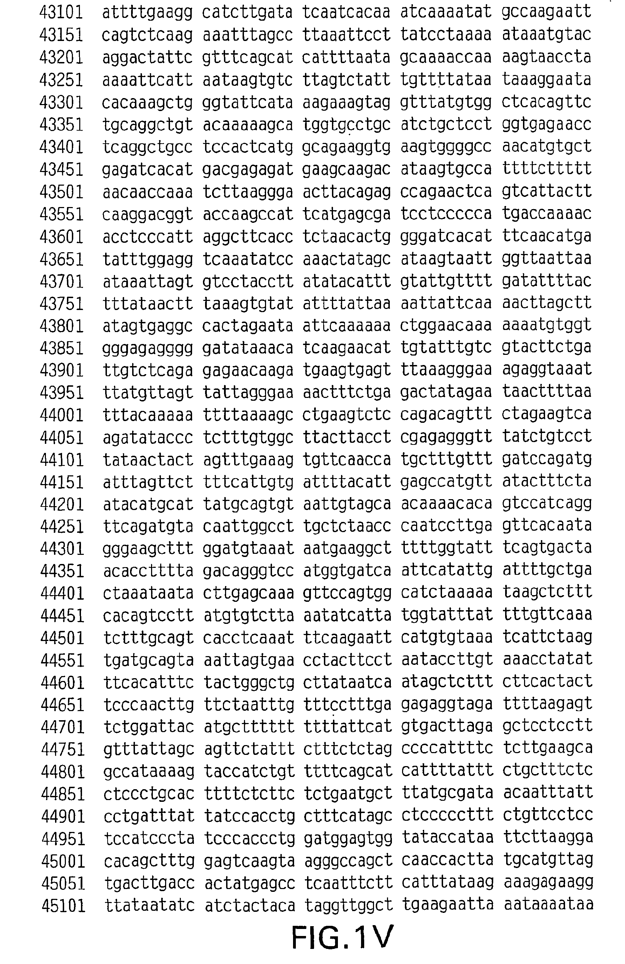

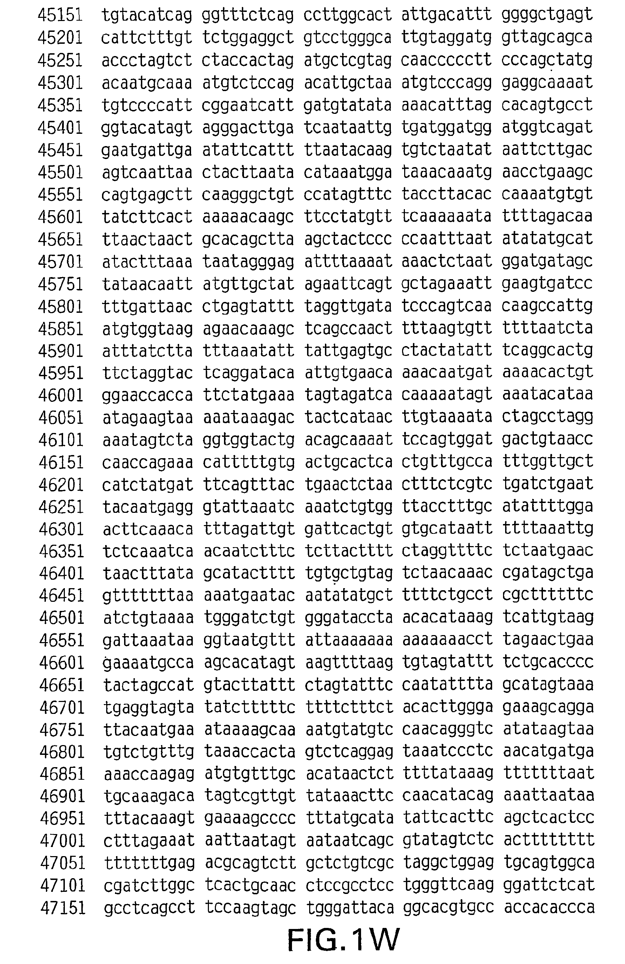

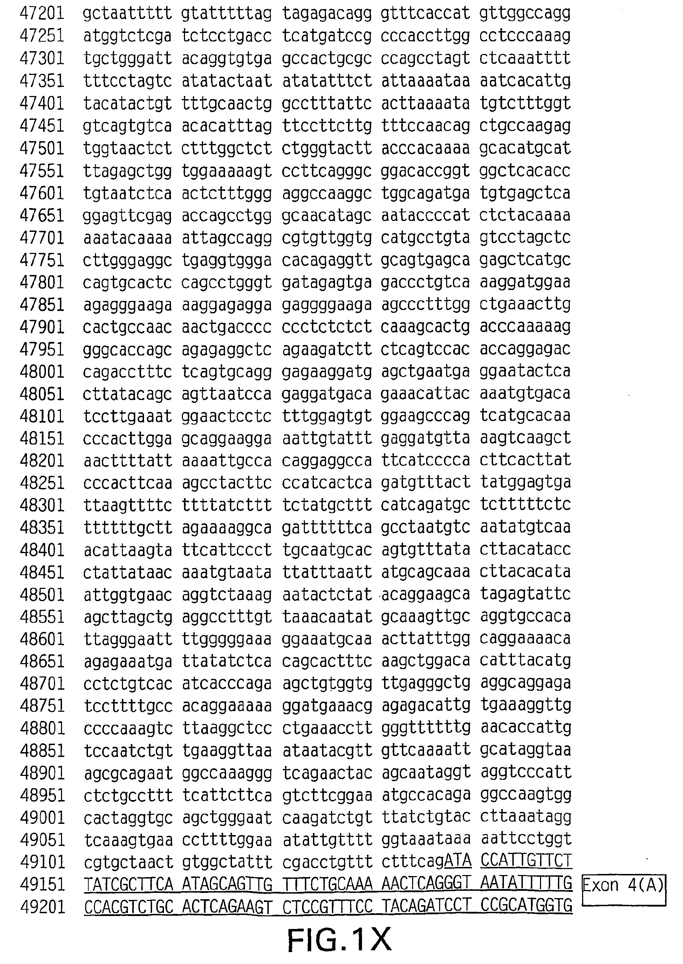

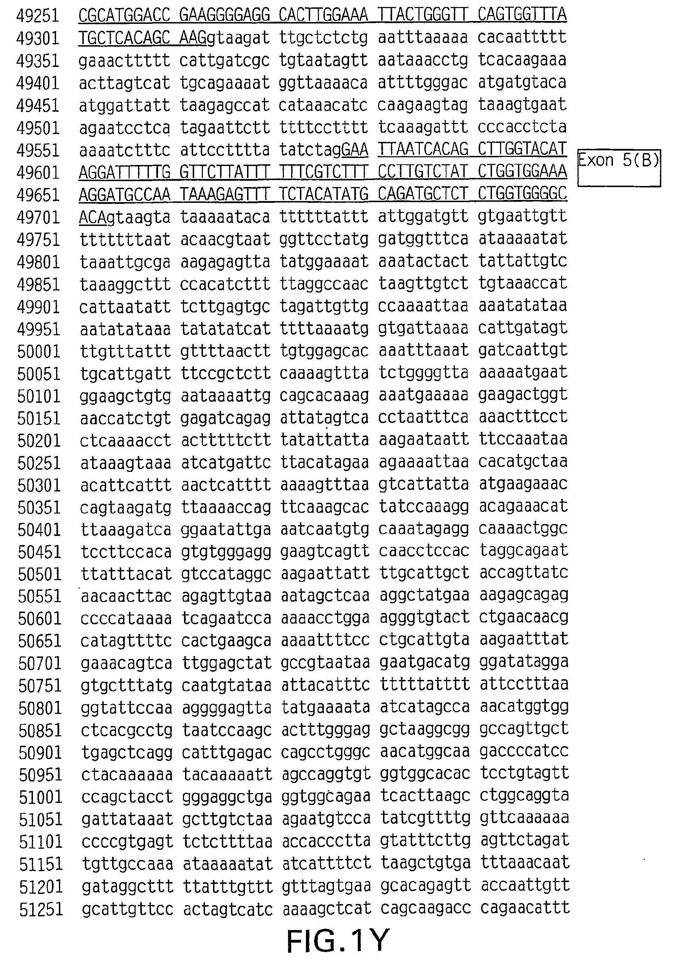

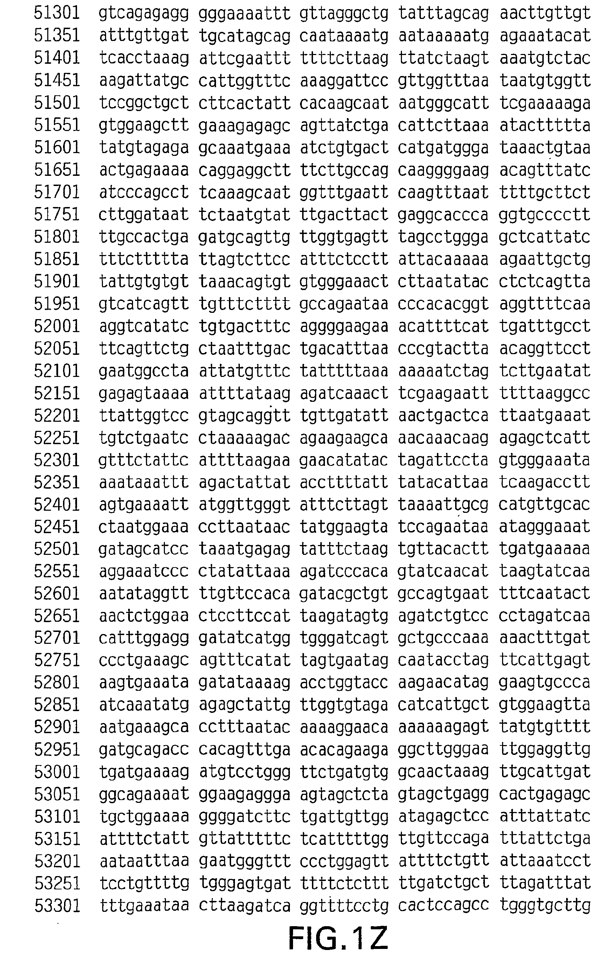

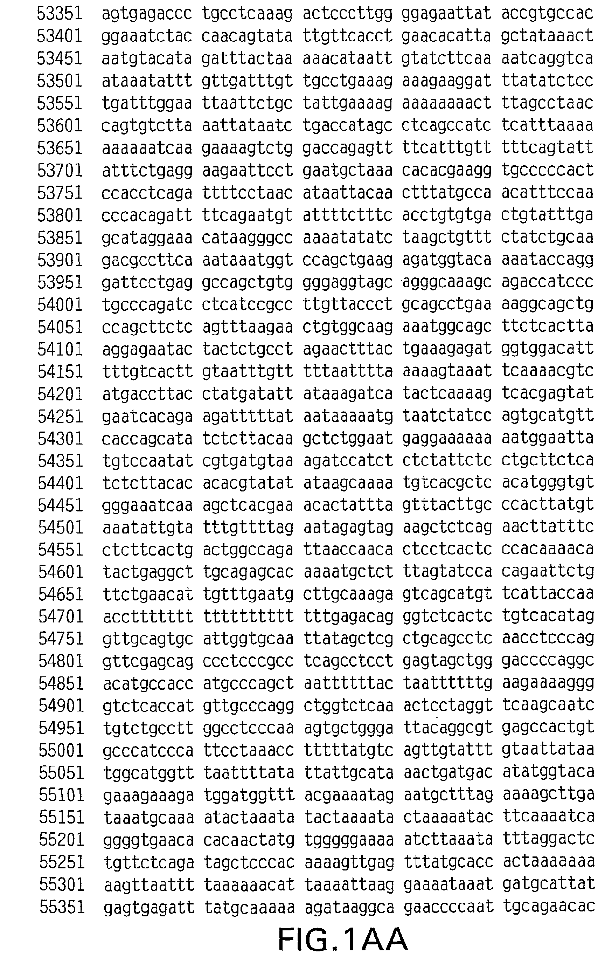

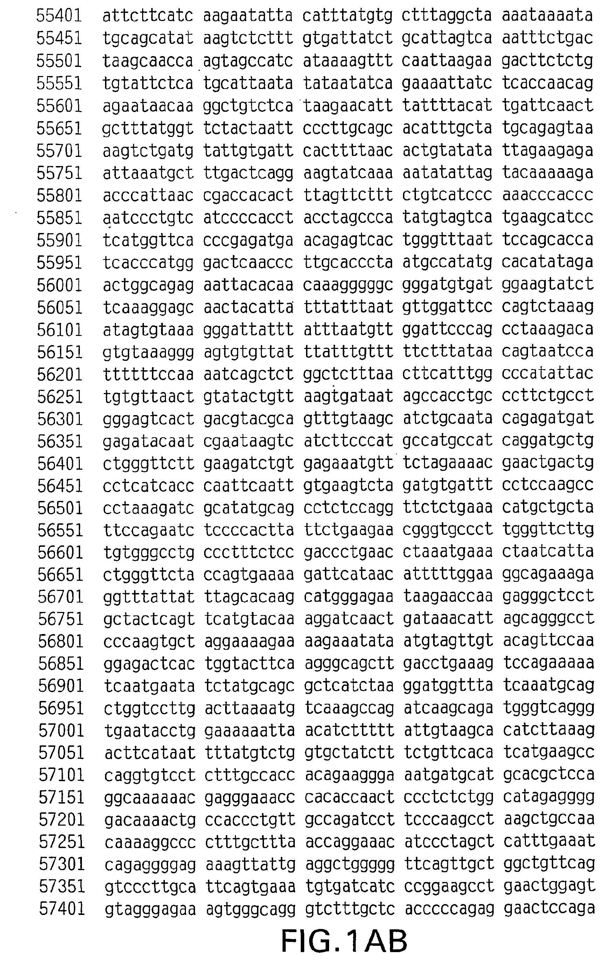

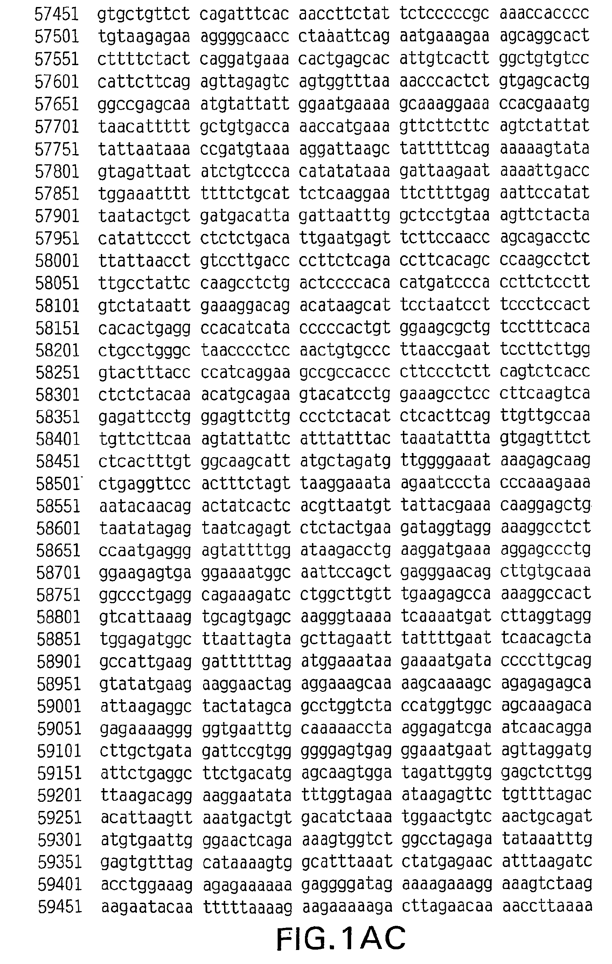

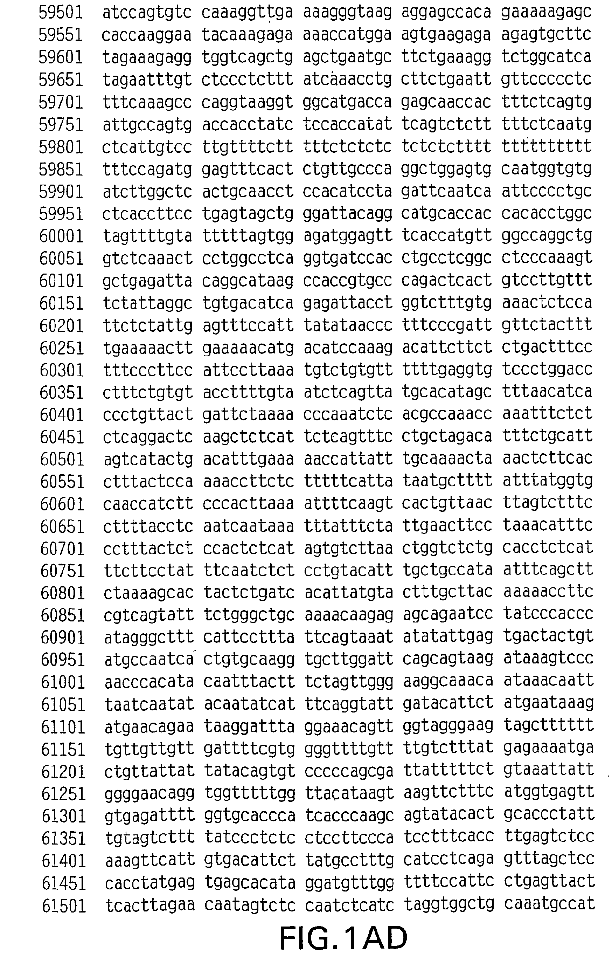

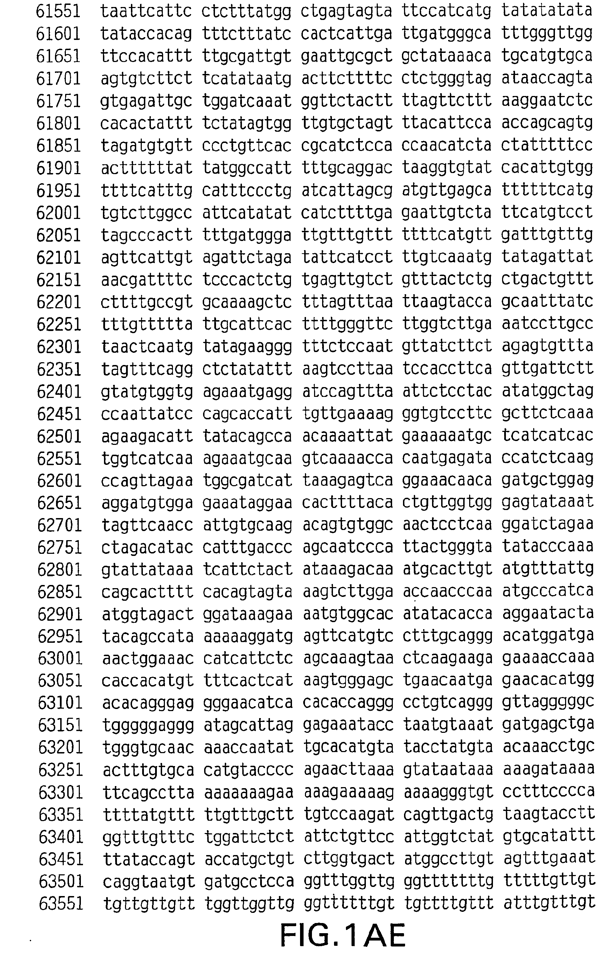

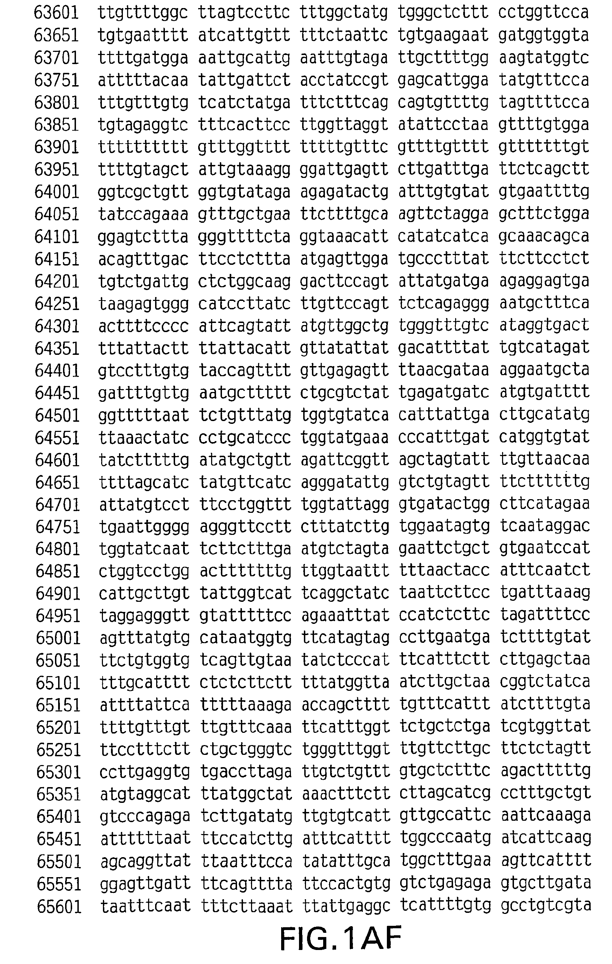

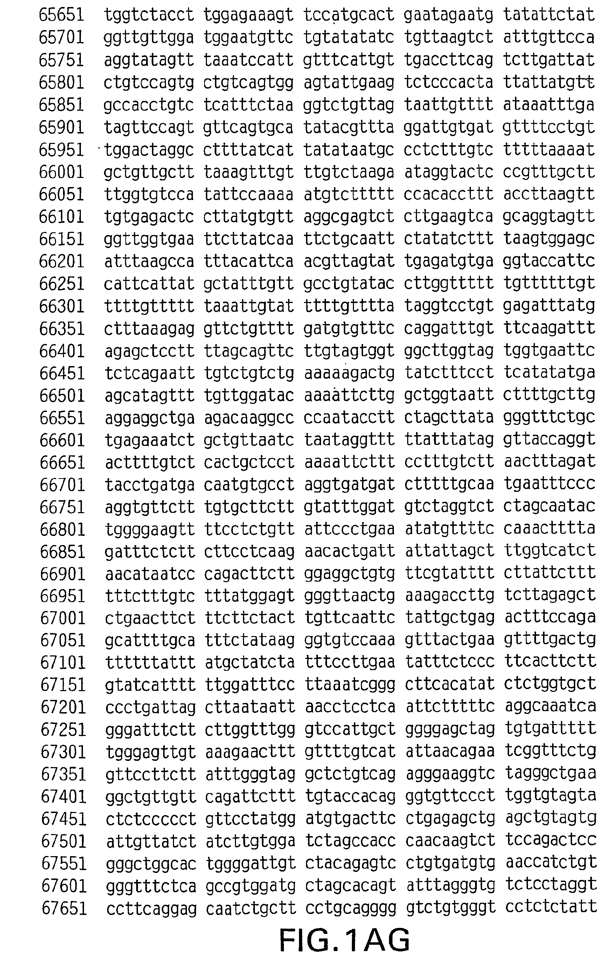

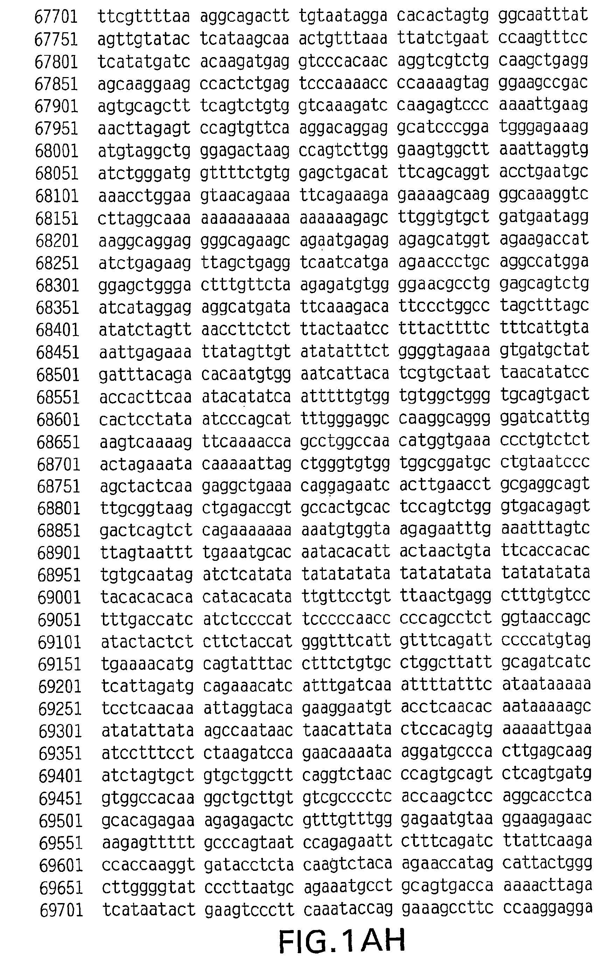

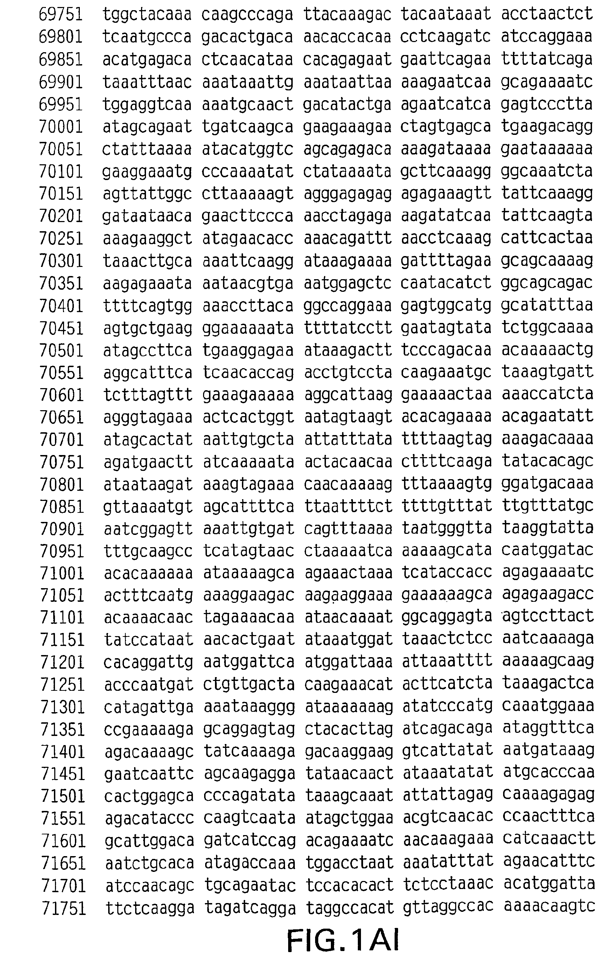

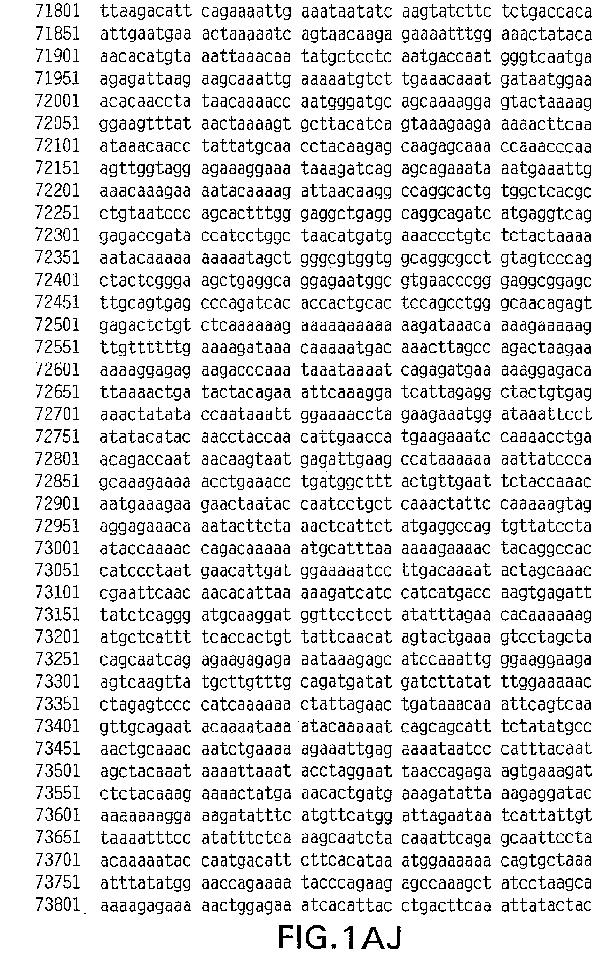

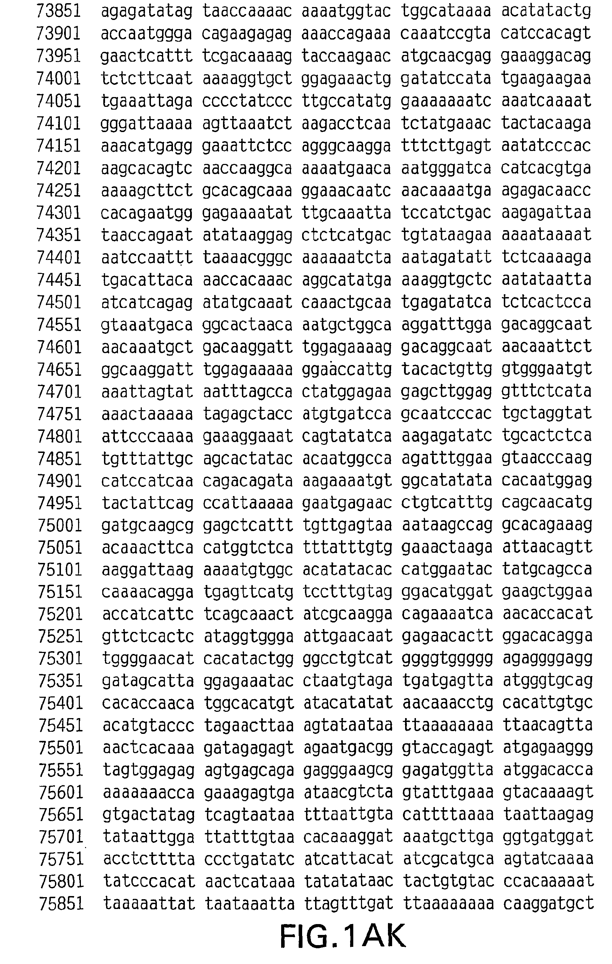

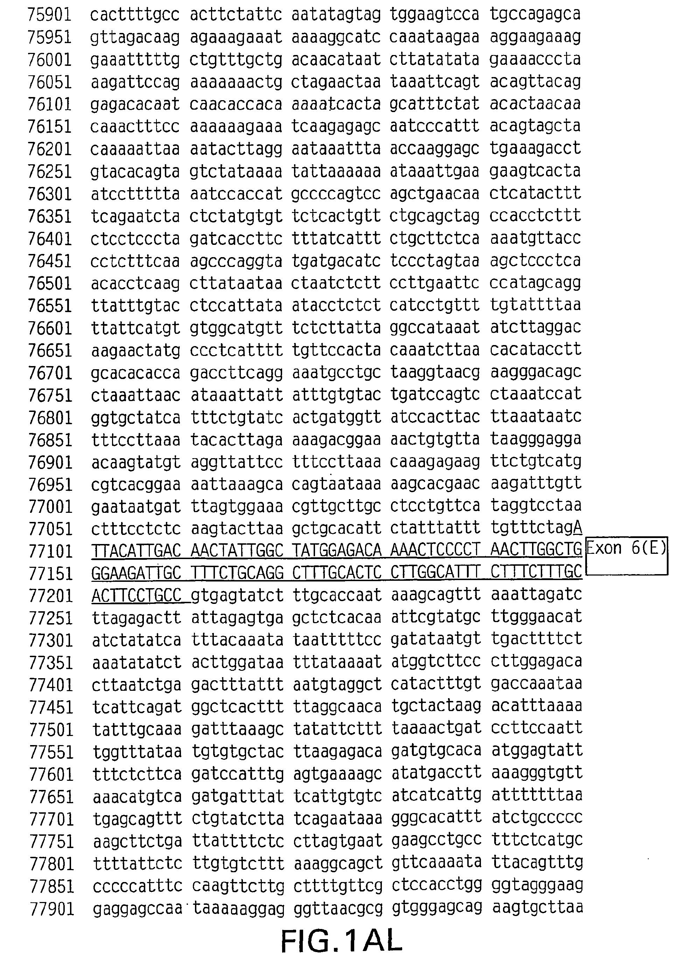

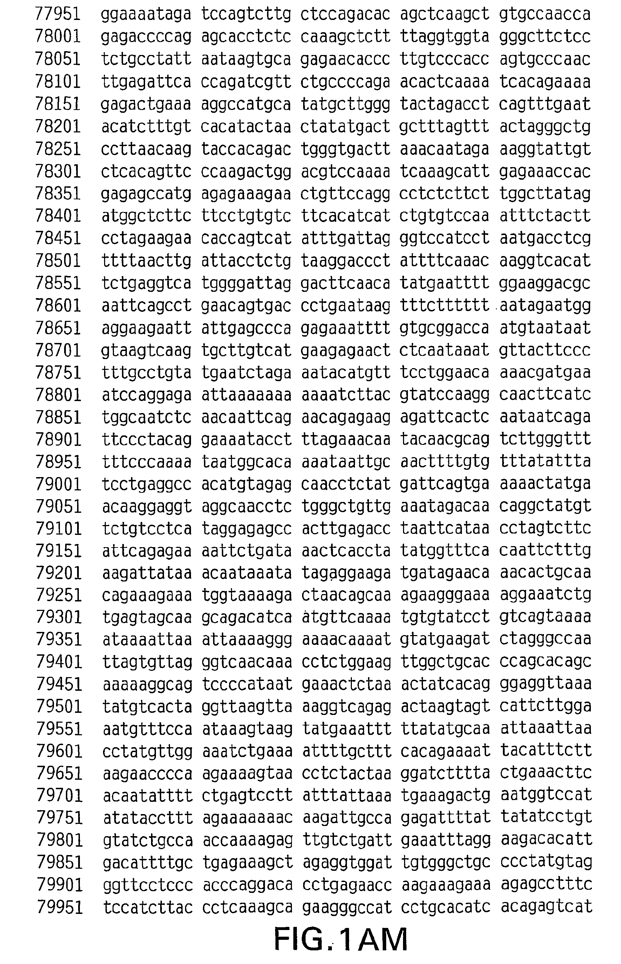

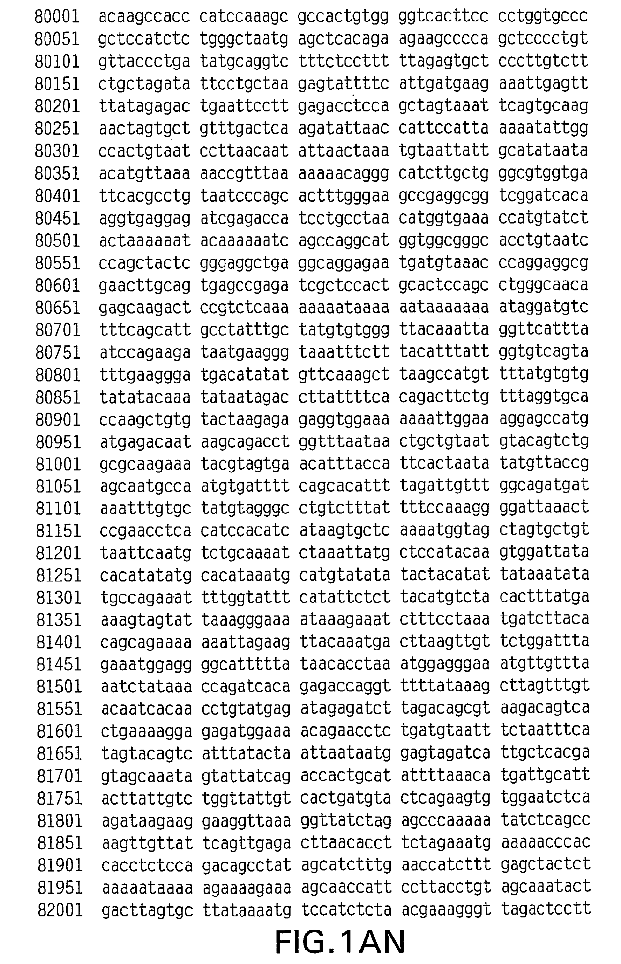

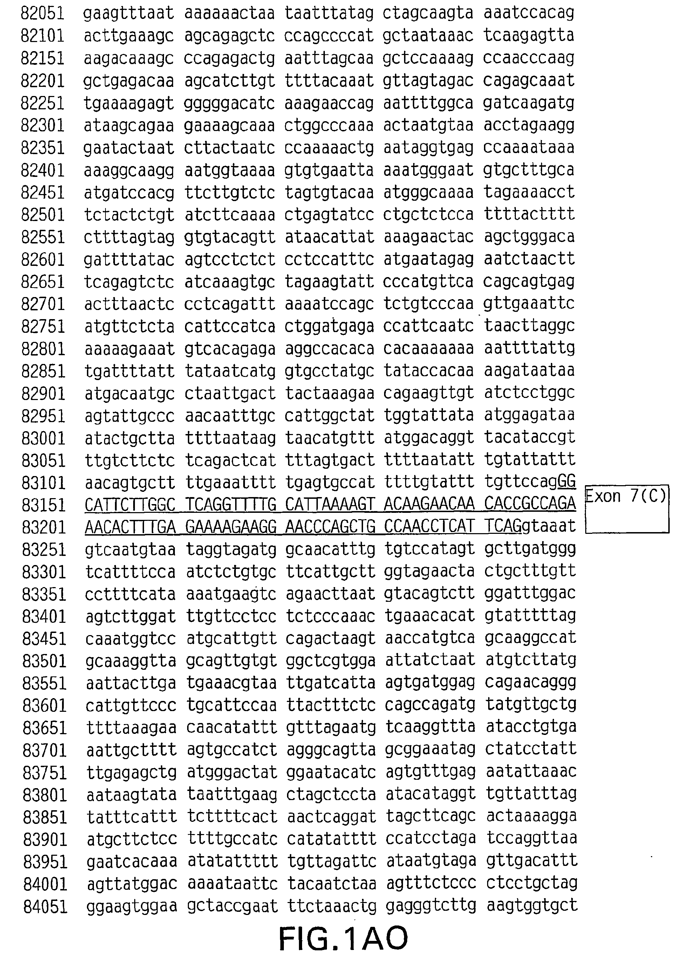

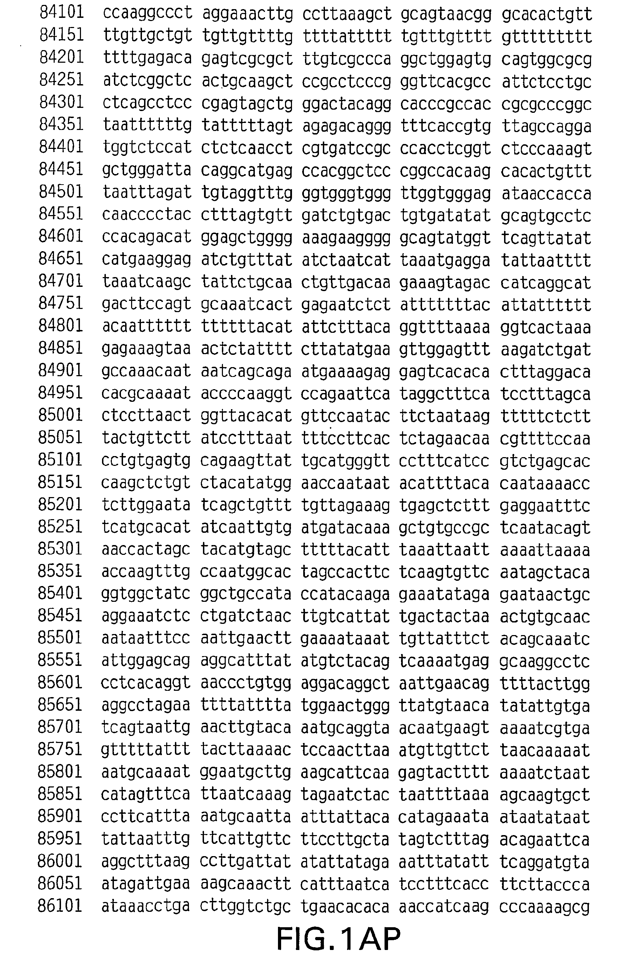

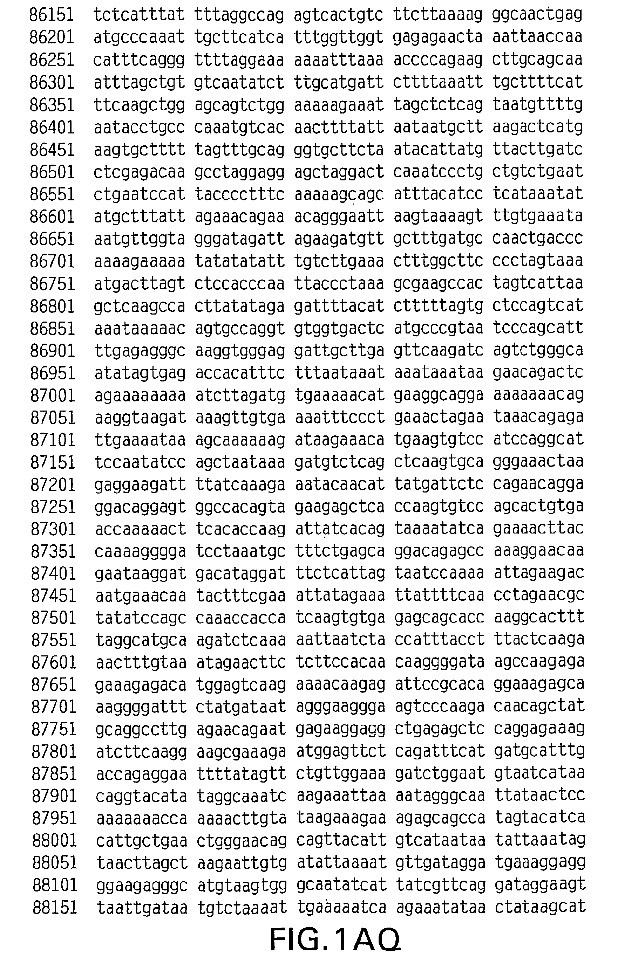

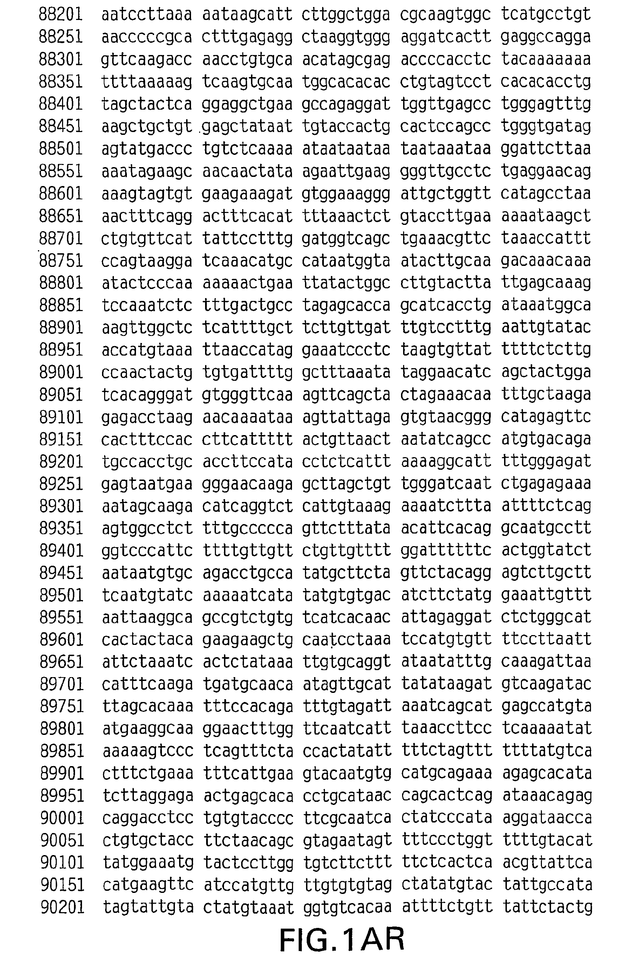

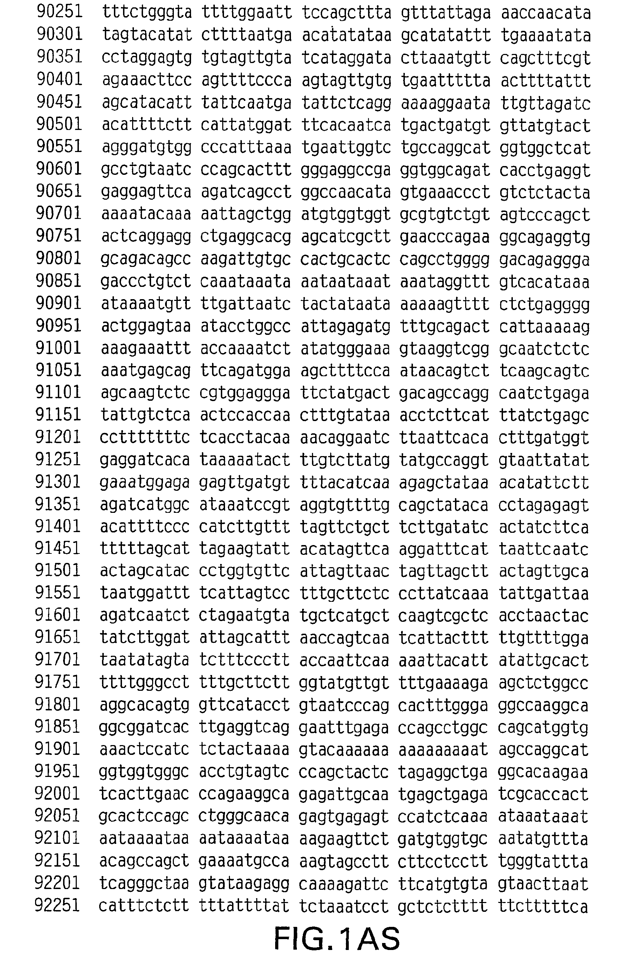

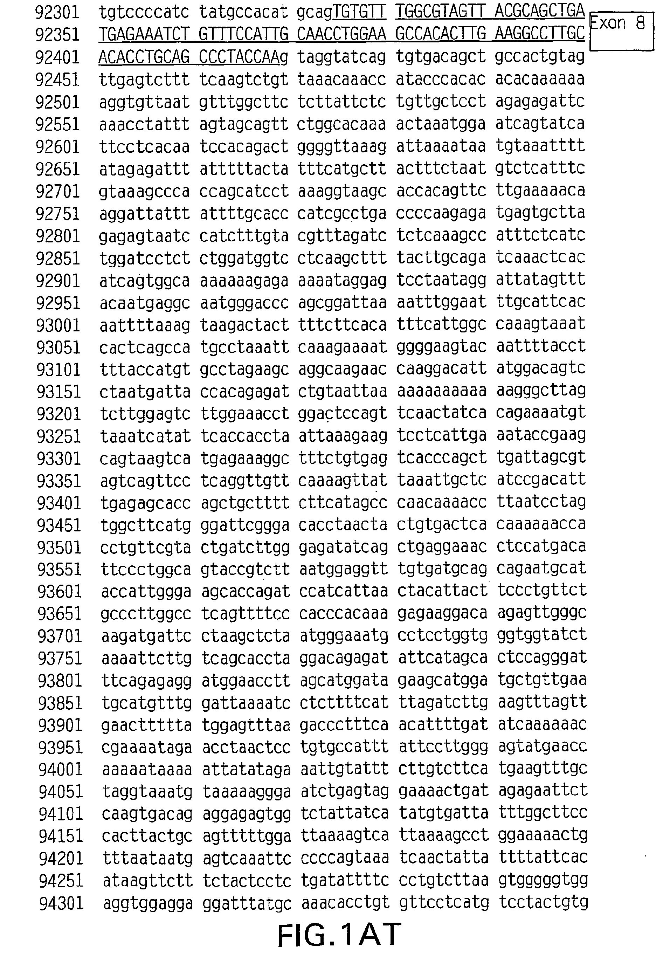

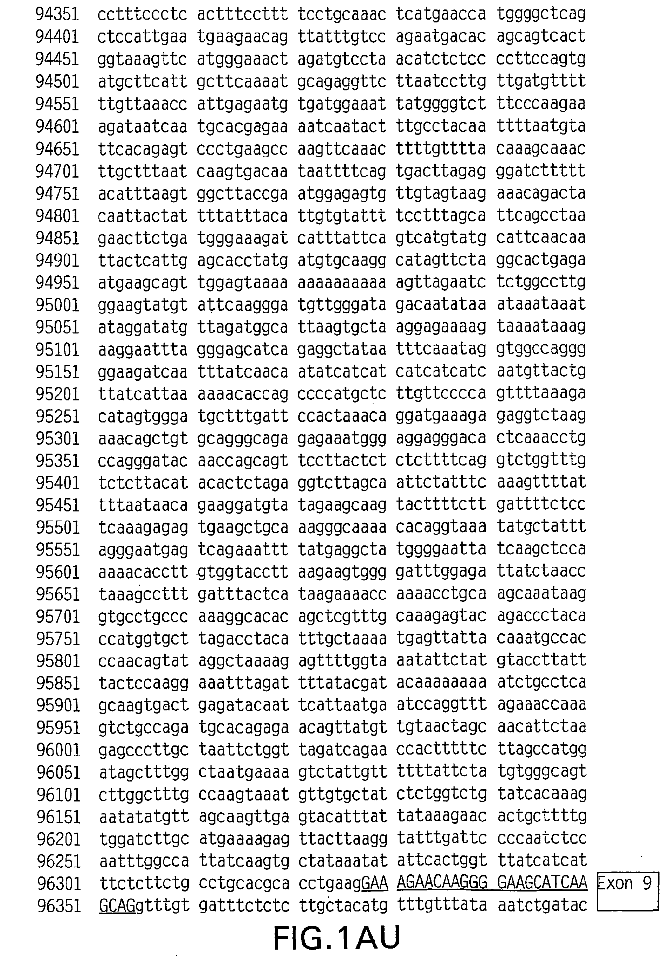

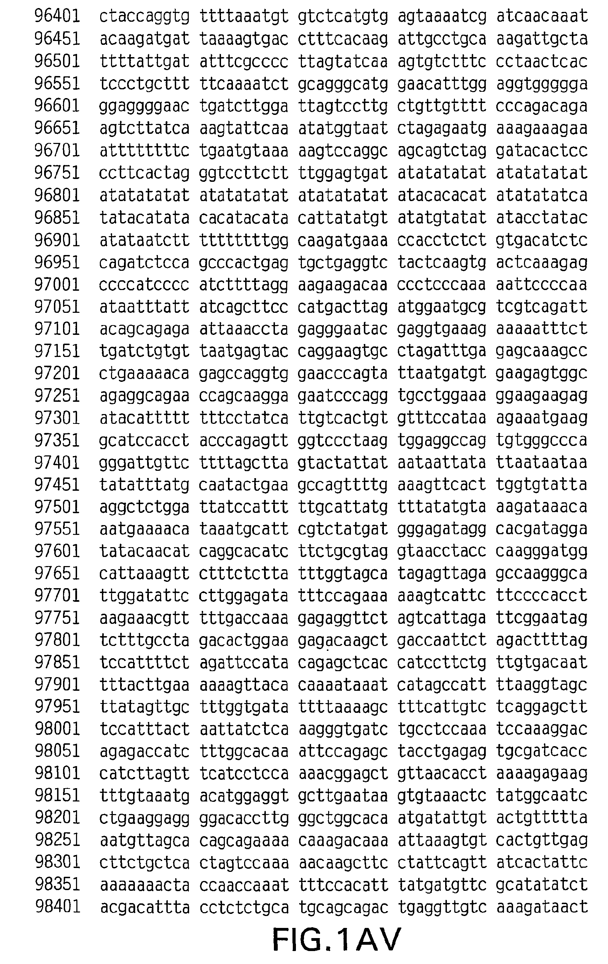

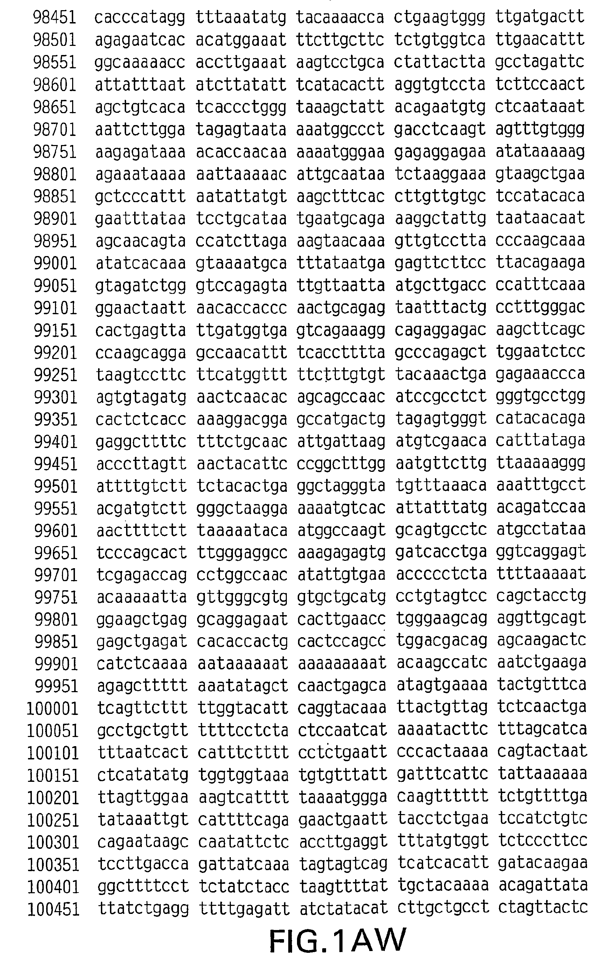

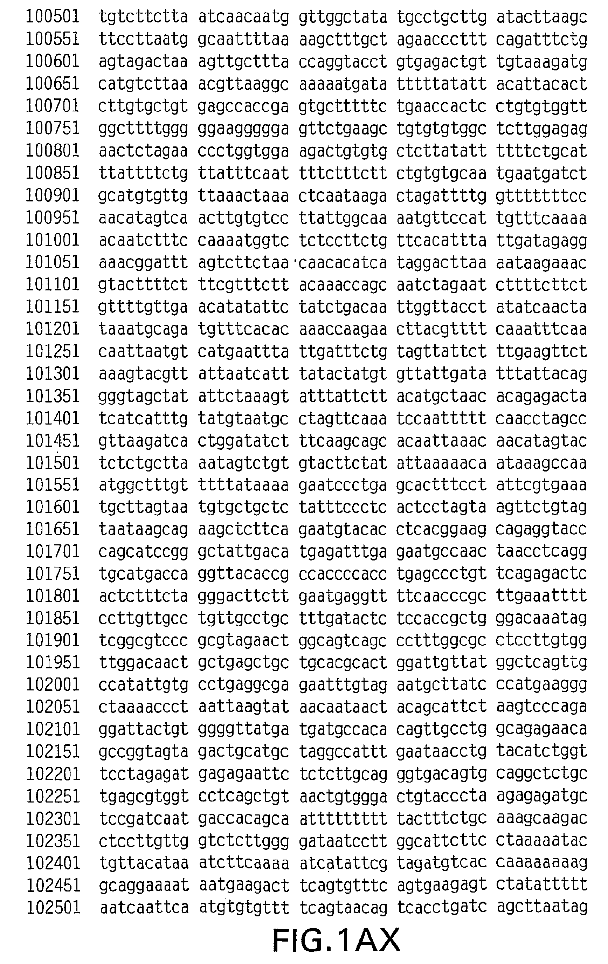

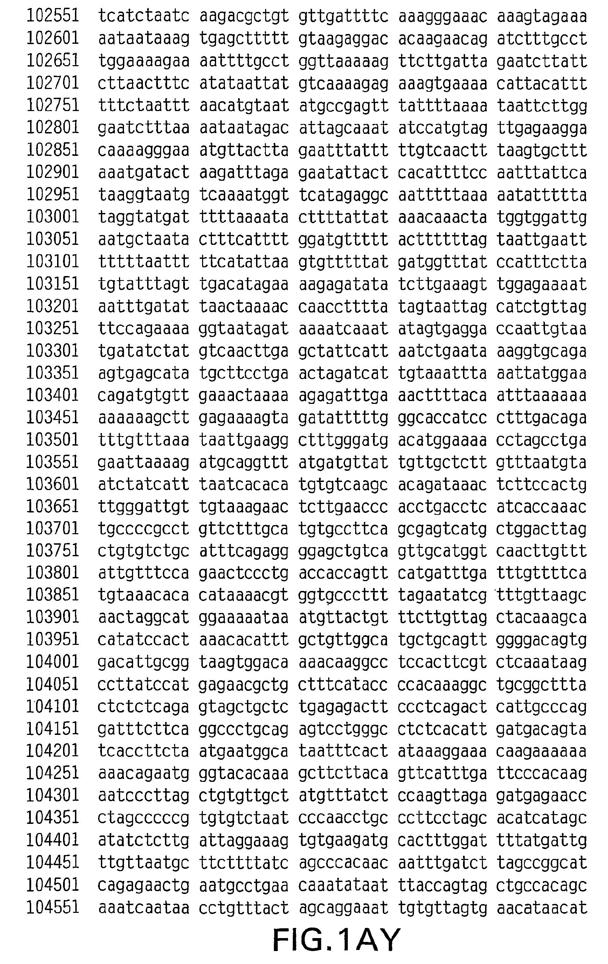

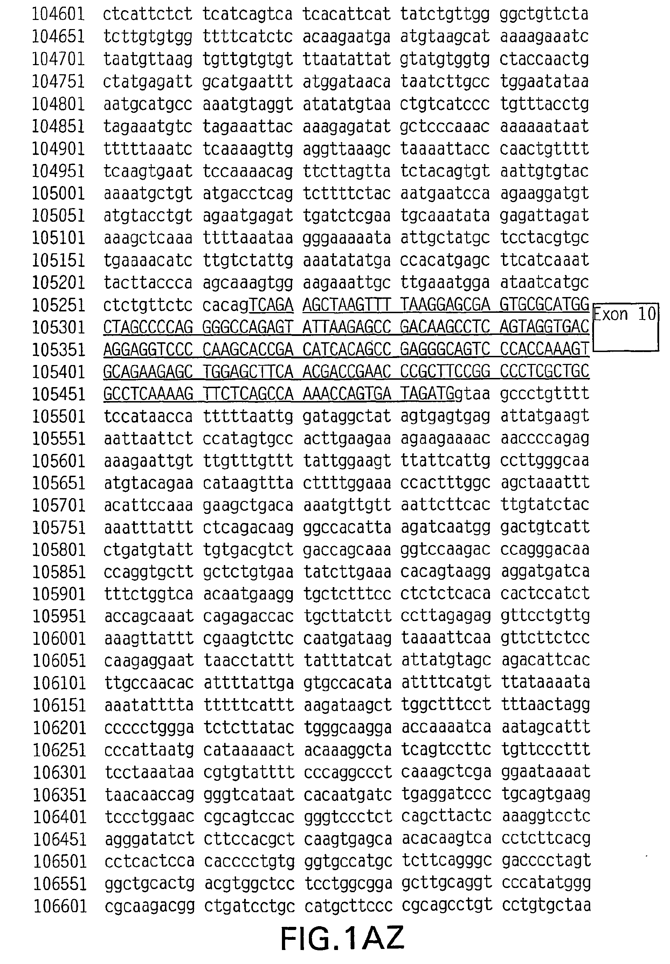

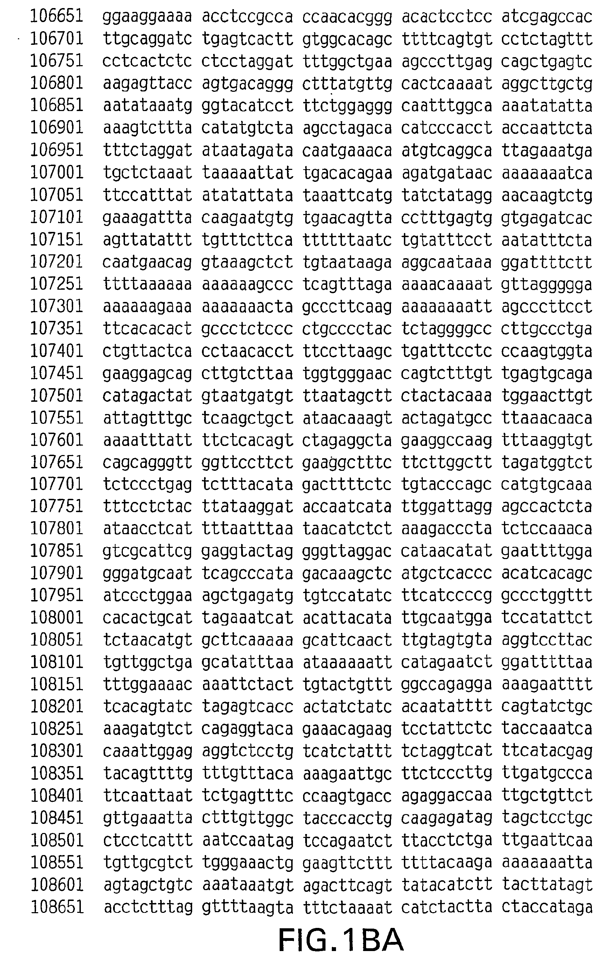

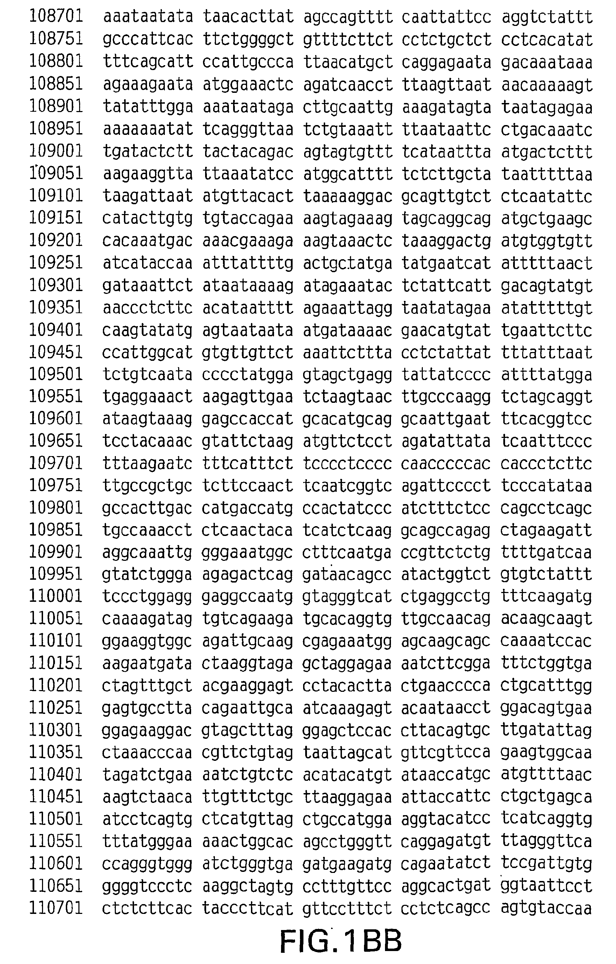

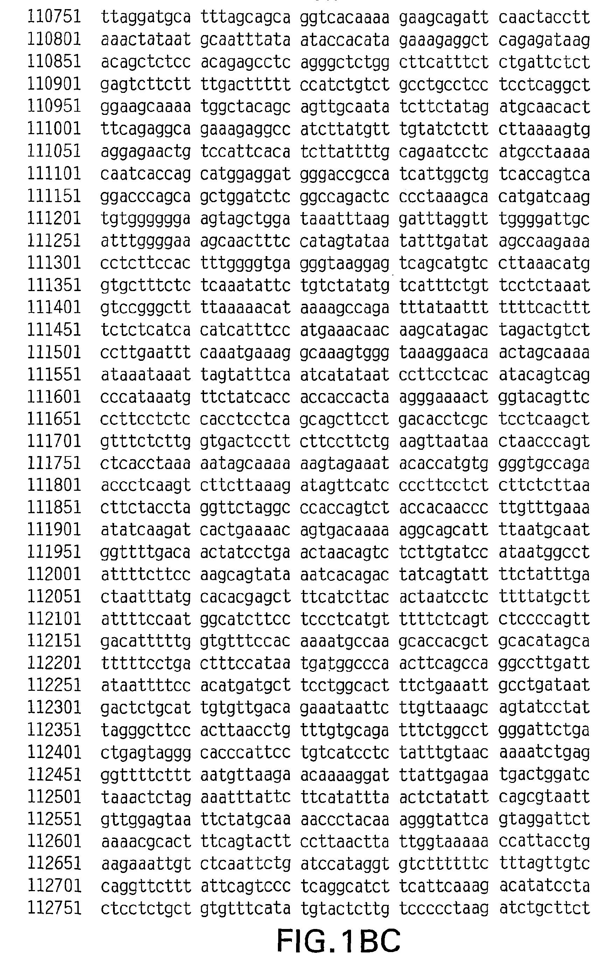

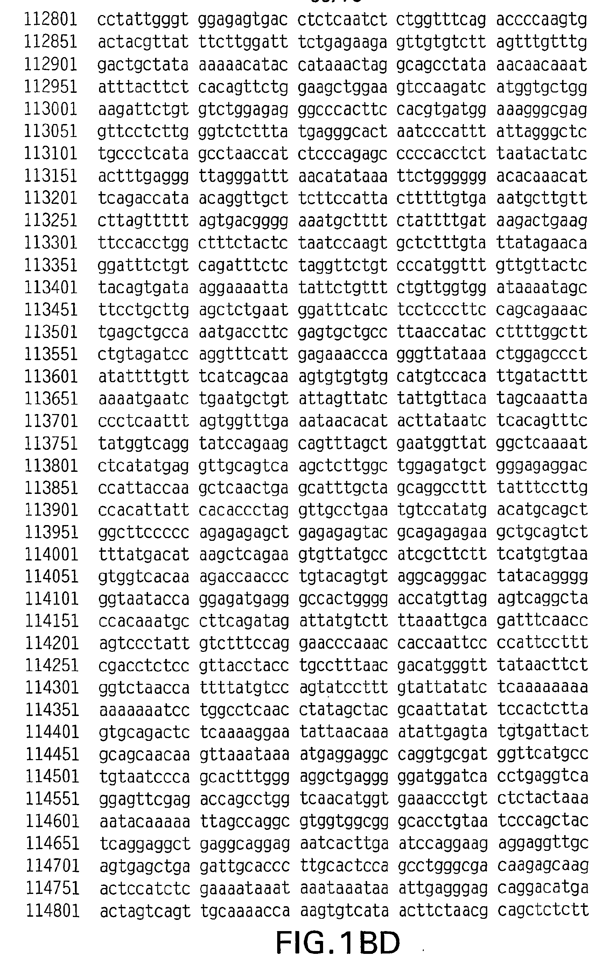

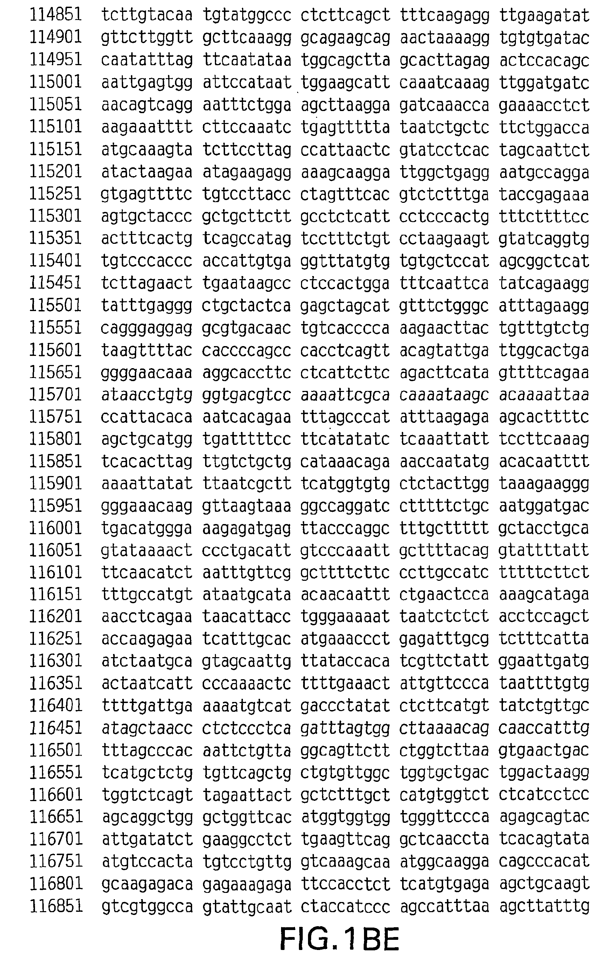

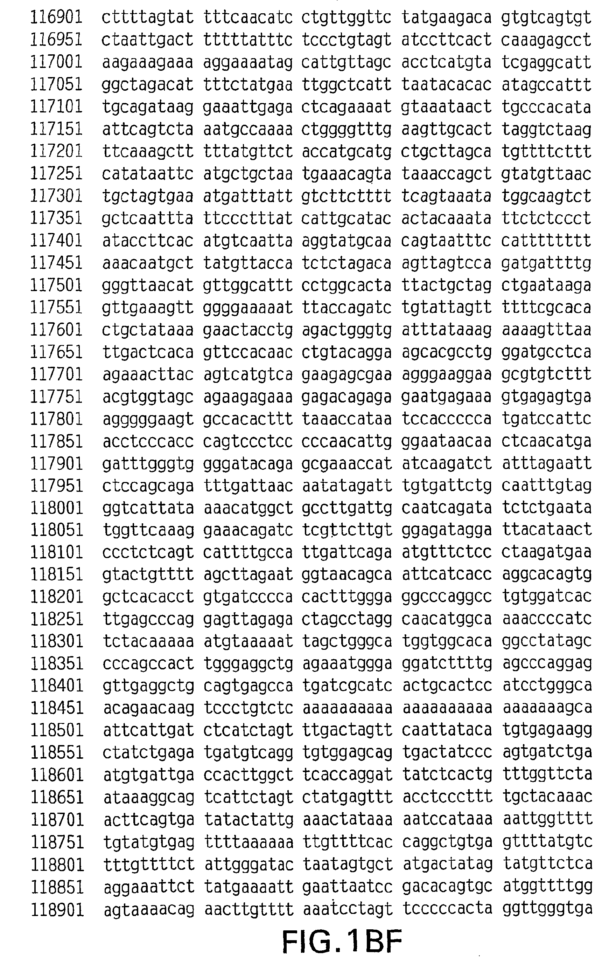

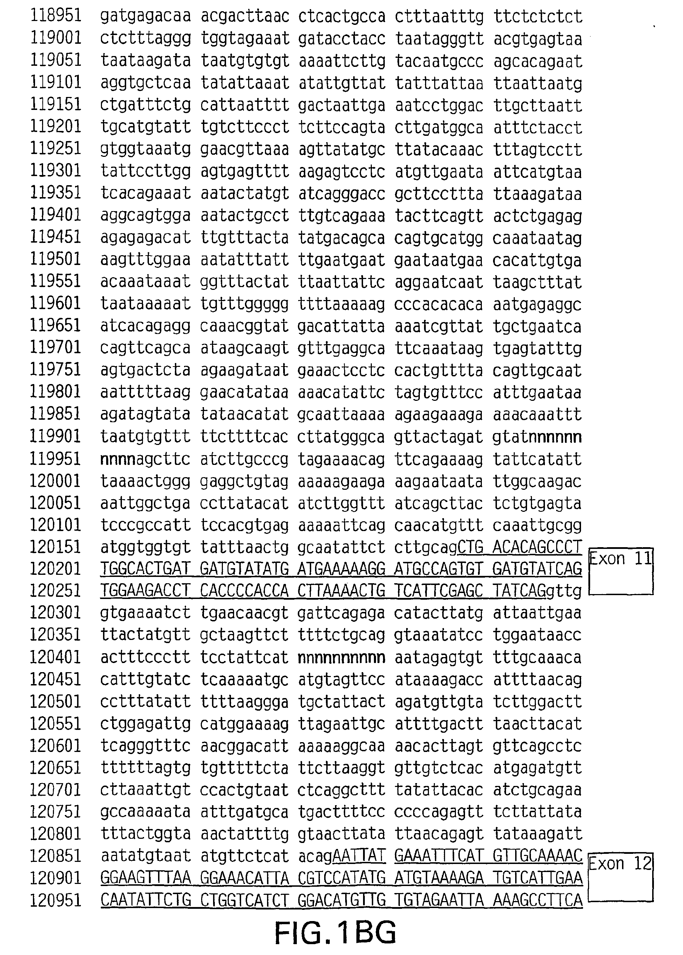

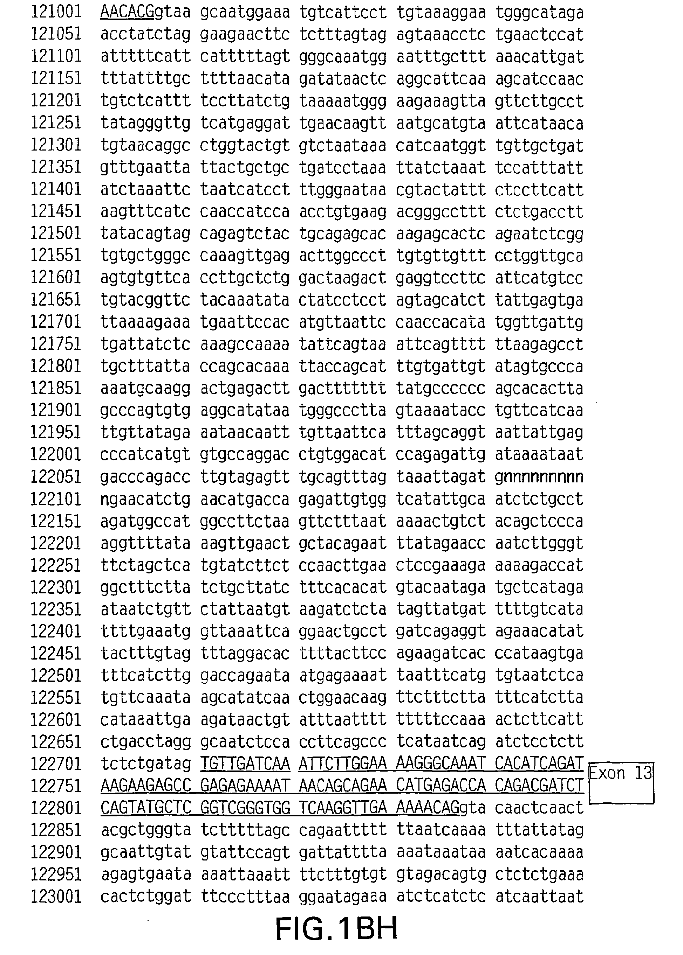

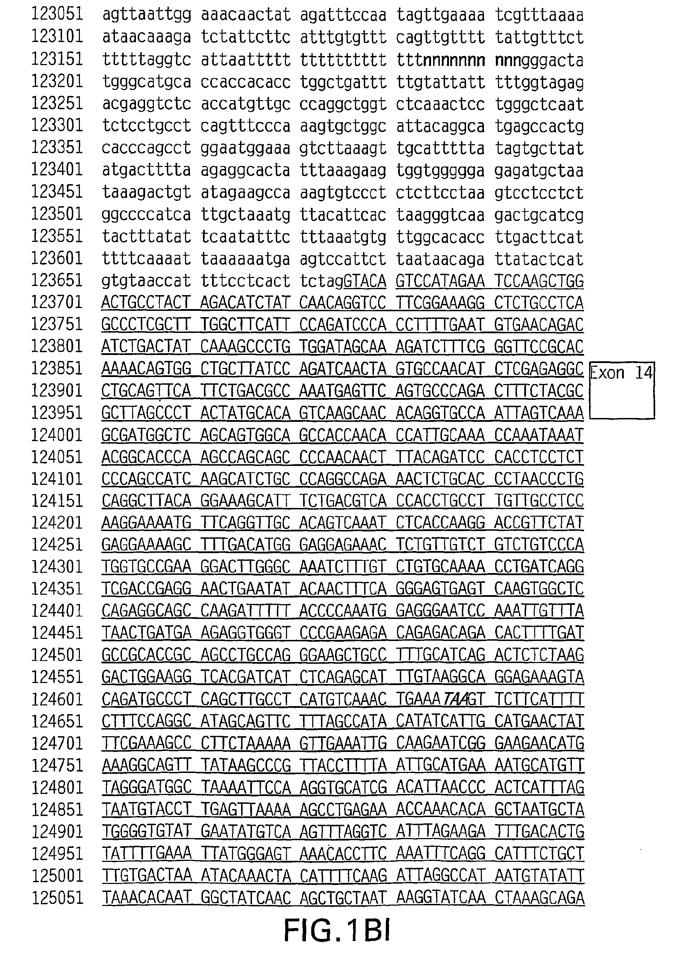

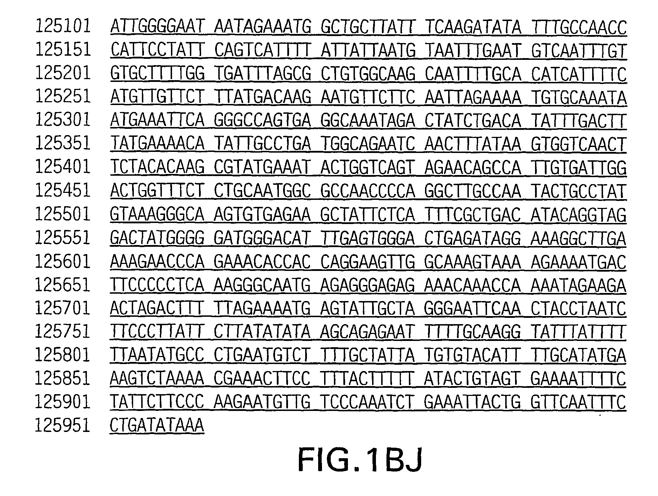

Figure 1A-AO shows the genomic DNA sequence of human KCNQ5 (SEQ.ID.NO.:1). Underlined nucleotides in capitals represent exons. The start ATG codon in exon 1 and the stop TAA codon in exon 14 are shown in bold italics. The D6D280 genetic marker and a phosphoglycerate pseudogene are underlined in bold. The exact lengths of the gaps between exons 1 and 2, 2 and 3, 10 and 11, 11 and 12, 12 and 13, and 13 and 14 are unknown. These gaps are represented as runs of ten bold ns for the sake of convenience.

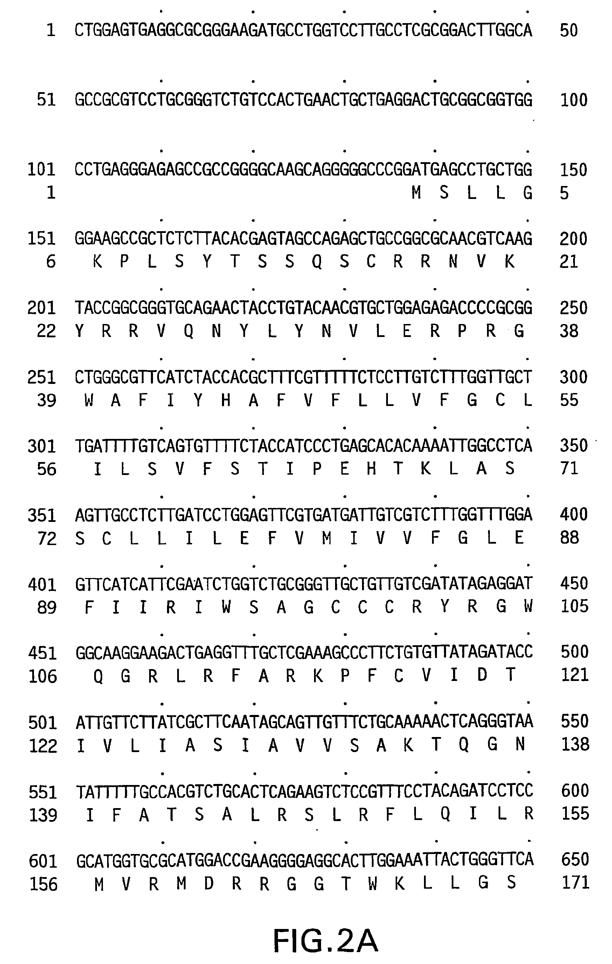

Figure 2A-E shows the nucleotide sequence (SEQ.ID.NO.:2) and encoded amino acid sequence (SEQ.ID.NO.:3) of human KCNQ5 cDNA. The ATG start codon is at position 138; the TAA stop codon is at position 2,676.

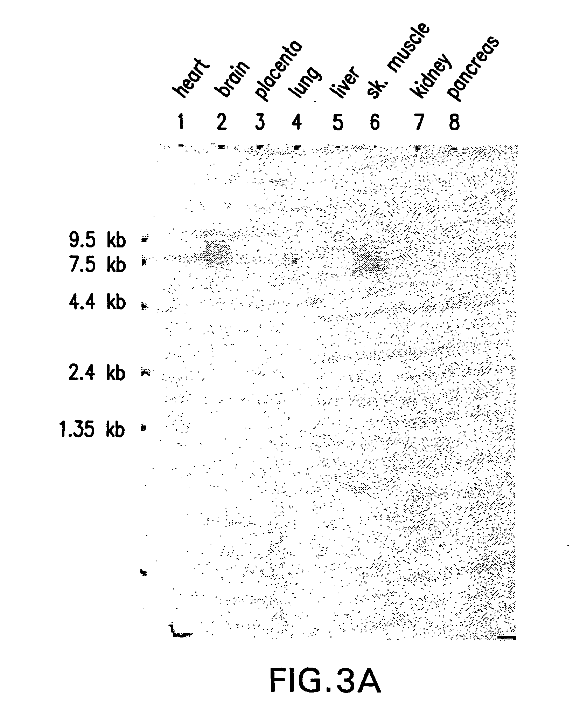

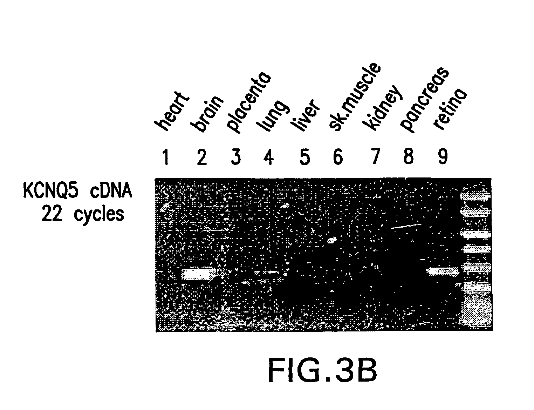

Figure 3A shows the results of a Northern blot of KCNQ5 mRNA expression in various human tissues. Figure 3B shows the results of RT-PCR analysis of KCNQ5 mRNA expression in various human tissues.

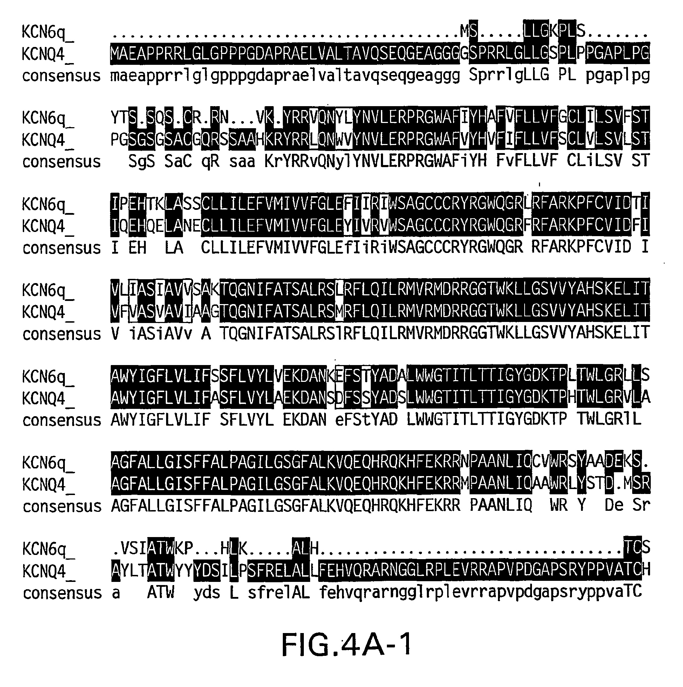

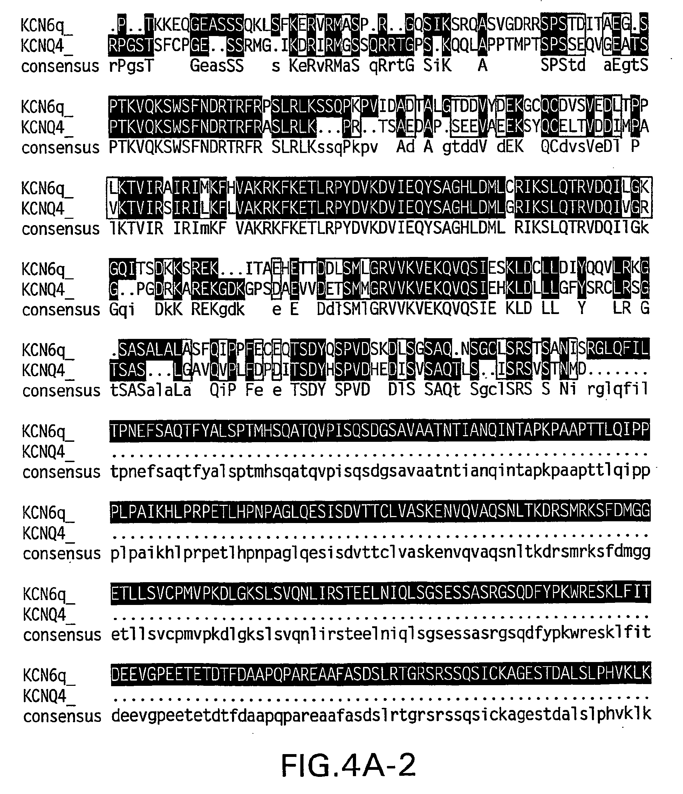

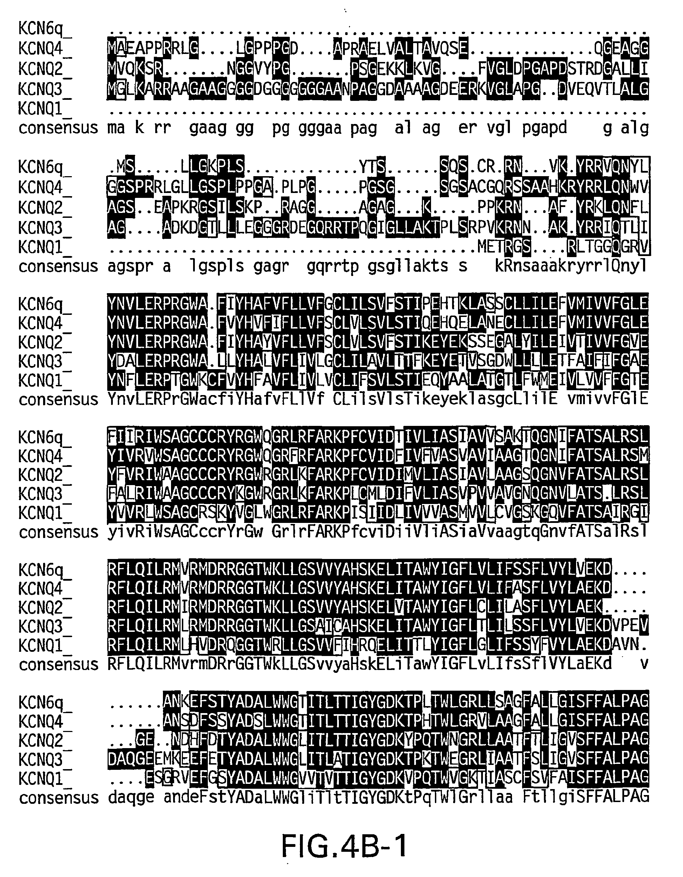

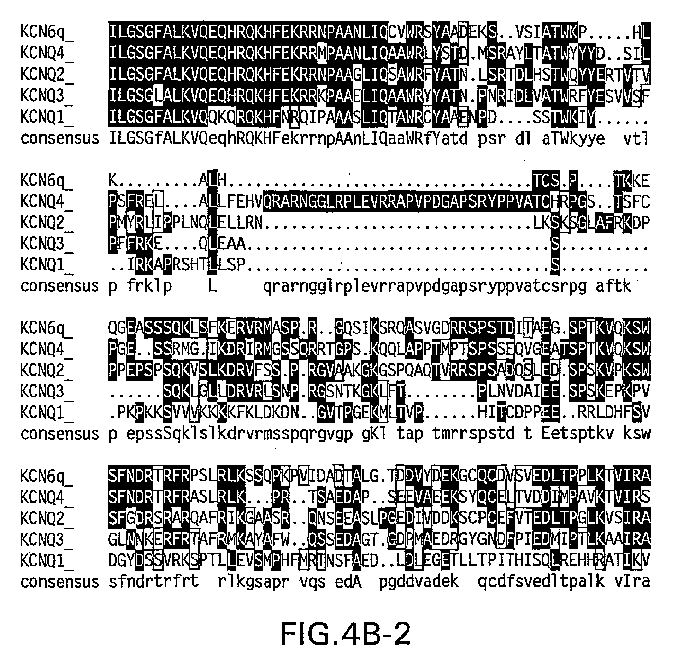

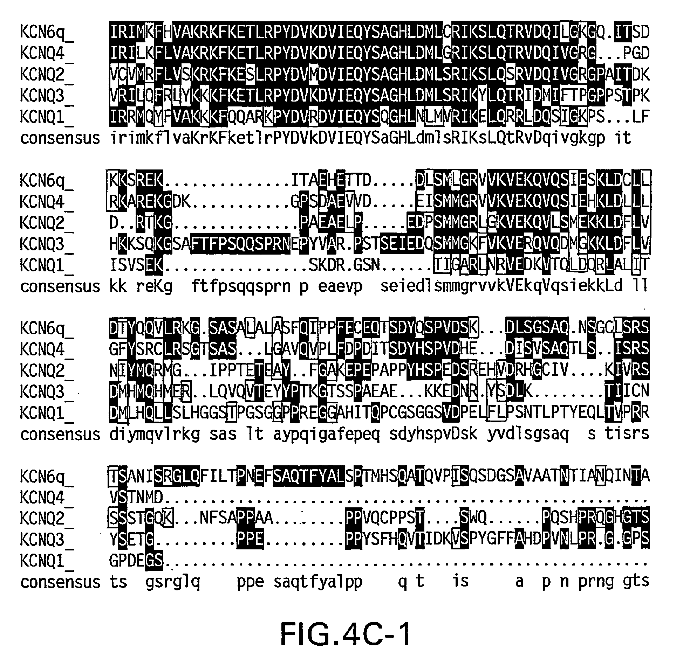

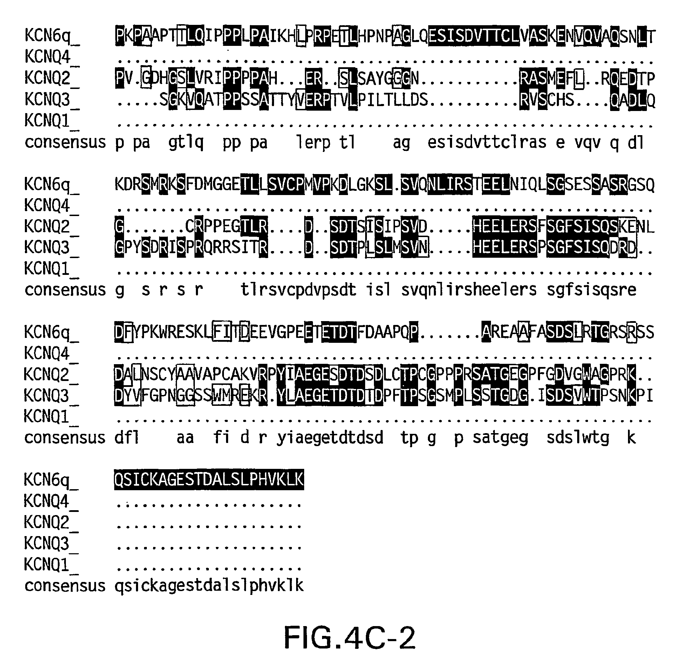

Figure 4A shows a sequence alignment of human KCNQ5 protein (SEQ.ID.NO.:3) with human KCNQ4 protein (SEQ.ID.NO.:4). The consensus sequence shown is (SEQ.ID.NO.:5). Figure 4B-C shows a multiple sequence alignment between human KCNQ5 protein (SEQ.ID.NO.:3), human KCNQ1 protein (SEQ.ID.NO.:43), human KCNQ2 protein (SEQ.ID.NO.:6), human KCNQ3 protein (SEQ.ID.NO.:7), and human KCNQ4 protein (SEQ.ID.NO.:4). The consensus sequence shown is (SEQ.ID.NO.:8).

### DETAILED DESCRIPTION OF THE INVENTION

For the purposes of this invention:

"Substantially free from other proteins" means at least 90%, preferably 95%, more preferably 99%, and even more preferably 99.9%, free of other proteins. Thus, a KCNQ5 protein preparation that is substantially free from other proteins will contain, as a percent of its total protein, no more than 10%, preferably no more than 5%, more preferably no more than 1%, and even more preferably no more than 0.1%, of non- KCNQ5 proteins. Whether a given KCNQ5 protein preparation is substantially free from other proteins can be determined by such conventional techniques of assessing protein purity as, *e.g.*, sodium dodecyl sulfate polyacrylamide gel electrophoresis (SDS-PAGE) combined with appropriate detection methods, *e.g.*, silver staining or immunoblotting.

"Substantially free from other nucleic acids" means at least 90%, preferably 95%, more preferably 99%, and even more preferably 99.9%, free of other nucleic acids. Thus, a KCNQ5 DNA preparation that is substantially free from other nucleic acids will contain, as a percent of its total nucleic acid, no more than 10%, preferably no more than 5%, more preferably no more than 1%, and even more preferably no more than 0.1%, of non- KCNQ5 nucleic acids. Whether a given KCNQ5 DNA preparation is substantially free from other nucleic acids can be determined by such conventional techniques of assessing nucleic acid purity as, *e.g.*, agarose gel electrophoresis combined with appropriate staining methods, *e.g.*, ethidium bromide staining, or by sequencing.

A "conservative amino acid substitution" refers to the replacement of one amino acid residue by another, chemically similar, amino acid residue. Examples of such conservative substitutions are: substitution of one hydrophobic residue (isoleucine, leucine, valine, or methionine) for another; substitution of one polar residue for another polar residue of the same charge (*e.g*., arginine for lysine; glutamic acid for aspartic acid); substitution of one aromatic amino acid (tryptophan, tyrosine, or phenylalanine) for another.

A polypeptide has "substantially the same biological activity as KCNQ5" if that polypeptide conducts a voltage-gated potassium current when expressed in appropriate cell types and has an amino acid sequence that is at least about 50% identical to SEQ.ID.NO.:3 when measured by such standard programs as BLAST or FASTA.

The present invention relates to the identification and cloning of KCNQ5, a gene encoding a novel voltage-gated potassium channel. The human KCNQ5 gene is located on chromosome 6q14, in a chromosomal region that contains genes that have been linked with the occurrence of at least three diseases: Stargardt-like macular dystrophy, cone-rod dystrophy, and Salla disease.

The human KCNQ5 gene is present on PAC clones from chromosomal region 6q14. PAC141B1 was sequenced and KCNQ5 was found based on homology between the genomic sequences of KCNQ5 present in PAC 141B and the sequences of known potassium channel genes. PAC 141B1 is available from Research Genetics, Inc., Huntsville, AL, as an individual clone from the RPCI4,5,6 Library (catalog number CTLI.C). Using PCR primers derived from the KCNQ5 sequence, a cDNA sequence representing the coding region as well as a large portion of the 3'-UTR of KCNQ5 was isolated from a human fetal brain cDNA library. Comparison of this cDNA clone with the genomic sequences present in PAC141B1, as well as KCNQ5 sequences found in PAC224H23, showed that exons 3-11 and portions of flanking intronic regions are present in PAC141B1. Exon 2 and flanking intronic regions were found in PAC224H23, while the rest of the KCNQ5 gene (exons 1, 12-14, and flanking intronic regions) was recovered from total human genomic DNA by using cDNA primers and a GenomeWalker kit from Clontech, Palo Alto, CA.

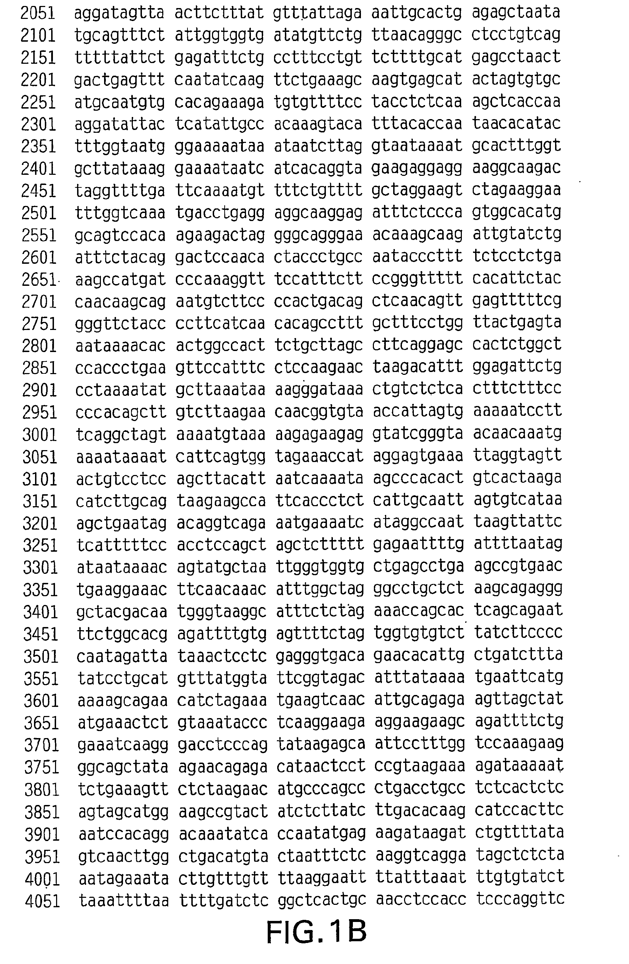

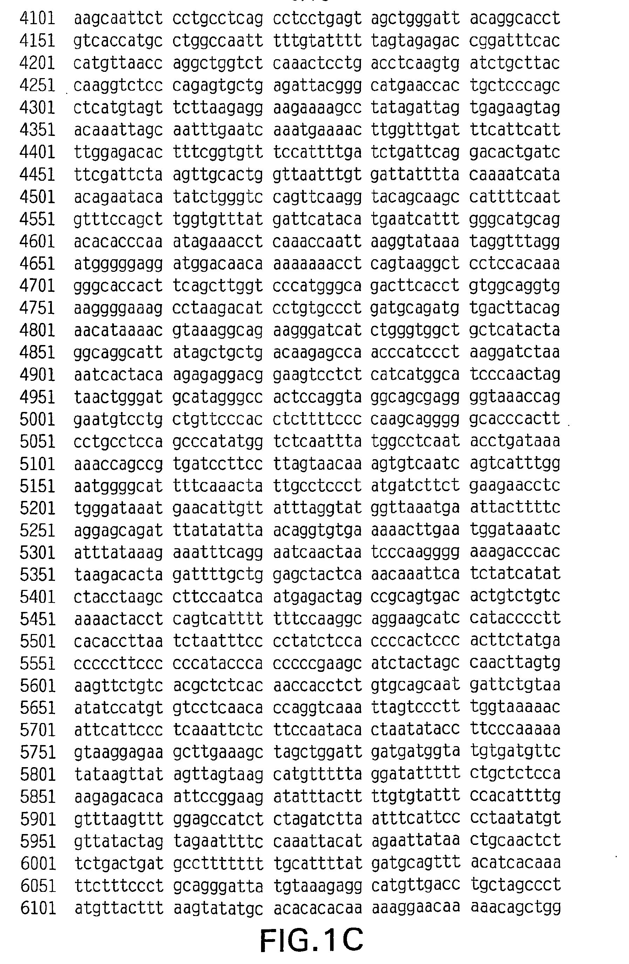

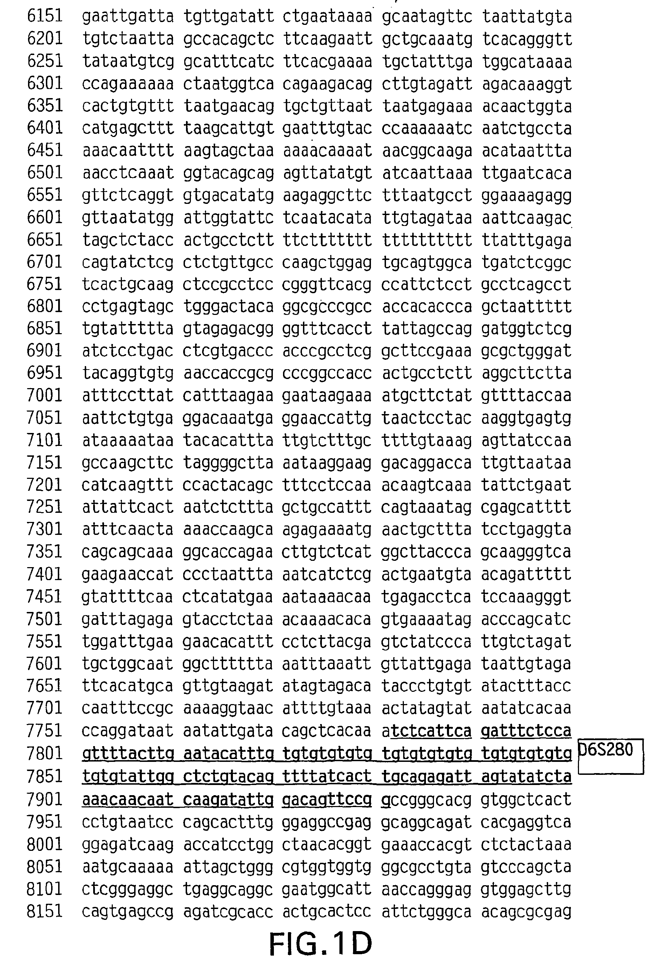

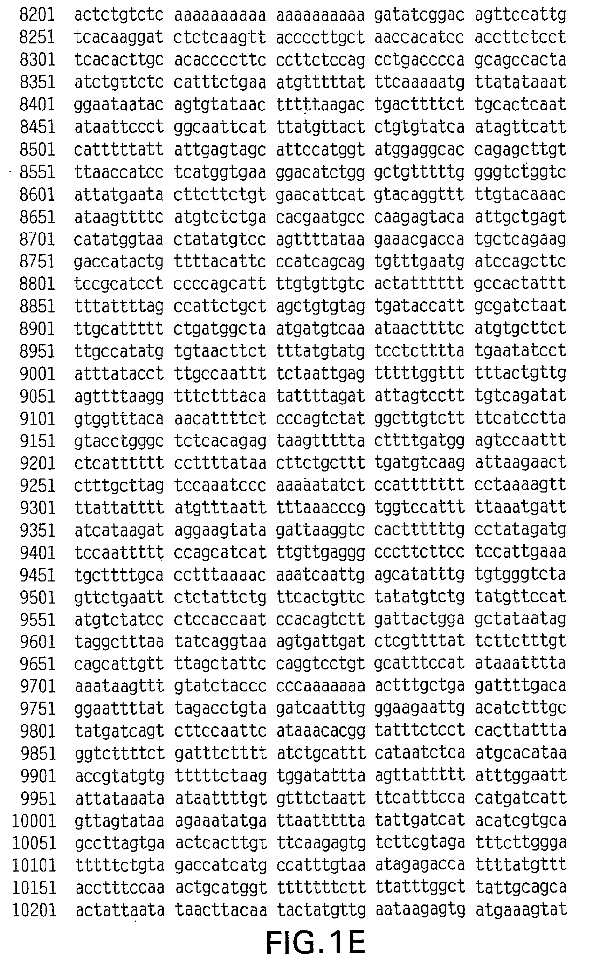

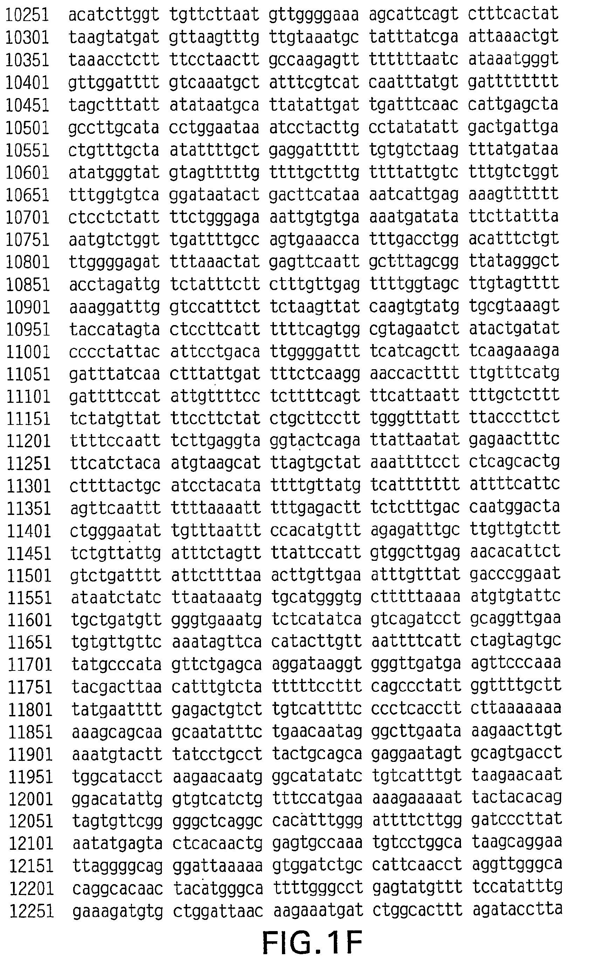

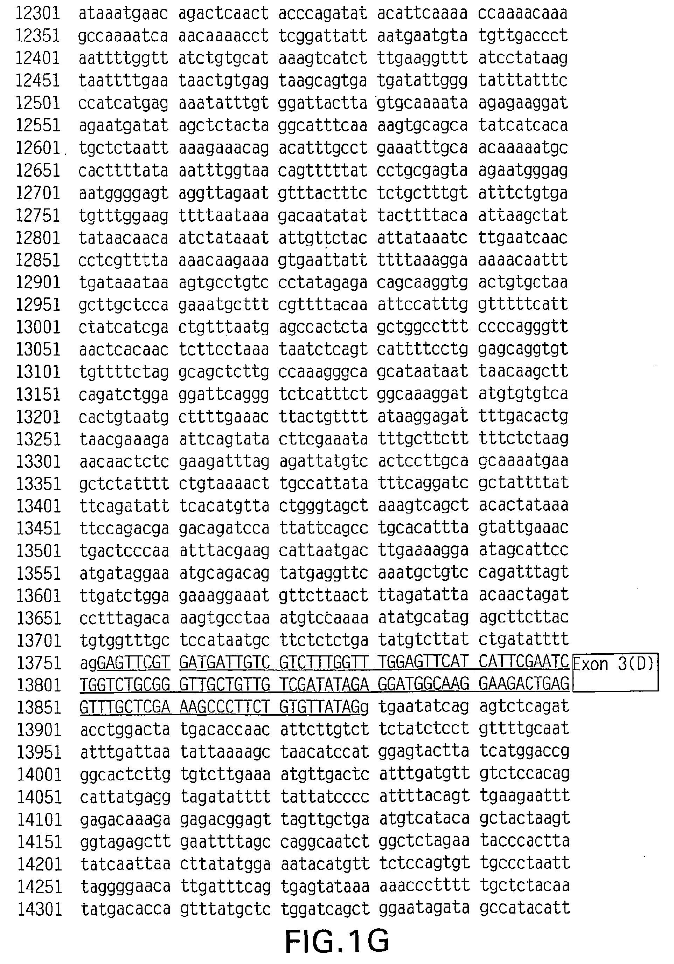

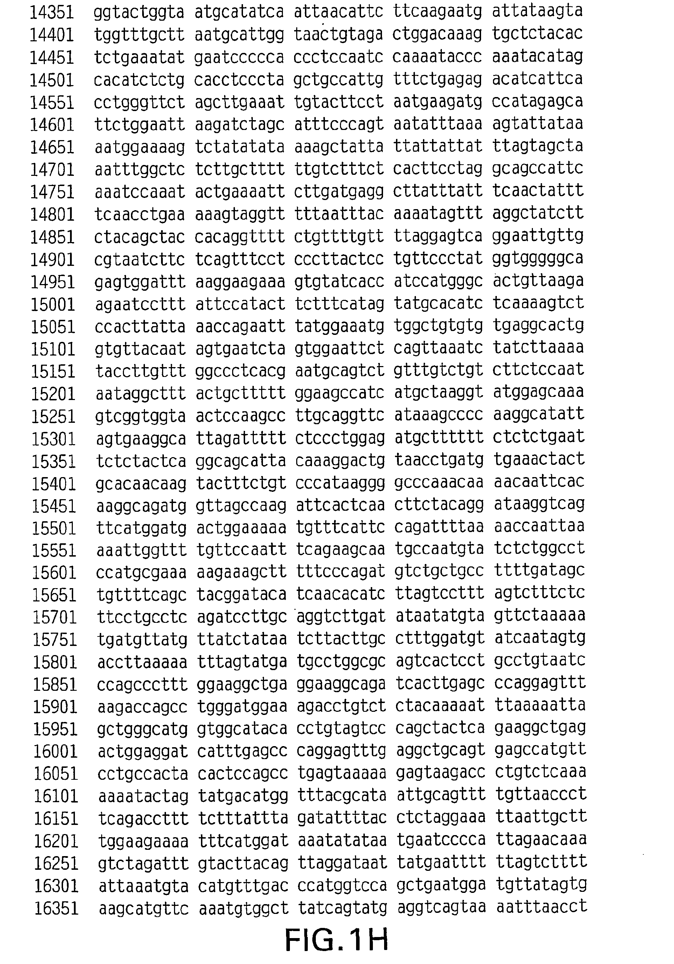

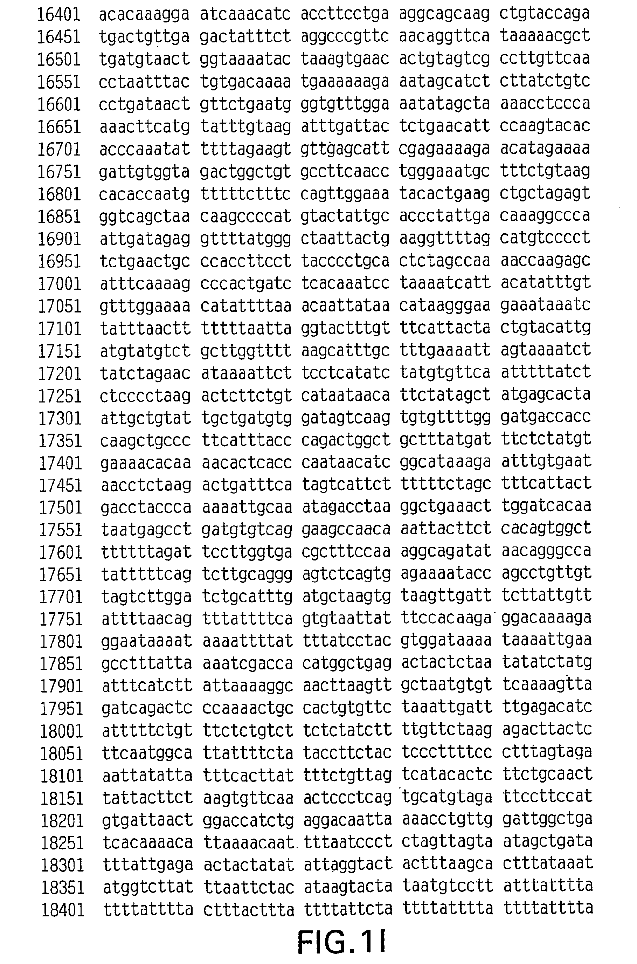

PAC141B1 and PAC224H23 are located in the region of the Salla disease gene (Leppänen et al., 1996, Genomics 37:62-67). PAC141B1 contains the polymorphic genetic marker D6S280 that is located in intron 3 of the KCNQ5 gene between exons 3 and 4 (Figure 1). D6S280 is the marker that detects the maximum LOD score of 5.5 (at genetic distance = 0) in families with Stargardt-like macular dystrophy (Stone et al., Arch. Ophthalmol. 112:765-772). D6S280 also detects a LOD score of 3.31 (at genetic distance = 0) in families with cone-rod dystrophy (Kelsell et al., 1998, Am. J. Hum. Genet. 63:274-279). These LOD scores indicate that D6S280 is very closely linked to, and probably is within, the gene for Stargardt-like macular dystrophy and cone-rod dystrophy. In view of these findings, it is likely that KCNQ5 is involved in Salla disease, Stargardt-like macular dystrophy, and cone-rod dystrophy.

That KCNQ5 should be involved with these three diseases is consistent with its expression pattern (see Figure 3A-B) which shows that KCNQ5 is expressed predominately in the retina and brain, in addition to being expressed in the skeletal muscle. Stargardt-like macular dystrophy and cone-rod dystrophy are inherited retinal diseases while Salla disease is a disorder that is characterized by early onset psychomotor retardation and ataxia.

Bioinformatic analysis revealed a striking homology of the KCNQ5 protein to a group of voltage gated potassium channels (KCNQ1, KCNQ2, KCNQ3, and KCNQ4; see Figure 4A-B). All of the typical amino acid motifs of these potassium channels are preserved in KCNQ5. A Kyte-Doolittle algorithm analysis predicts a transmembrane organization for KCNQ5 that is typical for this group of potassium channels. Mutations in members of this family of potassium channels have been shown to result in inherited disease (KCNQ2 and KCNQ3, epilepsy [Biervert et al., 1998, Science 279:403-406; Singh et al., 1998, Nature Genet. 18:25-29; Schroeder et al., Nature 1998, 396:687-690]; KCNQ4, a form of nonsyndromic dominant deafness [Kubisch et al., 1999, Cell 96:437-446], KCNQ1, congenital long QT syndrome which causes cardiac arrhythmias and sudden death [Splawski et al., 1997, N. Engl. J. Med. 336:1562-1567]).

The present invention provides DNA encoding KCNQ5 that is substantially free from other nucleic acids. The present invention also provides recombinant DNA molecules encoding KCNQ5. The present invention provides DNA molecules substantially free from other nucleic acids comprising the nucleotide sequence shown in Figure 1 as SEQ.ID.NO.:1. Analysis of SEQ.ID.NO.:1 revealed that this genomic sequence defines a gene having 14 exons. These exons collectively have an open reading frame that encodes a protein of 846 amino acids.

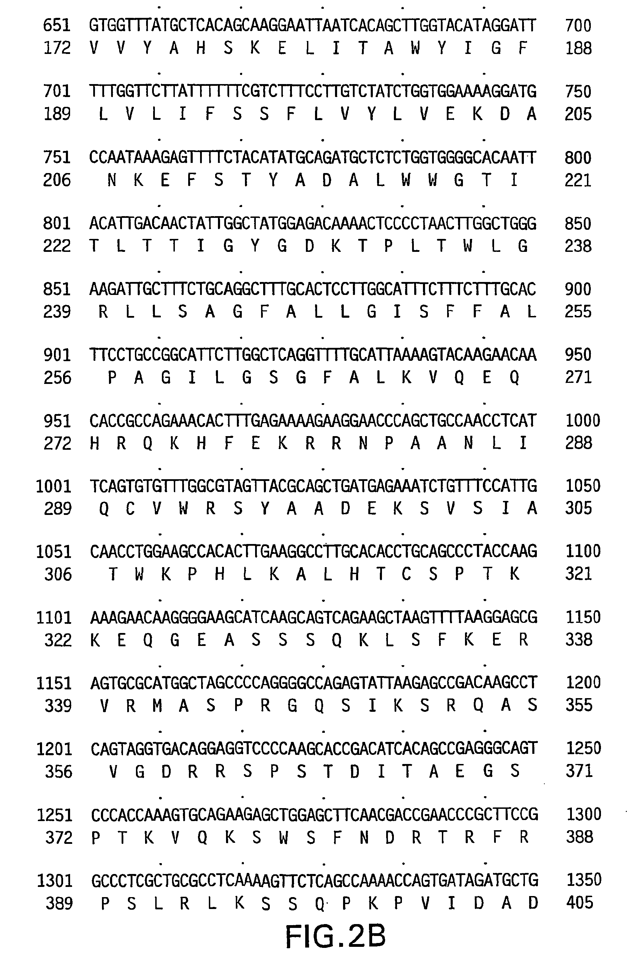

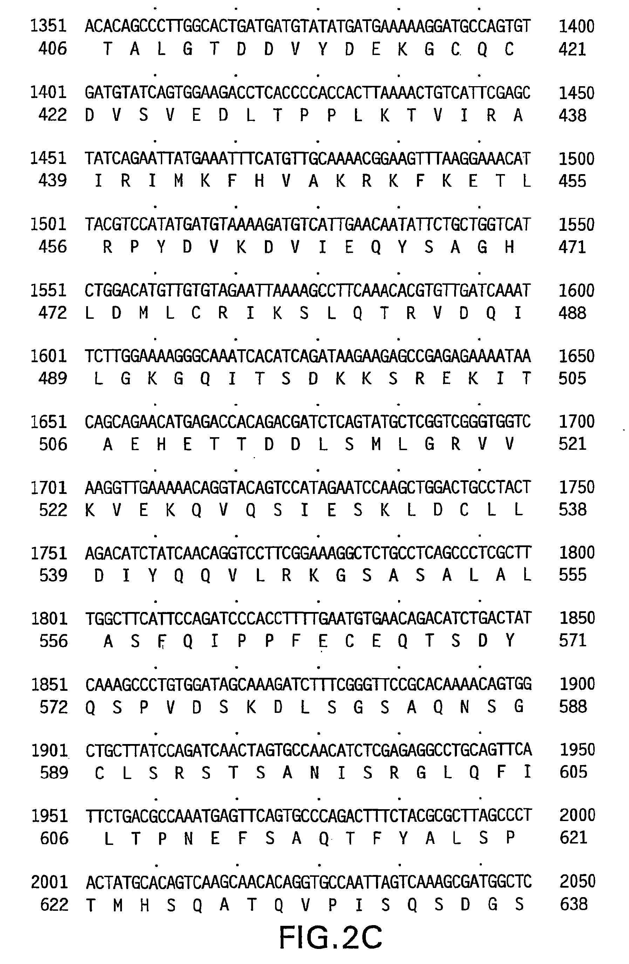

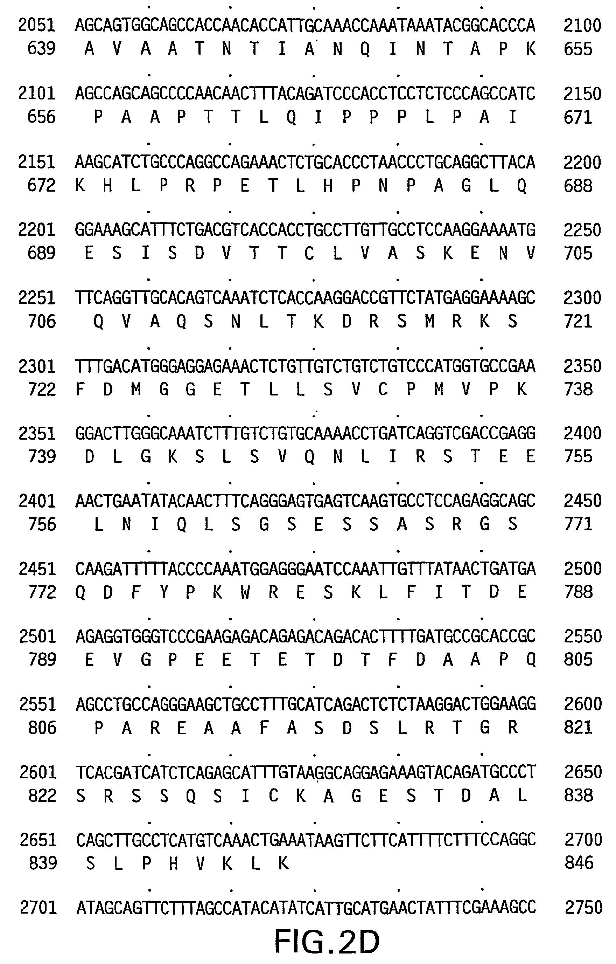

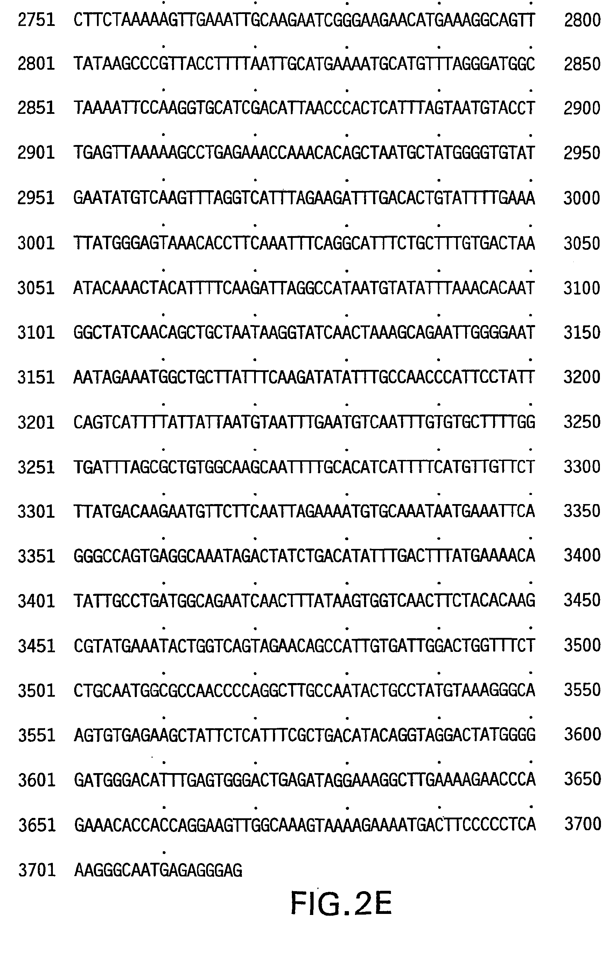

The present invention includes cDNA encoding KCNQ5 protein. Such a cDNA is shown in Figure 2 as SEQ.ID.NO.:2. The present invention therefore includes DNA comprising the nucleotide sequence SEQ.ID.NO.:2. The DNA can be isolated or substantially free of other DNA sequences.

The present invention includes DNA molecules substantially free from other nucleic acids comprising the coding region of SEQ.ID.NO.:2. Accordingly, the present invention includes DNA molecules substantially free from other nucleic acids having a sequence comprising positions 138-2,675 of SEQ.ID.NO.:2. Also included are recombinant DNA molecules having a nucleotide sequence comprising positions 138-2,675 of SEQ.ID.NO.:2 and isolated DNA molecules having a nucleotide sequence comprising positions 138-2,675 of SEQ.ID.NO.:2.

The novel DNA sequences of the present invention encoding KCNQ5, in whole or in part, can be linked with other DNA sequences, *i.e.,* DNA sequences to which KCNQ5 is not naturally linked, to form "recombinant DNA molecules" encoding KCNQ5. Such other sequences can include DNA sequences that control transcription or translation such as, *e.g.,* translation initiation sequences, promoters for RNA polymerase II, transcription or translation termination sequences, enhancer sequences, sequences that control replication in microorganisms, sequences that confer antibiotic resistance, or sequences that encode a polypeptide "tag" such as, *e.g.*, a polyhistidine tract or the myc epitope. The novel DNA sequences of the present invention can be inserted into vectors such as plasmids, cosmids, viral vectors, P1 artificial chromosomes, or yeast artificial chromosomes.

Included in the present disclosure are DNA sequences that hybridize to at least one of SEQ.ID.NO:1 or SEQ.ID.NO:2 under stringent conditions. By way of example, and not limitation, a procedure using conditions of high stringency is as follows: Prehybridization of filters containing DNA is carried out for 2 hr. to overnight at 65°C in buffer composed of 6X SSC, 5X Denhardt's solution, and 100 µg/ml denatured salmon sperm DNA. Filters are hybridized for 12 to 48 hrs at 65°C in prehybridization mixture containing 100 µg/ml denatured salmon sperm DNA and 5-20 X 10⁶ cpm of ³²P-labeled probe. Washing of filters is done at 37°C for 1 hr in a solution containing 2X SSC, 0.1% SDS. This is followed by a wash in 0.1X SSC, 0.1% SDS at 50°C for 45 min. before autoradiography.

Other procedures using conditions of high stringency would include either a hybridization carried out in 5XSSC, 5X Denhardt's solution, 50% formamide at 42°C for 12 to 48 hours or a washing step carried out in 0.2X SSPE, 0.2% SDS at 65°C for 30 to 60 minutes.

Reagents mentioned in the foregoing procedures for carrying out high stringency hybridization are well known in the art. Details of the composition of these reagents can be found in, *e.g.*, Sambrook, Fritsch, and Maniatis, 1989, Molecular Cloning: A Laboratory Manual, second edition, Cold Spring Harbor Laboratory Press. In addition to the foregoing, other conditions of high stringency which may be used are well known in the art.

The degeneracy of the genetic code is such that, for all but two amino acids, more than a single codon encodes a particular amino acid. This allows for the construction of synthetic DNA that encodes the KCNQ5 protein where the nucleotide sequence of the synthetic DNA differs significantly from the nucleotide sequences of SEQ.ID.NO:2, but still encodes the same KCNQ5 protein as SEQ.ID.NO:2. Such synthetic DNAs are intended to be within the scope of the present invention.

Mutated forms of SEQ.ID.NO:1 or SEQ.ID.NO:2 are intended to be within the scope of the present invention. In particular, mutated forms of SEQ.ID.NO:1 or SEQ.ID.NO:2 which give rise to Stargardt-like macular dystrophy, cone-rod dystrophy, Salla disease, or age-related macular degeneration are within the scope of the present invention.

Another aspect of the present invention includes host cells that have been engineered to contain and/or express DNA sequences encoding KCNQ5 protein. Such recombinant host cells can be cultured under suitable conditions to produce KCNQ5 protein. An expression vector containing DNA encoding KCNQ5 protein can be used for expression of KCNQ5 protein in a recombinant host cell. Recombinant host cells may be prokaryotic or eukaryotic, including but not limited to, bacteria such as *E. coli,* fungal cells such as yeast, mammalian cells including, but not limited to, cell lines of human, bovine, porcine, monkey and rodent origin, amphibian cells such as *Xenopus* oocytes, and insect cells including but not limited to *Drosophila* and silkworm derived cell lines. Cells and cell lines which are suitable for recombinant expression of KCNQ5 protein and which are widely mavailable, include but are not limited to, L cells L-M(TK-) (ATCC CCL 1.3), L cells L-M (ATCC CCL 1.2), 293 (ATCC CRL 1573), Raji (ATCC CCL 86), CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26), MRC-5 (ATCC CCL 171), ARPE-19 human retinal pigment epithelium (ATCC CRL-2302), *Xenopus* melanophores, and *Xenopus* oocytes.

A variety of mammalian expression vectors can be used to express recombinant KCNQ5 in mammalian cells. Commercially available mammalian expression vectors which are suitable include, but are not limited to, pMC1neo (Stratagene), pSG5 (Stratagene), pcDNAI and pcDNAIamp, pcDNA3, pcDNA3.1, pCR3.1 (Invitrogen), EBO-pSV2-neo (ATCC 37593), pBPV-1(8-2) (ATCC 37110), pdBPV-MMTneo(342-12) (ATCC 37224), pRSVgpt (ATCC 37199), pRSVneo (ATCC 37198), and pSV2-dhfr (ATCC 37146). Another suitable vector is the PT7TS oocyte expression vector. Following expression in recombinant cells, KCNQ5 can be purified by conventional techniques to a level that is substantially free from other proteins.

Certain voltage-gated potassium channel subunits have been found to require the expression of other voltage-gated potassium channel subunits as "chaperones" in order to be properly expressed at high levels and inserted in membranes. For example, co-expression of KCNQ3 appears to enhance the expression of KCNQ2 in *Xenopus* oocytes (Wang et al., 1998, Science 282:1890-1893). Also, some voltage-gated potassium channel Kv1α subunits require other related alpha subunits or Kvβ2 subunits (Shi et al., 1995, Neuron 16:843-852). Accordingly, the recombinant expression of the KCNQ5 protein may under certain circumstances benefit from the co-expression of other voltage-gated potassium channel proteins and such co-expression is intended to be within the scope of the present invention.

The present invention includes KCNQ5 protein substantially free from other proteins. The amino acid sequence of the full-length KCNQ5 protein is shown in Figure 2 as SEQ.ID.NO.:3. Thus, the present invention includes KCNQ5 protein substantially free from other proteins having the amino acid sequence SEQ.ID.NO.:3. The present invention also includes isolated KCNQ5 protein having the amino acid sequence SEQ.ID.NO.:3.

Mutated forms of KCNQ5 proteins are intended to be within the scope of the present invention. In particular, mutated forms of SEQ.ID.NO:3 that give rise to Stargardt-like macular dystrophy, cone-rod dystrophy, Salla disease, or age-related macular degeneration are within the scope of the present invention.

As with many proteins, it is possible to modify many of the amino acids of KCNQ5 and still retain substantially the same biological activity as the original protein. Thus, the present invention includes modified KCNQ5 proteins which have amino acid deletions, additions, or substitutions but that still retain substantially the same biological activity as KCNQ5. It is generally accepted that single amino acid substitutions do not usually alter the biological activity of a protein (see, *e.g.,* Molecular Biology of the Gene, Watson *et al.,* 1987, Fourth Ed., The Benjamin/Cummings Publishing Co., Inc., page 226; and Cunningham & Wells, 1989, Science 244:1081-1085). Accordingly, the present invention includes polypeptides where one amino acid substitution has been made in SEQ.ID.NO:3 wherein the polypeptides still retain substantially the same biological activity as KCNQ5. The present invention also includes polypeptides where two or more amino acid substitutions have been made in SEQ.ID.NO:3 wherein the polypeptides still retain substantially the same biological activity as KCNQ5. In particular, the present invention includes embodiments where the above-described substitutions are conservative substitutions. In particular, the present invention includes embodiments where the above-described substitutions do not occur in positions where the amino acid present in KCNQ5 is also present in the corresponding position of any one of KCNQ1, KCNQ2, KCNQ3, or KCNQ4 (see Figure 4A-B).

The KCNQ5 proteins of the present invention may contain post-translational modifications, *e.g.,* covalently linked carbohydrate, phosphorylation, myristoylation, etc..

The present invention also includes chimeric KCNQ5 proteins. Chimeric KCNQ5 proteins consist of a contiguous polypeptide sequence of at least a portion of KCNQ5 protein fused to a polypeptide sequence of a non-KCNQ5 protein.

The present invention also includes isolated forms of KCNQ5 proteins and KCNQ5 DNA. Use of the term "isolated" indicates that KCNQ5 protein or KCNQ5 DNA has been removed from its normal cellular environment. Thus, an isolated KCNQ5 protein may be in a cell-free solution or placed in a different cellular environment from that in which it occurs naturally. The term isolated does not imply that an isolated KCNQ5 protein is the only protein present. but instead means that an isolated KCNQ5 protein is at least 95% free of non-amino acid material (*e.g.,* nucleic acids, lipids, carbohydrates) naturally associated with the KCNQ5 protein. Thus, a KCNQ5 protein that is expressed in bacteria or even in eukaryotic cells which do not naturally (*i.e.,* without human intervention) express it through recombinant means is an "isolated KCNQ5 protein."

It is known that other members of the family of potassium channels to which KCNQ5 belongs can interact to form heteromeric structures resulting in functional potassium channels. For example, KCNQ2 and KCNQ3 can assemble to form a heteromeric functional potassium channel (Wang et al., 1998, Science 282:1890-1893). Accordingly, it is believed likely that KCNQ5 will also be able to form heteromeric structures with other proteins where such heteromeric structures constitute functional potassium channels. Thus, the present invention includes such heteromers comprising KCNQ5. Preferred heteromers are those in which KCNQ5 forms heteromers with at least one of KCNQ1, KCNQ2, KCNQ3, or KCNQ4.

A cDNA fragment encoding full-length KCNQ5 can be isolated from a human retinal or brain cDNA library by using the polymerase chain reaction (PCR) employing suitable primer pairs. Such primer pairs can be selected based upon the cDNA sequence for KCNQ5 shown in Figure 2 as SEQ.ID.NO.:2. Suitable primer pairs would be, *e.g.*:
5'-GGGGGCCCGGATGAGCC-3' (SEQ.ID.NO.:9) and
5'-GAAGAACTTATTTCAGTTTGA-3'(SEQ.ID.NO.:10)

The above primers are meant to be illustrative only; one skilled in the art would readily be able to design other suitable primers based upon SEQ.ID.NO.:2. Such primers could be produced by methods of oligonucleotide synthesis that are well known in the art.

PCR reactions can be carried out with a variety of thermostable enzymes including but not limited to AmpliTaq, AmpliTaq Gold, or Vent polymerase. For AmpliTaq, reactions can be carried out in 10 mM Tris-Cl, pH 8.3, 2.0 mM MgCl₂, 200 µM for each dNTP, 50 mM KCl, 0.2 µM for each primer, 10 ng of DNA template, 0.05 units/µl of AmpliTaq. The reactions are heated at 95°C for 3 minutes and then cycled 35 times using the cycling parameters of 95°C, 20 seconds, 62°C, 20 seconds, 72°C, 3 minutes. In addition to these conditions, a variety of suitable PCR protocols can be found in PCR Primer, A Laboratory Manual, edited by C.W. Dieffenbach and G.S. Dveksler, 1995, Cold Spring Harbor Laboratory Press; or PCR Protocols: A Guide to Methods and Applications, Michael *et al.,* eds., 1990, Academic Press.

A suitable cDNA library from which a clone encoding KCNQ5 can be isolated would be Human Retina 5'-stretch cDNA library in lambda gt10 or lambda gt11 vectors (catalog numbers HL1143a and HL1132b, Clontech, Palo Alto, CA) or human fetal brain 5-stretch plus cDNA library (catalog number HL5024t, Clontech, Palo Alto, CA). The primary clones of such a library can be subdivided into pools with each pool containing approximately 20,000 clones and each pool can be amplified separately.

By this method, a cDNA fragment encoding an open reading frame of 846 amino acids (SEQ.ID.NO.:3) can be obtained. This cDNA fragment can be cloned into a suitable cloning vector or expression vector. For example, the fragment can be cloned into the mammalian expression vector pcDNA3.1 (Invitrogen, San Diego, CA). KCNQ5 protein can then be produced by transferring an expression vector encoding KCNQ5 or portions thereof into a suitable host cell and growing the host cell under appropriate conditions. KCNQ5 protein can then be isolated by methods well known in the art.

As an alternative to the above-described PCR method, a cDNA clone encoding KCNQ5 can be isolated from a cDNA library using as a probe oligonucleotides specific for KCNQ5 and methods well known in the art for screening cDNA libraries with oligonucleotide probes. Such methods are described in, *e.g.,* Sambrook *et al.,* 1989, *Molecular Cloning: A Laboratory Manual;* Cold Spring Harbor Laboratory, Cold Spring Harbor, New York; Glover, D.M. (ed.), 1985, *DNA Cloning: A Practical Approach,* MRL Press, Ltd., Oxford, U.K., Vol. L II. Oligonucleotides that are specific for KCNQ5 and that can be used to screen cDNA libraries can be readily designed based upon the cDNA sequence of KCNQ5 shown in Figure 2 as SEQ.ID.NO.:2 and can be synthesized by methods well-known in the art.

Genomic clones containing the KCNQ5 gene can be obtained from commercially available human PAC or BAC libraries available from Research Genetics, Huntsville, AL. PAC clones containing the KCNQ5 gene (*e.g.,* PAC141B1, PAC224H23) are commercially available from Research Genetics, Huntsville, AL (catalog number for individual PAC clones is RPCLC). Alternatively, one may prepare genomic libraries, especially in P1 artificial chromosome vectors, from which genomic clones containing the KCNQ5 can be isolated, using probes based upon the KCNQ5 sequences disclosed herein. Methods of preparing such libraries are known in the art (Ioannou *et al.,* 1994, Nature Genet. 6:84-89).

The novel DNA sequences of the present invention can be used in various diagnostic methods relating to Stargardt-like macular dystrophy, cone-rod dystrophy, Salla disease, or age-related macular degeneration. Disclosed herein are diagnostic methods for determining whether a patient carries a mutation in the KCNQ5 gene that predisposes that patient toward the development of Stargardt-like macular dystrophy, cone-rod dystrophy, Salla disease, or age-related macular degeneration. In broad terms, such methods comprise determining the DNA sequence of a region of the KCNQ5 gene from the patient and comparing that sequence to the sequence from the corresponding region of the KCNQ5 gene from a non-affected person, *i.e.,* a person who does not suffer from Stargardt-like macular dystrophy, cone-rod dystrophy, Salla disease, or age-related macular degeneration, where a difference in sequence between the DNA sequence of the KCNQ5 gene from the patient and the DNA sequence of the KCNQ5 gene from the non-affected person indicates that the patient has Stargardt-like macular dystrophy, cone-rod dystrophy, Salla disease, or age-related macular degeneration.

Such methods of diagnosis may be carried out in a variety of ways. For example, one embodiment comprises:
(a) providing PCR primers from a region of the KCNQ5 gene;
(b) performing PCR on a DNA sample from the patient to produce a PCR fragment from the patient;
(c) performing PCR on a control DNA sample comprising a nucleotide sequence selected from the group consisting of SEQ.ID.NO:1 and SEQ.ID.NO.:2 to produce a control PCR fragment;
(d) determining the nucleotide sequence of the PCR fragment from the patient and the nucleotide sequence of the control PCR fragment;
(e) comparing the nucleotide sequence of the PCR fragment from the patient to the nucleotide sequence of the control PCR fragment;
where a difference between the nucleotide sequence of the PCR fragment from the patient and the nucleotide sequence of the control PCR fragment indicates that the patient has a mutation in the KCNQ5 gene and thus is likely to have Stargardt-like macular dystrophy, cone-rod dystrophy, Salla disease, or age-related macular degeneration.

In a particular embodiment, the PCR primers are from a region of the KCNQ5 gene where it is suspected that a patient harbors a mutation. In a particular embodiment, the PCR primers are from the coding region of the KCNQ5 gene, *i.e.*, from the coding region of SEQ.ID.NO:1 or SEQ.ID.NO:2. In a particular embodiment, the PCR primers amplify a region that includes the marker D6S280.

In a particular embodiment, the DNA sample from the patient is cDNA that has been prepared from an RNA sample from the patient. In another embodiment, the DNA sample from the patient is genomic DNA. In a particular embodiment, the control DNA sample is DNA from a person who does not have Stargardt-like macular dystrophy, cone-rod dystrophy, Salla disease, or age-related macular degeneration.

In a particular embodiment, the nucleotide sequences of the PCR fragment from the patient and the control PCR fragment are determined by DNA sequencing.

In a particular embodiment, the nucleotide sequences of the PCR fragment from the patient and the control PCR fragment are compared by direct comparison after DNA sequencing. In another embodiment, step (d) is omitted and the comparison in step (e) is made by a process that includes hybridizing the PCR fragment from the patient and the control PCR fragment and then using an endonuclease that cleaves at any mismatched positions in the hybrid but does not cleave the hybrid if the two fragments match perfectly. Such an endonuclease is, *e.g.,* S1. In this embodiment, the conversion of the PCR fragment from the patient to smaller fragments after endonuclease treatment indicates that the patient carries a mutation in the KCNQ5 gene. In such embodiments, it may be advantageous to label (radioactively, enzymatically, immunologically, *etc.*) the PCR fragment from the patient or the control PCR fragment.

Disclosed herein is a method of diagnosing whether a patient carries a mutation in the KCNQ5 gene that comprises:
(a) obtaining an RNA sample from the patient;
(b) performing reverse transcription-PCR (RT-PCR) on the RNA sample using primers that span a region of the coding sequence of the KCNQ5 gene to produce a PCR fragment from the patient where the PCR fragment from the patient has a defined length, the length being dependent upon the identity of the primers that were used in the RT-PCR;
(c) hybridizing the PCR fragment to DNA comprising a sequence selected from the group consisting of SEQ.ID.NO:1 and SEQ.ID.NO.:2, or to portions of SEQ.ID.NO:1 or SEQ.ID.NO.:2 that are sufficiently long to give rise to bands that can be seen on polyacrylamide gels, to form a hybrid;
(d) treating the hybrid produced in step (c) with an endonuclease that cleaves at any mismatched positions in the hybrid but does not cleave the hybrid if the two fragments match perfectly;
(e) determining whether the endonuclease cleaved the.hybrid by determining the length of the PCR fragment from the patient after endonuclease treatment where a reduction in the length of the PCR fragment from the patient after endonuclease treatment indicates that the patient carries a mutation in the KCNQ5 gene.

In a variation of the above-described method, instead of determining the length of the PCR fragment from the patient after endonuclease treatment, the length of the DNA comprising a sequence selected from the group consisting of SEQ.ID.NO:1 and SEQ.ID.NO.:2, or the DNA comprising portions of SEQ.ID.NO:1 or SEQ.ID.NO.:2 that are sufficiently long to give rise to bands that can be seen on polyacrylamide gels is determined after endonuclease treatment. In such a variation, a reduction in the length of the DNA comprising a sequence selected from the group consisting of SEQ.ID.NO: and SEQ.ID.NO.:2, or the DNA comprising portions of SEQ.ID.NO:1 or SEQ.ID.NO.:2 that are sufficiently long to give rise to bands that can be seen on polyacrylamide gels indicates that the patient carries a mutation in the KCNQ5 gene.

Disclosed herein is a method of diagnosing whether a patient carries a mutation in the KCNQ5 gene that comprises:
(a) making cDNA from an RNA sample from the patient;
(b) providing a set of PCR primers based upon SEQ.ID.NO.: 1 or SEQ.ID.NO.:2;.
(c) performing PCR on the cDNA to produce a PCR fragment from the patient;
(d) determining the nucleotide sequence of the PCR fragment from the patient;
(e) comparing the nucleotide sequence of the PCR fragment from the patient with the nucleotide sequence of SEQ.ID.NO.: 1 or SEQ.ID.NO.:2;
where a difference between the nucleotide sequence of the PCR fragment from the patient with the nucleotide sequence of SEQ.ID.NO.:1 or SEQ.ID.NO.:2 indicates that the patient carries a mutation in the KCNQ5 gene.

Disclosed herein is a method of diagnosing whether a patient carries a mutation in the KCNQ5 gene that comprises:
(a) preparing genomic DNA from the patient;
(b) providing a set of PCR primers based upon SEQ.ID.NO.:1 or SEQ.ID.NO.:2;
(c) performing PCR on the genomic DNA to produce a PCR fragment from the patient;
(d) determining the nucleotide sequence of the PCR fragment from the patient;
(e) comparing the nucleotide sequence of the PCR fragment from the patient with the nucleotide sequence of SEQ.ID.NO.: 1 or SEQ.ID.NO.:2;
where a difference between the nucleotide sequence of the PCR fragment from the patient with the nucleotide sequence of SEQ.ID.NO.: 1 or SEQ.ID.NO.:2 indicates that the patient carries a mutation in the KCNQ5 gene.

Also disclosed herein are oligonucleotide probes, based upon the sequences of SEQ.ID.NO:1 or SEQ.ID.NO:2, that can be used in diagnostic methods related to Stargardt-like macular dystrophy, cone-rod dystrophy, Salla disease, or age-related macular degeneration. In particular, there are disclosed DNA oligonucleotides comprising at least about 10, 15, or 18 contiguous nucleotides of a sequence selected from the group consisting of: SEQ.ID.NO: 1 and SEQ.ID.:NO.2 where the oligonucleotide probe comprises no stretch of contiguous nucleotides longer than 5 of a sequence selected from the group consisting of: SEQ.ID.NO:1 and SEQ.ID.:NO.2 other than the said at least about 10, 15, or 18 contiguous nucleotides. The oligonucleotides can be substantially free from other nucleic acids. Also provided by the present invention are corresponding RNA oligonucleotides. The DNA or RNA oligonucleotide probes can be packaged in kits.

In addition to the diagnostic utilities described above, the present disclosure makes possible the recombinant expression of the KCNQ5 protein in various cell types. Such recombinant expression makes possible the study of this protein so that its biochemical activity and its role in Stargardt-like macular dystrophy, cone-rod dystrophy, Salla disease, or age-related macular degeneration can be elucidated.

The present invention also makes possible the development of assays which measure the biological activity of the KCNQ5 protein. Such assays using recombinantly expressed KCNQ5 protein are especially of interest. Assays for KCNQ5 protein activity can be used to screen libraries of compounds or other sources of compounds to identify compounds that are activators or inhibitors of the activity of KCNQ5 protein. Such identified compounds can serve as "leads" for the development of pharmaceuticals that can be used to treat patients having Stargardt-like macular dystrophy, cone-rod dystrophy, Salla disease, or age-related macular degeneration. In versions of the above-described assays, mutant KCNQ5 proteins are used and inhibitors or activators of the activity of the mutant KCNQ5 proteins are identified.

Preferred cell lines for recombinant expression of KCNQ5 are those which do not express endogenous potassium channels (*e.g.*, CV-1, NIH-3T3). Such cell lines can be loaded with 86Rb, an ion which can pass through potassium channels. The ⁸⁶Rb-loaded cells can be exposed to collections of substances (*e.g.,* combinatorial libraries, natural products) and those substances that are able to alter 86Rb efflux identified. Such substances are likely to be activators or inhibitors of KCNQ5.

The present invention includes a method of identifying activators or inhibitors of KCNQ5 comprising:
(a) recombinantly expressing a KCNQ5 polypeptide comprising a sequence of amino acids substantially identical to those set forth in SEQ.ID. NO.:3 of a mutant thereof;
(b) measuring the biological activity of KCNQ5 protein or mutant KCNQ5 protein in the presence and in the absence of a substance suspected of being an activator or an inhibitor of KCNQ5 protein or mutant KCNQ5 protein;
where a change in the biological activity of the KCNQ5 protein or the mutant KCNQ5 protein in the presence as compared to the absence of the substance indicates that the substance is an activator or an inhibitor of KCNQ5 protein or mutant KCNQ5 protein.

In particular embodiments, the biological activity is the production of a voltage-gated potassium current, or efflux of ⁸⁶Rb.

In particular embodiments, a vector encoding KCNQ5 is transferred into *Xenopus* oocytes in order to cause the expression of KCNQ5 protein in the oocytes. Alternatively, RNA encoding KCNQ5 protein can be prepared *in vitro* and injected into the oocytes, also resulting in the expression of KCNQ5 protein in the oocytes. Following expression of KCNQ5 in the oocytes, membrane currents are measured after the transmembrane voltage is changed in steps. A change in membrane current is observed when the KCNQ5 channels opens, allowing potassium ion flow. Similar oocytes studies were reported for KCNQ2 and KCNQ3 potassium channels in Wang et al., 1998, Science 282:1890-1893.

Inhibitors of KCNQ5 can be identified by exposing the oocytes expressing KCNQ5 to collections of substances and determining whether the substances can block or diminish the membrane currents observed in the absence of the substance.

Accordingly, the present invention provides a method of identifying inhibitors of KCNQ5 comprising:
(a) expressing a KCNQ5 polypeptide comprising a sequence of amino acids substantially identical to those set forth in SEQ.ID. NO.: 3 or a mutant thereof in *Xenopus* oocytes;
(b) changing the transmembrane potential of the oocytes in the presence and the absence of a substance suspected of being an inhibitor of KCNQ5;
(c) measuring membrane potassium currents following step (b);
where if the potassium membrane currents measured in step (c) are greater in the absence rather than in the presence of the substance, then the substance is an inhibitor of KCNQ5.

The present invention also includes assays for the identification of activators and inhibitors of KCNQ5 that are based upon FRET between a first and a second fluorescent dye where the first dye is bound to one side of the plasma membrane of a cell expressing KCNQ5 and the second dye is free to shuttle from one face of the membrane to the other face in response to changes in membrane potential. In certain embodiments, the first dye is impenetrable to the plasma membrane of the cells and is bound predominately to the extracellular surface of the plasma membrane. The second dye is trapped within the plasma membrane but is free to diffuse within the membrane. At normal (*i.e.,* negative) resting potentials of the membrane, the second dye is bound predominately to the inner surface of the extracellular face of the plasma membrane, thus placing the second dye in close proximity to the first dye. This close proximity allows for the generation of a large amount of FRET between the two dyes. Following membrane depolarization, the second dye moves from the extracellular face of the membrane to the intracellular face, thus increasing the distance between the dyes. This increased distance results in a decrease in FRET, with a corresponding increase in fluorescent emission derived from the first dye and a corresponding decrease in the fluorescent emission from the second dye. See figure 1 of González & Tsien, 1997, Chemistry & Biology 4:269-277. See also González & Tsien, 1995, Biophys. J. 69:1272-1280 and U.S. Patent No. 5,661,035.

In certain embodiments, the first dye is a fluorescent lectin or a fluorescent phospholipid that acts as the fluorescent donor. Examples of such a first dye are: a coumarin-labeled phosphatidylethanolamine (*e.g.,* N-(6-chloro-7-hydroxy-2-oxo-2H--1-benzopyran-3-carboxamidoacetyl)-dimyristoylphosphatidyl-ethanolamine) or N-(7-nitrobenz-2-oxa-1,3-diazol-4-yl)-dipalmitoylphosphatidylethanolamine); a fluorescently-labeled lectin (*e.g.,* fluorescein-labeled wheat germ agglutinin). In certain embodiments, the second dye is an oxonol that acts as the fluorescent acceptor. Examples of such a second dye are: bis(1,3-dialkyl-2-thiobarbiturate)trimethineoxonols (*e.g.*, bis(1,3-dihexyl-2-thiobarbiturate)trimethineoxonol) or pentamethineoxonol analogues (*e.g.,* bis(1,3-dihexyl-2-thiobarbiturate)pentamethineoxonol; or bis(1,3-dibutyl-2-thiobarbiturate)pentamethineoxonol). See González & Tsien, 1997, Chemistry & Biology 4:269-277 for methods of synthesizing various dyes suitable for use in the present invention. In certain embodiments, the assay may comprise a natural carotenoid, *e.g.*, astaxanthin, in order to reduce photodynamic damage due to singlet oxygen.

The above described assays can be utilized to discover activators and inhibitors of KCNQ5. Such assays will generally utilize cells that express KCNQ5, *e.g.*, by transfection with expression vectors encoding KCNQ5. In assays for inhibitors, such cells will generally have a resting membrane potential that is roughly equal to the threshhold for activation of the KCNSQ channel. This is because most untransfected cells will have membrane potentials that are depolarized relative to the threshhold potential of KCNQ5 channels. Therefore, when KCNQ5 is expressed in these cells, the KCNQ5 channels open. This lets K⁺ out of the cells, which tends to hyperpolarize the membrane potential. This closes some of the KCNQ5 channels, leading to relative depolarization. In this way, a steady state develops around the threshhold for activation of the KCNQ5 channel. Inhibitors of KCNQ5. will, therefore, disturb this steady state and depolarize the cell. In assays for activators, KCNQ5 will be transfected into a cell line that also expresses a counteracting, depolarizing current. The membrane potential in these cells will therefore be set by contributions of both the KCNQ5 channel and the endogenous depolarizing current, resulting in a more depolarized resting potential. Ideally, the endogenous current will play the major role in the absence of a KCNQ5 activator. Activators of KCNQ5 will open this channel and increase the contribution of KCNQ5 to the membrane potential relative to the other current and the potential will, therefore, hyperpolarize in response to an activator of KCNQ5. Changes in membrane potential (depolarizations and hyperpolarizations) that are caused by activators and inhibitors of KCNQ5 can be monitored by the assays using FRET described above.

Accordingly, the present invention provides a method of identifying activators of KCNQ5 comprising:
(a) providing test cells comprising:
   (1) an expression vector that directs the expression of a KCNQ5 polypeptide comprising a sequence of amino acids substantially identical to those set forth in SEQ.ID. NO.:3 or a mutant thereof in the cells;
   (2) a first fluorescent dye, where the first dye is bound to one side of the plasma membrane; and
   (3) a second fluorescent dye, where the second fluorescent dye is free to shuttle from one face of the plasma membrane to the other face in response to changes in membrane potential;
(b) exposing the test cells to a substance that is suspected of being an activator of KCNQ5;
(c) measuring the amount of fluorescence resonance energy transfer (FRET) in the test cells that have been exposed to the substance;
(d) comparing the amount of FRET exhibited by the test cells that have been exposed to the substance with the amount of FRET exhibited by control cells;
wherein if the amount of FRET exhibited by the test cells is greater than the amount of FRET exhibited by the control cells, the substance is an activator of KCNQ5;
where the control cells are either (1) cells that are essentially the same as the test cells except that they do not comprise at least one of the items listed at (a) (1)-(3) but have been exposed to the substance; or (2) test cells that have not been exposed to the substance.

The present invention also provides a method of identifying inhibitors of KCNQ5 comprising:
(a) providing test cells comprising:
   (1) an expression vector that directs the expression of a KCNQ5 polypeptide comprising a sequence of amino acids substantially identical to those set forth in SEQ, ID. NO.: 3 or a mutant thereof in the cells;
   (2) a first fluorescent dye, where the first dye is bound to one side of the plasma membrane; and
   (3) a second fluorescent dye, where the second fluorescent dye is free to shuttle from one face of the plasma membrane to the other face in response to changes in membrane potential;
(b) exposing the test cells to a substance that is suspected of being an inhibitor of KCNQ5;
(c) measuring the amount of fluorescence resonance energy transfer (FRET) in the test cells that have been exposed to the substance;
(d) comparing the amount of FRET exhibited by the test cells that have been exposed to the substance with the amount of FRET exhibited by control cells;
wherein if the amount of FRET exhibited by the test cells is less than the amount of FRET exhibited by the control cells, the substance is an inhibitor of KCNQ5;
where the control cells are either (1) cells that are essentially the same as the test cells except that they do not comprise at least one of the items listed at (a) (1)-(3) but have been exposed to the substance; or (2) test cells that have not been exposed to the substance.

In a variation of the assay described above, instead of the transfected cell's membrane potential being allowed to reach steady state on its own, the membrane potential is artificially set at a potential in which the KCNQ5 channel is open. This can be done, *e.g.,* by variation of the external K⁺ concentration in a known manner (*e.g.,* increased concentrations of external K⁺). If such cells, having open KCNQ5 channels, are exposed to inhibitors of KCNQ5, the KCNQ5 channels will close, and the cells' membrane potentials will be depolarized. This depolarization can be observed as a decrease in FRET.

Accordingly, there is disclosed a method of identifying inhibitors of KCNQ5 comprising:
(a) providing cells comprising:
   (1) an expression vector that directs the expression of KCNQ5 in the cells;
   (2) a first fluorescent dye, where the first dye is bound to one side of the plasma membrane; and
   (3) a second fluorescent dye, where the second fluorescent dye is free to shuttle from one face of the plasma membrane to the other face in response to changes in membrane potential;
(b) adjusting the membrane potential of the cells such that the ion channel formed by KCNQ5 is open;
(c) measuring the amount of fluorescence resonance energy transfer (FRET) in the test cells;
(d) repeating step (b) and step (c) while the cells are exposed to a substance that is suspected of being an inhibitor of KCNQ5;
where if the amount of FRET exhibited by the cells that are exposed to the substance is less than the amount of FRET exhibited by the cells that have not been exposed to the substance, then the substance is an inhibitor of KCNQ5.

In particular embodiments of the above-described methods, the expression vectors are transfected into the test cells.

In particular embodiments of the above-described methods, KCNQ5 has an amino acid sequence of SEQ.ID.NO.:3.

In particular embodiments of the above-described methods, the first fluorescent dye is selected from the group consisting of: a fluorescent lectin; a fluorescent phospholipid; a coumarin-labeled phosphatidylethanolamine; N-(6-chloro-7-hydroxy-2-oxo-2H-1-benzopyran-3-carboxamidoacetyl)-dimyristoylphosphatidyl-ethanolamine); N-(7-nitrobenz-2-oxa-1,3-diazol-4-yl)-dipalmitoylphosphatidylethanolamine); and fluorescein-labeled wheat germ agglutinin.

In particular embodiments of the above-described methods, the second fluorescent dye is selected from the group consisting of: an oxonol that acts as the fluorescent acceptor; bis(1,3-dialkyl-2-thiobarbiturate)trimethineoxonols; bis(1,3-dihexyl-2-thiobarbiturate)trimethineoxonol; bis(1,3-dialkyl-2-thiobarbiturate)quatramethineoxonols; bis(1,3-dialkyl-2-thiobarbiturate)pentamethineoxonols; bis(1,3-dihexyl-2-thiobarbiturate)pentamethineoxonol; bis(1,3-dibutyl-2-thiobarbiturate)pentamethineoxonol); and bis(1,3-dialkyl-2-thiobarbiturate)hexamethineoxonols.

In a particular embodiment of the above-described methods, the cells are eukaryotic cells. In another embodiment, the cells are mammalian cells. In other embodiments, the cells are L cells L-M(TK-) (ATCC CCL 1.3), L cells L-M (ATCC CCL 1.2), 293 (ATCC CRL 1573), Raji (ATCC CCL 86), CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26), MRC-5 (ATCC CCL 171), *Xenopus* melanophores, or *Xenopus* oocytes.

In particular embodiments of the above-described methods, the control cells do not comprise item (a)(1) but do comprise items (a)(2) and (a)(3).

In assays to identify activators or inhibitors of KCNQ5, it may be advantageous to co-express another potassium channel, *e.g.,* KCNQ1, KCNQ2, KCNQ3, or KCNQ4, together with KCNQ5, or with an accessory subunit, such as the IsK protein or one of its homologues, in order to form a functional heteromeric potassium channel.

While the above-described methods are explicitly directed to testing whether "a" substance is an activator or inhibitor of KCNQ5, it will be clear to one skilled in the art that such methods can be adapted to test collections of substances, *e.g.,* combinatorial libraries, to determine whether any members of such collections are activators or inhibitors of KCNQ5. Accordingly, the use of collections of substances, or individual members of such collections, as the substance in the above-described methods is within the scope of the present invention.

Disclosed herein are pharmaceutical compositions comprising activators or inhibitors of KCNQ5 protein that have been identified by the herein-described methods. The activators or inhibitors are generally combined with pharmaceutically acceptable carriers to form pharmaceutical compositions. Examples of such carriers and methods of formulation of pharmaceutical compositions containing activators or inhibitors and carriers can be found in Remington's Pharmaceutical Sciences. To form a pharmaceutically acceptable composition suitable for effective administration, such compositions will contain a therapeutically effective amount of the activators or inhibitors.

Therapeutic or prophylactic compositions are administered to an individual in amounts sufficient to treat or prevent conditions where KCNQ5 activity is abnormal. The effective amount can vary according to a variety of factors such as the individual's condition, weight, gender, and age: Other factors include the mode of administration. The appropriate amount can be determined by a skilled physician.

Compositions can be used alone at appropriate dosages. Alternatively, co-administration or sequential administration of other agents can be desirable.

The compositions can be administered in a wide variety of therapeutic dosage forms in conventional vehicles for administration. For example, the compositions can be administered in such oral dosage forms as tablets, capsules (each including timed release and sustained release formulations), pills, powders, granules, elixirs, tinctures, solutions, suspensions, syrups and emulsions, or by injection. Likewise, they can also be administered in intravenous (both bolus and infusion), intraperitoneal, subcutaneous, topical with or without occlusion, or intramuscular form, all using forms well known to those of ordinary skill in the pharmaceutical arts.

Advantageously, compositions can be administered in a single daily dose, or the total daily dosage can be administered in divided doses of two, three or four times daily. Furthermore, compositions can be administered in intranasal form via topical use of suitable intranasal vehicles, or via transdermal routes, using those forms of transdermal skin patches well known to those of ordinary skill in that art. To be administered in the form of a transdermal delivery system, the dosage administration will, of course, be continuous rather than intermittent throughout the dosage regimen.

The dosage regimen utilizing the compositions is selected in accordance with a variety of factors including type, species, age, weight, sex and medical condition of the patient; the severity of the condition to be treated; the route of administration; the renal, hepatic and cardiovascular function of the patient; and the particular composition thereof employed. A physician of ordinary skill can readily determine and prescribe the effective amount of the composition required to prevent, counter or arrest the progress of the condition. Optimal precision in achieving concentrations of composition within the range that yields efficacy without toxicity requires a regimen based on the kinetics of the composition's availability to target sites. This involves a consideration of the distribution, equilibrium, and elimination of a composition.

Disclosed herein is a method of treating Stargardt-like macular dystrophy, cone-rod dystrophy, Salla disease, age-related macular degeneration and other forms of macular degeneration, deafness, epilepsy, and different forms of neuropsychiatric, heart, gastrointestinal, and muscle disorders by administering to a patient a therapeutically effective amount of a substance that is an activator or an inhibitor of a voltage-gated potassium channel containing the KCNQ5 protein.

When screening compounds in order to identify potential pharmaceuticals that specifically interact with a target ion channel, it is necessary to ensure that the compounds identified are as specific as possible for the target ion channel. To do this, it is necessary to screen the compounds against as wide an array as possible of ion channels that are similar to the target ion channel. Thus, in order to find compounds that are potential pharmaceuticals that interact with ion channel A, it is not enough to ensure that the compounds interact with ion channel A (the "plus target") and produce the desired pharmacological effect through ion channel A. It is also necessary to determine that the compounds do not interact with ion channels B, C, D, *etc* .(the "minus targets"). In general, as part of a screening program, it is important to have as many minus targets as possible (see Hodgson, 1992, Bio/Technology 10:973-980, at 980). KCNQ5 protein, DNA encoding KCNQ5 protein, and recombinant cells that have been engineered to express KCNQ5 protein have utility in that they can be used as "minus targets" in screens designed to identify compounds that specifically interact with other ion channels. For example, Wang et al., 1998, Science 282:1890-1893 have shown that KCNQ2 and KCNQ3 form a heteromeric potassium ion channel know as the "M-channel." The M-channel is an important target for drug discovery since mutations in KCNQ2 and KCNQ3 are responsible for causing epilepsy (Biervert et al., 1998, Science 279:403-406; Singh et al., 1998, Nature Genet. 18:25-29; Schroeder et al., Nature 1998, 396:687-690). A screening program designed to identify activators or inhibitors of the M-channel would benefit greatly by the use of KCNQ5 as a minus target.

The present invention also includes antibodies to the KCNQ5 protein. Such antibodies may be polyclonal antibodies or monoclonal antibodies. The antibodies of the present invention are raised against the entire KCNQ5 protein or against suitable antigenic fragments of the protein that are coupled to suitable carriers, *e.g.*, serum albumin or keyhole limpet hemocyanin, by methods well known in the art. Methods of identifying suitable antigenic fragments of a protein are known in the art. See, *e.g.,* Hopp & Woods, 1981, Proc. Natl. Acad. Sci. USA 78:3824-3828; and Jameson & Wolf, 1988, CABIOS (Computer Applications in the Biosciences) 4:181-186.

For the production of polyclonal antibodies, KCNQ5 protein or an antigenic fragment, coupled to a suitable carrier, is injected on a periodic basis into an appropriate non-human host animal such as, *e.g.*, rabbits, sheep, goats, rats, mice. The animals are bled periodically and sera obtained are tested for the presence of antibodies to the injected antigen. The injections can be intramuscular, intraperitoneal, subcutaneous, and the like, and can be accompanied with adjuvant.

For the production of monoclonal antibodies, KCNQ5 protein or an antigenic fragment, coupled to a suitable carrier, is injected into an appropriate non-human host animal as above for the production of polyclonal antibodies. In the case of monoclonal antibodies, the animal is generally a mouse. The animal's spleen cells are then immortalized, often by fusion with a myeloma cell, as described in Kohler & Milstein, 1975, Nature 256:495-497. For a fuller description of the production of monoclonal antibodies, see Antibodies: A Laboratory Manual, Harlow & Lane, eds., Cold Spring Harbor Laboratory Press, 1988.

Gene therapy may be used to introduce KCNQ5 polypeptides into the cells of target organs, *e.g.,* the pigmented epithelium of the retina or other parts of the retina. Nucleotides encoding KCNQ5 polypeptides can be ligated into viral vectors which mediate transfer of the nucleotides by infection of recipient cells. Suitable viral vectors include retrovirus, adenovirus, adeno-associated virus, herpes virus, vaccinia virus, lentivirus, and polio virus based vectors. Alternatively, nucleotides encoding KCNQ5 polypeptides can be transferred into cells for gene therapy by non-viral techniques including receptor-mediated targeted transfer using ligand-nucleotide conjugates, lipofection, membrane fusion, or direct microinjection. These procedures and variations thereof are suitable for *ex vivo* as well as *in vivo* gene therapy. Gene therapy with KCNQ5 polypeptides will be particularly useful for the treatment of diseases where it is beneficial to elevate KCNQ5 activity.

Disclosed herein are processes for cloning orthologues of human KCNQ5 from non-human species. In general, such processes include preparing a PCR primer or a hybridization probe based upon SEQ.ID.NO.:1 or SEQ.ID.NO.:2 that can be used to amplify a fragment containing the non-human KCNQ5 (in the case of PCR) from a suitable DNA preparation or to select a cDNA or genomic clone containing the non-human KCNQ5 from a suitable library. A preferred embodiment of this process is a process for cloning the KCNQ5 gene from mouse.

By providing DNA encoding mouse KCNQ5, the present disclosure allows for the generation of an animal model of Stargardt-like macular dystrophy, cone-rod dystrophy, Salla disease, or age-related macular degeneration. Such animal models can be generated by making transgenic "knockout" or "knockin" mice containing altered KCNQ5 genes. Knockout mice can be generated in which portions of the mouse KCNQ5 gene have been deleted. Knockin mice can be generated in which mutations that have been shown to lead to Stargardt-like macular dystrophy, cone-rod dystrophy, Salla disease, or age-related macular degeneration when present in the human KCNQ5 gene are introduced into the mouse gene. Such knockout and knockin mice will be valuable tools in the study of Stargardt-like macular dystrophy, cone-rod dystrophy, Salla disease, or age-related macular degeneration and will provide important model systems in which to test potential pharmaceuticals or treatments for Stargardt-like macular dystrophy, cone-rod dystrophy, Salla disease, or age-related macular degeneration.

Accordingly, there is disclosed a method of producing a mouse model of Stargardt-like macular dystrophy, cone-rod dystrophy, Salla disease, or age-related macular degeneration comprising:
(a) designing PCR primers or an oligonucleotide probe based upon SEQ.ID.NO.:1 or SEQ.ID.NO.:2 for use in cloning the mouse KCNQ5 gene;
(b) using the PCR primers or the oligonucleotide probe to clone at least a portion of the mouse KCNQ5 gene, the portion being large enough to use in making a transgenic mouse;
(c) producing a transgenic mouse having at least one copy of the mouse KCNQ5 gene altered from its native state.

Methods of producing knockout and knockin mice are well known in the art. One method involves the use of gene-targeted ES cells in the generation of gene-targeted transgenic knockout mice and is described in, *e.g.*, Thomas et al., 1987, Cell 51:503-512, and is reviewed elsewhere (Frohman et al., 1989, Cell 56:145-147; Capecchi, 1989, Trends in Genet. 5:70-76; Baribault et al., 1989, Mol. Biol. Med. 6:481-492).

Techniques are available to inactivate or alter any genetic region to virtually any mutation desired by using targeted homologous recombination to insert specific changes into chromosomal genes. Generally, use is made of a "targeting vector," *i.e.,* a plasmid containing part of the genetic region it is desired to mutate. By virtue of the homology between this part of the genetic region on the plasmid and the corresponding genetic region on the chromosome, homologous recombination can be used to insert the plasmid into the genetic region, thus disrupting the genetic region. Usually, the targeting vector contains a selectable marker gene as well.

In comparison with homologous extrachromosomal recombination, which occurs at frequencies approaching 100%, homologous plasmid-chromosome recombination was originally reported to only be detected at frequencies between 10-6 and 10⁻³ (Lin et al., 1985, Proc. Natl. Acad. Sci. USA 82:1391-1395; Smithies et al., 1985, Nature 317: 230-234; Thomas et al., 1986, Cell 44:419-428). Nonhomologous plasmid-chromosome interactions are more frequent, occurring at levels 10⁵-fold (Lin et al., 1985, Proc. Natl. Acad. Sci. USA 82:1391-1395) to 10²⁻fold (Thomas et al., 1986, Cell 44:419-428) greater than comparable homologous insertion.

To overcome this low proportion of targeted recombination in murine ES cells, various strategies have been developed to detect or select rare homologous recombinants. One approach for detecting homologous alteration events uses the polymerase chain reaction (PCR) to screen pools of transformant cells for homologous insertion, followed by screening individual clones (Kim et al., 1988, Nucleic Acids Res. 16:8887-8903; Kim et al., 1991, Gene 103:227-233). Alternatively, a positive genetic selection approach has been developed in which a marker gene is constructed which will only be active if homologous insertion occurs, allowing these recombinants to be selected directly (Sedivy et al., 1989, Proc. Natl. Acad. Sci. USA 86:227-231). One of the most powerful approaches developed for selecting homologous recombinants is the positive-negative selection (PNS) method developed for genes for which no direct selection of the alteration exists (Mansour et al., 1988, Nature 336:348-352; Capecchi, 1989, Science 244:1288-1292; Capecchi, 1989, Trends in Genet. 5:70-76). The PNS method is more efficient for targeting genes which are not expressed at high levels because the marker gene has its own promoter. Nonhomologous recombinants are selected against by using the Herpes Simplex virus thymidine kinase (HSV-TK) gene and selecting against its nonhomologous insertion with herpes drugs such as gancyclovir (GANC) or FIAU (1-(2-deoxy 2-fluoro-B-D-arabinofluranosyl)-5-iodouracil). By this counter-selection, the percentage of homologous recombinants in the surviving transformants can be increased.

Other methods of producing transgenic mice involve microinjecting the male pronuclei of fertilized eggs. Such methods are well known in the art.

The following non-limiting examples are presented to better illustrate the invention.

### EXAMPLE 1

### Identification of the human KCNQ5 gene and cDNA cloning

### Construction of Libraries for Shotgun Sequencing from PAC Clones

Bacterial strains containing the KCNQ5 PACs (P1 Artificial Chromosomes) were received from Research Genetics (Huntsville, AL). Cells were streaked on Luria-Bertani (LB) agar plates supplemented with the appropriate antibiotic. A single colony was used to prepare a 5-ml starter culture and then 1-L overnight culture in LB medium. The cells were pelleted by centrifugation and PAC DNA was purified by equilibrium centrifugation in cesium chloride-ethidium bromide gradient (Sambrook, Fritsch, and Maniatis, 1989, Molecular Cloning: A Laboratory Manual, second edition, Cold Spring Harbor Laboratory Press). Purified PAC DNA was brought to 50 mM Tris pH 8.0, 15 mM MgCl₂, and 25% glycerol in a volume of 2 ml and placed in a AERO-MIST nebulizer (CIS-US, Bedford, MA). The nebulizer was attached to a nitrogen gas source and the DNA was randomly sheared at 10 psi for 30 sec. The sheared DNA was ethanol precipitated and resuspended in TE (10 mM Tris, 1 mM EDTA). The ends were made blunt by treatment with Mung Bean Nuclease (Promega, Madison, WI) at 30°C for 30 min, followed by phenol/chloroform extraction, and treatment with T4 DNA polymerase (GIBCO/BRL, Gaithersburg, MD) in multicore buffer (Promega, Madison, WI) in the presence of 40 uM dNTPs at 16°C. To facilitate subcloning of the DNA fragments, BstX I adapters (Invitrogen, Carlsbad, CA) were ligated to the fragments at 14°C overnight with T4 DNA ligase (Promega, Madison, WI). Adapters and DNA fragments less than 500 bp were removed by column chromatography using a cDNA sizing column (GIBCO/BRL, Gaithersburg, MD) according to the instructions provided by the manufacturer. Fractions containing DNA greater than 1 kb were pooled and concentrated by ethanol precipitation. The DNA fragments containing BstX I adapters were ligated into the BstX I sites of pSHOT II which was constructed by subcloning the BstX I sites from pcDNA II (Invitrogen, Carlsbad, CA) into the BssH II sites of pBlueScript (Stratagene, La Jolla, CA). pSHOT II was prepared by digestion with BstX I restriction endonuclease and purified by agarose gel electrophoresis. The gel purified vector DNA was extracted from the agarose by following the Prep-A-Gene (BioRad, Richmond, CA) protocol. To reduce ligation of the vector to itself, the digested vector was treated with calf intestinal phosphatase (GIBCO/BRL, Gaithersburg, MD. Ligation reactions of the DNA fragments with the cloning vector were transformed into ultra-competent XL-2 Blue cells (Stratagene, La Jolla, CA), and plated on LB agar plates supplemented with 100 µg/ml ampicillin. Individual colonies were picked into a 96 well plate containing 100 µl/well of LB broth supplemented with ampicillin and grown overnight at 37°C. Approximately 25 µl of 80% sterile glycerol was added to each well and the cultures stored at -80°C.

### Preparation of plasmid DNA

Glycerol stocks were used to inoculate 5 ml of LB broth supplemented with 100 µg/ml ampicillin either manually or by using a Tecan Genesis RSP 150 robot (Tecan AG, Hombrechtikon, Switzerland) programmed to inoculate 96 tubes containing 5 ml broth from the 96 wells. The cultures were grown overnight at 37°C with shaking to provide aeration. Bacterial cells were pelleted by centrifugation , the supernatant decanted, and the cell pellet stored at -20°C. Plasmid DNA was prepared with a QIAGEN Bio Robot 9600 (QIAGEN, Chatsworth, CA) according to the Qiawell Ultra protocol. To test the frequency and size of inserts, plasmid DNA was digested with the restriction endonuclease Pvu II. The size of the restriction endonuclease products was examined by agarose gel electrophoresis with the average insert size being 1 to 2 kb.

### DNA Sequence Analysis of Shotgun clones

DNA sequence analysis was performed using the ABI PRISM^{™} dye terminator cycle sequencing ready reaction kit with AmpliTaq DNA polymerase, FS (Perkin Elmer, Norwalk, CT). DNA sequence analysis was performed with M13 forward and reverse primers. Following amplification in a Perkin-Elmer 9600, the extension products were purified and analyzed on an ABI PRISM 377 automated sequencer (Perkin Elmer, Norwalk, CT). Approximately 4 sequencing reactions were performed per kb of DNA to be examined (384 sequencing reactions per each of nine PACs).

### Assembly of DNA sequences

Phred/Phrap was used for DNA sequences assembly. This program was developed by Dr. Phil Green and licensed from the University of Washington (Seattle, WA). Phred/Phrap consists of the following programs: Phred for base-calling, Phrap for sequence assembly, Crossmatch for sequence comparisons, Consed and Phrapview for visualization of data, Repeatmasker for screening repetitive sequences. Vector and *E. coli* DNA sequences were identified by Crossmatch and removed from the DNA sequence assembly process. DNA sequence assembly was on a SUN Enterprise 4000 server running a Solaris 2.51 operating system (Sun Microsystems Inc., Mountain View, CA) using default Phrap parameters. The sequence assemblies were further analyzed using Consed and Phrapview.

### Genomic sequence of the KCNQ5 gene and its exon/intron organization

Genomic DNA sequence from PAC 141B1 was compared with GenBank database entries using the BLASTN and BLASTX algorithms of the AceDB package. This comparison originally revealed a total of 5 exons (exons 3(D), 4 (A), 5(B), 6(E), and 7(C) delineated in Figurel), based on their homology to the known potassium channel genes KCNQ1, KCNQ2, KCNQ3, and KCNQ4. Full-length cDNA was rescued from the pools of the human fetal brain cDNA library using the RCCA technique described in Example 2. Comparison of the cDNA sequence and genomic sequence of PAC141B1 revealed a total of 8 exons (exons 3-10 delineated in Figure1). Genomic regions corresponding to exons 1,2, and 11-14 were not present in PAC141B1.

In order to identify the genomic region corresponding to exon 2 and its right flanking intron, oligonucleotide KCN-2L2 (TTTTCTCCTTGTCTTTGGTTGCTTG; SEQ.ID.NO.:11) from the KCNQ5 cDNA in combination with the adaptor primer AP1 (CCATCCTAATACGACTCACTATAGGGC; SEQ.ID.NO.: 12) was used to PCR-amplify the DNA from a GenomeWalker kit purchased from Clontech (Palo Alto, CA). Diluted PCR product was subjected to PCR amplification with nested primer KCN-2L1 (CCTCAAGTTGCCTCTTGATCCTG; SEQ.ID.NO.:13) in combination with the nested adaptor primer AP2 (ACTCACTATAGGGCTCGAGCGGC; SEQ.ID.NO.:14).

In order to identify the genomic region corresponding to exon 2 and its left flanking intron, oligonucleotide KCN-2R1 (CAGGATCAAGAGGCAACTTGAGG; SEQ.ID.NO.:15) from the KCNQ5 cDNA in combination with the adaptor primer AP1 (CCATCCTAATACGACTCACTATAGGGC; SEQ.ID.NO.:12) was used to PCR-amplify the DNA from a GenomeWalker kit purchased from Clontech (Palo Alto, CA). Diluted PCR product was subjected to PCR amplification with nested primer KCN-2R2 (CCAATTTTGTGTGCTCAGGGATGGTAGA; SEQ.ID.NO.:16) in combination with the nested adaptor primer AP2 (ACTCACTATAGGGCTCGAGCGGC; SEQ.ID.N0.:14).

In order to identify the genomic region corresponding to exon 11 and its right flanking intron, oligonucleotide KCN-11L1 (GACACAGCCCTTGGCACT; SEQ.ID.NO.:17) from the KCNQ5 cDNA in combination with the adaptor primer AP1 (CCATCCTAATACGACTCACTATAGGGC; SEQ.ID.NO.:12) was used to PCR-amplify the DNA from a GenomeWalker kit purchased from Clontech (Palo Alto, CA). Diluted PCR product was subjected to PCR amplification with nested primer KCN-11L2 (GATGATGTATATGATGAAAAAGGATG; SEQ.ID.NO.:18) in combination with the nested adaptor primer AP2 (ACTCACTATAGGGCTCGAGCGGC; SEQ.ID.NO.:14).

In order to identify the genomic region corresponding to exon 11 and its left flanking intron, oligonucleotide KCN-11R1 (CTGATAGCTCGAATGACAGTTTT; SEQ.ID.NO.:19) from the KCNQ5 cDNA in combination with the adaptor primer AP1 (CCATCCTAATACGACTCACTATAGGGC; SEQ.ID.NO.:12) was used to PCR-amplify the DNA from a GenomeWalker kit purchased from Clontech (Palo Alto, CA). Diluted PCR product was subjected to PCR amplification with nested primer KCN11-R2 (AAGTGGTGGGGTGAGGTCTTCCACTG; SEQ.ID.NO.:20) in combination with the nested adaptor primer AP2 (ACTCACTATAGGGCTCGAGCGGC; SEQ.ID.NO.:14).

In order to identify the genomic region corresponding to exon 12 and its right flanking intron, oligonucleotide KCN-12L1 (AGA ATT ATG AAA TTT CAT GTT GCA; SEQ.ID.NO.:21) from the KCNQ5 cDNA in combination with the adaptor primer AP1 (CCATCCTAATACGACTCACTATAGGGC; SEQ.ID.NO.:12) was used to PCR-amplify the DNA from a GenomeWalker kit purchased from Clontech (Palo Alto, CA). Diluted PCR product was subjected to PCR amplification with nested primer KCN-12L2 (AAA CGG AAG TTT AAG GAA ACA TT; SEQ.ID.NO.:22) in combination with the nested adaptor primer AP2 (ACTCACTATAGGGCTCGAGCGGC; SEQ.ID.NO.:14).

In order to identify the genomic region corresponding to exon 12 and its left flanking intron, oligonucleotide KCN-12R1 (ACG TGT TTG TTG GCT TTT AAT TC; SEQ.ID.NO.:23) from the KCNQ5 cDNA in combination with the adaptor primer AP1 (CCATCCTAATACGACTCACTATAGGGC; SEQ.ID.NO.:12) was used to PCR-amplify the DNA from a GenomeWalker kit purchased from Clontech (Palo Alto, CA). Diluted PCR product was subjected to PCR amplification with nested primer KCN-12R2 ( TAC ACA ACA TGT CCA GAT GAC; SEQ.ID.NO.:24) in combination with the nested adaptor primer AP2 (ACTCACTATAGGGCTCGAGCGGC; SEQ.ID.NO.:14).

In order to identify the genomic region corresponding to exon 13 and its right flanking intron, oligonucleotide KCN-13L1 (TGATCAAATTCTTGGAAAAGGG; SEQ.ID.NO.:25) from the KCNQ5 cDNA in combination with the adaptor primer AP1 (CCATCCTAATACGACTCACTATAGGGC; SEQ.ID.NO.:12) was used to PCR-amplify the DNA from a GenomeWalker kit purchased from Clontech (Palo Alto, CA). Diluted PCR product was subjected to PCR amplification with nested primer KCN-13L2 (TCACATCAGATAAGAAGAGCCGA; SEQ.ID.NO.:26) in combination with the nested adaptor primer AP2 (ACTCACTATAGGGCTCGAGCGGC; SEQ.ID.NO.:14).

In order to identify the genomic region corresponding to exon 13 and its left flanking intron, oligonucleotide KCN-13R1 (GTTTTTCAACCTTGACCACCC; SEQ.ID.NO.:27) from the KCNQ5 cDNA in combination with the adaptor primer AP1 (CCATCCTAATACGACTCACTATAGGGC; SEQ.ID.NO.:12) was used to PCR-amplify the DNA from a GenomeWalker kit purchased from Clontech (Palo Alto, CA). Diluted PCR product was subjected to PCR amplification with nested primer KCN-13R2 (AGCATACTGAGATCGTCTGTGGT; SEQ.ID.NO.:28) in combination with the nested adaptor primer AP2 (ACTCACTATAGGGCTCGAGCGGC; SEQ.ID.NO.:14).

In order to identify the genomic region corresponding to exon 14 and its left flanking intron, oligonucleotide KCN-2543R(AATTCCAAAAGTGTCTGTCTCTGTC; SEQ.ID.NO.:29) from the KCNQ5 cDNA in combination with the adaptor primer AP1 (CCATCCTAATACGACTCACTATAGGGC; SEQ.ID.NO.:12) was used to PCR-amplify the DNA from a GenomeWalker kit purchased from Clontech (Palo Alto, CA). Diluted PCR product was subjected to PCR amplification with nested primer KCN-2512R (GGACCCACCTCTTCATCAGTTA; SEQ.ID.NO.:30) in combination with the nested adaptor primer AP2 (ACTCACTATAGGGCTCGAGCGGC; SEQ.ID.NO.:14).

Products obtained from these PCR amplifications were analyzed using ABI 377 sequencers according to standard protocols. Comparison of the full-length KCNQ5 cDNA sequence with the sequences of PAC141B1and sequences obtained in PCR reactions with DNA from the GenomeWalker kit revealed all 14 exons of the KCNQ5 gene. Exact sequence of exon/intron boundaries within the KCNQ5 gene were determined for exons 2-14. The splice signals in all introns conform to published consensus sequences.

### EXAMPLE 2

### Cloning of KCNQ5 cDNA

The DNA sequence of the cDNA fragment that matches exons 3(D), 4(A), 5(B), 6(E), and 7(C) of the KCNQ5 was deduced from the genomic sequence of PAC 141B1. Subsequent sequencing of PCR fragments obtained in RCCA reactions confirmed the presence of this fragment in the cDNA library from human fetal brain. This original cDNA fragment corresponds to the cDNA region with coordinates 368-1,004 in Figure 2.

A PCR based technique termed Reduced Complexity cDNA Analysis (RCCA) was used to extend this original cDNA fragment. RCCA is similar to procedures reported by Munroe et al., 1995, Proc. Natl. Acad. Sci. USA 92: 2209-2213 and Wilfinger et al., 1997, BioTechniques 22:481-486 and relies upon a PCR template that is a pool of approximately 20,000 cDNA clones; this reduces the complexity of the template and increases the probability of obtaining longer PCR extensions.

96 wells of a human fetal brain plasmid library were scanned, 20,000 clones per well, by amplifying a 483 bp PCR product using primers KCN-DL (GGAAGACTGAGGTTTGCTCG; SEQ.ID.NO.31) and KCN-ER (GGCAGGAAGTGCAAAGAAAG; SEQ.ID.NO.32). Eight wells were found to contain the correct 483 bp fragment by PCR analysis. 5' and 3' RACE was subsequently performed on the positive wells containing the plasmid cDNA library using a vector specific primer and a gene specific primer. The vector specific primers, PBS 543R (GGGGATGTGCTGCAAGGCGA; SEQ.ID.NO.33) and PBS 873F (CCCAGGCTTTACACTTTATGCTTCC; SEQ.ID.NO.34) were both used in combination with gene specific primers KCN-DL and KCN-ER because the orientation of the insert was not known. After the initial PCR amplification, a nested PCR reaction was performed using nested vector primers PBS 578R (CCAGGGTTTTCCCAGTCACGAC; SEQ.ID.NO.35) and PBS 838F (TTGTGTGGAATTGTGAGCGGATAAC; SEQ.ID.NO.36) and gene specific primers KCN-EL (CTTTCTTTGCACTTCCTGCC; SEQ.ID.NO.37) and KCN-DR1 (AACACAGAAGGGCTTTCGAG; SEQ.ID.NO.38). The PCR products were separated from the unincorporated dNTP's and primers using Qiagen, QIAquick PCR purification spin columns using standard protocols and resuspended in 30 µl of water. The products were analyzed on ABI 377 sequencers according to standard protocols.

PCR fragments were assembled into a contig termed "KCN consensus 2_16_99" that corresponds to the cDNA region with coordinates 278-1,456 in Figure 2. A second round of the RCCA analysis was performed to obtain the clones extending to the 3' end of the cDNA contig termed "KCN consensus 2_16_99". 96 wells of a human fetal brain plasmid library were scanned, 20,000 clones per well, by amplifying a 117 bp PCR product using primers KCN-11L1 (GACACAGCCCTTGGCACT; SEQ.ID.NO.17) and KCN-11R1 (CTGATAGCTCGAATGACAGTTTT; SEQ.ID.NO.19) that were derived from the 3' sequence of the cDNA contig termed "KCN consensus 2_16_99". A number of wells were found to contain the correct 117 bp fragment by PCR analysis. 3' RACE was subsequently performed on the positive wells containing the plasmid cDNA library using a vector specific primer and a gene specific primer. The vector specific primers, PBS 543R (GGGGATGTGCTGCAAGGCGA; SEQ.ID.NO.33) and PBS 873F (CCCAGGCTTTACACTTTATGCTTCC; SEQ.ID.NO.34) were both used in combination with gene specific primer KCN-11L1 (GACACAGCCCTTGGCACT; SEQ.ID.NO.17) because the orientation of the insert was not known. After the initial PCR amplification, a nested PCR reaction was performed using nested vector primers PBS 578R (CCAGGGTTTTCCCAGTCACGAC; SEQ.ID.NO.35) and PBS 838F (TTGTGTGGAATTGTGAGCGGATAAC; SEQ.ID.NO.36) and gene specific primer KCN11-R2 (AAGTGGTGGGGTGAGGTCTTCCACTG; SEQ.ID.NO.20). The PCR products were separated from the unincorporated dNTPs and primers using Qiagen, QIAquick PCR purification spin columns using standard protocols and resuspended in 30 µl of water. The products were analyzed on ABI 377 sequencers according to standard protocols.

PCR fragments were assembled into a contig termed "KCN consensus 2_26_99" that corresponds to the cDNA region with coordinates 278-2,527 in Figure 2. A third round of RCCA analysis was performed to obtain the clones extending to the 5' end of the cDNA contig termed "KCN consensus 2_26_99". 96 wells of a human fetal brain plasmid library were scanned, 20,000 clones per well, by amplifying a 214 bp PCR product using primers KCN-2L2 (TTTTCTCCTTGTCTTTGGTTGCTTG; SEQ.ID.NO.11) and KCN-DR1 (AACACAGAAGGGCTTTCGAG; SEQ.ID.NO.38) that were derived from the 5' sequence of the cDNA contig termed "KCN consensus 2_26_99". A number of wells were found to contain the correct 214 bp fragment by PCR analysis. 5'RACE was subsequently performed on the positive wells containing the plasmid cDNA library using a vector specific primer and a gene specific primer. The vector specific primers, PBS 543R (GGGGATGTGCTGCAAGGCGA; SEQ.ID.NO.33) and PBS 873F (CCCAGGCTTTACACTTTATGCTTCC; SEQ.ID.NO.34) were both used in combination with gene specific primer KCN-DR1 (AACACAGAAGGGCTTTCGAG; SEQ.ID.NO.38) because the orientation of the insert was not known. After the initial PCR amplification, a nested PCR reaction was performed using nested vector primers PBS 578R (CCAGGGTTTTCCCAGTCACGAC; SEQ.ID.NO.35) and PBS 838F (TTGTGTGGAATTGTGAGCGGATAAC; SEQ.ID.NO.36) and gene specific primer KCN-DR2 (CAGTCTTCCTTGCCATCCTC; SEQ.ID.NO.39). The PCR products were separated from the unincorporated dNTPs and primers using Qiagen, QIAquick PCR purification spin columns using standard protocols and resuspended in 30 µl of water. The products were analyzed on ABI 377 sequencers according to standard protocols.

PCR fragments were assembled into a contig termed "KCN consensus 3_3_99" that corresponds to the cDNA region with coordinates 1-2,527 in Figure 2. A fourth round of RCCA analysis was performed to obtain the clones extending to the 3' end of the cDNA contig termed "KCN consensus 3_3_99".96 wells of a human fetal brain plasmid library were scanned, 20,000 clones per well, by amplifying a 145 bp PCR product using primers KCN-2106L (GCAGCCCCAACAACTTTACA; SEQ.ID.NO.40) and KCN-2250R (CATTTTCCTTGGAGGCAACA; SEQ.ID.NO.41) that were derived from the 3' sequence of the cDNA contig termed "KCN consensus 3_3_99". A number of wells were found to contain the correct 214 bp fragment by PCR analysis. 5' RACE was subsequently performed on the positive wells containing the plasmid cDNA library using a vector specific primer and a gene specific primer. The vector specific primers , PBS 543R (GGGGATGTGCTGCAAGGCGA; SEQ.ID.NO.33) and PBS 873F (CCCAGGCTTTACACTTTATGCTTCC; SEQ.ID.NO.34) were both used in combination with gene specific primer KCN-2106L (GCAGCCCCAACAACTTTACA; SEQ.ID.NO.40) because the orientation of the insert was not known. After the initial PCR amplification, a nested PCR reaction was performed using nested vector primers PBS 578R (CCAGGGTTTTCCCAGTCACGAC; SEQ.ID.NO.35) and PBS 838F (TTGTGTGGAATTGTGAGCGGATAAC; SEQ.ID.NO.36) and gene specific primer KCN-2165L (GCCAGAAACTCTGCACCCTA; SEQ.ID.NO.42). The PCR products were separated from the unincorporated dNTP's and primers using Qiagen, QIAquick PCR purification spin columns using standard protocols and resuspended in 30 µl of water. The products were analyzed on ABI 377 sequencers according to standard protocols; PCR fragments were assembled into a contig termed "KCN consensus 3_15_99" that corresponds to the cDNA sequence depicted in Figure 2.

### EXAMPLE 3

### Analysis of expression of KCNQ5

*RT-PCR:* RT-PCR experiments were performed on "quick-clone" human cDNA samples available from Clontech, Palo Alto, CA. cDNA samples from heart, brain, placenta, lung, liver, skeletal muscle, kidney, pancreas, and retina were amplified with primers KCN-DL (GGAAGACTGAGGTTTGCTCG; SEQ.ID.NO.31) and KCN-ER (GGCAGGAAGTGCAAAGAAAG; SEQ.ID.NO.32) in the following PCR conditions:

| | |
|---|---|
| 1. | 94°C 10 min |
| 2. | 94°C 30 sec |
| 3. | 72°C 2 min (decrease this temperature by 1.1°C per cycle) |
| 4. | 72°C 2 min |
| 5. | Go to step 221 more times |
| 6. | 94°C 30 sec |
| 7. | 55°C 2 min |
| 8. | 72°C 2 min |
| 9. | Go to step 6 19 more times |
| 10. | 72°C 7 min |
| 11. | 4°C |

The KCNQ5 gene was found to be predominantly expressed in human retina and brain (Figure 3B).

*Northern blot analysis:* Northern blots containing poly(A+)-RNA from human heart, brain, placenta, lung, liver, skeletal muscle, kidney, pancreas were purchased from Clontech, Palo Alto, CA. Primers KCN-DL (GGAAGACTGAGGTTTGCTCG; SEQ.ID.NO.31) and KCN-ER (GGCAGGAAGTGCAAAGAAAG; SEQ.ID.NO.32) were used to amplify a PCR product of 483 bp from a quick-clone human retina cDNA available from Clontech, Palo Alto, CA. This fragment was purified on an agarose gel, the DNA extracted and used as a probe for Northern blot hybridization.

The probe was labeled by random priming with the Amersham Rediprime kit (Arlington Heights, IL) in the presence of 50-100 µCi of 3000 Ci/mmole [alpha 32P]dCTP (Dupont/NEN, Boston, MA). Unincorporated nucleotides were removed with a ProbeQuant G-50 spin column (Pharmacia/Biotech, Piscataway, NJ). The radiolabeled probe at a concentration of greater than 1 x 10⁶ cpm/ml in rapid hybridization buffer (Clontech, Palo Alto, CA) was incubated overnight at 65°C. The blots were washed by two 15 min incubations in 2X SSC, 0.1% SDS (prepared from 20X SSC and 20 % SDS stock solutions, Fisher, Pittsburgh, PA) at room temperature, followed by two 15 min incubations in 1X SSC, 0.1% SDS at room temperature, and two 30 min incubations in 0.1X SSC, 0.1% SDS at 60°C. Autoradiography of the blots was done to visualize the bands that specifically hybridized to the radiolabeled probe.

The probe hybridized to an mRNA transcript that is predominately expressed in brain and retina (Figure 3A).

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

## Claims

1. An isolated DNA molecule comprising a nucleotide sequence encoding a KCNQ5 polypeptide comprising the sequence of amino acids as set forth in SEQ ID. NO: 3.

2. The DNA of claim 1 comprising a nucleotide sequence selected from the group consisting of: SEQ.ID.NO.:1, SEQ-ID.NO.:2, and positions 138-2,675 of SEQ.ID.NO.:2.

3. An expression vector comprising the DNA of claim 1.

4. A recombinant host cell comprising the expression vector of claim 3.

5. An isolated KCNQ5 protein having the amino acid sequence SEQ.ID.NO.:3.

6. The KCNQ5 protein of claim 5 containing a single amino acid substitution.

7. The KCNQ5 protein of claim 5 having conservative substitutions and which retains the biological activity of KCNQ5.

8. An antibody that binds specifically to a KCNQ5 protein where the KCNQ5 protein has the amino acid sequence SEQ.ID.NO.:3.

9. A method of determining whether a substance is an activator or an inhibitor of a KCNQ5 protein or a mutant KCNQ5 protein comprising:
(a) recombinantly expressing the KCNQ5 polypeptide of Claims 5 to 7;
(b) measuring the biological activity of KCNQ5 protein or mutant KCNQ5 protein in the presence and in the absence of a substance suspected of being an activator or an inhibitor of KCNQ5 protein or mutant KCNQ5 protein;
where a change in the biological activity of the KCNQ5 protein or the mutant KCNQ5 protein in the presence as compared to the absence of the substance indicates that the substance is an activator or an inhibitor of KCNQ5 protein or mutant KCNQ5 protein.

10. A method of identifying inhibitors of KCNQ5 comprising.
(a) expressing the KCNQ5 polypeptide of Claims 5 to 7 in *Xenopus* oocytes:
(b) changing the transmembrane potential of the oocytes in the presence and the absence of a substance suspected of being an inhibitor of KCNQ5;
(c) measuring membrane potassium currents following step (b);
where if the potassium membrane currents measured in step (c) are greater in the absence rather than in the presence of the substance, then the substance is an inhibitor of KCNQ5.

11. A method of identifying activators or inhibitors of KCNQ5 comprising:
(a) providing test cells comprising:
(1) an expression vector that directs the expression of the KCNQ5 polypeptide of Claims 5 to 7 in the cells;
(2) a first fluorescent dye, where the first dye is bound to one side of the plasma membrane; and
(3) a second fluorescent dye, where the second fluorescent dye is free to shuttle from one face of the plasma membrane to the other face in response to changes in membrane potential;
(b) exposing the test cells to a substance that is suspected of being an activator or an inhibitor of KCNQ5;
(c) measuring the amount of fluorescence resonance energy transfer (FRET) in the test cells that have been exposed to the substance;
(d) comparing the amount of FRET exhibited by the test cells that have been exposed to the substance with the amount of FRET exhibited by control cells;
wherein if the amount of FRET exhibited by the test cells is greater than the amount of FRET exhibited by control cells, the substance is an activator of KCNQ5;
wherein if the amount of FRET exhibited by the test cells is less than the amount of FRET exhibited by the control cells, the substance is an inhibitor of KCNQ5;
where the control cells are either (1) cells that are essentially the same as the test cells except that they do not comprise at least one of the items listed at (a) (1)-(3) but have been exposed to the substance; or (2) test cells that have not been exposed to the substance.

## Patentansprüche

1. Isoliertes DNA-Molekül, umfassend eine Nukleotidsequenz, welche für ein KCNQ5-Polypeptid, umfassend die in SEQ-ID-Nr. 3 angegebene Aminosäuresequenz, codiert.

2. DNA nach Anspruch 1, umfassend eine Nukleotidsequenz, welche aus der Gruppe, die aus SEQ-ID-Nr. 1, SEQ-ID-Nr. 2 und den Positionen 138-2675 von SEQ-ID-Nr. 2 besteht, ausgewählt ist.

3. Expressionsvektor, umfassend die DNA nach Anspruch 1.

4. Rekombinante Wirtszelle, umfassend den Expressionsvektor nach Anspruch 3.

5. Isoliertes KCNQ5-Protein mit der Aminosäuresequenz SEQ-ID-Nr. 3.

6. KCNQ5-Protein nach Anspruch 5, welches eine einzige Aminosäuresubstitution enthält.

7. KCNQ5-Protein nach Anspruch 5, welches konservative Substitutionen aufweist und die biologische Aktivität von KCNQ5 behält.

8. Antikörper, der spezifisch an ein KCNQ5-Protein bindet, wobei das KCNQ5-Protein die Aminosäuresequenz SEQ-ID-Nr. 3 besitzt.

9. Verfahren zur Feststellung, ob eine Substanz ein Aktivator oder Inhibitor eines KCNQ5-Proteins oder eines Mutanten-KCNQ5-Proteins ist, umfassend:
(a) rekombinantes Exprimieren des KCNQ5-Polypeptids nach den Ansprüchen 5 bis 7;
(b) Messen der biologischen Aktivität des KCNQ5-Proteins oder Mutanten-KCNQ5-Proteins in Anwesenheit und in Abwesenheit einer Substanz, von der vermutet wird, dass es sich um einen Aktivator oder Inhibitor eines KCNQ5-Proteins oder eines Mutanten-KCNQ5-Proteins handelt;
wobei eine Veränderung in der biologischen Aktivität des KCNQ5-Proteins oder des Mutanten-KCNQ5-Proteins in Anwesenheit der Substanz im Vergleich zur Abwesenheit der Substanz anzeigt, dass die Substanz ein Aktivator oder Inhibitor eines KCNQ5-Proteins oder Mutanten-KCNQ5-Proteins ist.

10. Verfahren zur Identifizierung von Inhibitoren von KCNQ5, umfassend:
(a) Exprimieren des KCNQ5-Polypeptids nach den Ansprüchen 5 bis 7 in *Xenopus-*Oozyten;
(b) Ändern des Transmembranpotentials der Oozyten in Anwesenheit und Abwesenheit einer Substanz, von der vermutet wird, dass es sich um einen Inhibitor von KCNQ5 handelt;
(c) Messen von Membrankaliumströmen nach Schritt (b);
wobei die Substanz ein Inhibitor von KCNQ5 ist, wenn die in Schritt (c) gemessenen Kaliummembranströme in Abwesenheit der Substanz größer als in Anwesenheit der Substanz sind.

11. Verfahren zur Identifizierung von Aktivatoren oder Inhibitoren von KCNQ5, umfassend:
(a) Bereitstellen von Testzellen, umfassend:
(1) einen Expressionsvektor, der die Expression des KCNQ5-Polypeptids nach den Ansprüchen 5 bis 7 in den Zellen steuert;
(2) einen ersten Fluoreszenzfarbstoff, wobei der erste Farbstoff an eine Seite der Plasmamembran gebunden ist; und
(3) einen zweiten Fluoreszenzfarbstoff, wobei der zweite Fluoreszenzfarbstoff frei ist, um von einer Seite der Plasmamembran zu der anderen Seite als Reaktion auf Änderungen des Membranpotentials zu pendeln;
(b) Aussetzen der Testzellen einer Substanz, von der vermutet wird, dass es sich um einen Aktivator oder Inhibitor von KCNQ5 handelt;
(c) Messen des Ausmaßes an Fluoreszenzresonanzenergietransfer (FRET) in den Testzellen, die der Substanz ausgesetzt wurden;
(d) Vergleichen des Ausmaßes an FRET, das von den Testzellen gezeigt wird, die der Substanz ausgesetzt wurden, mit dem Ausmaß an FRET, das von Kontrollzellen gezeigt wird;
wobei die Substanz ein Aktivator von KCNQ5 ist, wenn das Ausmaß an FRET, das von den Testzellen gezeigt wird, größer ist als das Ausmaß an FRET, das von Kontrollzellen gezeigt wird;
wobei die Substanz ein Inhibitor von KCNQ5 ist, wenn das Ausmaß an FRET, das von den Testzellen gezeigt wird, geringer ist als das Ausmaß an FRET, das von den Kontrollzellen gezeigt wird;
wobei die Kontrollzellen entweder (1) Zellen sind, welche im Wesentlichen dieselben wie die Testzellen sind, mit der Ausnahme, dass sie nicht mindestens eines der in (a) (1)-(3) aufgeführten Materialien umfassen, jedoch der Substanz ausgesetzt wurden; oder (2) Testzellen, die nicht der Substanz ausgesetzt wurden.

## Revendications

1. Molécule d'ADN isolée comprenant une séquence de nucléotides codant un polypeptide KCNQ5 comprenant la séquence d'acides aminés telle qu'indiquée dans la SEQ ID N° 3.

2. ADN selon la revendication 1, comprenant une séquence de nucléotides choisie dans le groupe constitué par : la SEQ ID N° 1, la SEQ ID N° 2, et les positions 138-2675 de la SEQ ID N° 2.

3. Vecteur d'expression comprenant l'ADN de la revendication 1.

4. Cellule hôte recombinée comprenant le vecteur d'expression de la revendication 3.

5. Protéine KCNQ5 isolée ayant la séquence d'acides aminés SEQ ID N° 3.

6. Protéine KCNQ5 selon la revendication 5, contenant une seule substitution d'acide aminé.

7. Protéine KCNQ5 selon la revendication 5, ayant des substitutions conservatrices et qui conserve l'activité biologique de KCNQ5.

8. Anticorps qui se fixe spécifiquement à une protéine KCNQ5, où la protéine KCNQ5 a la séquence d'acides aminés SEQ ID N° 3.

9. Procédé pour déterminer si une substance est un activateur ou un inhibiteur d'une protéine KCNQ5 ou d'une protéine KCNQ5 mutante, comprenant :
(a) l'expression par recombinaison du polypeptide KCNQ5 des revendications 5 à 7 ;
(b) la mesure de l'activité biologique de la protéine KCNQ5 ou de la protéine KCNQ5 mutante en présence et en l'absence d'une substance suspectée d'être un activateur ou un inhibiteur de protéine KCNQ5 ou de protéine KCNQ5 mutante ;
où un changement de l'activité biologique de la protéine KCNQ5 ou de la protéine KCNQ5 mutante en présence de la substance, par comparaison avec l'activité en son absence, indique que la substance est un activateur ou un inhibiteur de protéine KCNQ5 ou de protéine KCNQ5 mutante.

10. Procédé pour identifier des inhibiteurs de KCNQ5, comprenant :
(a) l'expression du polypeptide KCNQ5 des revendications 5 à 7 dans des oocytes de *Xenopus ;*
(b) le changement du potentiel transmembranaire des oocytes en présence et en l'absence d'une substance suspectée d'être un inhibiteur de KCNQ5 ;
(c) la mesure des potentiels potassiques membranaires après l'étape (b) ;
où, si les potentiels membranaires potassiques mesurés dans l'étape (c) sont supérieurs en l'absence de la substance, à ceux en sa présence, alors la substance est un inhibiteur de KCNQ5.

11. Procédé pour identifier des activateurs ou des inhibiteurs de KCNQ5, comprenant :
(a) le fait de disposer de cellules de test comprenant :
(1) un vecteur d'expression qui dirige l'expression du polypeptide KCNQ5 des revendications 5 à 7 dans les cellules ;
(2) un premier colorant fluorescent, lequel premier colorant est fixé à un côté de la membrane plasmatique ; et
(3) un deuxième colorant fluorescent, lequel deuxième fluorescent est libre de faire la navette entre une face de la membrane plasmatique et l'autre face en réponse à des changements du potentiel membranaire ;
(b) l'exposition des cellules de test à une substance qui est suspectée d'être un activateur ou un inhibiteur de KCNQ5 ;
(c) la mesure de la quantité de transfert d'énergie par résonance en fluorescence (FRET) dans les cellules de test qui ont été exposées à la substance ;
(d) la comparaison de la quantité de FRET que présentent les cellules de test qui ont été exposées à la substance avec la quantité de FRET que présentent des cellules témoins ;
dans lequel, si la quantité de FRET que présentent les cellules de test est supérieure à la quantité de FRET que présentent les cellules témoins, la substance est un activateur de KCNQ5 ;
dans lequel, si la quantité de FRET que présentent les cellules de test est inférieure à la quantité de FRET que présentent les cellules témoins, la substance est un inhibiteur de KCNQ5 ;
où les cellules témoins sont soit (1) des cellules qui sont pratiquement identiques aux cellules de test sauf qu'elles ne comprennent pas au moins l'un des éléments listés en (a) (1)-(3) mais ont été exposées à la substance ; soit (2) des cellules de test qui n'ont pas été exposées à la substance.
